(19)

Europäisches
Patentamt
European
Patent Office
Office européen
des brevets

(11) **EP 4 296 373 B1**

(12) **EUROPEAN PATENT SPECIFICATION**

(45) Date of publication and mention
of the grant of the patent:
**19.02.2025  Bulletin 2025/08**

(21) Application number: **23189043.5**

(22) Date of filing: **04.10.2019**

(51) International Patent Classification (IPC):
*C12Q 1/6806* (2018.01)    *C12Q 1/6865* (2018.01)

(52) Cooperative Patent Classification (CPC):
(C-Sets available)
**C12Q 1/6806; C12Q 1/6865; C12Q 1/6876;**
C12Q 2525/131; C12Q 2525/143; C12Q 2535/122;
C12Q 2545/101; C12Q 2600/166         (Cont.)

(54) **NORMALIZATION CONTROLS FOR MANAGING LOW SAMPLE INPUTS IN NEXT GENERATION SEQUENCING**

NORMALISIERUNGSSTEUERUNG ZUR VERWALTUNG GERINGER PROBENEINGABEN IN DER NEXT-GENERATION-SEQUENZIERUNG

COMMANDES DE NORMALISATION POUR GÉRER DE FAIBLES ENTRÉES D'ÉCHANTILLON DANS LE SÉQUENÇAGE DE NOUVELLE GÉNÉRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **04.10.2018   US 201862741466 P**
**11.04.2019   US 201962832560 P**

(43) Date of publication of application:
**27.12.2023   Bulletin 2023/52**

(62) Document number(s) of the earlier application(s) in accordance with Art. 76 EPC:
**19794317.8 / 3 861 135**

(73) Proprietor: **Arc Bio, LLC**
**Cambridge, Massachusetts 02141 (US)**

(72) Inventors:
• HARNESS, Eric
  CA 94086 Sunnyvale (US)
• NAGESH, Vaishnavi
  MA 02141, Cambridge (US)
• BENTLEY, Lloyd Gordon
  MA 02141 Cambridge (US)
• CARPENTER, Meredith L.
  CA 94401 San Mateo (US)

(74) Representative: **Boult Wade Tennant LLP**
**Salisbury Square House**
**8 Salisbury Square**
**London EC4Y 8AP (GB)**

(56) References cited:
• **LESHKOWITZ DENA ET AL: "Using Synthetic Mouse Spike-In Transcripts to Evaluate RNA-Seq Analysis Tools", PLOS ONE, vol. 11, no. 4, 21 April 2016 (2016-04-21), pages e0153782, XP093100733, Retrieved from the Internet <URL:https://www.ncbi.nlm.nih. gov/pmc/articles/PMC4839710/pdf/ pone.0153782.pdf> DOI: 10.1371/ journal.pone.0153782**
• **SIMON A HARDWICK ET AL: "Spliced synthetic genes as internal controls in RNA sequencing experiments", NATURE METHODS, vol. 13, no. 9, 1 September 2016 (2016-09-01), New York, pages 792 - 798, XP055535717, ISSN: 1548-7091, DOI: 10.1038/nmeth.3958**
• **MAURO D. LOCATI ET AL: "Improving small RNA-seq by using a synthetic spike-in set for size-range quality control together with a set for data normalization", NUCLEIC ACIDS RESEARCH ADVANCE ACCESS, vol. 43, no. 14, 18 August 2015 (2015-08-18), GB, pages e89 - e89, XP055386708, ISSN: 0305-1048, DOI: 10.1093/nar/gkv303**

EP 4 296 373 B1

**(Cont. next page)**

(52) Cooperative Patent Classification (CPC): (Cont.)

C-Sets
**C12Q 1/6806, C12Q 2525/131, C12Q 2525/143,
C12Q 2525/204, C12Q 2535/122, C12Q 2545/101,
C12Q 2545/113;
C12Q 1/6865, C12Q 2525/131, C12Q 2525/143,
C12Q 2535/122, C12Q 2545/101**

**Description**

**FIELD**

The invention relates to methods for the quantification of nucleic acids in samples using high throughput sequencing.

**BACKGROUND**

Next generation sequencing (NGS), also called high throughput sequencing, is a powerful tool for research and diagnostics. NGS technologies allow for massively high throughput sequencing of nucleic acids. Using NGS technologies, millions of DNA or RNA molecules (polynucleotides) from a single sample can be sequenced cheaply and efficiently. The degree to which polynucleotides are sampled during NGS sequencing is proportional to the initial concentration of the polynucleotides in a sample. Therefore, NGS can be used to determine the quantity of an individual polynucleotide or population of polynucleotides in a sequencing sample. However, the number of reads generated by NGS and the starting concentration of a polynucleotide in a sample are not necessarily well correlated. Correlating NGS read number and starting concentration is particularly problematic when starting with low sample inputs, as stochastic processes during sample preparation and sequencing can have a disproportionate effect on the number of nucleic acid molecules that are sequenced in the NGS process when starting sample inputs are low. Starting sample inputs can be low in a variety of clinical applications, for example when trying to detect pathogens present in a sample at low titers I-eshkowitz et al (2016) PLOS ONE, vol. 11, no. 4, page e0153782 discloses in vitro transcription methods comprising spike in transcripts for normalization.

There exists a need in the art for compositions and methods to accurately quantify the abundance of polynucleotides in a sample when using NGS. Provided herein are methods and compositions useful for such quantification of polynucleotides in a sample using NGS.

**SUMMARY**

The invention is defined in the appended claims. The invention provides a method of making a normalization control comprising RNA molecules, comprising:

> a. synthesizing at least three groups of DNA molecules, wherein each DNA molecule in the at least three groups of DNA molecules comprises, from 5' to 3', a first component sequence, a sequence complementary to a promoter sequence, a first restriction enzyme site, a second component sequence, a second sequence complementary to a promoter sequence, a second restriction site, a third component sequence and a third sequence complementary to a promoter sequence;
> b. digesting each of the at least three groups of DNA molecules with a restriction enzyme that cuts at the first and second restriction enzyme sites to produce at least three groups of digested DNA molecules;
> c. *in vitro* transcribing each of the at least three groups of digested DNA molecules to produce at least three populations of RNA molecules;
> d. quantifying the amount of RNA in each of the at least three populations of RNA molecules; and
> e. mixing a pre-determined amount of each of the at least three populations of RNA molecules, thereby producing a normalization control comprising RNA molecules.

Provided herein are methods of making normalization controls for next generation sequencing. Also described herein are normalization controls for next generation sequencing and methods of using the same.

Described herein are normalization controls comprising at least three groups of polynucleotides, wherein the polynucleotides within each group are of the same length. In some examples, the lengths of all of the polynucleotides in the normalization control are the same. In some examples, the polynucleotides within each group are of a different length when compared to the polynucleotides within any other group.

In some examples of the normalization controls of the disclosure, the polynucleotides within each group comprise the same sequence. In some examples, the polynucleotides within each group do not comprise the same sequence. In some examples, every group of polynucleotides comprises at least three subgroups of polynucleotides, wherein the polynucleotides within each subgroup comprise the same sequence, and wherein the polynucleotides within each subgroup do not comprise the same sequence as any other subgroup.

In some examples of the normalization controls of the disclosure, the sequence of at least one group of polynucleotides comprises a component sequence. In some examples, the sequence of every group of polynucleotides comprises a component sequence. In some examples, the component sequence comprises or consists of a random sequence.

In some examples of the normalization controls of the disclosure, the sequence of at least one group of polynucleotides comprises an isolated sequence. In some examples, the sequence of every group of polynucleotides comprises an isolated sequence. In some examples, the isolated sequence is isolated or derived from a virus, a bacterium, a fungus or a eukaryote. In some examples, the isolated sequence is not the same as a target sequence in a sequencing sample.

In some examples of the normalization controls of the disclosure, the sequence of at least one subgroup or at least one group of polynucleotides in the normalization control shares at least one sequence property with a target sequence in the sequencing sample. The at least one sequence property is, for example, a sequence property of a transposable element sequence, viral sequence, bacterial sequence, a fungal sequence, a eukaryotic parasite sequence or one or more human genes sequence(s).

In some examples of the normalization controls of the disclosure, the sequencing sample comprises a mixture of host and non-host nucleic acids. In some examples, the host is a eukaryote, such as an insect, a plant or an animal. In some examples, the host is human. In some examples, the non-host comprises a symbiote, a commensal organism, a parasite or a pathogen. The non-host can comprise multiple species. In some examples, the target sequence is a non-host sequence in the sequencing sample. In exemplary examples, the non-host is a virus, a bacterium or a fungus and the target sequence comprises a sequence of a virulence factor of the non-host.

Also described herein are methods of making normalization controls, comprising: (a) synthesizing at least three groups of DNA molecules, wherein the DNA molecules within a group have the same sequence, and wherein the sequence of each of the DNA molecules comprises, from 5' to 3', a first component sequence, a first restriction enzyme site, a second component sequence, a second restriction site, and a third component sequence; (b) quantifying the amount of the each of the groups of DNA molecules in a solution; (c) digesting each of the groups of DNA molecules with a restriction enzyme that cuts the first and second restriction sites to produce at least three groups of digested DNA molecules; and (d) mixing a pre-determined amount of each of the groups of digested DNA molecules thereby resulting in a normalization control.

Also described herein are methods making normalization controls comprising at least three groups of polynucleotides, wherein the polynucleotides within each group are of the same length, the methods comprising: (a) extracting DNA from a sample; (b) digesting the DNA with a restriction enzyme to produce a collection of DNA fragments; (c) separating the collection of DNA fragments; (d) purifying the DNA fragments to produce at least three groups of polynucleotides wherein the polynucleotides within each group are of the same length; and (e) mixing a pre-determined amount of each of groups of polynucleotides to produce the normalization control.

Also described herein are methods quantifying the level of expression of at least one target nucleic acid molecule in a sample, comprising: (a) mixing a known amount of the normalization controls of the disclosure with the sample, (b) preparing a high throughput sequencing library, (c) sequencing said library to produce a collection of reads, (d) mapping each read to the sample or the normalization control, (e) determining the number of reads produced by each of the groups or subgroups polynucleotides in the normalization control, (f) calculating relationship between the starting concentration of each of the plurality of nucleic acid molecules in the normalization control mixed with the sample in (a) and the number of reads produced in (c), (g) determining the relationship between reads and concentration in the sample, and (h) calculating the initial concentration of the at least one target nucleic acid molecule in the sample from the number of reads produced by the target nucleic acid molecule using the model in (g).

Also described herein are multi-analyte controls for next generation sequencing, methods of making and of using the same. The normalization controls described herein may be used to normalize reads from multi-analyte controls and from a sample, and the normalized reads from may be used to determine the titer of one or more target organisms in a sample.

Also described herein are multi-analyte controls comprising a mixture of at least three different species of organisms, wherein the organisms have been inactivated. In some examples, the multi-analyte controls comprise at least ten different species of organisms.

Also described herein are methods of quantifying at least one target sequence in a sample, wherein the sample comprises a multi-analyte control comprising a mixture of at least three different species of organisms, wherein the organisms have been inactivated.

Also described herein are methods of quantifying at least one target sequence in a sample, the methods comprising mixing a known amount of a multi-analyte control with the sample, wherein the multi-analyte control comprises a mixture of at least three different species of organisms, and wherein the organisms have been inactivated.

Also described herein are methods quantifying a titer of a target organism in a sample, comprising: (a) providing a sample comprising the target organism, wherein the target organism comprises at least one target sequence; (b) providing a multi-analyte control comprising known titers of at least three species of organisms, wherein the organisms have been inactivated; (c) mixing a known amount of the normalization controls of the disclosure with the sample and with the multi-analyte control; (d) preparing high throughput sequencing libraries from the sample and the multi-analyte control; (e) sequencing said libraries to produce a collection of sample reads and a collection of multi-analyte control reads; (f) normalizing the collection of sample reads and the collection of multi-analyte control reads from (e) using the normalization controls; (g) determining a relationship between normalized reads and the known titers of organisms from the at least three species of organisms in the multi-analyte control; and (h) calculating a titer of the target organism in the sample using the relationship determined in (g).

In some examples, normalizing at step (f) comprises: (i) mapping reads from the collection of sample reads to the sample or the normalization control; (ii) mapping reads from the collection of multi-analyte control reads to the multi-analyte control or the normalization control; (iii) determining the number of reads produced by each of the groups or subgroups of polynucleotides in the normalization control for the collection of sample reads and the collection of multi-analyte control reads; (iv) calculating a relationship between the starting concentration of each of the groups or subgroups of polynucleotides in the normalization control and the number of reads produced at step (e) for both the sample and the multi-analyte control; and (v) determining the relationship between reads and concentration in the sample and in the multi-analyte control.

Also described herein are methods of making a plurality of normalization control oligonucleotides, comprising: (a) generating a plurality of reference sequence fragments from at least one reference sequence; (b) generating a distribution of at least one parameter as a function of number of reference sequence fragments; (c) dividing the distribution into at least 5 bins; (d) selecting at least one reference sequence fragment from at least 3 of the at least 5 bins; (e) shuffling the at least 3 reference sequences to generate shuffled sequences; and (f) synthesizing oligonucleotides comprising the shuffled sequences; thereby generating a plurality of normalization control oligonucleotides.

Also described herein are methods of making a plurality of normalization control oligonucleotides, comprising: (a) generating a plurality of reference sequence fragments from at least one reference sequence using a sliding window; (b) generating a distribution of at least one parameter as a function of number of reference sequence fragments; (c) dividing the distribution into at least 5 bins; (d) selecting at least two reference sequence fragments from at least 3 of the at least 5 bins, wherein the at least two reference sequence fragments are either non-contiguous in the reference sequence, or from different reference sequences; (e) concatenating the at least two reference sequence fragments from each of the at least 3 bins; and (f) synthesizing oligonucleotides comprising the concatenated reference sequence fragments; thereby generating a plurality of normalization control oligonucleotides. In some examples, the the sliding window comprises a 1 bp, 2 bp, 3 bp, 4 bp or 5 bp sliding window. In some examples, the reference sequence fragments are about 15-60, about 20-40, about 20-30, about 15-32, 20-32 or about 25-35 contiguous nucleotides of the corresponding reference sequence. In some examples, the reference sequence fragments are 29, 30, 31, 32, 33 or 34 contiguous nucleotides of the corresponding reference sequence.

In some examples of the methods of the disclosure, the parameter comprises least one of (1) percent GC content, (2) entropy, (3) complexity, (4) EIIP, or a combination thereof.

In some examples of the methods of the disclosure, the at least one reference sequence comprises at least 2, at least 10, at least 20, at least 50, at least 100, at least 200, at least 300, at least 400, at least 500, at least 600, at least 700, at least 800, at least 900, at least 1,000, at least 1,200, at least 1,300, at least 1,400, at least 1,500, at least 1,600, at least 1,700, at least 1,800, at least 1,900, at least 2,000, at least 2,200, at least 2,400, at least 2,600, at least 2,800, at least 3,000, at least 4,000, at least 5,000 , at least 6,000, at least 7,000, at least 8,000, at least 9,000 or at least 10,000 reference sequences. The at least one reference sequence may comprise about 2,000 reference sequences.

In some examples of the methods of the disclosure, each normalization control oligo has at least three of: (1) similar percent GC content, (2) similar entropy, (3) similar EIIP, (4) similar length or (5) similar complexity as the reference sequence fragment(s) from a corresponding bin in the reference sequence distribution.

Described herein are methods of enriching a sample for target nucleic acids of interest for use in combination with the normalization controls and multi-analyte controls provided herein. In some examples, the methods comprise nucleotide modification based enrichment methods. In some examples, the methods comprise nucleic acid-guide nuclease based enrichment methods. In some examples, the methods comprise both nucleotide modification based enrichment methods and nucleic acid-guide nuclease based enrichment methods.

Described herein are methods of enriching a sample for nucleic acids of interest comprising: (a) providing a sample comprising nucleic acids of interest and nucleic acids targeted for depletion, wherein at least a subset of the nucleic acids of interest or a subset of the nucleic acids targeted for depletion comprise a plurality of first recognition sites for a first modification-sensitive restriction enzyme; (b) terminally dephosphorylating a plurality of the nucleic acids in the sample; (c) contacting the sample from (b) with the first modification-sensitive restriction enzyme under conditions that allow for cleavage of at least some of the first modification-sensitive restriction sites in the nucleic acids in the sample; and (d) contacting the sample from (c) with adapters under conditions that allow for the ligation of the adapters to a 5' and 3' end of a plurality of the nucleic acids of interest; thereby generating a sample enriched for nucleic acids of interest that are adapter-ligated on their 5' and 3' ends.

In some examples of the methods of the disclosure, the methods comprise contacting the sample with a plurality of nucleic acid-guided nuclease-guide nucleic acid (gNA) complexes, wherein the gNAs are complementary to targeted sites in the nucleic acids targeted for depletion, thereby generating cut nucleic acids targeted for depletion that are adapter-ligated on one end and target nucleic acids of interest that are adapter-ligated on both the 5' and 3' ends. In some examples, the method comprises contacting the sample with at least $10^2$ unique nucleic acid-guided nuclease-gNA complexes, at least $10^3$ unique nucleic acid-guided nuclease-gNA complexes, $10^4$ unique nucleic acid-guided nuclease-gNA complexes or $10^5$ unique nucleic acid-guided nuclease-gNA complexes. In some examples, the nucleic acid-guided nuclease is Cas9, Cpf1 or a combination thereof.

Also described herein are kits comprising the multi-analyte controls of the disclosure, kits comprising the normalization controls of the disclosure, and kits comprising both the normalization controls and the multi-analyte controls of the disclosure. In some examples, the kits further comprise reagents for nucleotide modification based enrichment of target nucleic acids of interest, reagents for nucleic acid-guide nuclease based enrichment of target nucleic acids of interest, or both.

Also described are kits comprising the normalization controls of the disclosure.

Also described are kits comprising multi-analyte controls of the disclosure.

Also described are kits comprising normalization controls, multi-analyte controls, reagents, and instructions for use. In some examples, the kits further comprise reagents for depleting a sequence targeted for depletion, and instructions for use.

Also described are systems for designing a plurality of normalization control polynucleotide sequences. In some examples, the system comprises a computer-readable storage medium which stores computer-executable instructions comprising: (i) instructions for importing at least one reference sequence; (ii) instructions for generating a plurality of reference sequence fragments from at least one reference sequence; (iii) instructions for generating a distribution of at least one parameter as a function of number of reference sequence fragments; (iv) instructions for dividing the distribution into bins; (v) instructions for selecting a plurality of reference sequence fragment from at least a subset of the bins; and (vi) instructions for shuffling the plurality of reference sequence fragments to generate shuffled sequences; thereby generating a plurality of normalization polynucleotide sequences. In some examples the system comprises a computer-readable storage medium which stores computer-executable instructions comprising: (i) instructions for importing at least one reference sequence; (ii) instructions for generating a plurality of reference sequence fragments from at least one reference sequence; (iii) instructions for generating a distribution of at least one parameter as a function of number of reference sequence fragments; (iv) instructions for dividing the distribution into bins; (v) instructions for selecting at least two reference sequence fragments from each of at least a subset of the bins, wherein the at least two reference sequence fragments are either non-contiguous in the reference sequence, or from different reference sequences; and (vi) instructions for concatenating the at least two reference sequence fragments from each bin; thereby generating a plurality of normalization polynucleotide sequences. In some examples, the system further comprises a processor which is configured to perform steps comprising: (a) receiving a set of input files comprising the at least at least one reference sequence; and (b) executing the computer-executable instructions stored in the computer-readable storage medium. In some embodiments, the parameter comprises least one of (1) percent GC content, (2) entropy, (3) complexity, (4) EIIP, (5) length, or a combination thereof.

## BRIEF DESCRIPTION OF THE DRAWINGS

FIG. 1 is a table showing a quality control analysis of normalization control (NC) reads.

FIG. 2 is a graph plotting Cytomegalovirus (CMV) abundance (x-axis) versus titer (y-axis). Without normalization, the $R^2$ value is 0.89.

FIG. 3 is a graph plotting normalization control (NC) normalized CMV abundance (x-axis) versus titer (y-axis). With normalization, the $R^2$ value is 0.98.

FIG. 4 is a bar chart showing the percent abundance of a virus in sample (y-axis, arbitrary units) analyzed with three hypothetical next generation sequencing libraries to demonstrate the percent abundance of virus calculated using different transformations. For each library, the bars, from left to right, represent known viral load, viral load calculated using raw read evidence following next generation sequencing, and viral load calculated using f(r) read evidence and f(r) + Δr read evidence.

FIG. 5 is a plot showing the expected decrease in variability when calculating a viral load using f(r) versus calculating viral load using raw reads.

FIG. 6 is an outline of a protocol for quantifying the level of a target sequence in a sample where normalization controls are added during the RNA extraction process.

FIG. 7 is a graph showing the normalization control percent reads in libraries of Exact Diagnostics Negative Plasma samples where the NCs were added to the samples during sample extraction in low, medium and high amounts.

FIG. 8 is a table of a protocol for annealing dual indexing primers to normalization controls.

**FIG. 9** is a graph showing normalization control percent reads in libraries with spiked in normalization controls where DNA NCs were spiked in at high (left two bars), medium (right two bars) and low (middle two bars) amounts.

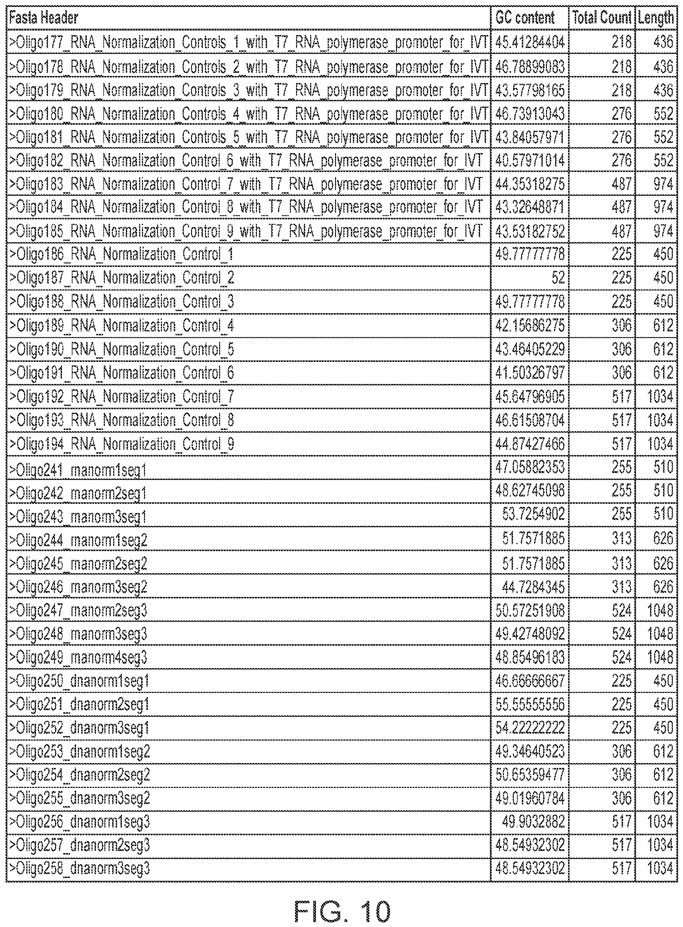

**FIG. 10** is a table showing GC content, total count and lengths of exemplary normalization controls.

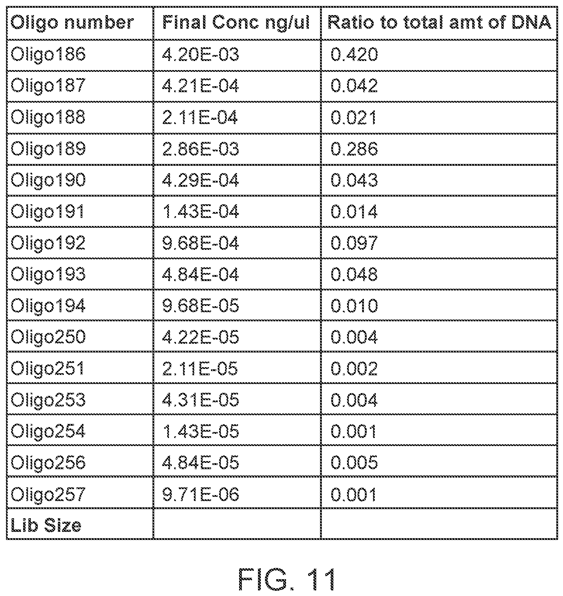

**FIG. 11** is a table showing concentrations and ratios of exemplary normalization controls of the disclosure. From left to right, columns show groups of polynucleotides (referred to in FIG. 11 as "oligos" and with corresponding oligo numbers) in the normalization control composition, the final concentration (ng/µL) of each group of polynucleotides, and for the population of normalization control polynucleotides in the composition, the ratio of the amount of DNA of the specified group of polynucleotides in the normalization control composition to the total amount of all DNA for all the polynucleotides in the normalization control composition.

**FIG. 12** is a table showing size, number of input molecules and concentrations of exemplary RNA normalization controls of the disclosure. Columns, from left to right, show individual RNA fragments in the normalization control, groups of RNAs in the normalization control, the length of the RNA molecules (in kb and bp), the number of input molecules, the number of attomoles of each RNA molecule in its original gBlock, and the resulting concentration of each RNA molecule in attomoles/microliter following gBlock fragmentation.

**FIG. 13** is a graph showing titer in log units as a function of observed signal for calibration and experimental samples. Dark gray circles indicate species in multi-analyte control samples used for calibration, while light gray circles are experimental samples.

**FIG. 14A** is a flow chart showing an exemplary process for designing normalization controls of the disclosure.

**FIG. 14B** is a graph showing signal (y-axis) and log concentration (x-axis) of one design of normalization controls of the disclosure.

**FIG. 14C** is a table showing the concentrations of one set of normalization controls of the disclosure. Each oligo is a different sequence.

**FIG. 14D** is a graph showing signal (y-axis) and log concentration (x-axis) of one design of normalization controls of the disclosure. Each order of magnitude in concentration is represented by 6 concentrations with at least one different oligo per log, and 7-8 orders of magnitude are covered.

**FIG. 15** is a ternary plot showing the interactions between entropy, electron-ion interaction potential (EIIP) and GC content for the approximately 1200 reference genomes.

**FIG. 16** is a series of three plots showing the distribution of Kmers from reference sequences generated using the shuffling method. From left to right: electron EIIP, entropy and GC content. Values for EIIP, entropy and GC content are shown on the x-axis, from left to right, respectively. On the y-axis, Kmer density is shown. The line represents the distribution and the histogram bars represent actual counts of the number of Kmers in a given bin.

**FIG. 17A** is a series of three plots showing the distribution 10 million Kmers generated using the shuffling method. From left to right: electron EIIP, entropy and GC content. Values for EIIP, entropy and GC content are shown on the x-axis, from left to right, respectively. On the y-axis, Kmer density is shown.

**FIG. 17B** is a series of three plots showing the distribution 1.3 million Kmers generated using the shuffling method. From left to right: electron EIIP, entropy and GC content. Values for EIIP, entropy and GC content are shown on the x-axis, from left to right, respectively. On the y-axis, Kmer density is shown.

**FIG. 18** is a diagram showing a process for concatenating Kmers prior to shuffling to generate normalization control sequences.

**FIG. 19A** is a series of three graphs showing the correspondence between normalization controls generated using the Cantor shuffling method and BK polyomavirus isolate CH-1 for EIIP (top left), GC content (top right) and complexity (bottom row). Unshuffled Kmers are plotted on the x-axis, Cantor shuffled Kmers are on the y-axis, lines indicate 1:1 correspondence.

**FIG. 19B** is a series of three graphs showing the correspondence between normalization controls generated using the Cantor shuffling method and JC polyomavirus strain NIID 12-31 for EIIP (top left), GC content (top right) and complexity (bottom row). Unshuffled Kmers are plotted on the x-axis, Cantor shuffled Kmers are on the y-axis, lines indicate 1:1 correspondence.

**FIG. 19C** is a series of three graphs showing the correspondence between normalization controls generated using the Cantor shuffling method and human polyomavirus 1 strain BK 2 for EIIP (top left), GC content (top right) and complexity (bottom row). Unshuffled Kmers are plotted on the x-axis, Cantor shuffled Kmers are on the y-axis, lines indicate 1:1 correspondence.

**FIG. 19D** is a series of three graphs showing the correspondence between normalization controls generated using the Cantor shuffling method and human Adenovirus C for EIIP (top left), GC content (top right) and complexity (bottom row). Unshuffled Kmers are plotted on the x-axis, Cantor shuffled Kmers are on the y-axis, lines indicate 1:1 correspondence.

**FIG. 20A** is a Basic Local Alignment Search Tool (BLAST) alignment showing that Cantor shuffled normalization control sequences do not align to BK polyoma virus isolate CH-1 using NCBI BLAST Nucleotide Sequence.

**FIG. 20B** is a blast alignment showing that Cantor shuffled normalization control sequences do not align to JC polyomavirus strain NIID 12-31 using NCBI BLAST Nucleotide Sequence.

FIG. 20C is a blast alignment showing that Cantor shuffled normalization control sequences do not align to human polyomavirus 1 strain BK 2 using NCBI BLAST Nucleotide Sequence.

FIG. 20D is a BLAST alignment showing that Cantor shuffled normalization control sequences do not align to human Adenovirus C using NCBI BLAST Nucleotide Sequence.

FIG. 21A is a series of three plots showing EIIP, entropy and GC content for Kmers of selected percent GC content.

FIG. 21B is a series of three plots showing EIIP, entropy and GC content for Kmers of selected percent GC content.

FIG. 21C is a series of three plots showing EIIP, entropy and GC content for Kmers of selected percent GC content.

FIG. 22 is a pair of plots showing the first and second principle components (x and y axes, respectively) for EIPP, GC content and entropy. PC1 and PC2 were calculated for sequences of the Reference Genomes (left) and normalization control sequences generated using a sliding window/exhaustive Kmer approach (right).

FIG. 23 are a series of three Q-Q plots comparing GC content (top), EIIP (bottom left) and entropy (bottom right) of Reference Genome Sequences (x-axis) and normalization control oligos generated using a sliding window/exhaustive Kmer approach (y-axis).

FIG. 24 is a Kolmogorov-Smirnov (KS) test plot comparing entropy in random sub-sampled populations of the Reference Genome sequences and the normalization control sequences generated using a sliding window/exhaustive Kmer approach. ECDF = empirical cumulative distribution function. Arrow indicates entropy of the normalization controls. Dots indicate the K-S statistic.

FIG. 25A is a BLAST alignment showing that sequences at the head of the file of 785,000 normalization control sequences generated using a sliding window/exhaustive Kmer approach align to Pseudomonas at the ~750 base pair (bp) position for about 50-80 bp.

FIG. 25B is a BLAST alignment showing that sequences at the tail of the file of 785,000 normalization control sequences generated using a sliding window/exhaustive Kmer approach do not align to any sequences using BLAST.

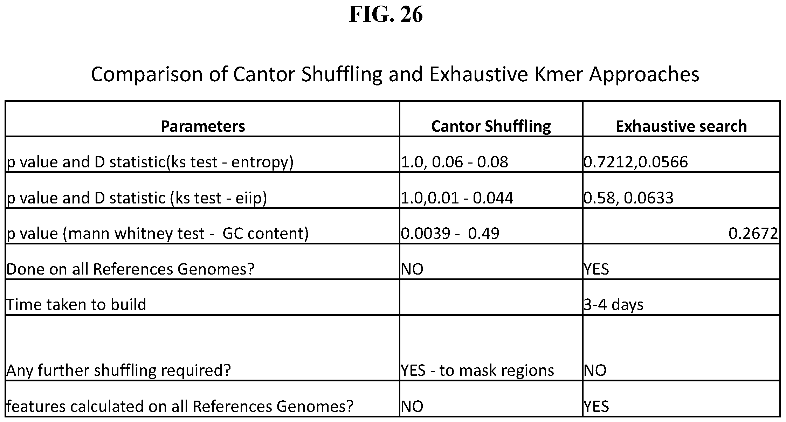

FIG. 26 is a table comparing normalization controls designed using the shuffling and exhaustive Kmer/sliding window approaches.

FIG. 27 is a diagram illustrating an exemplary method of the invention. Nucleic acids in the sample are dephosphorylated, and then digested with a restriction enzyme that is blocked by the presence of modifications at the restriction enzyme recognition site. The exposed phosphates from the resulting digestion are then used to ligate adapters to the nucleic acids of interest.

FIG. 28 is a diagram illustrating an exemplary method of the invention. Nucleic acids in the sample are dephosphorylated, and then digested with a restriction enzyme that recognizes a restriction enzyme site comprising one or more modified nucleotides. Cut nucleic acids are then digested with an exonuclease that uses the exposed terminal phosphates, and adapters are ligated to the remaining nucleic acids of interest.

FIG. 29 is a diagram illustrating an exemplary method of the invention. Nucleic acids in the sample are adapter ligated, and then digested with a restriction enzyme that recognizes a restriction enzyme site comprising one or more modified nucleotides, resulting in nucleic acids of interest that are adapter ligated on both ends.

FIG. 30 is a diagram illustrating an exemplary method of the disclosure. Nucleic acids in the sample are adapter ligated, and then cleaved with a nucleic acid-guided nuclease that cleaves the nucleic acids targeted for depletion, resulting in nucleic acids of interest that are adapter ligated on both ends. This method can be used in conjunction with the nucleotide modification-based methods of the disclosure.

## DETAILED DESCRIPTION

Described herein are compositions useful as normalization controls for the quantification of samples in next generation sequencing (NGS). The normalization controls may be used when working with low sample inputs for NGS. The normalization controls may be used to calculate the titer of an organism in a sample, for example using a multi-analyte control as described herein. The normalization controls may be combined with methods of enriching a sample for a target sequence, as described herein.

The invention provides methods of making normalization controls. Methods of using normalization controls during NGS are also described herein.

Unless defined otherwise herein, all technical and scientific terms used herein have the same meaning as commonly understood by one of ordinary skill in the art to which this disclosure belongs. Although any methods and materials similar or equivalent to those described herein can be used in the practice or testing of the present disclosure, exemplary methods and materials are described.

The term "next-generation sequencing" (NGS) refers to the so-called parallelized sequencing-by-synthesis or sequencing-by-ligation platforms, for example, those currently employed by Illumina, Life Technologies, and Roche, etc. Next-generation sequencing methods may also include nanopore sequencing methods or electronic-detection based methods such as Ion Torrent technology commercialized by Life Technologies. NGS is also referred to herein as "high throughput sequencing."

A "sequencing sample" is a sample containing nucleic acids that are sequenced using NGS. The nucleic acids can be deoxyribonucleic acids (DNA), ribonucleic acids (RNA) or both.

A "target sequence" refers to an individual sequence or set of sequences in a sequencing sample whose abundance in the sample is determined by use of the NCs of the disclosure, in an NGS reaction. The abundance of a target sequence may be non-zero (*i.e.,* the target sequence is present in the sample with some abundance), or zero (*i.e.,* no target sequence is present, or is below levels of detection).

As used herein, a "reference sequence" refers to one or more sequences that is identical or similar to any known target sequence. A target sequence can comprise or consist of one reference sequence, or a plurality of reference sequences.

A "target organism" refers to an organism in a sample comprising a target sequence.

As used herein, the term "component sequence" refers to a portion or entirety of a polynucleotide found in a normalization control. A normalization control polynucleotide comprises or consists of the component sequence. The component sequence can be isolated from a naturally occurring sequence, an engineered plasmid, or synthetic. The term "isolated sequence" refers to a type of component sequence that has been isolated or derived from an organism or an otherwise preexisting sequence. Isolated sequences may be similar to the sequences of the organism from which they are isolated or derived, or may also be subject to one or more transformations such as shuffling or concatenation.

The term "low sample" or "low input sample" refers to samples which comprise amounts of nucleic acids that are lower than used in conventional library preparation protocols. A low sample input can comprise as much as 100 ng of nucleic acids, and as little as 1 picogram of nucleic acids of total nucleic acids in the sample. In certain embodiments, the term "low sample" or "low sample input" refers to the amount of a nucleic acid encoding one or more target sequences in a sample. A low sample input can comprise as much as 100 ng of nucleic acids comprising the target sequence, or less than 1 femtogram of nucleic acids comprising the target sequence. For particularly rare target sequences, concentrations of the target sequences in the femtomolar and attomolar range are considered as being within the scope of the disclosure.

As used throughout the disclosure, "sequence identity" or "sequence similarity" may be determined by using the stand-alone executable BLAST engine program for blasting two sequences (bl2seq), which can be retrieved from the National Center for Biotechnology Information (NCBI) ftp site, using the default parameters (Tatusova and Madden, FEMS Microbiol Lett., 1999, 174, 247-250). The terms "identical" or "identity" when used in the context of two or more nucleic acids or polypeptide sequences, refer to a specified percentage of residues that are the same over a specified region of each of the sequences. The percentage can be calculated by optimally aligning the two sequences, comparing the two sequences over the specified region, determining the number of positions at which the identical residue occurs in both sequences to yield the number of matched positions, dividing the number of matched positions by the total number of positions in the specified region, and multiplying the result by 100 to yield the percentage of sequence identity. In cases where the two sequences are of different lengths or the alignment produces one or more staggered ends and the specified region of comparison includes only a single sequence, the residues of single sequence are included in the denominator but not the numerator of the calculation. When comparing DNA and RNA, thymine (T) and uracil (U) can be considered equivalent. Identity can be performed manually or by using a computer sequence algorithm such as BLAST or BLAST 2.0.

Sequences can be "similar" if they are at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% or at least 99.9% identical by alignment.

Described herein are normalization controls tobe added to a sample for NGS. Optionally, the sample is a low input sample.

The disclosure provides methods of making normalization controls. Described herein are methods of using the same.

In some embodiments, the normalization controls are added at the same quantity to every sample.

In some embodiments, the normalization controls are not added at the same quantity to every sample.

In some embodiments, normalization controls are added to the sample prior to nucleic acid extraction. In other embodiments, normalization controls are added after nucleic acid extraction and during library preparation. In other alternatives, normalization controls are added to the sample after library preparation and prior to sequencing.

The disclosure describes normalization controls comprising at least three groups of polynucleotides, wherein the polynucleotides within each of the at least three groups are of the same length. In some embodiments, normalization control polynucleotides comprise a component sequence, in some embodiments designed to resemble or recapitulate some feature or characteristic of a target sample to be sequenced. In some embodiments, the component sequence is a random sequence. In some embodiments, normalization control polynucleotides comprise an isolated sequence. In some embodiments, normalization control polynucleotides comprise both isolated sequences and component sequences. In some embodiments, the sequences of the normalization control polynucleotides share at least one sequence property with a target sequence in a sequencing sample.

Described herein are methods of making normalization controls comprising: (a) synthesizing at least three

populations of DNA molecules; wherein each of the DNA molecules within a population has the same sequence, wherein the sequence of each of the DNA molecules comprises, from 5' to 3', a first component sequence, a first restriction enzyme site, a second component sequence, a second restriction site, and a third component sequence; (b) quantifying the amount of the each of the populations of DNA molecules in a solution; (c) digesting each of the populations of DNA molecules with a restriction enzyme that cuts the first and second restriction sites to produce at least three populations of digested DNA molecules; and (d) mixing a pre-determined amount of each of the populations of digested DNA molecules in a single normalization control composition.

In accordance with the invention, the normalization controls are RNA normalization controls, and the disclosure provides methods of making normalization controls comprising: (a) synthesizing populations of at least three DNA molecules; wherein each of the at least three synthetic DNA molecules comprises, from 5' to 3', a first component sequence, a sequence complementary to promoter sequence, a first restriction enzyme site, a second component sequence, a second sequence complementary to a promoter sequence, a second restriction site, a third component sequence and a third sequence complementary to a promoter sequence; (b) digesting each population of DNA molecules with a restriction enzyme that cuts the first and second restriction enzyme sites to produce at least three populations of digested DNA molecules; (c) *in vitro* transcribing each of the at least three digested populations of DNA molecules to produce at least three populations of RNA molecules; (d) quantifying the amount of RNA produced in each of the populations of RNA molecules; and (e) mixing a pre-determined amount of each of the populations of RNA molecules to produce a single normalization control composition.

Also described herein are methods of making normalization controls comprising: (a) extracting DNA from a sample; (b) digesting the DNA with a restriction enzyme to produce a collection of DNA fragments; and (c) separating the collection of DNA fragments; (d) purifying at least three DNA fragments to produce at least three groups of polynucleotides, wherein each of the at least three groups of polynucleotides comprises a plurality of polynucleotides; and (e) mixing a pre-determined amount of each of the at least three groups of polynucleotides to produce the normalization control.

Also described herein are methods of making normalization control compositions wherein the normalization controls comprise both polynucleotides that are synthesized using the methods of the disclosure, and polynucleotides comprising isolated sequences that are isolated and/or derived using the methods of disclosure.

Also described herein are methods of quantifying the level of expression of at least one target nucleic acid molecule in a sample using normalization controls comprising: (a) mixing a known amount of a normalization control of the disclosure with the sample, (b) preparing a high throughput sequencing library, (c) sequencing said library to produce a collection of reads, (d) mapping each read to the sample or the normalization control, (e) determining the number of reads produced by each of the plurality of nucleic acid molecules in the normalization control, (f) calculating relationship between the starting concentration of each of the plurality of nucleic acid molecules in the normalization control mixed with the sample in (a) and the number of reads produced in (c), (g) modeling the relationship between reads and concentration in the sample, and h. calculating the initial concentration of the at least one target nucleic acid molecule in the sample from the number of reads produced by the target nucleic acid molecule using the model in (g).

### *Normalization Control Compositions*

The disclosure provides normalization control compositions (interchangeably referred to herein as normalization controls, or (NCs) useful for the quantification of sample input in NGS applications. As provided herein, the NCs of the disclosure can comprise at least two groups of polynucleotides, wherein the polynucleotides within each of the at least two groups are of the same length. In some embodiments, NCs are *de novo* synthesized as polynucleotides. In some embodiments, NCs are isolated or derived from sample, such as a DNA or RNA sample from an organism, or a vector. In some embodiments, NCs comprise both *de novo* synthesized polynucleotides and polynucleotides that are isolated or derived from a sample. In some embodiments, NCs comprise at least three group of polynucleotides, wherein the polynucleotides within each group of the at least three groups are of the same length.

In some embodiments, NCs comprise at least 3 groups of polynucleotide sequences of different lengths, and each group of polynucleotides is present in the normalization control at a different concentration.

In some embodiments, NCs comprise at least 15 groups of polynucleotide sequences of different lengths, and each group of polynucleotides is present in the normalization control at a different concentration.

In some embodiments, NCs comprise 15 groups of polynucleotide sequences of different lengths, and each group of polynucleotides is present in the normalization control at a different concentration.

Polynucleotide Lengths in the NCs

The lengths of the polynucleotides within the normalization controls can be varied throughout the concentration range to allow for bias in the fragmentation rates or clustering (as is sometimes seen in the case of Illumina based NGS methods). For example, a normalization control comprises multiple groups of polynucleotides, and each group of polynucleotides is a different length and present at a different concentration within the normalization control.

Groups of polynucleotides of differing lengths and combinations of lengths are envisaged as being within the scope of normalization controls of the disclosure. All of the individual polynucleotides within a given group are of the same length. However, within a normalization control composition comprising at least three groups, the lengths of the polynucleotides within a group can be either the same as or different from the lengths of the polynucleotides within any other group in the normalization control composition.

Accordingly, in some embodiments, the lengths of all of the polynucleotides in the normalization control are the same. In some embodiments, the polynucleotides within each of the at least three groups are of a different length than the polynucleotides within any other group.

In some embodiments, the lengths of the at least three groups of polynucleotides are distributed in a linear sequence or a geometric sequence.

A linear sequence is a sequence of numbers in which the next number in the sequence increases or decreases by the same amount each time relative to the previous number. An exemplary linear sequence comprises the sequence of "1, 2, 3, 4, 5, 6." In general, linear sequences are represented by the formula:

$$u_n = d \times n + c$$

where d is the first difference between successive terms in the sequence, n is the term in the sequence, and c is a constant.

A geometric sequence is a sequence of numbers where each term after the first is found by multiply the previous term by a fixed, non-zero number called the common ratio. An exemplary geometric sequence comprises "2, 4, 8, 16" (each number is 2x the previous). In general, geometric sequences are represented by the formula:

$$u_n = u_1^{r-1}$$

where r is the common ratio.

In some embodiments, for example in those embodiments wherein the polynucleotides comprise a component sequence (as further described herein), the lengths of the polynucleotides in each of the groups are between about 15 bp and about 3000 bp, between about 50 bp and about 3000 bp, between about 100 bp and about 3000 bp, between about 1000 bp and about 3000 bp, between about 1200 and about 3000 bp, between about 1500 bp and about 3000 bp, between about 15 bp and about 50 bp, between about 15 bp and about 100 bp, between about 15 bp and about 150 bp, between about 15 bp and about 200 bp, between about 15 bp and about 300 bp, between about 15 bp and about 400 bp, between about 15 and about 500 bp, between about 50 bp and about 1200 bp, between about 100 bp and about 1200 bp, between about 150 bp and about 1200 bp or between about 150 and about 1100 bp. In exemplary embodiments, the lengths of the polynucleotides in the groups of polynucleotides are between about 15 bp and about 3000 bp. In exemplary embodiments, the lengths of the polynucleotides in the groups of polynucleotides are between about 500 bp and about 1500 bp. In exemplary embodiments, the lengths of the polynucleotides in the groups of polynucleotides are between about 100 bp and about 1200 bp. In exemplary embodiments, the lengths of the polynucleotides in the groups of polynucleotides are between about 150 bp and about 600 bp.

In some embodiments, the at least three groups of polynucleotides comprise polynucleotides with lengths selected from the lengths consisting of: 175 bp, 250 bp and 450 bp; 192 bp, 250 bp and 450 bp; 200 bp, 300 bp and 500 bp; 217 bp, 300 bp and 517 bp; 436 bp, 552 bp and 974 bp; 450 bp, 612 bp and 1034 bp; 510 bp, 626 bp and 1048 bp; and 450 bp, 612 bp and 1034 bp.

As described herein, the groups of polynucleotides can be synthesized *in vitro,* or can be isolated or derived from already synthesized nucleic acids, or can be isolated or derived from naturally occurring nucleic acids. It is contemplated that polynucleotides that are synthesized *in vitro* may have a maximum length of approximately 3 kb, but a skilled artisan will understand such length may be dictated by current oligonucleotide synthesis technologies. It is contemplated that normalization control polynucleotides isolated or derived from already synthesized, or naturally occurring nucleic acids may encompass a broader range of lengths than normalization controls that are made using synthesis methods, but here too, this is dictated by current oligonucleotide synthesis technologies.

In some embodiments, for example where the normalization control polynucleotides comprise an isolated sequence (as described further herein), the lengths of the at least three different groups of polynucleotides are between about 15 bp and about 500 kb. In some embodiments, the lengths of the at least three different groups of polynucleotides are between about 15 bp and about 50 kb. In some embodiments, the lengths of the at least three different groups of polynucleotides are between about 50 bp and about 50 kb. In some embodiments, the lengths of the at least three different groups of polynucleotides are between about 500 bp and about 50 kb. In some embodiments, the lengths of the at least three different groups of polynucleotides are between about 1000 bp and about 50 kb. In some embodiments, the longest of the each of the at least three different groups of polynucleotides is at least 15 kb in length, at least 20 kb in length, at least 25 kb in length, at least 30 kb in length, at least 35 kb in length or at least 40 kb in length, inclusive of the endpoints.

Polynucleotide Sequences in the NCs

Groups of polynucleotides of the disclosure are envisaged as either comprising polynucleotides of the same sequence, or comprising polynucleotides in which the sequences of all the polynucleotides in the group are not all the same.

In some embodiments, the polynucleotides within each group consist of the same sequence.

In some embodiments, the polynucleotides within each group comprise the same sequence. Non-limiting examples of sequences that can be the same for all the polynucleotides within each group include adaptor sequences, primer sequences, indexing sequences, restriction sites, promoter sequences, component sequences, isolated sequences or a combination thereof.

In some embodiments, the polynucleotides within each group comprise both a sequence that is the same sequence in all the polynucleotides and a sequence that is not the same in all the polynucleotides. For example, all the polynucleotides within each group can comprise the same adaptor and/or indexing sequence, but differ in their component sequences. In other embodiments, all the polynucleotides within each group can comprise different adaptor and/or indexing sequences, but share the same component or isolated sequence.

In some embodiments, the sequences of polynucleotides within each group are at least 5% identical, at least 10% identical, at least 20% identical, at least 30% identical, at least 40% identical, at least 50% identical, are at least 60% identical, at least 70% identical, at least 80% identical, are at least 90% identical, at least 95% identical, at least 97% identical, at least 98% identical or at least 99% identical.

In some embodiments, the polynucleotides within each group do not comprise the same sequence.

In some embodiments, one or more, or every group of polynucleotides comprises at least three subgroups of polynucleotides, wherein the polynucleotides within each subgroup comprise the same sequence, and the polynucleotides within each subgroup do not comprise the same sequence as any other subgroup.

In some embodiments of the normalization controls of the disclosure, each polynucleotide within each of the at least three groups comprises the same sequence.

In some embodiments, the sequence of the plurality of polynucleotides within each one of the at least three groups does not comprise the same sequence as the sequence of the plurality of polynucleotides within any other group.

In some embodiments, each of the pluralities of polynucleotides within each of the at least three groups comprises polynucleotides that do not have the same sequence.

In some embodiments, each of the at least three groups of polynucleotides comprises at least three subgroups of polynucleotides, and each subgroup of polynucleotides comprises a plurality of polynucleotides of the same sequence, and the sequence of the plurality of polynucleotides in each subgroup is not the same as the other subgroups in the group.

Component Sequence

In some embodiments of the normalization controls of the disclosure, the sequence of at least one group of polynucleotides comprises a component sequence, designed, selected, or known to have certain properties, such as length, sequence, or GC content. This can result in that group of polynucleotides having a designed, selected, or known range of those properties represented. The properties of the component sequence can be designed or selected to match the properties of a target sequence in a sequencing sample.

As used herein, the term "component sequence" refers to a portion or entirety of the polynucleotide found in the normalization control. The normalization control polynucleotide comprises or consists of the component sequence. In some embodiments, the component sequence is designed *in silico* and is not isolated or derived from any organism.

In some embodiments, the component sequence comprises a random sequence. In some embodiments, the component sequence is designed to mimic, or share, one or more sequence properties of a target sequence in a sequencing sample.

In some embodiments, the sequence of every group of polynucleotides comprises a component sequence. In some embodiments, the component sequence of each group of polynucleotides is not the same as the component sequence of any other group.

In some embodiments, the sequence of at least one subgroup of at least one group of polynucleotides comprises a component sequence. In some embodiments, the sequence of each of the at least three subgroups of at least one group comprises a component sequence. In some embodiments, the sequence of each of at least three subgroups of every group comprises a component sequence. In some embodiments, the component sequence of each subgroup is not the same as the component sequence of any other subgroup.

For example, in a normalization control composition with three groups, each of which has three subgroups, each subgroup has a different component sequence, none of which is the same. In this example, the population of polynucleotides in the normalization control composition comprises polynucleotides with nine different component sequences.

In some embodiments, the component sequence comprises a sequence of between about 15 bp and about 3000 bp, between about 50 bp and about 3000 bp, between about 100 bp and about 3000 bp, between about 1000 bp and about 3000 bp, between about 1200 and about 3000 bp, between about 1500 bp and about 3000 bp, between about 15 bp and about 50 bp, between about 15 bp and about 100 bp, between about 15 bp and about 150 bp, between about 15 bp and about 200 bp, between about 15 bp and about 300 bp, between about 15 bp and about 400 bp, between about 15 and about 500 bp, between about 50 bp and about 1200 bp, between about 100 bp and about 1200 bp, between about 150 bp and about 1200 bp or between about 150 and about 1100 bp. In some embodiments, the component sequence comprises a sequence of between about 6 bp and about 3000 bp. In some embodiments, the component sequence comprises a sequence of between about 150 bp and about 500 bp.

In some embodiments of the methods of the disclosure, the component sequence is not the same as a target sequence in a sequencing sample. In some embodiments, the component sequence has less than or equal to 1%, 2, %, 3%, 4%, 5%, 6%, 7%, 8%, 9%, 10%, 11%, 12%, 13%, 14%, 15%, 16%, 17%, 18%, 19%, 20%, 30%, 40%, 50%, 60%, 70%, 80%, 85%, 90%, 95%, 96%, 97%, 98%, 99% or 99.5%, 99.8% or 99.9% identity to a target sequence in a sequencing sample. In some embodiments, the component sequence may differ from the target sequence by about 1 bp in every 20 bp, by about 1 bp in every 50 bp, by about 1 bp in every 150 bp, by about 1 bp in every 250 bp or by about 1 bp in every 300 bp.

In some embodiments, the component sequence comprises or consists of a random sequence. In some embodiments, random sequences are generated using a random sequence generator. In some embodiments, random sequences do not map to reference sequences. In some embodiments, random sequences do not map to the genome of any organism of interest, for example, any organism in the NCBI Nucleotide database, or any organism which the normalization controls will be used to detect, quantify, or otherwise analyze. In some embodiments, the random sequence is not cut by any CRISPR-associated (Cas) guide RNAs (gRNAs) in a 90k gRNA array designed to cut human DNA. In some embodiments, random sequences can be designed to exclude specific sequence features. For example, a random sequence can exclude features such as CRISPR/Cas gRNA recognition sites, restriction sites, transcription factor binding sites or repetitive sequences. In some embodiments, random sequences can be designed to mimic properties of a target sequence. For example, a random sequence can be designed with the same GC content, or the same frequency of a particular sequence motif (e.g. a trinucleotide repeat) as a target sequence.

Isolated Sequence

In some embodiments of the normalization controls of the disclosure, the normalization control polynucleotides comprise an isolated sequence. As used herein, an "isolated sequence" is a type of component sequence. The term "isolated sequence" refers to a sequence that has been isolated or derived from an organism or an otherwise preexisting sequence. Isolated sequences can comprise genomic, mitochondrial or chloroplast DNA sequences. Isolated sequences can comprise RNA sequences, for example messenger RNAs (mRNAs), transfer RNAs (tRNAs), non-coding RNAs (ncRNAs) or microRNAs. Isolated sequences can be isolated from an organism or pre-existing sequence, and comprise a fragment or fragments of a pre-existing sequence. Alternatively, or in addition, isolated sequences may be subject to one or more transformations to derive them from pre-existing sequences, such as sequence shuffling, sequence concatenation, inverting the order of nucleotides, or nesting within other sequences.

In some embodiments, the sequence of at least one group of polynucleotides comprises an isolated sequence. In some embodiments, the sequence of every group of polynucleotides comprises an isolated sequence.

In some embodiments, sequence of at least one subgroup of at least one group of polynucleotides comprises an isolated sequence. In some embodiments, sequence of every subgroup of at least one group comprises an isolated sequence. In some embodiments, the sequence every subgroup of every group comprises an isolated sequence. In some embodiments, the isolated sequence of each subgroup is not the same as the isolated sequence of any other subgroup.

In some embodiments, the length of the isolated sequence is between about 15 bp and about 500 kb. In some embodiments, the length of the isolated sequence is between about 15 bp and about 100 kb. In some embodiments, the length of the isolated sequence is between about 15 bp and about 50 kb. In some embodiments, the length of the isolated sequence is between about 50 bp and about 50 kb. In some embodiments, the length of the isolated sequence is between about 500 bp and about 50 kb. In some embodiments, the length of the isolated sequence is between about 1000 bp and about 50 kb.

In some embodiments, the isolated sequence is between about 6 bp and about 200,000 bp, between about 15 bp and about 50,000 bp, between about 500 bp and about 1500 bp, between about 100 bp and about 1200 bp, or between about 150 bp and about 600 bp.

In some embodiments, the isolated sequence is at least about 50 bp, at least about 100 bp, at least about 150 bp, at least about 200 bp, at least about 250 bp, at least about 300 bp, at least about 350 bp, at least about 400 bp, at least about 450 bp, at least about 500 bp, at least about 550 bp, at least about 600 bp, at least about 650 bp, at least about 600 bp, at least about 750 bp, at least about 800 bp, at least about 850 bp, at least about 900 bp, at least about 950 bp, at least about 1000 bp, at least about 1250 bp, at least about 1500 bp, at least about 2000 bp, at least about 2500 bp or at least about 3000 bp.

In some embodiments, the isolated sequence is about 100 bp, about 200 bp, about 300 bp, about 400 bp, about 500 bp, about 600 bp, about 700 bp, about 800 bp, about 900 bp, about 1000 bp, about 1100 bp, about 1200 bp, about 1300 bp, about 1400 bp or about 1500 bp.

In some embodiments, the isolated sequence is isolated or derived from a virus, a bacterium, a fungus or a eukaryote. In some embodiments, the virus is a T4 bacteriophage (T4) or cytomegalovirus (CMV). In some embodiments, the isolated sequence is isolated from cloning vector, such as a plasmid.

In some embodiments, the nucleic acid sample from which the isolated sequences of the normalization controls are purified is a cloning vector. In some embodiments, the cloning vector is a bacterial component chromosome (BAC), a yeast artificial chromosome (YAC), a cosmid, a fosmid or a plasmid. In some embodiments, the nucleic acid sample from which the isolated normalization controls are purified is a plasmid. In some embodiments, the normalization control sequences are cloned from a species of interest, for example a virus, a bacteria or a eukaryotic parasite, into a cloning vector, and the normalization controls are purified from the cloning vector using methods of the disclosure.

In some embodiments, the isolated sequence is not the same as a target sequence in a sequencing sample. In some embodiments, the sequence of any of the polynucleotides in the normalization control has less than or equal to 1%, 2, %, 3%, 4%, 5%, 6%, 7%, 8%, 9%, 10%, 11%, 12%, 13%, 14%, 15%, 16%, 17%, 18%, 19%, 20%, 30%, 40%, 50%, 60%, 70%, 80%, 85%, 90%, 95%, 96%, 97%, 98% or 99% identity to the target sequence in the sequencing sample.

In some embodiments, the isolated sequence is isolated or derived from a plurality of reference sequences. A "reference sequence" refers to one or more sequences that is identical or similar to target sequence that is known in the art. A reference sequence may refer to a single target sequence, for example, a gene or a genome of a target organism.

As a further example a "reference sequence" may refer to a plurality of sequences from a plurality of different organisms. The organisms can be viruses, bacteria, fungi, or single celled eukaryotes, such as parasitic or pathogenic eukaryotes, or a combination thereof. The reference sequence can be a genomic DNA sequence, a cDNA sequence, or a combination thereof.

In some embodiments, the normalization controls comprise a plurality of isolated sequences isolated or derived from a single reference sequence.

In some embodiments, the normalization controls comprise a plurality of isolated sequences that are isolated or derived from a plurality of reference sequences, for example from a plurality of different organisms. In some embodiments, the plurality of reference sequences comprises a plurality of sequences from organisms generally regarded to be non-host organisms such as viruses, bacteria or fungi (*e.g.,* when the host is a mammal, plant or multi-cellular eukaryote). Pluralities of reference sequences can be used to model population level characteristics of panels of target organisms in the normalization controls described herein. Any of the panel of target organism may be present in the sequencing sample, and their presence and titer can be measured using the methods described herein. In those embodiments where the target sequence or target organism in the sequencing sample is not known prior to sequencing, using normalization controls that model pluralities of organisms can increase accuracy of identification and titer measurements.

In some embodiments, the plurality of isolated sequences are isolated or derived from at least 2, at least 3, at least 4, at least 5, at least 6, at least 7, at least 8, at least 9, at least 10, at least 20, at least 50, least 100, at least 200, at least 300, at least 400, at least 500, at least 600, at least 700, at least 800, at least 900, at least 1,000, at least 1,200, at least 1,300, at least 1,400, at least 1,500, at least 1,600, at least 1,700, at least 1,800, at least 1,900, at least 2,000, at least 2,200, at least 2,400, at least 2,600, at least 2,800, at least 3,000, at least 4,000, at least 5,000 , at least 6,000, at least 7,000, at least 8,000, at least 9,000, at least 10,000 reference sequences, at least 11,000 reference sequences, or at least 12,000 reference sequences. In some embodiments, the plurality of isolated sequences are isolated or derived from at least 10 reference sequences. In some embodiments, the plurality of isolated sequences are isolated or derived from at least 100 reference sequences. In some embodiments, the plurality of isolated sequences are isolated or derived from at least 500 reference sequences. In some embodiments, the plurality of isolated sequences are isolated or derived from at least 1000 reference sequences. In some embodiments, the plurality of isolated sequences are isolated or derived from at least 1,200 reference sequences. In some embodiments, the plurality of isolated sequences are isolated or derived from at least 1,500 reference sequences. In some embodiments, the plurality of isolated sequences are isolated or derived from at least 2,000 reference sequences. In some embodiments, each reference sequence is from a different organism, or different strain or subspecies of the same organism. In some embodiments, the plurality of reference sequences are genomic DNA sequences from different organisms or different strains or subspecies of the same organism.

In some embodiments, the plurality of reference sequences comprises reference sequences from a plurality of organisms. In some embodiments, the plurality of reference sequences comprise sequences from at least 2, at least 10, at least 20, at least 50, least 100, at least 200, at least 300, at least 400, at least 500, at least 600, at least 700, at least 800, at least 900, at least 1,000, at least 1,200, at least 1,300, at least 1,400, at least 1,500, at least 1,600, at least 1,700, at least 1,800, at least 1,900, at least 2,000, at least 2,200, at least 2,400, at least 2,600, at least 2,800, at least 3,000, at least 4,000, at least 5,000 , at least 6,000, at least 7,000, at least 8,000, at least 9,000 or at least 10,000 organisms. In some embodiments, the plurality of reference sequences comprise sequences from about 1,000 organisms, from about 1,200 organisms, from about 1,400 organisms, from about 1,500 organisms, from about 1,600 organisms, from about 1,700 organisms, from about 1,800 organisms, from about 2,000 organisms, from about 2,200 organisms, from about 2,400 organisms, from about 2,500 organisms, from about 3,000 organisms, from about 5,000 organisms, from about 7,000 organisms, or from about 10,000 organisms.

In some embodiments, the plurality of isolated sequences comprises sequences that are isolated or derived from fragments of reference sequences. In some embodiments, the reference sequence fragments are between about 10-100, about 10-80, about 10-70, about 10-60, about 10-50, about 10-40, about 10-30, about 10-20, about 20-100, about 20-80, about 20-60, about 20-50, about 20-40, about 20-30, about 10-35, about 20-35, or about 20-25 contiguous nucleotides of the reference sequence. In some embodiments, the reference sequence fragments are between about 15-60, about 20-40, about 20-30, about 15-32, 20-32 or about 25-35 contiguous nucleotides of the reference sequence. In some embodiments, the reference sequence fragments comprise 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 or 65 contiguous base pairs of the reference sequence. In some embodiments, the reference sequence fragments comprise 25, 26, 27, 28, 29, 30, 31, 32, 33, 34 or 35 contiguous bp of the reference sequence. In some embodiments, the reference sequence fragments comprise 31 or 32 contiguous bp of the reference sequence.

In some embodiments, the reference sequence fragments are less than half the average length of a sequencing read. For example, if the NGS sequencing method produces reads that are an average of 120 bp each, then the reference sequence fragments are less than 60 bp. Different NGS platforms produce sequencing reads of different lengths. For example, HiSeq and HiSeq X have average read lengths of around 300 bp, while the MiSeq platforms have average read lengths of between about 300 and 600 bp. In some embodiments, the average NGS read length is about 100 bp, about 150 bp, about 200 bp, about 250 bp, about 300 bp, about 350 bp, about 400 bp, about 450 bp, about 500 bp, about 550 bp, about 600 bp, about 650 bp, about 700 bp, about 750 bp, about 800 bp, about 850 bp, about 900 bp, about 950 bp, about 1000 bp, about 1100 bp, about 1200 bp, about 1300, about 1400 bp, about 1500 bp, about 1600 bp, about 1700 bp, about 1800 bp, about 1900 bp or about 2000 bp.

In some embodiments, the plurality of isolated sequences comprises fragments of reference sequences that have been shuffled. Methods of shuffling sequences are known in the art, and include random shuffling and Cantor shuffling. A variety of programs can be used to shuffle nucleotide sequences and will be known to persons of skill in the art (see, for example, www.bioinformatics.org/sms2/shuffle_dna.html). As contemplated herein, shuffled reference sequence fragments retain the one or more characteristics of the reference sequences, while at the same time are not identical to a target sequence in a sample, and hence is expected to be distinguishable from target sequences by methods such as blast.

In some embodiments, the plurality of isolated sequences comprises reference sequence fragments that have been concatenated. In some embodiments, the reference sequence fragments are shuffled and concatenated, *e.g.* to produce isolated sequences of a desired length or lengths. In some embodiments, the isolated sequence comprises at least two fragments of at least a first reference and second reference sequences that have been shuffled and concatenated. In some embodiments, the first and second reference sequences are non-adjacent in a genome of an organism. In some embodiments, the first and second reference sequences are from different organisms. An isolated sequence can comprise at least 1, 2, 3, 4, 5, 6, 7, 8, 9, 10, 11, 12, 13, 14, 15, 16, 17, 18, 19 or 20 reference sequence fragments that have been shuffled an concatenated, any of which may be non-adjacent fragments from the same reference sequence or reference organism, or from different reference sequences or reference organisms.

In some embodiments, the isolated sequence is derived from the reference sequence by a process of (a) representing at least one reference sequence by a distribution of reference sequence fragments; (b) dividing the into bins; (c) selecting a plurality of reference sequence fragments from at least a subset of the bins; and (d) shuffling, and optionally concatenating, the reference sequence fragments to generate isolated sequences.

In some embodiments, isolated sequences of the normalization controls are generated by (a) generating a distribution of overlapping reference sequence fragments generated using a sliding window over at least one reference sequence; (b) dividing the distribution into bins; (c) selecting a plurality of reference sequence fragments from at least a subset of the bins; and (d) concatenating the reference fragment sequences thereby generating isolated sequences.

In some embodiments, the sliding window comprises a 1 base pair (bp), 2 bp, 3 bp, 4 bp , 5 bp, 6 bp, 7 bp, 8 bp, 9 bp, 10 bp ,11 bp, 12 bp, 13 bp, 14 bp, 15 bp, 16 bp, 17, bp, 18 bp, 19 bp or 20 bp sliding window. A sliding window is a window of length N that is slid along the reference sequence to generate reference sequence fragments that are offset by the length of the window, *e.g.* 1, 2, 3, 4, 5 bp, or more.

The distribution can be a distribution of any one of (1) percent GC content, (2) entropy, (3) complexity, (4) electron-ion interaction potential (EIIP), (5) length, or a combination thereof. To generate the distribution, the reference sequences are broken into fragments of a specified size or sizes (sometimes referred to herein as Kmers), and the number of reference sequence fragments is binned across a given parameter such as percent GC content, EIIP, length or entropy. Each bin represents a percentage of the parameter distribution, for example 1%, 2%, 3%, 4%, 5%, 6%, 7%, 8%, 9%, 10%, 15%, 20%, or 25%.

The distribution can be divided into any number of bins. For example, the distribution can be divided into 2, 3, 4, 5, 6, 7, 8, 9, 10, 20, 50, 100 or 1000 bins. In some embodiments, each bin represents 0.1%, 0.5%, 1%, 2%, 5%, 10%, 15%, or 20% of the distribution of a parameter such as percent GC content, entropy, complexity, length or EIIP.

In some embodiments, polynucleotides within each group of the normalization control are generated using reference sequence fragments selected from one of the at least 3 bins of a distribution divided into 5 bins. In some embodiments, polynucleotides within each group of the normalization control are generated by using reference sequence fragments selected from at least 1, 2, 3, 4, 5, 6, 7, 8, 9, 10, 11, 12, 13, 14, 15, 16, 17, 18, 19 or 20 bins in a distribution. In some embodiments, the distribution is divided into 10 bins and the reference sequence fragments are selected from the 10th, 30th, 50th, 70th, 80th and 90th percentile bins from the percent GC content distribution. In some embodiments, polynucleotides within each group of the normalization control are generated by using reference sequence fragments selected from every bin in the distribution.

In some embodiments, one or more reference sequence fragments are selected from a bin of the distribution, for example a percent GC content distribution. In some embodiments, one or more reference sequence fragments are selected from every bin in the distribution. In some embodiments, one or more reference sequence fragments are selected from a subset of bins of the distribution. In some embodiments, reference sequence fragments selected from the same bin are concatenated to form an isolated sequence of desired length. This allows for isolated sequences in the normalization controls to model the distribution of various parameters such as GC content, EIIP, length and entropy across the full distribution by subsampling selected bins, without needing a prohibitively high number of normalization control oligos to model the entire distribution reference sequences with a 1:1 correspondence between reference sequences and normalization controls.

In some embodiments, the polynucleotides within each group have at least three of: (1) similar percent GC content, (2) similar entropy, (3) similar EIIP, (4) similar length or (5) similar complexity as the reference sequence fragments from the corresponding bin in the reference sequence distribution.

Similar percent GC content, EIIP, length, complexity or entropy can refer a comparison of the average GC content, EIIP, length, complexity or entropy of the reference sequences and normalization controls. For example, normalization controls and reference sequences may have similar percent GC content, EIIP, length, complexity or entropy if the average GC content, EIIP, length, complexity or entropy of the references sequences is within 0.1, 0.5, 1, 2, 3, 4, 5, 6, 7, 8, 9, 10, 11, 12, 13, 14, or 15 percentage points of the average GC content, EIIP, length, complexity or entropy of the normalization controls. Alternatively, or in addition, similar GC content, EIIP, length, complexity or entropy may refer to the distribution of GC content, EIIP, length, complexity or entropy across some or all sequences in the reference sequences and the sequences of the normalization controls. Methods of determining the degree of similarity will be known in the art and include, but are not limited to, Kolmogorov-Smirnov tests, Z-tests, Q-Q plots and Analysis of Variance (ANOVA). The ordinarily skilled artisan will be able to select the appropriate test based on the characteristics of the distribution(s) in question (*e.g.,* normal, Poisson, Gaussian, or skewed).

In some embodiments, the plurality of isolated sequences comprises reference sequence fragments that have been concatenated. In some embodiments, the reference sequence fragments are concatenated without being shuffled. If the reference sequence fragments that are concatenated to form the isolated sequences are non-adjacent in the reference sequence, or are from different reference sequences, then the presence of the "joins" between non-adjacent sequences or sequences from different sources should allow the isolated sequences to be identified via blast alignment after NGS. The presence of the joins allows the isolated sequences in the normalization control to be distinguished from the target sequence in the sample. Without wishing to be bound by theory, it is expected that sequence fragments between about 15 to about 50 bp, preferably about 20 to about 35 bp, may be preferred for this approach, as sequences that are too small, such as sequences below the seed length of the aligner, will likely not be sufficiently unique to produce robust alignments, while sequences that are too large will not have joins that are captured by individual NGS reads.

In some embodiments, the isolated sequence comprises at least two fragments of at least one reference sequence that are concatenated, and the at least two fragments are non-adjacent in the at least one reference sequence. *I.e.,* the at least two fragments are separated by at least 1 bp, at least 2 bp, at least 3 bp, at least 4 bp, at least 5 bp or any number of bp.

In some embodiments, the isolated sequence comprises at least a first fragment of at least a first reference sequence, and at least a second fragment of at least a second reference sequence, wherein the at least first and second fragments are concatenated, and wherein the first and second reference sequences are not the same reference sequence. For example, the first fragment is from human adenovirus, and the second fragment is from *Candida albicans.* As a further example, the isolated sequence can, comprise nested reference sequence fragments: *e.g.,* a reference sequence fragment from a first reference organism within a reference sequence fragment from a second reference organism, optionally concatenated to a reference sequence fragment from yet another different organism. An isolated sequence can comprise at least 1, 2, 3, 4, 5, 6, 7, 8, 9, 10, 11, 12, 13, 14, 15, 16, 17, 18, 19 or 20 reference sequence fragments that have been concatenated, any of which may be non-adjacent fragments from the same reference sequence or reference organism, or from different reference sequences or reference organisms.

In some embodiments, the isolated sequence (for example, the shuffled or concatenated isolate sequence) is not cut by any CRISPR-associated (Cas) guide RNAs (gRNAs) in a 90k gRNA array designed to cut human DNA. In some embodiments, isolated sequences can be designed to exclude specific sequence features. For example, a shuffled or concatenated isolated sequence can be selected that excludes features such as CRISPR/Cas gRNA recognition sites, restriction sites, transcription factor binding sites or repetitive sequences.

Sequence Properties

In some embodiments, the sequences of each of the at least three groups of polynucleotides have at least one property in common with a target sequence in a sequencing sample. In some embodiments, a component sequence is designed to have at least one property in common with a target sequence in a sequencing sample. In some embodiments, the isolated sequence is isolated from an organism or sequence in a cloning vector that has been chosen because it is known to have at least one property in common with a target sequence in a sequencing sample.

In some embodiments, the at least one property comprises a sequence property of a transposable element sequence, viral sequence, bacterial sequence, a fungal sequence, a eukaryotic parasite sequence or one or more human genes sequence(s).

In some embodiments, the property of the transposable element comprises a transposable element sequence. Transposable elements are DNA sequences that can change positions in the genome. A transposable element (transposon) generally consists of a pair of inverted terminal repeats (ITRs) flanking an open reading frame encoding a transposase enzyme. Accordingly, exemplary properties of a transposable element comprise ITR sequences, transposase sequences, or any sequence of the transposon. Any sequence of a transposon or sequence property of a transposon can be incorporated into a component sequence of the disclosure. An isolated sequence of the disclosure can comprise any sequence of a transposon or sequence property of a transposon.

In some embodiments, the property of the viral sequence comprises a percent GC content, a repetitive sequence element, an inverted terminal repeat (ITR), an internal ribosome entry site (IRES), a protein coding sequence, a post-transcriptional regulatory element, a transcriptional regulatory element, a promoter sequence, a cis-acting RNA element, an RNA structural element, a genome packaging signal, a 5' untranslated region (5'UTR) sequence, a 3' untranslated region (3' UTR) sequence or a combination thereof. Any sequence of a virus or sequence property of a virus can be incorporated into a component sequence of the disclosure.

In some embodiments, the property of the bacterial sequence comprises percent GC content, a repetitive sequence, a microRNA binding site, an internal ribosome entry site (IRES), a protein coding sequence, a transcriptional regulatory element, a promoter sequence, a 5' UTR sequence, a 3' UTR sequence or a combination thereof. In some embodiments, the repetitive sequence element comprises a polyA motif, a polyT motif, a polyG motif, a polyC motif, dinucleotide motif, a trinucleotide motif, a tetranucleotide motif, a pentanucleotide motif, a hexanucleotide motif, a heptanucleotide motif, an octanucleotide motif, a nonanucleotide motif, an interspersed repetitive sequence element, a ribosomal RNA sequence, a transfer RNA (tRNA) sequence, a terminal inverted repeat (TIR), a non-autonomous miniature inverted repeat transposable element (MITE), a Clustered Regularly Interspaced Short Palindromic Repeat (CRISPR) or a combination thereof. PolyA motifs, polyT motifs, polyG motifs and polyC motifs are runs of As, Ts, Gs and Cs (for example, 4-50 As, Ts, Gs, or Cs). Any sequence of a bacterium or sequence property of a bacterium can be incorporated into a component sequence of the disclosure. An isolated sequence of the disclosure can comprise sequence of a bacterium or sequence property of a bacterium.

In some embodiments, the property of the fungal sequence comprises percent GC content, a repetitive sequence element, a microRNA binding site, an internal ribosome entry site (IRES), a protein coding sequence, a transcriptional regulatory element, a promoter sequence, a 5' UTR sequence, a 3' UTR sequence, a centromeric sequence, a telomeric sequence, a subtelomeric sequence, a mitochondrial sequence or a combination thereof. Any sequence of a fungus or sequence property of a fungus can be incorporated into a component sequence of the disclosure. An isolated sequence of the disclosure can comprise sequence of a fungus or sequence property of a fungus.

In some embodiments, the property of the one or more human genes comprises percent GC content, a repetitive sequence, a protein coding sequence, an intronic sequence, a 5' UTR sequence, a 3' UTR sequence, a transcriptional regulatory element sequence, a promoter sequence, a microRNA binding sequence or a combination thereof. In exemplary embodiments, the property of the one or more human genes is a sequence encoding a conserved protein domain shared by multiple proteins in a family. A non-limiting example of a protein domain is a DNA binding domain such as a zinc finger DNA binding domain or a homeodomain. In exemplary embodiments, the property of the one or more human genes is a microRNA binding site shared by all genes regulated by the cognate microRNA. Any sequence of human gene or genes or sequence property of human gene or genes can be incorporated into a component sequence of the disclosure. An isolated sequence of the disclosure can comprise sequence of a human gene or genes or sequence property of a human gene or genes.

In some embodiments, the property of the eukaryotic parasite sequence comprises percent GC content, a repetitive sequence, a microRNA binding site, a protein coding sequence, a transcriptional regulatory element, a promoter sequence, a 5' UTR sequence, a 3' UTR sequence, a centromeric sequence, a telomeric sequence, a subtelomeric sequence, a mitochondrial sequence or a combination thereof. Any sequence or sequence property of a eukaryotic parasite can be incorporated into a component sequence of the disclosure. An isolated sequence of the disclosure can comprise sequence of a eukaryotic parasite or sequence property of a eukaryotic parasite.

In some embodiments, the at least one property shared by the normalization control sequences and the target sequence comprises entropy. Entropy is a measure of the information content and complexity of a nucleotide sequence. Methods of calculating entropy will be known to persons of ordinary skill in the art (see, for example Bioinformatics, Vol. 27 2011, Pages 1061-1067).

In some embodiments, the at least one property shared by the normalization control sequences and the target sequence comprises complexity. Complexity (denoted by N) is the number of base pairs of unique or nonrepeating nucleotides in a given segment of polynucleotides, or component of the genome. This is different from the length (L) of the sequence if some of the polynucleotides are repeated.

In some embodiments, the at least one property shared by the normalization control sequences and the target sequence comprises percent GC content.

In some embodiments of the normalization controls of the disclosure, the GC content of one or more groups or subgroups of polynucleotides is the same as the target sequence in the sequencing sample. In some embodiments, the GC content of the entire polynucleotide in the group or subgroup is the same as the target sequence. In some embodiments, the GC content of the component sequence and/or the isolated sequence is the same as the target sequence. GC content is known to influence read count during NGS (GC bias). Sequences with low or high GC content tend to be underrepresented in the number of reads they produce during NGS. Thus, mimicking the GC content of a target sequence in an NC composition increases the ability of the NC to model the behavior of the target sequence during NGS.

In some embodiments, the polynucleotides within a group have the same percent GC content. In some embodiments, the polynucleotides within a group have the same percent GC content as the polynucleotides within every other group. In some embodiments, the polynucleotides within a group do not have the same percent GC content as the polynucleotides within any other group.

In some embodiments, the percent GC content of the polynucleotides within each group is between 40% and 60%, inclusive of the endpoints. In some embodiments, the percent GC content of the polynucleotides within each group is between 43% and 56%, inclusive of the endpoints.

In some embodiments, the at least one property shared by the normalization control sequences and the target sequence comprises electron-ion interference potential (EIIP). EIIP can be defined as the average energy of delocalized electrons of the nucleotides in a nucleotide sequence. The nucleotide sequence can be converted to the numerical EIIP sequence by replacing each nucleotide with the corresponding EIIP value. For example, A=0.1260, C=0.1340, T=0.1335, G=0.0806. In some embodiments, the EIIP values can be summed across all or part of the sequence, for example across an isolated sequence or a reference sequence fragment.

In some embodiments, the at least one property shared by the normalization control sequences and the target sequence comprises GC content, EIIP and entropy. GC content, EIIP and entropy all depend on sequence, and therefore covary. In some embodiments, normalization controls share all three of these parameters with the target sequences.

The degree to which a parameter such as percent GC content, EIIP, entropy or complexity is similar between normalization control sequences and target or reference sequences can be determined by a variety of statistical methods known in the art. These include, but are not limited to Analysis of Variance (ANOVA), t-tests such as Student's t-test, Kolmogorov Smirnov (KS) tests and Q-Q plots. In some embodiments, normalization controls comprise modified nucleotides. Any type of nucleotide modification is envisaged as within the scope of the disclosure. Exemplary but non-limiting examples of nucleotide modifications of the disclosure are described below.

Nucleotide modifications used by the methods of the disclosure can occur on any nucleotide (adenine, cytosine, guanine, thymine or uracil, *e.g.*). These nucleotide modifications can occur on deoxyribonucleic acids (DNA) or ribonucleic acids (RNA). These nucleotide modifications can occur on double or single stranded DNA molecules, or on double or single stranded RNA molecules.

In some embodiments, the nucleotide modification comprises adenine modification or cytosine modification.

In some embodiments, the adenine modification comprises adenine methylation. In some embodiments, the adenine methylation comprises $N^6$-methyladenine (6mA). $N^6$-methyladenine (6mA) is present in both prokaryotic and eukaryotic genomes.

In some embodiments, the adenine methylation comprises EcoKI methylation. EcoKI methylation is a type of DNA nucleotide modification that is carried out by the EcoKI methylase. The EcoKI methylase modifies adenine residues in the sequences AAC($N_6$)GTGC and GCAC($N_6$)GTT. EcoKI methylase, and EcoKI methylation, are found in prokaryotes.

In some embodiments, the adenine modification comprises adenine modified at $N^6$ by glycine (momylation). Momylation changes adenine for N6-(1-acetamido)-adenine. Momylation occurs in viruses, for example bacteriophages.

In some embodiments, the modification comprises cytosine modification. In some embodiments, the abundance and type of cytosine modification in a genome varies based on species. In some embodiments, the location of cytosine modifications (within a particular restriction enzyme recognition site, *e.g.*) in a genome varies based on species.

In some embodiments, the cytosine modification comprises 5-methylcytosine (5mC), 5-hydroxymethlcytosine (5hmC), 5-formylcytosine (5fC), 5-carboxylcytosine (5caC), 5-glucosylhydroxymethylcytosine (5ghmC) or 3-methylcytosine (3mC).

In some embodiments, the cytosine modification comprises cytosine methylation. In some embodiments, the cytosine methylation comprises 5-methylcytosine (5mC) or N4-methylcytosine (4mC). In some embodiments, 4mC cytosine methylation is found in bacteria. In some embodiments, the cytosine methylation comprises Dcm methylation. In some embodiments, the cytosine methylation comprises DNMT1 methylation, DNMT3A methylation or DNMT3B methylation.

In some embodiments, the cytosine methylation comprises CpG methylation, CpA methylation, CpT methylation, CpC methylation or a combination thereof. In some embodiments, the cytosine methylation comprises CpG methylation.

In some embodiments, the cytosine modification comprises 5-hydroxymethylcytosine (5hmC), 5-formylcytosine (5fC), 5-carboxylcytosine (5caC), 5-glucosylhydroxymethylcytosine, or 3-methylcytosine.

Target Organisms

The disclosure provides compositions and methods for determining the titer of one or more target organisms in sample. Target organisms are organisms that comprise one or more the target sequences described herein.

In some embodiments, the sample comprises a mixture of host organism and one or more non-host organisms, and the target organism is one or more of the non-host organisms. In some embodiments, the host organism is the target organism.

In some embodiments, the non-host target organism is a pathogen. In some embodiments, the target organism is a viral, bacterial, fungal or eukaryotic pathogen. For example, in some embodiments, the target organism is a viral pathogen in a human clinical sample, and the target sequence is a sequence of that viral pathogen.

In some embodiments, the target organism is a symbiote or a commensal organism.

In some embodiments, the target organism is a species in a mixture of species in a metagenomic sample.

Target Sequences

In some embodiments, the NCs are designed to account for possible variables that affect the conversion of the target sequence in a sequencing sample to sequencing reads after NGS. For example, NC sequences can be designed to match the GC content of the nucleic acids of the target sequence whose abundance in the starting sample is determined using the normalization controls and methods of the disclosure.

In some embodiments of the normalization controls of the disclosure, the component and/or the isolated sequence has at least one property in common with a target sequence in a sequencing sample. Common properties shared between the target sequence and the component sequence(s) enable the component sequence(s) of the NC to mimic the target sequence during sample and/or library preparation and sequencing. This allows NCs to model the transformation from an initial concentration of a target sequence in a sample to a number reads of the target sequence after NGS.

In some embodiments, the sequence of at least one subgroup of polynucleotides in the normalization control shares at least one sequence property with the target sequence in the sequencing sample. In some embodiments, the at least one sequence property comprises a sequence property of a transposable element sequence, viral sequence, bacterial sequence, a fungal sequence, a eukaryotic parasite sequence or one or more human genes sequence(s).

All sequence properties of the target sequence are envisaged as being within the scope of the normalization control compositions of the disclosure. In some embodiments, the at least one property comprises a sequence property of a transposable element sequence, viral sequence, a bacterial sequence, a fungal sequence, a eukaryotic parasite sequence or a sequence of one or more human genes. In some embodiments, the at least one property comprises GC content, entropy, complexity, length, EIIP or a combination thereof.

In some embodiments, the sequencing sample comprises a mixture of host and non-host nucleic acids. In some embodiments, the non-host comprises the target sequence or sequences.

In some embodiments, the host is a eukaryote. In some embodiments, the host is an insect, an animal or a plant. In some embodiments, the animal is a human.

In some embodiments, the host is any mammalian organism. In particular embodiments, the mammal is a human. In other embodiments, the mammal is a livestock animal, for example a horse, a sheep, a cow, a pig, or a donkey. In other embodiments, a mammalian organism is a domestic pet, for example a cat, a dog, a gerbil, a mouse or a rat. In other embodiments, the mammal is a type of a monkey.

In some embodiments, the host is any bird or avian organism. An avian organism includes but is not limited to a chicken, turkey, duck and goose.

In some embodiments, the host is an insect. Insects include, but are not limited to honeybees, solitary bees, ants, flies, wasps and mosquitoes.

In some embodiments, the host is a plant. In some particular embodiments, the plant is rice, maize, wheat, rose, grape, coffee, fruit, tomato, potato or cotton.

In some embodiments, the non-host comprises a symbiote, a commensal organism, a parasite or a pathogen. In some embodiments, the non-host comprises one or more species of symbiotes, commensal organisms, parasites or pathogens.

In some embodiments, the non-host is a pathogen. In some embodiments, the non-host is a species of virus, a species of bacteria, a species of fungus or a species eukaryotic parasite. In some embodiments, the non-host is a species of algae.

In some embodiments, the eukaryotic parasite is a mammalian parasite. In some embodiments, the parasite is a worm. In other embodiments, the parasite is a malaria-causing parasite. In other embodiments, the parasite is a Leishmaniasis-causing parasite. In other embodiments, the parasite is an amoeba.

In some embodiments, the non-host is a species of bacteria. In particular embodiments, the bacteria are tuberculosis-causing bacteria.

In some embodiments, the target sequence is a non-host sequence in the sequencing sample.

In some embodiments, the target sequence comprises a reference sequence. In some embodiments, the target sequence comprises a plurality of reference sequences. In some embodiments, the target sequence comprises at least 2, at least 10, at least 20, at least 50, least 100, at least 200, at least 300, at least 400, at least 500, at least 600, at least 700, at least 800, at least 900, at least 1,000, at least 1,200, at least 1,300, at least 1,400, at least 1,500, at least 1,600, at least 1,700, at least 1,800, at least 1,900, at least 2,000, at least 2,200, at least 2,400, at least 2,600, at least 2,800, at least 3,000, at least 4,000, at least 5,000 , at least 6,000, at least 7,000, at least 8,000, at least 9,000 or at least 10,000 reference sequences. In some embodiments, each reference sequence is from a different organism. In some embodiments, the plurality of reference sequences are genomic DNA or cDNA sequences from different organisms. In some embodiments, the target sequence comprises a plurality of reference sequences from at least 2, at least 10, at least 20, at least 50, least 100, at least 200, at least 300, at least 400, at least 500, at least 600, at least 700, at least 800, at least 900, at least 1,000, at least 1,200, at least 1,300, at least 1,400, at least 1,500, at least 1,600, at least 1,700, at least 1,800, at least 1,900, at least 2,000, at least 2,200, at least 2,400, at least 2,600, at least 2,800, at least 3,000, at least 4,000, at least 5,000 , at least 6,000, at least 7,000, at least 8,000, at least 9,000 or at least 10,000 organisms.

In some embodiments, the target sequence comprises a non-host sequence, and the non-host is a species of virus, a species of bacteria, a species of fungus or a species eukaryotic parasite.

In some embodiments, the non-host comprises a symbiote. A symbiote, sometimes called a mutual or mutualistic organism, is an organism in a relationship with another organism that provides mutual benefit to both parties.

In some embodiments, the non-host comprises a commensal organism. In commensal organismal interactions, one organism benefits (*e.g.,* the non-host) while the other *(e.g.* the host) is unaffected. A commensal or mutual organism may depend on its host for food shelter, support, transportation of a combination thereof. The host can receive a variety of benefits from the interaction, including but not limited to protection from infection, improved digestion and immune modulation. A non-limiting example of a mutual and/or commensal relationship is that of a human host with the population of microorganisms populating the gut (the gut microbiota, or microbiome). Gut microbiota help maintain the mucosal barrier in the gut, provide nutrients such as vitamins, protect against pathogens, and help maintain proper immune function.

In some embodiments, the non-host comprises multiple species, or different organisms from the same species. In some embodiments, the non-host comprises, at least 5, at least 10, at least 50, at least 100, at least 200, at least 500, at least 1000, at least 1,500 or at least 2,000 species or different organism from the same species, or combinations thereof. In some embodiments, the non-host comprises at least 2,000 species, for example the at least 2,000 gut microbiome species or microbiome organisms.

In some embodiments, the target sequence is a non-host sequence associated with pathogenicity. Exemplary non-host sequences associated with pathogenicity include, but are not limited to antibiotic resistance genes, virulence factors, or markers pathogenicity associated with eukaryotic parasites.

A virulence factor is a gene or a group of genes which contribute to pathogenicity of a microorganism such as a bacterium, a virus, or a fungus in infecting a host. Virulence refers to the degree of damage caused to the host by the microorganism, with greater virulence causing greater damage.

Exemplary, but non-limiting virulence factors include genes that contribute to colonization of a niche in the host (for example, attachment of the non-host to host cells), immunoevasion (evasion of the host immune response), immunosuppression (inhibition of the host immune response by the non-host), cellular entry and exit (of intracellular non-hosts), and obtaining nutrition from the host. Exemplary virulence factors that are used to assist and promote colonization of the host, include, but are not limited to, adhesins, invasins and antiphagocytic factors.

Exemplary virulence factors include factors that increase damage to the host include, but are not limited to toxins, destructive enzymes and proteins that regulate host GTPases. Exemplary toxins include endotoxin, and exotoxins. Endotoxin (lipopolysaccharide, or LPS) is a bacterial cell wall component capable of triggering inflammation in the host. Exotoxins are toxins that are actively secreted by some bacteria and have a wide range of effects on the host, including, but not limited to, inhibition of specific biochemical pathways. Exemplary exotoxins include, but are not limited to tetanus toxin secreted by *Clostridium tetani,* botulin toxin secreted by *Clostridium botulinum,* as well as toxins secreted *Escherichia coli, Clostridium perfringens, Bacillus anthracis* and *Clostridium difficile.* In some embodiments, endotoxins are secreted by fungi (called mycotoxins). An exemplary mycotoxin comprises aflatoxin produced by *Aspergillus.*

In some embodiments, the virulence factor is a destructive enzyme. Destructive enzymes are enzymes which cause virulence by damaging host cells and tissues. Exemplary destructive enzymes include, but are not limited to proteases, lipases, and DNases. For example, hemolysins produced by bacteria such as E. coli and *Staphylococcus aureus* break down host cells, including red blood cells.

In some embodiments, the virulence factor is a protein which regulates host GTPase activity. Virulence factors can act as a guanine nucleotide exchange factor (GEF) or a GTPase activating protein (GAP) to modify host GTPase activity, or, alternatively, covalently modify the host GTPase itself.

Exemplary viral virulence genes include viral genes that control viral latency. Additional exemplary viral virulence genes include viral genes that contribute to viral evasion of the host immune response and aspects of the viral life cycle such as binding and entry to host cells or virion production and release.

In some embodiments, virulence factors are chromosomal and intrinsic. For example, bacterial virulence factors such as lipopolysaccharides (endotoxin) are chromosomal.

In some embodiments, virulence factors are encoded by mobile genetic elements that are spread through horizontal gene transfer. In some embodiments, the mobile genetic element is a plasmid, a transposable element, a bacteriophage element or a ribozyme. In some embodiments, the horizontal gene transfer of a virulence factor converts a microorganism that was a commensal or mutualistic (symbiotic) microorganism prior to the acquisition of the virulence factor into a pathogenic microorganism.

In some embodiments, the non-host is a virus, a bacterium or a fungus and the target sequence comprises a sequence of a virulence factor.

In some embodiments, the non-host is a bacterium and the target sequence comprises a sequence of an antibiotic resistance gene. In some embodiments, the antibiotic resistance gene is encoded by a mobile genetic element that is spread through horizontal gene transfer. In some embodiments, the mobile genetic element is a plasmid, a transposable element, a bacteriophage element or a ribozyme.

In some embodiments, the antibiotic resistance gene conveys resistance to quinolones, β-lactams, macrolides, tetracyclines, sulfonamides, aminoglycosides or vancomycins.

In some embodiments, the sequencing sample comprises a metagenomics sample. As used herein, the term "metagenomics" indicates the direct analysis, for example using the compositions and methods of the disclosure, of a sample of mixed organisms.

In some embodiments, the metagenomics sample is an environmental sample. Exemplary environmental samples include, but are not limited to, soil, water and air samples. In some embodiments, the environmental sample comprises a sample taken from a man-made surface, for example from a surface in a hospital.

In some embodiments, the sample comprises an environmental sample and the target sequence comprises a sequence of a virulence factor, an antibiotic resistance gene or a sequence associated with pathogenicity. For example, the development of antibiotic resistance through lateral gene transfer is a major public health problem. It is therefore beneficial to quantify the frequency of antibiotic resistance genes in a metagenomic sample taken from an environment in which antibiotics are used, for example a hospital or farm, using the compositions and methods of the disclosure.

In some embodiments, normalization controls of the disclosure are designed to mimic the properties of a target sequence, *i.e.* the non-host sequence, in a sequencing sample during sample extraction and/or library preparation. In some embodiments, the population of polynucleotides of the normalization controls are purified from a nucleic acid sample, and the nucleic acid sample is isolated or derived from a virus, a bacterium, a fungus or a eukaryotic parasite. In some embodiments, the virus, bacterium, fungus or eukaryote from which the NCs are purified is similar but not identical to the virus, a bacterium, a fungus or a eukaryotic parasite of the non-host.

In some embodiments, the non-host is a virus, and polynucleotides of the normalization controls are purified from a related virus or a virus with properties similar to the non-host. For example, if the non-host is a lentivirus, the polynucleotides of the normalization control are purified from a related species lentivirus. In some embodiments, the virus is a T4 bacteriophage or cytomegalovirus (CMV). Alternatively, if the non-host is a bacterium, the normalization control polynucleotides are purified from a related species of bacteria or a species of bacteria with properties similar to the non-host.

The NC can be from one or more organisms. For example, the NC can comprise polynucleotides extracted from one or more organisms or species of organisms, such as viruses, bacteria, fungi or eukaryotes, that simulate one or more properties of the non-host sequences in a mixed host/non-host sample.

In certain embodiments, the NC is used to determine the relative amounts of different nucleic acids in a sample. For example, normalization controls can be used to determine the abundance value of a pathogen (a non-host) in a sample from an infected host *(e.g.* a human host infected with a virus or bacteria). The abundance value for the pathogen can be determined by dividing NGS reads that map to the pathogen by the number of NGS reads that map to the NCs to compensate for differing background host content. This value can then be mapped to a standard curve, which was generated and normalized the same way, and the pathogen titer can be inferred from the standard curve. Using such methods, there would then not necessarily be a need to determine an absolute pathogen load.

In certain embodiments, the NC is used to determine the absolute amounts of different nucleic acids in a sample. For example, the NC can be used to determine the absolute amount of a pathogen (a non-host) in a sample from an infected host *(e.g.,* a sample from a human host infected with a virus or bacteria). An abundance value for the pathogen can be determined by dividing the number of NGS reads that map to the pathogen by the number of NGS reads that map to the NCs to compensate for differing background host contents. Based on this ratio and the input quantity of NC *(e.g.,* copies of bacteriophage or polynucleotides), the relative pathogen genome copy can be determined. This value can then be corrected for pathogen genome size to derive an "absolute" pathogen abundance in the starting material.

In some embodiments, the species of interest from which the normalization control polynucleotides are purified is similar to, but not identical to the species of the non-host.

In some embodiments, the sequencing sample comprises a cancer-related sample from a subject who has been diagnosed to have a type of cancer. The progression of cancer is a multiple step process that requires the accumulation of genetic changes over time. In some embodiments, the genetic changes leading to cancer comprise genetic changes that activate proto-oncogenes and deregulate tumor suppressor genes and DNA repair genes. Genetic changes leading to cancer allow previously normal cells to escape regulation of cell division and divide unchecked. Cancers thus comprise heterogeneous populations of cells with different mutations. Accordingly, in some embodiments, the cancer-related sample comprises a population of cells comprising nucleic acids *(e.g.* genes, DNA or RNA) with at least one genetic change relative to nucleic acids in a non-cancerous cell of the subject, or a cell from a subject that does not have cancer, or from a subject that has a different type of cancer.

In some embodiments, a mutation of interest occurs only rarely in the heterogeneous population of cells in the cancer sample. However, specific mutations in a cancer sample are predictive of clinical responses to treatment. It is therefore beneficial to identify and quantify the occurrence of mutations in heterogeneous cancer samples using the compositions and methods of the disclosure. The compositions and methods of the disclosure are particularly informative and useful when either the cell type and/or the mutation is rare.

In some embodiments, the at least one genetic change in the cancer cells comprises a single nucleotide polymorphism (SNP), an insertion, a deletion, an inversion or a chromosomal rearrangement.

In some embodiments, the at least one genetic chance is associated with the activation of a proto-oncogene or the inactivation of a tumor suppressor gene. Exemplary proto-oncogenes include, but are not limited to human epidermal growth factor receptor 2 (HER2), RAS, MYC proto-oncogene (MYC), SRC proto-oncogene (SRC), telomerase, BCL-2, apoptosis regulator (BCL-2) and Epidermal Growth Factor Receptor (EGFR). Exemplary tumor suppressor genes include, but are not limited to, tumor protein p53 (p53), retinoblastoma (Rb), adenomatous polyposis coli (APC), breast cancer susceptibility gene 1 (BRCA1) and breast cancer susceptibility gene 2 (BRCA2).

In some embodiments, the target sequence comprises the sequence with at least one genetic change in the cancer.

In some embodiments, the sequences of the nucleic acids in the nucleic acid sample from which the normalization control polynucleotides are purified are not the same as the target sequence in the sequencing sample. In some embodiments, the sequences of the nucleic acids in the nucleic acid sample have less than or equal to 1%, 2, %, 3%, 4%, 5%, 6%, 7%, 8%, 9%, 10%, 11%, 12%, 13%, 14%, 15%, 16%, 17%, 18%, 19%, 20%, 30%, 40%, 50%, 60%, 70%, 80%, 85%, 90%, 95%, 96%, 97%, 98% or 99% identity to a target sequence in a sequencing sample.

Concentrations

In some embodiments, NCs are designed to be added to a host *(e.g.* a patient) sample during DNA extraction for normalization of sequence reads as part of a non-host *(e.g.,* a pathogen) identification pipeline. The normalization control fragments, collectively, are designed such that they represent a small fraction of the total sequence reads obtained from a sample. The normalization control fragments, collectively, should represent a small fraction (<5%) of the sequence reads obtained from a sample. In some embodiments, the frequency of the normalization control fragments matches the frequency of the target sequence in the sample.

Every possible arrangement of concentrations between groups of polynucleotides is envisaged as within the scope of the disclosure.

In some embodiments, the polynucleotides of every group are at the same concentration. For example, in some embodiments, each of three groups is at the same concentration of 1 picogram (pg) per microliter ($\mu$L), for a total concentration of 3 pg/$\mu$L in the normalization control composition.

In some embodiments, the polynucleotides of every group are not at the same concentration. In some embodiments, each of the groups of polynucleotides can be present at a different concentration. For example, in some embodiments, if three groups are present at a concentration ratio of 1:2:4, and the first group is at concentration of 1 pg/$\mu$L, then the second group is at a concentration of 2 pg/$\mu$L, and the third group is at a concentration of 4 pg/$\mu$L producing a total concentration of 7 pg/$\mu$L in the normalization control composition.

Every possible arrangement of concentrations between subgroups within a group, and between subgroups of different groups, is envisaged as within the scope of the disclosure.

In some embodiments, the at least three groups comprise at least one group wherein the concentrations of polynucleotides in the at least three subgroups in the group are equal and at least one group wherein the concentrations subgroups in the group are not equal.

In some embodiments, the concentrations of the polynucleotides in each of the least three subgroups in each of the at least three groups are not equal to the concentrations of the other subgroups in the group. In some embodiments, the concentrations of each of the at least three subgroups in a group are distributed in a linear sequence or in a geometric sequence. A linear sequence is a sequence of numbers in which the next number in the sequence increases or decreases by the same amount each time relative to the previous number. In general, linear sequences are represented by the formula:

$$u_n = d \; x \; n + c$$

where d is the first difference between successive terms in the sequence, n is the term in the sequence, and c is a constant. In an exemplary, non-limiting embodiment where there are three subgroups within a group whose concentrations are a linear sequence, the concentrations of the three subgroups are, for example, 1 pg/$\mu$L, 2 pg/$\mu$L, and 3 pg/$\mu$L.

A geometric sequence is a sequence of numbers where each term after the first is found by multiply the previous term by a fixed, non-zero number called the common ratio. In general, geometric sequences are represented by the formula:

$$u_n = u_1^{r-1}$$

where r is the common ratio. In an exemplary, non-limiting set of embodiments, where there are three subgroups within a group whose concentrations are a geometric sequence, the concentrations of the three subgroups are, for example, 2 pg/$\mu$L, 4 pg/$\mu$L, and 8 pg/$\mu$L. The relative concentrations of the subgroups within each group of polynucleotides is an advantageous feature of the normalization controls of the disclosure. Following NGS, the relative abundances of sequencing reads that map back to each of the subgroups and/or groups of polynucleotides will reflect the relative concentrations of the subgroups and/or groups in the normalization control composition, and therefore the sample, prior to NGS. Calculating the relative abundances of reads within groups or subgroups thus provides an internal quality control measure and allows for a more accurate model of an initial concentration of a target sequence in a sequencing sample.

In some embodiments, one subgroup in a group has a baseline concentration, and the concentrations of the non-baseline subgroups in the group are integer multiples of the baseline. In some embodiments, one subgroup in a group has a baseline concentration, and the concentration of each subgroup in the group is twice the concentration of another subgroup in the group, excluding the baseline. In some embodiments, the at least three subgroups in the group are present at a concentration ratio of 1:2:4.

In some embodiments, the pluralities of polynucleotides are mixed in a way that allows for a wide dynamic range in the concentrations of individual NC sequences. The concentrations can span from 101 to 107 in concentration range with some fragments having concentrations at intermediate levels. The normalization controls are validated using a variety of methods, including QPCR.

In some embodiments, the population of polynucleotides are at a concentration of between about 0.005 and about 5000 picograms (pg) per microliter ($\mu$L), between about 0.005 and 1000 pg/$\mu$L about, between about 0.005 and about 700 pg/$\mu$L, between about 0.005 and about 500 pg/$\mu$L, between about 0.005 and about 100 pg/$\mu$L, between about 0.005 and about 50 pg/ $\mu$L, between about 0.05 and about 5000 pg/$\mu$L, between about 0.05 and about 1000 pg/$\mu$L, between about 0.05 and about 700 pg/$\mu$L, between about 0.05 and about 500 pg/$\mu$L, between about 0.05 and about 100 pg/$\mu$L, between about 0.05 and about 50 pg/$\mu$L, between about 0.5 and about 1000 pg/$\mu$L, between about 0.5 and about 700 pg/$\mu$L, between about 0.5 and about 500 pg/$\mu$L, between about 0.5 and about 100 pg/$\mu$L, between about 0.05 and

about 50 pg/$\mu$L, between about 5 and about 1000 pg/$\mu$L, between about 5 and about 700 pg/$\mu$L, between about 5 and about 500 pg/$\mu$L, between about 5 and about 100 pg/$\mu$L or between about 5 and about 50 pg/$\mu$L in the normalization control composition. In some embodiments, the population of polynucleotides are at a concentration of between about 0.005 and 500 picograms (pg) per microliter ($\mu$L) in the normalization control composition. In some embodiments, the population of polynucleotides are at a concentration of between about 0.5 and about 50 pg/$\mu$L in the normalization control composition. In some embodiments, the population of polynucleotides are at a concentration of between about 10 and about 20 pg/$\mu$L in the normalization control composition. In some embodiments, the population of polynucleotides are at a concentration of between about 1 and about 2 pg/$\mu$L in the normalization control composition. In some embodiments, the population of polynucleotides are at a concentration of between about 0.1 and about 0.2 pg/$\mu$L in the normalization control composition. In some embodiments, the population of polynucleotides are at a concentration of 1.4 pg/$\mu$L in the normalization control composition, inclusive of the endpoints.

Indexing

In some embodiments, normalization control polynucleotides comprise a sequence encoding a unique molecular identifier. In some embodiments, the sequence encoding the unique molecular identifier comprises a first indexing sequence or a second indexing sequence.

An exemplary, non-limiting indexing strategy of the disclosure uses two indices, a first index and second index. In some embodiments, the sequence encoding the unique molecular identifier comprises a common primer sequence, an index sequence and an adaptor sequence. In some embodiments, the common primer sequence comprises a flow cell attachment site for NGS.

In some embodiments, the index sequence is a unique 4-20 bp sequence. In some embodiments, the index sequence is a unique 6 bp, 8 bp or 10 bp sequence. In some embodiments, the index sequence is a unique 6 bp sequence. The length of the index will depend on the complexity of the library. An appropriate index sequence length will be readily apparent to one of skill in the art. In some embodiments, a sequence comprising a first primer sequence, a first index sequence and a first adaptor sequence is attached to the 5' end of a polynucleotide, and a sequence comprising a second primer sequence, a second index sequence, and a second adaptor sequence is attached to the 3' end of the same polynucleotide. The polynucleotide is, for example a polynucleotide of a normalization control composition of the disclosure. In some embodiments, every group of polynucleotides comprises a unique dual index on the 5' and 3' ends of the plurality of polynucleotides in the group. In this embodiment, the dual index is the same for all of the plurality of polynucleotides within the group, and different for polynucleotides between groups. In some embodiments, every subgroup of polynucleotides comprises a unique dual index on the 5' and 3' ends of the plurality of polynucleotides in the subgroup. In this embodiment, the dual index is the same for all of the plurality of polynucleotides within the subgroup but different for polynucleotides in different subgroups. In some embodiments, the plurality of polynucleotides within each group and within each subgroup have a unique dual index specific to the group and/or the subgroup.

A non-limiting exemplary dual indexing system of the disclosure is the Illumina i5 and i7 dual indexing system.

In some embodiments, normalization controls are designed to measure the conversion of a DNA or RNA target sequence in a sequencing sample to a sequence-able library molecule. In some embodiments, conversion rates of the starting material to the ending material during library preparation are poor and difficult to measure reliably in all samples. By dual indexing the ends of the NC polynucleotide, this allows for tracking which molecules are converted and how they are converted per sample at any time during the library preparation. This allows for the calculation of a sample type agnostic library conversion factor. This also allows for quality control to be built into the sample preparation. Using this technique, it is possible to compare reagent lots, the efficiency of enzymatic reactions and many other metrics that affect NGS. These comparisons can be done in line with steps of the sequencing reaction that would normally have to wait until the end of the library preparation process to determine if problems had arisen.

Proteins

Normalization control polynucleotides described herein comprise deoxyribonucleic acid (DNA) molecules, ribonucleic acid (RNA) molecules, or DNA-RNA hybrid molecules.

Association with proteins is a significant source of variability and error in calculating target sequence concentration from NGS read counts. For example, in some embodiments, the target sequence is a viral sequence, and association of the polynucleotide comprising the viral sequence with a capsid prevents purification and/or fragmentation of the target sequence during sample and library preparation. Therefore, the addition of proteins to the normalization control compositions of the disclosure that mimic nucleic acid/protein interactions of the target sequence in the sequencing sample increases the ability of the NCs to model the behavior of the target sequence during NGS.

In some embodiments of the normalization controls of the disclosure, the normalization controls further comprise at least one protein. In some embodiments, the at least one protein is isolated or derived from a bacteria, a virus or a eukaryotic parasite. In some embodiments, the at least one protein is a viral capsid protein or a bacterial cell wall protein. Exemplary but non-limiting viral capsid proteins comprise viral capsid proteins isolated or derived from adenovirus, adeno-associated virus (AAV), lentiviruses or retrovirus.

In some embodiments, the population of polynucleotides and the protein are operably linked. In exemplary embodiments, the protein is a viral capsid protein and the polynucleotides are encapsulated within the viral capsid proteins. In other embodiments, the proteins and the polynucleotides are bound.

Additional Sequences

In some embodiments, normalization control polynucleotides comprise a promoter sequence. In some embodiments, the promoter sequence comprises a promoter sequence selected from the group consisting of a T7 promoter sequence, an SP6 promoter sequence or a T3 promoter sequence. In some embodiments, in particular those embodiments wherein the promoter is a T7 promoter, the T7 promoter comprises a sequence of 5'-TAATACGACTCACTATAGG-3' (SEQ ID NO: 14). In some embodiments, the T7 promoter comprises a sequence of 5'-TAATACGACTCACTATAGGG-3'(SEQ ID NO: 15). In some embodiments, the T7 promoter comprises a sequence of 5'-GCCTCGAGCTAATACGACTCACTATAGAG-3' (SEQ ID NO: 16). In some embodiments, the SP6 promoter comprises a sequence of 5' -ATTTAGGTGACACTATAG-3' (SEQ (ID NO: 17). In some embodiments, the SP6 promoter comprises a sequence of 5' -CATACGATTTAGGTGACACTATAG-3 (SEQ ID NO: 18). In some embodiments, the T3 promoter comprises a sequence of 5' AATTAACCCTCACTAAAG 3' (SEQ ID NO: 19).

In some embodiments, normalization control polynucleotides comprise additional primer sequences. Primer sequences can be located 5' and 3' of the component and/or isolated sequences. Primer sequences can be added as part of the *in silico* design of the component sequence or added secondarily to the component and/or isolated sequence. Primer sequences can be added to the component and/or isolated sequences by, for example, adaptor ligation or random priming and extension of the component and/or isolated sequences.

Mixed Compositions

In some embodiments, normalization controls of the disclosure comprise a mixture of synthetic polynucleotides and polynucleotides that have been isolated or derived from one or more organisms. For example, a set of target sequences in a sample may cover a range of lengths that extend beyond the lengths of current manufacturing methods of synthetic polynucleotides (e.g., a length of 10 kb). In this example, to fully model the range of lengths of the target sequences with the normalization controls, the normalization controls comprise a mixture of component and isolated polynucleotides.

In some embodiments, at least one group of polynucleotides in the normalization controls are purified from an organism using the methods of the disclosure. In some embodiments, the organism is a virus, a bacterium, a fungus or a eukaryotic parasite. In some embodiments, the organism shares one or more features with the non-host in a mixed host/non-host sequencing sample. In some embodiments, the virus is bacteriophage T4.

In some embodiments, bacteriophage T4 is also added at a known concentration as an addition, or alternative, to an NC based on synthesized polynucleotides (an oligo-based NC).

The addition of bacteriophage T4 to normalization controls allows the normalization controls to control for extraction variation. Bacteriophage T4 has the added benefit that it is cheaper and easier to manufacture than the oligo-based NC, especially for long sequences.

*Methods of Making Normalization Controls*

*de novo* Synthesis

The disclosure provides a method of making normalization controls comprising at least three groups of polynucleotides, wherein the polynucleotides within each group are of the same length. In some embodiments, each of the at least three groups of polynucleotides further comprise at least three subgroups of polynucleotides.

In some embodiments, the at least three groups comprise polynucleotides of the same sequence. In some embodiments, the at least three groups comprise polynucleotides that are not the same sequence

In some embodiments of the methods of the disclosure, the methods comprise synthesizing as oligonucleotides the polynucleotides of each unique group and/or subgroup, quantifying the concentration of each unique group and/or subgroup, and mixing pre-determined amounts of each unique group and/or subgroup to produce the NC composition.

In some embodiments, the polynucleotide is a DNA polynucleotide. In some embodiments, the polynucleotide is between about 10 bp and about 3000 bp. In some embodiments, the polynucleotide is between about 10 bp and about 1200 bp. In some embodiments, the polynucleotide is between 10 bp and 250 bp. In some embodiments, the polynucleotide is between about 150 bp and about 1500 bp. In some embodiments, the polynucleotide is between about 400 bp and about 1100 bp. In some embodiments, the polynucleotide is between about 500 bp and about 1500 bp.

In some embodiments, the polynucleotide has at least one property in common with a target sequence in a sequencing sample. All sequence properties of the target sequence are envisaged as being within the scope of the normalization controls of the disclosure. In some embodiments, the target sequence comprises a transposable element sequence, viral sequence, a bacterial sequence, a fungal sequence, a eukaryotic parasite sequence or a sequence of one or more human genes. In some embodiments, the at least one property comprises a sequence property of a transposable element sequence, viral sequence, a bacterial sequence, a fungal sequence, a eukaryotic parasite sequence or a sequence of one or more human genes.

In some embodiments, the polynucleotides of the normalization controls comprise RNAs. In some embodiments, the DNA polynucleotide further comprises a sequence encoding a promoter, and the method further comprises *in vitro* transcribing each DNA polynucleotide to produce pluralities of RNAs, quantifying concentration of the each of the plurality of RNAs, and mixing the pluralities of RNAs in predetermined amounts to produce the normalization controls. Exemplary promoters include, but are not limited T3, SP6 and T7.

Every possible arrangement of concentrations between groups and subgroups of polynucleotides is envisaged as within the scope of the disclosure.

Every possible arrangement of concentrations between the subgroups within a group, and between subgroups of different groups, is envisaged as within the scope of the disclosure.

Concentrations can be expressed as a weight per volume (e.g. pictograms (pg per microliter ($\mu$L), or nanograms (ng) per $\mu$L) or alternatively, as the number of molecules of the polynucleotide per volume (a molar concentration). It will be readily apparent to one of ordinary skill in the art how to convert the measures of concentration.

In some embodiments, each of the polynucleotides are synthesized as DNA oligonucleotides, and optionally, *in vitro* transcribed to produce a plurality of RNA polynucleotides. Each of the pluralities of polynucleotides of each group or subgroup is quantified, and the pluralities are mixed in a concentration ratio of the disclosure. The final normalization control composition is then diluted to a concentration of between about 0.001 and about 5000 picograms (pg) per microliter ($\mu$L), between about 0.005 and about 1000 pg/$\mu$L, between about 0.005 and about 700 pg/$\mu$L, between about 0.005 and about 500 pg/$\mu$L, between about 0.005 and about 100 pg/$\mu$L, between about 0.005 and about 50 pg/ $\mu$L, between about 0.05 and 5000 pg/$\mu$L, between about 0.05 and about 1000 pg/$\mu$L, between about 0.05 and about 700 pg/$\mu$L, between about 0.05 and about 500 pg/$\mu$L, between about 0.05 and about 00 pg/$\mu$L, between about 0.05 and about 50 pg/$\mu$L, between about 0.5 and about 1000 pg/$\mu$L, between about 0.5 and about 700 pg/$\mu$L, between about 0.5 and about 500 pg/$\mu$L, between about 0.5 and about 100 pg/$\mu$L, between about 0.05 and about 50 pg/$\mu$L, between about 5 and about 1000 pg/$\mu$L, between about 5 and about 700 pg/$\mu$L, between about 5 and about 500 pg/$\mu$L, between about 5 and about 100 pg/$\mu$L, or between about 5 and about 50 pg/$\mu$L. In some embodiments, the normalization controls are at a concentration of between about 0.005 and about 500 pg/$\mu$L. In some embodiments, the normalization controls are at a concentration of between about 0.5 and about 50 pg/$\mu$L. In some embodiments, the normalization controls are at a concentration of between about 10 and about 20 pg/$\mu$L. In some embodiments, the normalization controls are at a concentration of between about 1 and about 2 pg/$\mu$L. In some embodiments, the normalization controls are at a concentration of between about 0.1 and about 0.2 pg/ $\mu$L. In some embodiments, the normalization controls are at a concentration of 1.4 pg/$\mu$L.

Described herein are methods of making a plurality of normalization control oligos, comprising: (a) generating a plurality of reference sequence fragments from at least one reference sequence; (b) generating a distribution of at least one parameter as a function of number of reference sequence fragments; (c) dividing the distribution into bins; (d) selecting at least one reference sequence fragment from at least a subset of the bins; (e) shuffling the reference sequences to generate shuffled sequences; and (f) synthesizing oligos comprising the shuffled sequences; thereby generating a plurality of normalization control oligos.

Described herein are methods of making a plurality of normalization control oligos, comprising: (a) generating a plurality of reference sequence fragments from at least one reference sequence using a sliding window; (b) generating a distribution of at least one parameter as a function of number of reference sequence fragments; (c) dividing the distribution into a bins; (d) selecting at least two reference sequence fragments from at least a subset of the bins, wherein the at least two reference sequence fragments are either non-contiguous in the reference sequence, or from different reference sequences; (e) concatenating the at least two reference sequence fragments from each of the at least 3 bins; and (f) synthesizing oligos comprising the concatenated reference sequence fragments; thereby generating a plurality of normalization control oligos.In some embodiments, the sliding window comprises a 1 base pair (bp), 2 bp, 3 bp, 4 bp or 5 bp sliding window. In some embodiments, the reference sequence fragments are about 15-60, about 20-40, about 20-30, about 15-32, 20-32 or about 25-35 contiguous nucleotides of the corresponding reference sequence. In some embodiments, the reference sequence fragments are 29, 30, 31, 32, 33 or 34 base pairs of the corresponding reference sequence. In some embodiments, the reference sequence fragments are less than half the average length of a sequencing read.

In some embodiments, the parameter comprises least one of (1) percent GC content, (2) entropy, (3) complexity, (4) EIIP, or a combination thereof. In some embodiments, the parameter comprises percent GC content.

The distribution, for example distribution of GC content, can be divided into any number of suitable bins depending upon the desired resolution of the normalization controls. For example, each bin can represent 1 percent, 2 percent, 5 percent, 10 percent, 15 percent, or 20 percent of the distribution. The reference sequence fragments can be chosen from each bin, or from a subset of the bins. For example, in GC content distribution comprising 10 bins, the reference sequence fragments are chosen from the 10th, 30th, 50th, 70th, 80th and 90th percentile bins of a percent GC content distribution.

In some embodiments, step (d) comprises selecting at least 2 reference sequence fragments from each bin. For example, at least 2, 3, 4, 5, 6, 7, 8, 9, or 10, reference sequence fragments are selected from each bin. In some embodiments, step (e) further comprises concatenating the shuffled reference sequences from each bin. In some embodiments, only sequences from a given bin are concatenated, to mimic the properties of the reference sequence fragments in the corresponding bin and thus model multiple points along the distribution.

In some embodiments, the at least one reference sequence comprises at least 2, at least 10, at least 20, at least 50, at least 100, at least 200, at least 300, at least 400, at least 500, at least 600, at least 700, at least 800, at least 900, at least 1,000, at least 1,200, at least 1,300, at least 1,400, at least 1,500, at least 1,600, at least 1,700, at least 1,800, at least 1,900, at least 2,000, at least 2,200, at least 2,400, at least 2,600, at least 2,800, at least 3,000, at least 4,000, at least 5,000 , at least 6,000, at least 7,000, at least 8,000, at least 9,000 or at least 10,000 reference sequences. In some embodiments, the at least one reference sequence comprises about 2,000 reference sequences. In some embodiments, the at least one reference sequence comprises a genomic sequence or a cDNA sequence, or a combination thereof. For example, the reference sequence corresponding to a first organism may be a genomic sequence, and the reference sequence corresponding to as second organism may be cDNA sequences. Alternatively, the reference sequences for multiple organisms (all organisms) may be genomic sequences or cDNA sequences.

In some embodiments, the reference sequence fragments are between about 10-100, about 10-80, about 10-70, about 10-60, about 10-50, about 10-40, about 10-30, about 10-20, about 20-100, about 20-80, about 20-60, about 20-50, about 20-40, about 20-30, about 10-35, about 20-35, or about 20-25 contiguous nucleotides of the reference sequence. IN some embodiments, the reference sequence fragments are between about 15-60, about 20-40, about 20-30, about 15-32, 20-32 or about 25-35 contiguous nucleotides of the reference sequence. In some embodiments, the reference sequence fragments comprise 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 or 65 contiguous base pairs of the reference sequence.

In some embodiments, each normalization control oligo has at least three of: (1) similar percent GC content, (2) similar entropy, (3) similar EIIP, (4) similar complexity, or (5) similar length, as the reference sequence fragment(s) from a corresponding bin in the reference sequence distribution.

Multiplexed Synthesis

In some embodiments of the disclosure, the normalization controls comprise a mixture of synthetic DNA polynucleotides.

The disclosure describes a method of making a population of synthetic polynucleotides, comprising: (a) synthesizing at least three populations of DNA molecules; wherein each of the DNA molecules within each of the at least three populations has the same sequence, wherein the sequence of each of the DNA molecules comprises, from 5' to 3', a first component sequence, a first restriction enzyme site, a second component sequence, a second restriction site, and a third component sequence; (b) quantifying the amount of the each of the populations DNA molecules in a solution; (c) digesting each of the at least three populations of DNA molecules with a restriction enzyme that cuts the first and second restriction sites to produce at least three populations of digested DNA molecules; and (d) mixing a pre-determined amount of each of the at least three populations of digested synthetic DNA molecules in a single composition. In some embodiments, each of the at least three synthetic DNA molecules comprises first, second and third component sequences that are the same lengths as the first, second and third component sequences of any other of the at least three synthetic DNA molecules.

In some embodiments of the methods of the disclosure, the first, second and third component sequences are each between about 15 bp and about 3000 bp. In some embodiments, the first, second and third component sequences are each between about 15 bp and about 1200 bp. In some embodiments, the first, second and third component sequences are each between about 100 bp and about 600 bp. In some embodiments, the first, second and third component sequences are each between about 150 bp and about 500 bp. In some embodiments, the first, second and third component sequences are 175 bp, 250 bp and 450 bp, respectively.

In some embodiments, none of the first, second and third component sequences of any of the at least three DNA molecules are the same sequence. In some embodiments, the first, second and third component sequences are random sequences. In some embodiments, random sequences are generated using a random sequence generator. An exemplary, but non-limiting random sequence generator can be found at www.bioinformatics.org/sms2/random_dna.html, and is incorporated herein by reference. In some embodiments, random sequences do not map to the genome of any organism in the NCBI Nucleotide database. In some embodiments, random sequences can be designed that includes specific sequence properties, such as GC content, and excludes other (e.g., common restriction sites).

In some embodiments, the first, second and third component sequences of each of the at least three DNA molecules have at least one property in common with a target sequence in a sequencing sample.

In some embodiments, the sequences of the first and second restriction sites are the same. In some embodiments, the restriction sites are restriction sites that are recognized and cleaved by a type II restriction endonuclease. Choice of an appropriate restriction site will depend on the first, second and third component sequences, and will be readily apparent to one of ordinary skill in the art. In some exemplary embodiments, a restriction site is chosen that does not occur in any of the first, second and third component sequences.

In some embodiments, the sequence of the first and second restriction sites is selected from the group consisting of a sequence of an EcoRI site, a BamHI site, a HindIII site, a NotI site, a PvuII site, a SmaI site, an EcoRV site, a KpnI site, a PstI site, a SalI site, a ScaI site, an SpeI site, an SphI site, a StuI site and an XbaI site. In some embodiments, the restriction enzyme used to digest each of the at least three populations of at least three synthetic DNA molecules comprises EcoRI, BamHI, HindIII, NotI, PvuII, SmaI, EcoRV, KpnI, PstI, SalI, ScaI, SpeI, SphI, StuI or XbaI. In some embodiments, the sequences of the first and second restriction sites comprise XhoI sites and the restriction enzyme comprises XhoI.

In some embodiments, the sequence of each of the at least three DNA molecules further comprises a first primer sequence and sequence complementary to a second primer sequence, the first primer sequence is located 5' of the first random sequence, and the sequence complementary to the second primer sequence is located 3' of the third random sequence. In some embodiments, the first primer sequence comprises a sequence of 5'-TGAAGAACTGCGGCAGG-3' (SEQ ID NO: 12) (a Mito array primer). In some embodiments, the second primer sequence comprises a sequence of 5'-AGCTGGAAGTGCAGACC-3' (SEQ ID NO: 20) (a Ribo array primer sequence), and the sequence complementary to the second primer sequence comprises a sequence of 5'-GGTCTGCACTTCCAGCT-3' (SEQ ID NO: 13).

In some embodiments, the methods of the disclosure comprise quantifying the amount of the each of the at least three DNA molecules in a solution. In some embodiments, the methods of the disclosure further quantifying the amount of the each of the at least three populations of digested DNA molecules in a solution. Methods of quantifying the amount of the each of the populations of DNA molecules in a solution and/or quantifying the amount of each of at least three populations of digested DNA molecules in a solution will be readily apparent to one of ordinary skill in the art. Non-limiting examples of methods of DNA quantification include real time PCR, Droplet Digital PCR (ddPCR) and spectrophotometry.

In some embodiments, the methods of the disclosure comprise mixing a pre-determined amount of each of the at least three populations of digested DNA molecules in a single composition.

In some embodiments, the pre-determined amounts of the least three populations of digested DNA molecules are equal. In some embodiments, wherein the pre-determined amounts of the at least three populations of digested DNA molecules are not equal. In some embodiments, the pre-determined amounts of the at least three populations of digested DNA molecules are a linear sequence or in a geometric sequence.

In some embodiments, a pre-determined amount of one population of digested synthetic DNA molecules is a baseline, and the predetermined amounts of the at least two additional populations of digested DNA molecules are integer multiples of the baseline. In some embodiments, a pre-determined amount of one population of digested DNA molecules is a baseline, and wherein the pre-determined amount of each additional population of digested DNA molecules is twice the pre-determined amount of another population of digested DNA molecules, excluding the baseline.

In some embodiments, the pre-determined amounts of the at least three populations of digested synthetic DNA molecules are at a ratio of 1:2:4.

In exemplary embodiments, three populations of digested DNA molecules are mixed at a ratio of A:B:C, where 'A', 'B' and 'C' represent any pre-determined ratio of concentrations (for example, 1:2:4, or 1:3:5, or any linear or geometric sequence). In this embodiment, the first component sequence of each of the three DNA molecules is the same length, the second component sequence of each of the three DNA molecules is the same length and the third component sequence of each of the three synthetic DNA molecules is the same length. The resulting NC composition consists of three groups, each of which consists of three subgroups. Each subgroup of the first group consists of polynucleotides comprising the first component sequence each of the DNA molecules, each subgroup of the second group consists of polynucleotides comprising the second component sequence each of the three DNA molecules, and each subgroup of the third group consists of polynucleotides comprising the third component sequence each of the DNA molecules. The three pluralities of polynucleotides of the three subgroups of the first group are present at concentrations of a ratio of A:B:C as the three populations of digested DNA molecules were mixed at a ratio of A:B:C. As each of population of digested DNA molecules contributed equally to each group, each group is present in the composition at an equal concentration.

*In vitro Transcription*

In accordance with the claimed invention, the normalization controls comprise RNA polynucleotides. The disclosure provides methods of making such RNA normalization controls.

The disclosure provides for *in vitro* transcription methods to transcribe sequences that are 3' of a promoter sequence of a polymerase (for example, a T3, SP6 or T7 polymerase). Any sequence with a 5' promoter sequence is envisaged as being a suitable template for the *in vitro* transcription methods of the disclosure.

The disclosure provides methods of making a normalization control, comprising synthesizing at least three DNA polynucleotides, each polynucleotide comprising a sequence of a promoter and a component sequence, *in vitro* transcribing each polynucleotides to produce pluralities of RNAs, quantifying concentration of the each of the plurality of RNAs, and mixing the pluralities of RNAs in predetermined amounts to produce the NC composition.

The disclosure provides methods of making a population of synthetic nucleic acid molecules, comprising: (a) synthesizing at least three groups of DNA molecules; wherein the each of the DNA molecules comprises, from 5' to 3', a first component sequence, a promoter sequence, a first restriction enzyme site, a second component sequence, a second promoter sequence, a second restriction site, a third component sequence and a third promoter sequence; (b) digesting each of the groups of DNA molecules with a restriction enzyme that cuts the first and second restriction enzyme sites to produce at least three populations of digested synthetic DNA molecules; (c) *in vitro* transcribing each of the groups of DNA molecules to produce at least three populations of RNA molecules; (d) quantifying the amount of RNA produced in each of the populations of RNA molecules; and (e) mixing a pre-determined amount of each of the populations of RNA molecules to produce a single normalization composition.

In some embodiments, each of the DNA molecules comprises first, second and third component sequences that are the same lengths as the first, second and third component sequences of any other of the DNA molecules.

In some embodiments, the first, second and third component sequences are each between about 15 bp and about 1200 bp. In some embodiments, the first, second and third component sequences are each between about 150 bp and about 500 bp. In some embodiments, the first, second and third component sequences are each between about 100 bp and about 600 bp.

In some embodiments, none of the first, second and third component sequences of any of the DNA molecules are the same sequence. In some embodiments, the first, second and third component sequences of each of the DNA molecules are random sequences. In some embodiments, the first, second and third component sequences of each of the DNA molecules have at least one property in common with a target sequence in a sample.

The disclosure provides methods of digesting each of the at least three synthetic DNA molecules with a restriction enzyme such as a Type II restriction endonuclease that cuts the first and second restriction enzyme sites to produce at least three populations of digested DNA molecules.

In some embodiments, the sequences of the first and second restriction sites are the same. In some embodiments, the restriction sites are restriction sites that are recognized and cleaved by a type II restriction endonuclease. Choice of an appropriate restriction site will depend on the first, second and third component sequences, and will be readily apparent to one of ordinary skill in the art. In some exemplary embodiments, a restriction site is chosen that does not occur in any of the first, second and third component sequences.

In some embodiments, the sequence of the first and second restriction sites is selected from the group consisting of a sequence of an EcoRI site, a BamHI site, a HindIII site, a NotI site, a PvuII site, a SmaI site, an EcoRV site, a KpnI site, a PstI site, a SalI site, a ScaI site, an SpeI site, an SphI site, a StuI site and an XbaI site. In some embodiments, the restriction enzyme used to digest each of the at least three populations of at least three synthetic DNA molecules comprises EcoRI, BamHI, HindIII, NotI, PvuII, SmaI, EcoRV, KpnI, PstI, SalI, ScaI, SpeI, SphI, StuI or XbaI. In some embodiments, the sequences of the first and second restriction sites comprise XhoI sites and the restriction enzyme comprises XhoI.

In some embodiments, the resulting populations of digested DNA molecules comprise DNA molecules with a 5' promoter for *in vitro* transcription, and a component sequence. For example, each population of the at least three synthetic DNA molecules, upon digestion, produces three DNA fragments, each fragment comprising a 5' promoter operably linked to either the first, second or third component sequences. These DNA fragments can then be used as templates for *in vitro* transcription to produce RNA polynucleotides of the disclosure. In some embodiments, the fragments are purified and isolated separately prior to *in vitro* transcription. In some embodiments, the pool of fragments is use as a template for *in vitro* transcription.

The disclosure provides methods of *in vitro* transcribing each of the populations of DNA molecules to produce at least three populations of RNA molecules. In some embodiments, the first promoter sequence, the second promoter sequence and the third promoter sequence comprise the same promoter sequence. In some embodiments, the promoter sequence comprises a promoter sequence selected from the group consisting of a T7 promoter sequence, an SP6 promoter sequence or a T3 promoter sequence. In some embodiments, in particular those embodiments wherein the promoter is a T7 promoter, the T7 promoter comprises a sequence of 5'-TAATACGACTCACTATAGG-3' (SEQ ID NO: 14). In some embodiments, the T7 promoter comprises a sequence of 5'- TAATACGACTCACTATAGGG-3' (SEQ ID NO: 15). In some embodiments, the T7 promoter comprises a sequence of 5'-GCCTCGAGCTAATACGACTCACTATAGAG-3' (SEQ ID NO: 16). In some embodiments, the SP6 promoter comprises a sequence of 5'- ATTTAGGTGACACTATAG-3' (SEQ ID NO: 17). In some embodiments, the SP6 promoter comprises a sequence of 5' -CATACGATTTAGGTGACACTATAG-3 (SEQ ID NO: 18). In some embodiments, the T3 promoter comprises a sequence of 5' AATTAACCCTCACTAAAG 3' (SEQ ID NO: 19).

Polymerases of the disclosure can be RNA polymerase II or RNA polymerase III polymerases. In some embodiments, the polymerase is a T7 polymerase, an SP6 polymerase or a T3 polymerase. RNA polymerases of the disclosure may be wild type polymerases, component polymerases, or polymerases that have been optimized or engineered (*e.g.,* for *in vitro* transcription). The activity of polymerases of the disclosure may be highly specific for given promoter sequence (e.g., the T7 polymerase for the T7 promoter, the SP6 polymerase for the SP6 promoter, or the T3 polymerase for the T3 promoter).

The T7 promoter is recognized by and supports transcription by the T7 bacteriophage RNA polymerase. T7 polymerases of the disclosure may be wild type T7 polymerases, component T7 polymerases, or T7 polymerases that have been optimized or engineered (*e.g.,* for *in vitro* transcription). The T7 polymerase is a DNA dependent RNA polymerase that catalyzes the formation of RNA from a DNA template in the 5' to 3 direction. The DNA template may be double stranded or single stranded. T7 polymerase exhibits high specificity for the T7 promoter, can produce robust transcription *in vitro,* and is capable of incorporating modified nucleotides (*e.g.,* labeled nucleotides) into nascent RNA transcripts. These features of the T7 polymerase make it an excellent polymerase for synthesizing RNAs of the disclosure.

The disclosure provides methods of quantifying the amount of RNA produced in each of the at least three populations of synthetic RNA molecules. Methods of quantifying RNA will be readily apparent to one of ordinary skill in the art. Non-limiting exemplary methods of quantifying RNA include spectrophotometry or fluorescent tagging with a dye such as ethidium bromide.

The disclosure provides methods of mixing a pre-determined amount of each of populations of RNA molecules to produce a single composition.

In some embodiments, the pre-determined amounts of each of the populations of RNA molecules are equal. In some embodiments, the pre-determined amounts of each of the populations of RNA molecules are not equal. In some embodiments, the pre-determined amounts of the populations of RNA molecules are in a linear sequence or in a geometric sequence.

In some embodiments, the pre-determined amount of one population of RNA molecules is a baseline, and wherein the pre-determined amounts of the additional populations of RNA molecules are integer multiples of the baseline. In some embodiments, the pre-determined amount of one population of RNA molecules is a baseline, and wherein the pre-determined amount of each additional population of RNA molecules is twice the concentration of another population of RNA molecules excluding the baseline. In some embodiments, the pre-determined amounts of the at least three populations of RNA molecules are a ratio of 1:2:4.

In some embodiments, the methods further comprise diluting the normalization controls to a concentration of between about 0.001 and about 500 pg/$\mu$L. In some embodiments, the methods further comprise diluting the normalization controls to a concentration of between about 0.5 and about 500 pg/$\mu$L. In some embodiments, the methods further comprise diluting the normalization controls to a concentration of between about 0.5 and about 50 pg/$\mu$L. In some embodiments, the methods further comprise diluting the normalization controls to a concentration of between about 10 and about 20 pg/$\mu$L. In some embodiments, the methods further comprise diluting the normalization controls to a concentration of between about 1.0 and about 2.0 pg/$\mu$L. In some embodiments, the methods further comprise diluting the normalization controls to a concentration of between about 0.1 and about 0.2 pg/$\mu$L. In some embodiments, the concentration of the normalization controls is 1.4 pg/$\mu$L.

Isolated Sequences

The disclosure describes methods of making normalization control compositions wherein the normalization controls comprise an isolated sequence. In some embodiments, the isolated sequence is isolated from a naturally occurring sequence without additional steps, such as sequence shuffling or concatenation with additional isolated or component sequences.

Accordingly, the disclosure describes methods of making a normalization controls, comprising (a) extracting DNA from a sample; (b) digesting the DNA with a restriction enzyme to produce a collection of DNA fragments; (c) separating the collection of DNA fragments; (d) purifying at least three DNA fragments from the collection of DNA fragments to produce at least three groups of polynucleotides, wherein each of the at least three groups of polynucleotides comprises a plurality of polynucleotides; and (e) mixing a pre-determined amount of each of the at least three groups of polynucleotides to produce the composition.

The disclosure describes methods of extracting DNA from a sample. In some embodiments, sample is isolated or derived from a virus, a bacterium, a fungus or a eukaryotic parasite. In some embodiments, the sample comprises a cloning vector. In some embodiments, the cloning vector is a bacterial artificial chromosome (BAC), a yeast artificial chromosome (YAC), a cosmid, a fosmid or a plasmid. In some embodiments, the sample is a plasmid. In some embodiments, the normalization control sequences are cloned from a species of interest, for example a virus, a bacteria or a eukaryotic parasite, into a cloning vector, and DNA is extracted from a culture of cells producing the cloning vector. In some embodiments, the cells are bacteria (e.g. *Escherichia coli*). Exemplary methods of DNA extraction comprise kits *(e.g.* Qiagen MiniPrep) or phenol based extraction methods. Appropriate methods of DNA extraction will be readily apparent to one of ordinary skill in the art.

The disclosure describes methods of digesting the DNA with a restriction enzyme to produce a collection of DNA fragments. In some embodiments, the restriction enzyme is selected from the group consisting of EcoRI, BamHI, HindIII, NotI, PvuII, SmaI, EcoRV, KpnI, PstI, SalI, ScaI, SpeI, SphI, StuI and XbaI. In some embodiments, the restriction enzyme is selected from the group consisting of EcoRI, BamHI, HindIII, PvuII, SmaI, EcoRV, KpnI, PstI, SalI, ScaI, SpeI, SphI, StuI, XbaI, NotI, AscI, FseI, PacI, PmeI, BglII, BstBI, HincII and SgfI.

In some embodiments, the fragments produced by digesting the DNA with a restriction enzyme are between about 15 bp and about 100 kb. In some embodiments, fragments are between about 15 bp and about 50 kb. In some embodiments, fragments are between about 50 bp and about 50 kb. In some embodiments, fragments are between about 500 bp and about 50 kb. In some embodiments, fragments are between about 1000 bp and about 50 kb. In some embodiments, the longest of the each of the at least three different groups of polynucleotides is at least 15 kb in length, at least 20 kb in length, at least 25 kb in length, 30 kb in length, at least 35 kb in length or at least 40 kb in length.

The disclosure describes methods of purifying at least three DNA fragments from the collection of DNA fragments to produce at least three groups of polynucleotides, wherein each of the at least three groups of polynucleotides comprises a plurality of polynucleotides. In some embodiments, separating the collection of DNA fragments comprises a separation based on fragment size, fragment charge, or a combination thereof. In some embodiments, separating the collection of DNA fragments comprises a separation based on fragment size. In some embodiments, separating the DNA fragments based on size comprises gel electrophoresis, chromatography or tangential flow filtration (TFF).

In some embodiments, separating the DNA fragments based on size comprises gel electrophoresis. Gel electrophoresis separates polynucleotides according to their size. Polynucleotides samples are loaded into wells at one end of a gel, and an electric current is applied to pull them through the gel. Polynucleotides are negatively charged, and so move towards the positive electrode. The size of the polynucleotide determines the rate at which it migrates through the pores of the gel. Polynucleotides of varying sizes can be separated by varying agarose concentration of the gel (e.g., between 0.8% and 3% agarose). Alternatively, acrylamide gels can be used to separate smaller polynucleotides. In some embodiments, particularly those embodiments where large polynucleotides are being separated, changing the angle, strength and duration of the electromagnetic field can aid in separation. In some embodiments, the gel electrophoresis is pulse field gel electrophoresis.

In some embodiments, separating the DNA fragments based on size comprises chromatography. An exemplary chromatography method comprises anion exchange chromatography. Anion exchange chromatography separates polynucleotides based on their charge using an ion-exchange resin containing positively charged groups, which bind to negatively charged molecules. Tightness of binding of the polynucleotides to the column or matrix is based on the overall strength of the negative charge of the polynucleotide, and therefore size.

In some embodiments, separating the DNA fragments based on size comprises TFF. TFF is a membrane based filtration process. Based on membrane porosity, it can be classified as a microfiltration or an ultrafiltration process. Ultrafiltration membranes typically have pore sizes between 0.001 and 0.1 $\mu$m. Tangential Flow Filtration (TFF), also known as crossflow filtration, applies a feed stream parallel to the membrane face. One portion of the stream passes through the membrane (the permeate) while the remainder (the retentate) is recirculated back to the feed reservoir. Polynucleotides are separated by passing through the pores of the membrane.

In some embodiments, the methods further comprise ligating an adaptor to at least on end of each of the at least three different groups of polynucleotides.

In some embodiments, the methods further comprise ligating an adaptor to each end of the at least three different groups of polynucleotides.

In some embodiments, the sequences of the at least three different groups of polynucleotides in the normalization control further comprise synthetic polynucleotide sequences. Exemplary but non-limiting synthetic sequences comprise sequences of adaptors, primers for PCR amplification, promoter sequences for *in vitro* transcription, and binding sites for flow cell attachment during NGS.

In some embodiments, the adaptor sequence comprises a sequence encoding unique molecular identifier. In some embodiments, the sequence encoding the unique molecular identifier comprises a first indexing sequence or a second indexing sequence. An exemplary, non-limiting indexing strategy of the disclosure uses two indices, a first index and second index. In some embodiments, the sequence encoding the unique molecular identifier comprises a common primer sequence, an index sequence and an adaptor sequence. In some embodiments, the common primer sequence comprises a flow cell attachment site for NGS.

In some embodiments, the index sequence is a unique 4-20 bp sequence. In some embodiments, the index sequence is a unique 6 bp, 8 bp or 10 bp sequence. In some embodiments, the index sequence is a unique 6 bp sequence. The length of the index will depend on the complexity of the library. An appropriate index sequence length will be readily apparent to one of skill in the art. In some embodiments, a sequence comprising a first primer sequence, a first index sequence and a first adaptor sequence is attached to the 5' end of a polynucleotide, and a sequence comprising a second primer sequence, a second index sequence, and a second adaptor sequence is attached to the 3' end of the same polynucleotide. The polynucleotide is, for example a polynucleotide of a normalization control composition of the disclosure. In some embodiments, every group of polynucleotides comprises a unique dual index on the 5' and 3' ends of the plurality of polynucleotides in the group. In this embodiment, the dual index is the same for all of the plurality of polynucleotides within the group, and different for polynucleotides between groups.

In some embodiments, the synthetic sequence comprises a promoter sequence. In some embodiments, the promoter sequence comprises a promoter sequence selected from the group consisting of a T7 promoter sequence, an SP6 promoter sequence or a T3 promoter sequence.

Combining Isolation and *de novo* Synthesis

The disclosure describes methods of making normalization control compositions wherein the normalization controls comprise both polynucleotides comprising sequences that are synthesized and polynucleotides comprising sequences that are isolated from a sample, using the methods of disclosure.

The disclosure describes methods of making a normalization control, comprising: (a) synthesizing at least three groups of DNA molecules, wherein the DNA molecules within a group have the same sequence, and wherein the sequence of each of the DNA molecules comprises, from 5' to 3', a first component sequence, a first restriction enzyme site, a second component sequence, a second restriction site, and a third component sequence; (b) quantifying the amount of the each of the groups of DNA molecules in a solution; (c) digesting each of the groups of DNA molecules with a restriction enzyme that cuts the first and second restriction sites to produce at least three groups of digested DNA molecules; and (d) mixing a pre-determined amount of each of the groups of digested DNA molecules thereby generating a single normalization control composition.

In some embodiments, the mixing step of (d) further comprises mixing a pre-determined amount of at least one additional group of DNA molecules with the least three groups of digested DNA molecules from steps (a)-(c) to produce at least four groups of DNA molecules in a single normalization control, and wherein the at least one additional group of DNA molecules is isolated or derived from a sample. In certain embodiments, the at least one additional group of DNA molecules is isolated or derived from a sample using the methods of the disclosure.

In some embodiments, the pre-determined amounts of the at least four groups of DNA molecules are equal.

In some embodiments, the pre-determined amounts of the at least four groups of DNA molecules are not equal. In certain embodiments, the pre-determined amounts of the at least four groups of DNA molecules are in a linear sequence or a geometric sequence.

The disclosure describes methods of making a normalization control, comprising: (a) synthesizing at least one group of DNA molecules, wherein the DNA molecules within the group have the same sequence, and wherein the sequence of each of the DNA molecules comprises, from 5' to 3', a first component sequence, a first restriction enzyme site, a second component sequence, a second restriction site, and a third component sequence; (b) quantifying the amount of the groups of DNA molecules in a solution; (c) digesting the at least one group of DNA molecules with a restriction enzyme that cuts the first and second restriction sites to produce at least one group of digested DNA molecules; and (d) mixing a pre-determined amount of the group of digested DNA molecules with pre-determined amounts of at least two additional groups of DNA molecules to produce at least three groups of DNA molecules in a single normalization control composition, wherein the at least two additional groups of DNA molecules are isolated or derived from a sample.

In some embodiments, the pre-determined amounts of the at least three groups of DNA molecules are equal.

In some embodiments, the pre-determined amounts of the at least three groups of DNA molecules are not equal. In some embodiments, the pre-determined amounts of the at least three groups of DNA molecules are in a linear sequence or a geometric sequence.

In some embodiments, a pre-determined amount of one group of DNA molecules is a baseline, and the predetermined amounts of additional groups of DNA molecules are integer multiples of the baseline. In some embodiments, a pre-determined amount of one group of digested DNA molecules is a baseline, and wherein the pre-determined amount of each additional group of digested DNA molecules is twice the pre-determined amount of another group of digested DNA molecules, excluding the baseline. In some embodiments, the pre-determined amounts of the at least four groups of digested DNA molecules are at a ratio of 1:2:4, optionally 1:2:4:8, or 1:2:4:8:16 etc.

In some embodiments, the sample comprises plasmid DNA, mitochondrial DNA, chloroplast DNA or genomic DNA. In certain embodiments, the sample comprises DNA isolated from a cloning vector. In some embodiments, the cloning vector is a bacterial component chromosome (BAC), a yeast artificial chromosome (YAC), a cosmid, a fosmid or a plasmid.

*Sample Preparation*

Described herein are methods of quantifying the level of expression of at least one target nucleic acid molecule in a sample, comprising: (a) mixing a known amount of the normalization control composition of the disclosure with the sample, (b) preparing a high throughput sequencing library, (c) sequencing said library to produce a collection of reads, (d) mapping each read to the sample or the composition, (e) determining the number of reads produced by each of the plurality of nucleic acid molecules in the composition, (f) calculating relationship between the starting concentration of each of the plurality of nucleic acid molecules in the composition mixed with the sample in (a) and the number of reads produced in (c), (g) modeling (determining) the relationship between reads and concentration in the sample, and h. calculating the initial concentration of the at least one target nucleic acid molecule in the sample from the number of reads produced by the target nucleic acid molecule using the model in (g).

In some embodiments, the sample comprises nucleic acids. The nucleic acids can be DNA, RNA or a mixture of DNA and RNA. The nucleic acids can be single-stranded (e.g., single-stranded RNA, single-stranded DNA), double-stranded (e.g., double-stranded DNA, DNA:RNA hybrids), or a mix thereof.

In some embodiments, the sample comprises whole blood, plasma, serum, tears, saliva, mucous, cerebrospinal fluid, teeth, bone, fingernails, feces, urine, tissue or a biopsy taken from a mammal. In some embodiments, the sample comprises whole blood, plasma, serum, tears, saliva, mucous, cerebrospinal fluid, teeth, bone, fingernails, feces, urine, tissue or a biopsy taken from a human. In some embodiments, the sample comprises leaves, flowers or parts thereof, stems, roots, nuts or seeds from a plant. In some embodiments, the sample comprises body parts, tissues, feces or hemolymph from an insect.

In some embodiments, the method further comprises extracting nucleic acids from the sample. In some embodiments, the nucleic acids comprise DNA, RNA or a mixture thereof. Methods of extracting nucleic acids from a sample will be well known to a person of ordinary skill in the art. Exemplary extraction methods include, but are not limited to, phenol/chloroform based extraction methods, and kits such as the Qiagen Miniprep kit (Cat. No. 27104) and the Qiagen RNeasy mini kit (Cat. No. 74104).

In some embodiments, the sample is a mammalian sample. In some embodiments, the target nucleic acid molecule comprises a sequence of a virus, a bacterium, a fungus, or a eukaryotic parasite (e.g. a virus, a bacterium, a fungus, or a eukaryotic parasite found in the mammalian sample). In some embodiments, the target nucleic acid molecule comprises one or more mammalian genes.

In some embodiments, the sample is a human sample. In some embodiments, the target nucleic acid molecule comprises a sequence of a virus, a bacterium, a fungus, or a eukaryotic parasite (e.g. a virus, a bacterium, a fungus, or a eukaryotic parasite found in the human sample). In some embodiments, the target nucleic acid molecule comprises one or more human genes.

Described herein are methods of mixing a known amount of the normalization controls of the disclosure with the sample. In some embodiments, the normalization control composition is mixed with the sample such that the normalization controls represent a small fraction of the total sequence reads obtained from a sample. In some embodiments, the normalization controls, collectively, should represent a small fraction (<5%) of the sequence reads obtained from a sample using NGS.

In some embodiments, each of the groups or subgroups of polynucleotides in the normalization control composition are at a ratio of between about 0.001 to about 0.500: 1 relative to the total amount of polynucleotides in the normalization control. In some embodiments, each of the groups or subgroups of polynucleotides in the normalization control composition are at a ratio of between about 0.001 to about 0.420: 1 relative to the total amount of polynucleotides in the normalization control. In some embodiments, each of the groups or subgroups of polynucleotides in the normalization control composition are at a ratio of between about 0.001 to about 0.450: 1, between about 0.001 to about 0.400: 1, between about 0.001 to about 0.400: 1, between about 0.001 to about 0.350: 1, between about 0.001 to about 0.300: 1, between about 0.001 to about 0.250: 1, between about 0.001 to about 0.200: 1, between about 0.001 to about 0.150: 1, between about 0.001 to about 0.100: 1, between about 0.001 to about 0.05: 1, between about 0.005 to about 0.500: 1, between about 0.010 to about 0.500: 1, between about 0.050 to about 0.500: 1, between about 0.100 to about 0.500: 1, between about 0.010 to about 0.400: 1, between about 0.010 to about 0.300: 1, between about 0.010 to about 0.200: 1, between about 0.010 to about 0.100: 1, between about 0.050 to about 0.400: 1, between about 0.050 to about 0.300: 1, between about 0.050 to about 0.200: 1 or between about 0.050 to about 0.100: 1.

In some embodiments, each of the groups or subgroups of polynucleotides in the composition are added to the sample at a final concentration of between about 0.0001 pg/ μL and about 45 pg/μL between about 0.001 pg/ μL and about 45 pg/μL, between about 0.001 pg/ μL and about 10 pg/μL, between about 0.005 pg/ μL and about 5 pg/μL or between about 0.009 pg/ μL and about 5 pg/μL. In some embodiments, each of the groups or subgroups of polynucleotides in the composition are added to the sample at a final concentration of between about 0.009 pg/ μL and about 5 pg/μL.

In some embodiments, each of the groups or subgroups of polynucleotides in the composition are added to the sample at a final concentration of between about 0.001 attomole/μL and about 50 attomole/μL. In some embodiments,

each of the groups or subgroups of polynucleotides in the composition are added to the sample at final concentration of between about 0.01 attomole/μL and 50 attomole/μL. In some embodiments, each of the groups of or subgroups polynucleotides in the composition are added to the sample at final concentration of between about 0.05 attomole/μL and 25 attomole/μL.

In some embodiments, the methods further comprise extracting nucleic acids from the sample, and mixing the normalization controls with the sample occurs prior to extracting nucleic acid from the sample. In some embodiments, mixing of normalization controls with the sample prior to nucleic acid extraction allows the normalization controls to measure bias in nucleic acid extraction during sample preparation. For example, target nucleic acids complexed with proteins may be resistant to nucleic acid extraction and thus be underrepresented in read abundance following NGS. This process can be mimicked through the addition of appropriate normalization controls, for example, normalization controls encapsulated by viral capsid proteins.

In some embodiments, mixing the normalization controls with the sample occurs prior to the preparation of the high throughput sequencing library. In some embodiments, particularly those embodiments where the mixing step occurs prior to library preparation, the NCs measure the conversion of a DNA or RNA target sequence in a sequencing sample to a sequence-able library molecule. Dual indexing the ends of the NC polynucleotides at the same time as sample polynucleotides allows for tracking which molecules are converted and how they are converted per sample at any time during the library preparation. This allows for the calculation of a sample type agnostic library conversion factor. This also allows for quality control to be built into the sample preparation. Using this technique, it is possible to compare reagent lots, the efficiency of enzymatic reactions and many other metrics about sequencing. These comparisons can be done in line with steps of the sequencing reaction that would normally have to wait until the end of the library preparation process to determine if problems had arisen.

In some embodiments, mixing the normalization controls with the sample occurs at the same time as the library preparation step. Normalization controls, with appropriate adaptors, as necessary, may be added during any point of the library preparation process.

In some embodiments, mixing the normalization controls with the sample occurs after as the library preparation step. Normalization controls, with appropriate indexing and/or sequencing adaptors, can be added to the library after library preparation but before NGS. In some embodiments, normalization controls added at this point in the process control for biases in the sequence reactions themselves. For example, normalization controls with high or low GC content can control for known GC bias in pyrosequencing methods.

Libraries

Sequencing methods of the disclosure involve the preparation of sequencing libraries. Sequencing library preparation in turn involves the production of a collection of adaptor-modified DNA fragments, which are ready to be sequenced. Sequencing libraries of polynucleotides can be prepared from DNA or RNA, including equivalents, analogs of either DNA or cDNA, that is complementary or copy DNA produced from an RNA template, for example by the action of reverse transcriptase. The polynucleotides may originate in double-stranded DNA (dsDNA) form *(e.g.* genomic DNA fragments, PCR and amplification products) or polynucleotides that may have originated in single-stranded form, as DNA or RNA, and been converted to dsDNA form. By way of example, mRNA molecules may be copied into double-stranded cDNAs suitable for use in preparing a sequencing library.

Preparation of sequencing libraries for some NGS sequencing platforms requires that the polynucleotides be of a specific range of fragment sizes *e.g.* 0-1200 bp. Therefore, fragmentation of large polynucleotides may be required. Whether polynucleotides are forcibly fragmented or naturally exists as fragments, they are converted to blunt-ended DNA having 5-phosphates and 3'-hydroxyl. The process of fragmentation in library preparation is one potential source of error in quantifying a target sequence using NGS, and can be modeled by the inclusion of normalization controls prior to a fragmentation step in library preparation.

Typically, the fragment ends are end-repaired, e.g. blunt-ended using methods or kits known in the art. The blunt-ended fragments can be phosphorylated by enzymatic treatment, for example using polynucleotide kinase. In some embodiments, a single deoxynucleotide e.g. deoxyadenosine (A) is added to the 3'-ends of the polynucleotides, for example, by the activity of certain types of DNA polymerase such as Taq polymerase or Klenow exo minus polymerase. dA-tailed products are compatible with 'T' overhang present on the 3' terminus of each duplex region of adaptors to which they are ligated in a subsequent step. dA-tailing prevents self-ligation of both of the blunt-ended polynucleotide such that there is a bias towards formation of the adaptor-ligated sequences. The dA-tailed polynucleotides are ligated to double-stranded adaptor polynucleotides sequences. The same adaptor can be used for both ends of the polynucleotide, or two sets of adaptors can be used. Ligation methods are known in the art and utilize ligase enzymes such as DNA ligase to covalently link the adaptor to the d-A-tailed polynucleotide. The adaptor may contain a 5'-phosphate moiety to facilitate ligation to the target 3'-OH. The dA-tailed polynucleotide contains a 5'-phosphate moiety, either residual from the shearing process, or added using an enzymatic treatment step, and has been end repaired, and optionally extended by an overhanging base or bases, to give a 3'-OH suitable for ligation. The products of the ligation reaction are purified to remove unligated adaptors, adaptors that may have ligated to one another, and to select a size range of templates for cluster generation, which can be preceded by an amplification e.g. a PCR amplification. Purification of the ligation products can be obtained by methods including gel electrophoresis and solid-phase reversible immobilization (SPRI).

Standard protocols, e.g. protocols for sequencing using, for example, the Illumina platform, may instruct users to purify the end-repaired products prior to dA-tailing, and to purify the dA-tailing products prior to the adaptor-ligating steps of the library preparation. Purification of the end-repaired products and dA-tailed products remove enzymes, buffers, salts and the like to provide favorable reaction conditions for the subsequent enzymatic step. In some embodiments, the steps of end-repairing, dA-tailing and adaptor ligating exclude the purification steps. Thus, in some embodiments, the method of the disclosure encompasses preparing a sequencing library that comprises the consecutive steps of end-repairing, dA-tailing and adaptor-ligating. In embodiments for preparing sequencing libraries that do not require the dA-tailing step, e.g. protocols for sequencing using Roche 454 and SOLID™ 3 platforms, the steps of end-repairing and adaptor-ligating exclude the purification step of the end-repaired products prior to the adaptor-ligating.

In some embodiments, an amplification reaction is prepared. The amplification step introduces to the adaptor ligated template molecules the polynucleotide sequences required for hybridization to the flow cell. The contents of an amplification reaction are known by one skilled in the art and include appropriate substrates (such as dNTPs), enzymes (e.g. a DNA polymerase) and buffer components required for an amplification reaction. Optionally, amplification of adaptor-ligated polynucleotides can be omitted. Generally amplification reactions require at least two amplification primers *i.e.* primer oligonucleotides, which may be identical, and include an 'adaptor-specific portion', capable of annealing to a primer-binding sequence in the polynucleotide molecule to be amplified (or the complement thereof if the template is viewed as a single strand) during the annealing step. Once formed, the library of templates prepared according to the methods described above can be used for solid-phase nucleic acid amplification. The term 'solid-phase amplification' as used herein refers to any nucleic acid amplification reaction carried out on or in association with a solid support such that all or a portion of the amplified products are immobilized on the solid support as they are formed. In particular, the term encompasses solid-phase polymerase chain reaction (solid-phase PCR) and solid phase isothermal amplification which are reactions analogous to standard solution phase amplification, except that one or both of the forward and reverse amplification primers is/are immobilized on the solid support. Solid phase PCR covers systems such as emulsions, wherein one primer is anchored to a bead and the other is in free solution, and colony formation in solid phase gel matrices wherein one primer is anchored to the surface, and one is in free solution. Following amplification, and sequencing libraries can be analyzed by microfluidic capillary electrophoresis to ensure that the library is free of adaptor dimers or single stranded DNA. The library of template polynucleotide molecules is particularly suitable for use in solid phase sequencing methods. In addition to providing templates for solid-phase sequencing and solid-phase PCR, library templates provide templates for whole genome amplification.

In some embodiments, the library of adaptor-ligated polynucleotides is subjected to massively parallel sequencing, which includes techniques for sequencing millions of fragments of nucleic acids, e.g., using attachment of randomly fragmented genomic DNA to a planar, optically transparent surface and solid phase amplification to create a high density sequencing flow cell with millions of clusters. The Solexa/Illumina method relies on the attachment of randomly fragmented genomic DNA to a planar, optically transparent surface. Attached DNA fragments are extended and bridge amplified to create an ultra-high density sequencing flow cell with millions of clusters each containing thousands of copies of the same template. The cluster templates are sequenced using a robust four-color DNA sequencing-by-synthesis technology that employs reversible terminators with removable fluorescent dyes. Alternatively, the library may be amplified on beads wherein each bead contains a forward and reverse amplification primer.

Sequencing of the amplified libraries can be carried out using any suitable sequencing technique as described herein.

### Sequencing

Described herein are methods of sequencing a sample, the sample comprising normalization controls, using next generation sequencing.

In some embodiments, the method described herein employs next generation sequencing technology (NGS) in which clonally amplified DNA templates or single DNA molecules are sequenced in a massively parallel fashion within a flow cell (*e.g.* as described in Volkerding et al. Clin Chem 55:641-658 [2009]; Metzker M Nature Rev 11:31-46 [2010]). In addition to high-throughput sequence information, NGS provides digital quantitative information, in that each sequence read is a countable "sequence tag" representing an individual clonal DNA template or a single DNA molecule. The sequencing technologies of NGS include pyrosequencing, sequencing-by-synthesis with reversible dye terminators, sequencing by oligonucleotide probe ligation and real time sequencing.

Some of the sequencing technologies are available commercially, such as the sequencing-by-hybridization platform from Affymetrix Inc. (Sunnyvale, Calif.) and the sequencing-by-synthesis platforms from 454 Life Sciences (Bradford, Conn.), Illumina/Solexa (Hayward, Calif.) and Helicos Biosciences (Cambridge, Mass.), and the sequencing-by-ligation platform from Applied Biosystems (Foster City, Calif.). In addition to the single molecule sequencing performed using sequencing-by-synthesis of Helicos Biosciences, other single molecule sequencing technologies are encompassed by the method of the disclosure and include the SMRT™ technology of Pacific Biosciences, the Ion Torrent™ technology, and nanopore sequencing being developed for example, by Oxford Nanopore Technologies. The present disclosure however is contemplated to be compatible with any future high throughput sequencing technology that is developed, that inherently poses the problem of quantification of low input samples.

While the automated Sanger method is considered as a 'first generation' technology, Sanger sequencing including the automated Sanger sequencing, can also be employed by the method of the disclosure.

In some embodiments of the methods of the disclosure, the DNA sequencing technology that is used is the 454 sequencing (Roche) (*e.g.* as described in Margulies, M. et al. Nature 437:376-380 [2005]). In 454 sequencing DNA fragments of approximately 300-800 base pairs, are blunt-ended and adaptors are ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is discerned and analyzed. 454 Sequencing can produce reads that are between 100-1000 bp in length.

In some embodiments of the methods of the disclosure, the DNA sequencing technology that is used is the SOLiD™ technology (Applied Biosystems). In SOLiD™ sequencing-by-ligation, 5' and 3' are attached to DNA fragments to generate a fragment library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated. SOLiD technology can produce reads of about 50 bp.

In some embodiments of the methods of the disclosure, the DNA sequencing technology that is used is the single molecule, real-time (SMRT™) sequencing technology of Pacific Biosciences. In SMRT sequencing, the continuous incorporation of dye-labeled nucleotides is imaged during DNA synthesis. Single DNA polymerase molecules are attached to the bottom surface of individual zero-mode wavelength identifiers (ZMW identifiers) that obtain sequence information while phospholinked nucleotides are being incorporated into the growing primer strand. A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Identification of the corresponding fluorescence of the dye indicates which base was incorporated. The process is repeated. The RS instrument has an average read length of about 1100 bp, sometimes with reads up to 2500 or 2900 bp. The RS II can produce read lengths of 8,500-60,000 bp.

In some embodiments of the methods of the disclosure, the DNA sequencing technology that is used is nanopore sequencing (e.g. as described in Soni G V and Meller A. Clin Chem 53: 1996-2001 [2007]). Nanopore sequencing DNA analysis techniques are being industrially developed by a number of companies, including Oxford Nanopore Technologies (Oxford, United Kingdom). Nanopore sequencing is a single-molecule sequencing technology whereby a single molecule of DNA is sequenced directly as it passes through a nanopore. A nanopore is a small hole, of the order of 1 nanometer in diameter Immersion of a nanopore in a conducting fluid and application of a potential (voltage) across it results in a slight electrical current due to conduction of ions through the nanopore. The amount of current which flows is sensitive to the size and shape of the nanopore. As a DNA molecule passes through a nanopore, each nucleotide on the DNA molecule obstructs the nanopore to a different degree, changing the magnitude of the current through the nanopore in different degrees. Thus, this change in the current as the DNA molecule passes through the nanopore represents a reading of the DNA sequence. Nanopore MinION can produce read lengths of over 10,000 bp.

In some embodiments, the DNA sequencing technology is the Ion Torrent single molecule sequencing, which pairs semiconductor technology with a simple sequencing chemistry to directly translate chemically encoded information (A, C, G, T) into digital information (0, 1) on a semiconductor chip. In nature, when a nucleotide is incorporated into a strand of DNA by a polymerase, a hydrogen ion is released as a byproduct. Ion Torrent uses a high-density array of micro-machined wells to perform this biochemical process in a massively parallel way. Each well holds a different DNA molecule.

Beneath the wells is an ion-sensitive layer and beneath that an ion sensor. When a nucleotide, for example a C, is added to a DNA template and is then incorporated into a strand of DNA, a hydrogen ion will be released. The charge from that ion will change the pH of the solution, which can be identified by Ion Torrent's ion sensor. The sequencer essentially the world's smallest solid-state pH meter-calls the base, going directly from chemical information to digital information. The Ion personal Genome Machine (PGM™) sequencer then sequentially floods the chip with one nucleotide after another. If the next nucleotide that floods the chip is not a match. No voltage change will be recorded and no base will be called. If there are two identical bases on the DNA strand, the voltage will be double, and the chip will record two identical bases called. Direct identification allows recordation of nucleotide incorporation in seconds. Ion Torrent can produce read lengths of about 400 bp.

In some embodiments, the method employs massively parallel sequencing of millions of DNA fragments using Illumina's sequencing-by-synthesis and reversible terminator-based sequencing chemistry (*e.g.* as described in Bentley et al., Nature 6:53-59 [2009]). Illumina's sequencing technology relies on the attachment of fragmented genomic DNA to a planar, optically transparent surface on which oligonucleotide anchors are bound. Template DNA is end-repaired to generate 5'-phosphorylated blunt ends, and the polymerase activity of Klenow fragment is used to add a single A base to the 3' end of the blunt phosphorylated DNA fragments. This addition prepares the DNA fragments for ligation to oligonucleotide adapters, which have an overhang of a single T base at their 3' end to increase ligation efficiency. The adapter oligonucleotides are complementary to the flow-cell anchors. Under limiting-dilution conditions, adapter-modified, single-stranded template DNA is added to the flow cell and immobilized by hybridization to the anchors. Attached DNA fragments are extended and bridge amplified to create an ultra-high density sequencing flow cell with hundreds of millions of clusters, each containing ~1,000 copies of the same template. The templates are sequenced using a robust four-color DNA sequencing-by-synthesis technology that employs reversible terminators with removable fluorescent dyes. High-sensitivity fluorescence identification is achieved using laser excitation and total internal reflection optics. Short sequence reads are aligned against a repeat-masked reference genome and genetic differences are called using specially developed data analysis pipeline software. After completion of the first read, the templates can be regenerated in situ to enable a second read from the opposite end of the fragments. Thus, either single-end or paired end sequencing of the DNA fragments is used according to the method. Illumina read lengths depend on the specific platform, but are generally between 50-400 bp.

### *Quantification*

A "formula," "algorithm," or "model" is any mathematical equation, algorithmic, analytical or programmed process, or statistical technique that takes one or more continuous or categorical inputs and calculates an output value, sometimes referred to as an "index" or "index value." Non-limiting examples of "formulas" include sums, ratios, and regression operators, such as coefficients or exponents, biomarker value transformations and normalizations, statistical classification models, and neural networks trained on historical populations. Of particular use in determining the relationship between starting concentration in a sample and output reads following NGS are linear and non-linear equations and statistical classification analyses to determine the relationship between starting concentration in a sample and sequence reads after NGS.

Of particular interest are structural and syntactic statistical classification algorithms, utilizing pattern recognition features, including established techniques such as cross-correlation, Principal Components Analysis (PCA), factor rotation, Logistic Regression (LogReg), Linear Discriminant Analysis (LDA), Support Vector Machines (SVM), Random Forest (RF), Recursive Partitioning Tree (RPART), as well as other related decision tree classification techniques, Shrunken Centroids (SC), StepAIC, Kth-Nearest Neighbor, Boosting, Decision Trees, Neural Networks, Bayesian Networks, and Hidden Markov Models, among others. These may be coupled with information criteria, such as Akaike's Information Criterion (AIC) or Bayes Information Criterion (BIC), in order to quantify the tradeoff between additional sequence data and model improvement, and to aid in minimizing overfit. The resulting predictive models may be validated in other studies, or cross-validated in the study they were originally trained in, using such techniques as Bootstrap, Leave-One-Out (LOO) and 10-Fold cross-validation (10-Fold CV). At various steps, false discovery rates may be estimated by value permutation according to techniques known in the art.

### Linear Correlations

In some embodiments of the methods of the disclosure, the concentrations of the groups and/or subgroups of the normalization controls comprise a linear sequence. In some embodiments, the linearity of the design of the normalization controls allows for correlation between the reads in the library. The concentrations of the NC molecules when added to a sample or during library preparation are perfectly correlated and follow a linear model. This relationship is expected to still hold for the output reads shared by these NC polynucleotides. When the starting concentration of NC is plotted against the corresponding normalized read count, and the $R^2$ value of the regression is determined, an $R^2$ of more than 0.8 indicates that the extraction and downstream library preparation process has been followed without any fallacies.

The NCs are added to a library at definite concentrations and at different sizes. The range of sizes of different polynucleotides are meant to be a representation of the insert sizes of the target DNA or RNA to be identified in the library. As the concentrations of the NCs that are added to a sample or a library during preparation are pre-determined, the concentration of a target sequence present in the sample can be determined by fitting the read counts of the target to a linear model determined by the NCs. In this model, biases that the target sequence, the background nucleic acids in the sample, or the NCs have during the library prep process are minimal. Each NC linearity has its own line equation, y = mx + c. Y is the log(reads per million, RPM) of the target sequence/NC in question which is given; M is the slope of line and C is the intercept. X is undetermined for the target sequence. Theoretically, the NCs follow linearity, which can be calculated as log(concentration) in attomoles/$\mu$L. This concentration converts to how many fragments of target sequence are present in the library per $\mu$L.

The success of this model depends on the linearity of the NC having an $R^2$ of above 0.8 to begin target titer estimation. An $R^2$ lower than 0.8 suggests a quality control fail of the NC during sample preparation and/or high throughput sequencing. In this model, the linearity of NC is established based on absolute concentrations. It further assumes that the insert size is one kind only, that there is 100% genome coverage and minimal sample variation. Sample variation can be determined by calculating the percent of reads that map to the background sample sequence (sample sequence other than the 'target'), the percent of reads mapping to the NC, and theoretically, the percent depletion. Although, a singular insert size of the target sequence is desired, this is seldom achievable. Ideally, in a histogram, the 3 - 5 most representative bins of target insert sizes are selected, and the percentage of reads in each bin is calculating. Doing this inherently takes care of the genome coverage issue. The number of insert molecules is then dependent on the numbers in each of these bins.

The number or concentration of target insert molecules can be calculated using the linear relationship Y = mx + C. The number of target molecules $\propto$ (is a function of) Titer; and Titer $\propto$ f(insert size bins, genome coverage, % human mapping, % NC mapping).

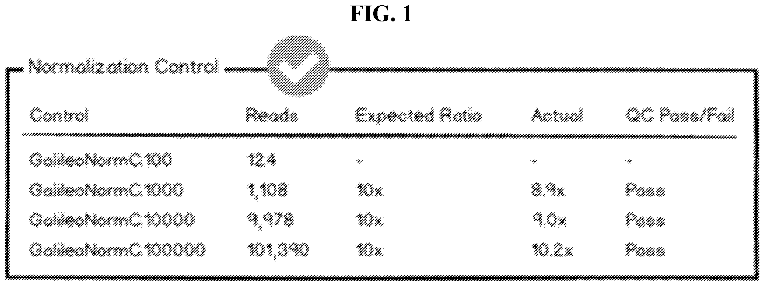

For example, to allow for quality control, normalization controls can be added in in ratios of each other. This allows for the computation of the observed ratios, and confirmation that the nucleic acids converted as expected throughout the entire process/assay. If the ratios come out as expected (for example, NC1, NC2, and NC3 are added to a sample at concentration ratios of 1:2:4, then the read count/evidence recovers that ratio - 100 reads : 200 reads: 400 reads). Fig. 1 shows an exemplary normalization control quality control check. For quality control of normalization controls that comprise genomic DNA rather than synthesized DNA polynucleotides, for example bacteriophage T4 normalization NC, a minimum number of reads a recovered from that genome (*e.g.* the bacteriophage T4 genome) is looked for.

In some cases, NC are used to normalize a standard curve. In a simple case, the observed reads whose abundance is to be determined (referred to herein as 'target' reads) are divided by the observed NC reads (either combined or separately, depending on which allows for the generation of a better standard curve). In this case, normalized target read abundance is calculated as follows:

Normalized read abundance = [(target observed) / (NC observed)] * C
(where C = a Constant, if needed)

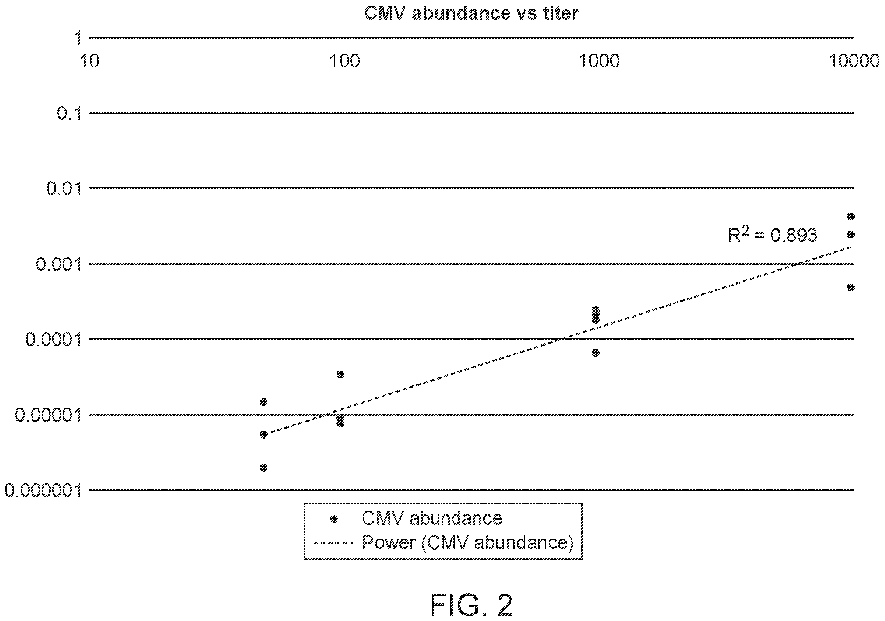

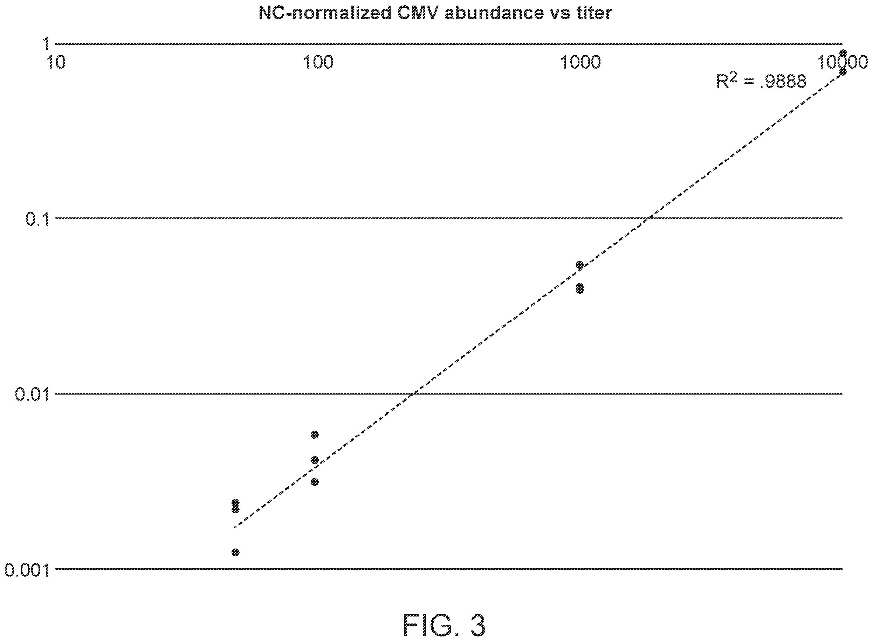

FIG. 2 shows a plot of Cytomegalovirus CMV (target) viral titer versus read abundance in next generation sequencing of DNA libraries in which the DNA was extracted from plasma to which CMV DNA was added (the target). The dotted line indicates the linear regression the CMV titer versus CMV read abundance. Without normalization, the $R^2$ value is 0.89. In contrast, with normalization, the $R^2$ value is 0.98 (FIG. 3). Normalization controls thus improve the correlation between the abundance of the target (here CMV) in the initial sample, and the percentage of reads that map to the target following library preparation and next generation sequencing.

Normalization controls added to sample before library preparation can account for error arising from different experimental conditions and systematic/random error that affects the nucleic acid in the sample. When designed to mimic actual biology as close as possible, many differences in preparation, conversion, sequencing and so forth can be accounted for and normalized.

However, in some cases, there are some biological factors cannot be mimicked and therefore cannot be accounted for by the normalization controls. When this is the case, the conversion of raw reads to target concentration can be described by the equation:

$$\tilde{r} = f(r) + \Delta r$$

where r is raw target read evidence (or counts), f(r) is the transformation function that allows one to normalize the reads for all the variables accounted for, and the $\Delta$ function is the residual error due to processes that cannot be mimicked or accounted for. Ideally, Δ(r) goes to zero, and the transformation converts raw target read evidence to an initial starting concentration (such as viral copies per milliliter). An example of a simple function f(r) is a simple multiplicative factor. In this case, f(r) is computed from the actual input percentage/abundance of normalization controls and the observed abundance (via read counts/evidence) of normalization controls:

$$f(r) = f \cdot r$$

where f can be computed as

$$f = \frac{\text{actual abundance}}{\text{observed abundance}}$$

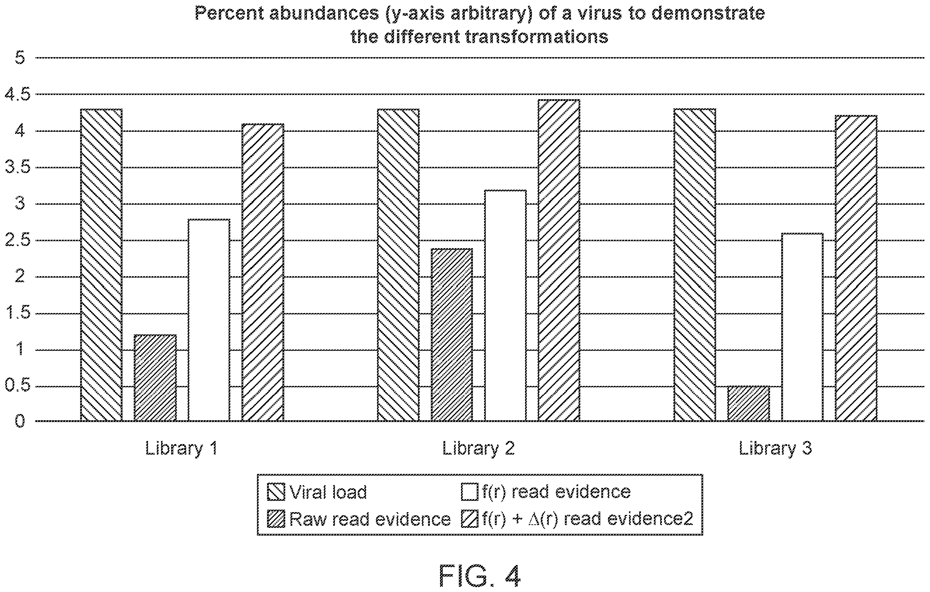

Other forms of f(r) can account for and mimic target properties such as target GC content, target size, fragmentation conditions, whether the target is associated with proteins (e.g. a viral genome in a capsid) or is a free DNA molecule, and so forth. If the normalization controls are designed to account for all these variables, then Δ will get smaller and smaller. A visual representation of this is sketched out in a hypothetical example in FIG. 4. In FIG. 4. a nucleic acid sample isolated from a host infected with a virus is analyzed in triplicate using next generation sequencing. In FIG. 4, the left most bar for each next generation sequencing library is the known non-host concentration (a viral load) in the starting sample (*i.e.,* the starting abundance of the 'target' in the sample). After library preparation, next generation sequencing and alignment of the reads, the read counts are transformed such that they recover the viral load. The second bar from the left in each library is an example of computing the percent abundance of the virus using only the obtained viral evidence. FIG. 4 demonstrates that there is huge variability between each library when the percent abundance of the virus in the starting sample (viral load) is calculated only from the obtained viral evidence. This calculation also does not reflect the actual viral load in the original sample. Using an f(r) function, it is possible to transform the raw read evidence to a quantity that is more comparable across libraries (FIG. 4, third bar from the left for each library). Using the f(r) function, the variability of the calculated percent abundance of the virus is decreased amongst the libraries, and the resulting value is closer to the actual viral load. The only remaining transformation that takes one directly to viral load is Δ(r). Accurately computing Δ(r) reduces the variability across libraries even more and result in the viral load (within error).

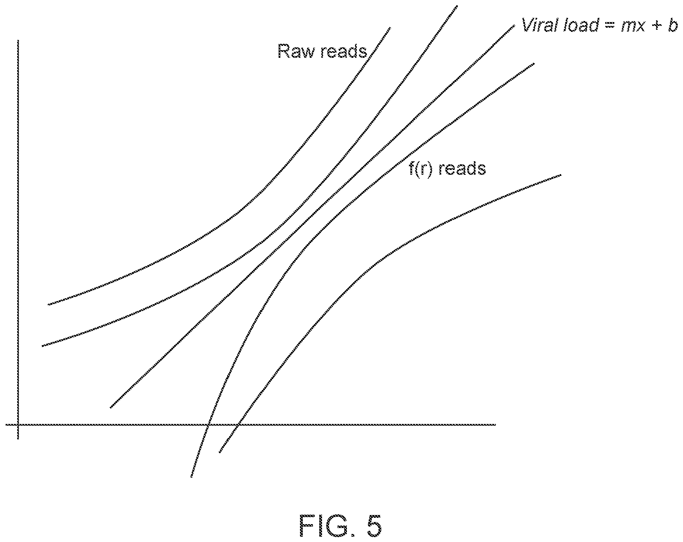

Carefully constructing the normalization controls allows for the estimation of f(r) for each library for and for each target. Random sequences can be used, but more biological information can also be added. A feature of this approach is that if f(r) can be computed, then one can avoid running a multiplicity of samples to directly transform raw reads into target abundance in the initial sample. f(r) closes the gap between read count and target abundance and lowers the variability. Δ(r) can be estimated by designing an experiment that accounts for all the variables that could not be included in the normalization controls. For example, Δ(r) could be due to a cell wall, for example a bacterial cell wall, or a capsid environment for viruses, the effect of which can be determined experimentally. By closing the gap between target abundance in the initial sample and output read count with f(r), Δ is minimized. Δ, likely a multiplicative factor (y=ax+b), can be computed from multiple samples. In certain embodiments, higher-order models for Δ may also be appropriate. FIG. 5 depicts the expected decrease in variability when using f(r) as opposed to raw reads to calculate viral load. Variability is decreased as much as possible through f(r) before running many samples to calculate Δr to get the equation for the line depicted in FIG. 5.

One potential source of variability in the post-computation of f(r) and Δr is the site of execution of the assay. In some cases, it may be that when the assay is executed at secondary site (for example, another hospital calculating viral titer in patient samples), the execution of the assay delivers data that doesn't quite fall on the line that was initially generated for that set of target and normalization controls. In this case, the assay is validated at the secondary site. For example, the secondary site can run enough samples to compute the viral load conversion, and input their equation into a web-app, which is used when computing the viral load from the real samples.

A table of some of the possible variables that effect the conversion of the sample to sequencing reads is set forth below as Table 1. Table 1 outlines possible variables to consider in NC control design. Table 1 outlines possible variable to consider in designing NCs when the target is virus. However, some of these possible variables may be applicable to other targets.

Table 1. Variables in NC Design

| Variable | Category/Noise | NC design strategy | Contributes to | Test description |
|---|---|---|---|---|
| GC content | Biology | Compute GC content in all TPx viruses and have NCs mimic that range | f(r) | Run libraries with these NCs, compute read evidence, see whether an average of the NC counts can be used for f(r), or a component of the NC, with GC content as close to the virus, produces a better f(r) multiplicative factor (or observe if f(r) is more complicated, but moves the raw read abundance closer to virus load with less variability. |
| Location bias | Random | None | Δ(r) | Send out enough samples at various locations to estimate the effect of location variability. |
| Viral fragmentation | Biology | Have NCs that are big enough to fragment | f(r) | Run libraries with these NCs, compute read evidence, see whether an average of the NC counts can be used for f(r), or a component of the NC, with fragmentation pattern as close to the virus, produces a better f(r) multiplicative factor (or observe if f(r) is more complicated, but moves the raw read abundance closer to viral load with less variability. |
| Extraction from capsid environment (Extraction bias) | Biology | None / Add NCs to capsids equivalent to viruses | Δ(r) | Run enough samples to determine if a linear range is observed, with high R2, and get the equation for the line. |
| Abundance of NC vs virus | Biology | Spike in NCs at clinically relevant levels for viruses | f(r) | Run libraries with these NCs, compute read evidence, see whether an average of the NC counts can be used for f(r), or a component of the NC, with abundances close to the virus, produces a better f(r) multiplicative factor (or observe if f(r) is more complicated, but moves the raw read abundance closer to viral load with less variability. |

Accordingly, described herein are methods of calculating relationship between the starting concentration of each of the plurality of nucleic acid molecules in the normalization controls mixed with the sample and the number of reads produced by NGS, modeling the relationship between reads and concentration in the sample, and calculating the initial concentration of the at least one target nucleic acid molecule in the sample from the number of reads produced by the target nucleic acid molecule using the model.

In some embodiments, the model is a linear model. In some embodiments, a linear regression of the initial concentration of the at least one target nucleic acid molecule in the sample versus the number of reads produced by the target nucleic acid molecule has an $R^2$ value of greater than 0.95, greater than 0.96, greater than 0.97, greater than 0.98 or greater than 0.99 following normalization. In some embodiments, the $R^2$ value of the linear regression improves by at least 0.01, 0.03, 0.05, 0.07, 0.09, 0.1, 0.13, 0.15 0.17, 0.19, 0.2, 0.23, 0.25, 0.27, 0.29, 0.3, 0.33, 0.35, 0.37, 0.39, 0.4, 0.43, 0.45, 0.47, 0.49, 0.5, 0.53, 0.55, 0.57, 0.59, 0.6, 0.63, 0.65, 0.67, 0.69, 0.7, 0.8, 0.9, 1.0 or any value in between following normalization.

Titer Determination Using Machine Learning

Support Vector Machines (SVM) are a known mechanism of supervised learning approaches to predict drug toxicity and antimicrobial resistance. They are a set of methods used for classification, regression and outlier-detection. For an SVM to make predictions on data, it must have been fit with the data. Given a set of training data, each data point marked as belong one of two categories, an SVM training algorithm builds a model that assigns new data to one of the categories. SVMs are non-probabilistic binary linear classifiers that use a supervised machine learning approach to train the classifier.

In some embodiments, the normalization control data is used for fitting the SVM model. In some embodiments, 80% of the data is used for training the SVM, and the remaining 20% of the data is used for testing the SVM. The fitting to model works as a feedback process, where only a linear model is not taken into consideration. The model with the prediction that is closest to the actual concentration of normalization controls that went into the NGS library determines the model chosen for predicting of titer of the target sequence in the sample.

Accordingly, described herein are methods of calculating relationship between the starting concentration of each of the plurality of nucleic acid molecules in the normalization controls mixed with the sample and the number of reads produced by NGS, modeling the relationship between reads and concentration in the sample, and calculating the initial concentration of the at least one target nucleic acid molecule in the sample from the number of reads produced by the target nucleic acid molecule using the model.

In some embodiments, the model is created using a machine learning classifier. In some embodiments, the machine learning classifier is supervised. In some embodiments, the machine learning classifier is a Vector Support Machine.

All percentages and ratios are calculated by weight or number of molecules (e.g. molar ratios) unless otherwise indicated. The appropriate units of measurement will be readily apparent to one of ordinary skill in the art.

All percentages and ratios are calculated based on the total normalization control composition unless otherwise indicated.

## *Multi-Analyte Controls*

Described herein are multi-analyte controls, compositions comprising multi-analyte controls, methods of making multi-analyte controls and methods of using multi-analyte controls. As described herein, multi-analyte controls comprise mixtures of species of organisms that have been inactivated. Without limiting the species of organisms that can be included in multi-analyte controls, viruses, bacteria, fungi and eukaryotic parasites (both unicellular and multicellular) are all envisaged as species of organisms that can be included in the mixture or species in the multi-analyte control. In some embodiments, multi-analyte controls further comprise a suitable carrier, diluent or excipient. Multi-analyte controls, and compositions comprising multi-analyte controls, are referred to interchangeably herein.

Without wishing to be bound to any particular application, the multi-analyte controls of the present disclosure have many applications in high throughput sequencing and sample analysis. In some embodiments, multi-analyte controls are used autonomously, *i.e.* without the use of the normalization controls described herein. For example, multi-analyte controls can be used a positive control during sample processing steps, such as library preparation and sequencing. In some embodiments, multi-analyte controls are used in concert with the normalization controls described herein. For example, the normalization controls can be used to normalize reads from one or more species in a multi-analyte control. Accordingly, in some embodiments, normalization controls are added to the multi-analyte control, which is processed and sequenced as described herein. As the concentrations (titers) of species in the multi-analyte control are pre-determined, multi-analyte controls can thus be used as positive controls for the normalization controls described herein. Alternatively, or in addition, normalization controls can be used to normalize reads from one or more species in the multi-analyte control, and the relationship between normalized read count and titer used to generate a calibration curve. This calibration curve can be used to determine the titer of target organism in a sample from a read count that has also been normalized using the normalization controls described herein. In some embodiments, the normalization controls are added to multi-analyte controls, and the multi-analyte controls are processed, sequenced and the reads normalized in parallel with a sample to generate a calibration curve. In some embodiments, the multi-analyte controls and normalization controls are used to generate a reference calibration curve. In some embodiments, normalization controls and multi-analyte controls are both added to a sample (i.e., spiked-in).In some embodiments, the calibration curve is used to calculate the titer of a target organism in the sample.

In some embodiments, normalization controls of the disclosure are used to normalize reads from multi-analyte controls. In some embodiments, the normalized reads from the multi-analyte controls are used to determine the titer of one or more target organisms in a sample.

Described herein are multi-analyte controls comprising a mixture of at least three different species of organisms. In some embodiments, the multi-analyte control comprises at least ten species of organisms. In some embodiments, the multi-analyte control comprises at least 4 species of organisms, at least 5 species of organisms, at least 6 species of organisms, at least 7 species of organisms, at least 8 species of organisms, at least 9 species of organisms, at least 10 species of organisms, at least 11 species of organisms, at least 12 species of organisms, at least 13 species of organisms, at least 14 species of organisms, at least 15 species of organisms, at least 16 species of organisms, at least 18 species of organisms, at least 19 species of organisms, at least 20 species of organisms, at least 25 species of organisms, at least 30 species of organisms, at least 35 species of organisms, at least 40 species of organisms, at least 45 species of organisms, at least 50 species of organisms, at least 75 species of organisms, at least 100 species of organisms, at least 150 species of organisms or at least 200 species of organisms. In some embodiments, the multi-analyte control consists essentially of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 species of organisms. In some embodiments, the multi-analyte control consists essentially of 10 species of organisms. In some embodiments, the multi-analyte control consists essentially of 11 species of organisms. In some embodiments, the organisms in the multi-analyte control have been inactivated. In some embodiments, the multi-analyte control further comprises an acceptable carrier, diluent or excipient.

In some embodiments, the multi-analyte control comprises at least 4 viruses, at least 5 viruses, at least 6 viruses, at least 7 viruses, at least 8 viruses, at least 9 viruses, at least 10 viruses, at least 11 viruses, at least 12 viruses, at least 13 viruses, at least 14 viruses, at least 15 viruses, at least 16 viruses, at least 18 viruses, at least 19 viruses, at least 20 viruses, at least 25 viruses, at least 30 viruses, at least 35 viruses, at least 40 viruses, at least 45 viruses, at least 50 viruses, at least 75 viruses, at least 100 viruses, at least 150 viruses or at least 200 viruses. In some embodiments, the multi-analyte control consists essentially of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 viruses. In some embodiments, the multi-analyte control consists essentially of 10 viruses. In some embodiments, the multi-analyte control consists essentially of 11 viruses. In some embodiments, the organisms in the multi-analyte control have been inactivated. In some embodiments, the multi-analyte control further comprises an acceptable carrier, diluent or excipient.

In some embodiments of the multi-analyte controls of the disclosure, each species in the mixture of species in the multi-analyte control is present at the same titer.

In some embodiments, each species is present in the multi-analyte control at a different titer. In some embodiments, the titers of the species of organisms in the multi-analyte control are present in a pre-determined desired ratio. If the pre-determined desired ratio is 1:1, each species is present in the same titer. In an alternative example, each organism in a multi-analyte control comprising three species can be present in a ratio of 1:5:35 or 1:20:90. Any predetermined desired ratio of titers is envisaged as within the scope of the disclosure. In some embodiments, the titers of the species of organisms in the multi-analyte control are in a linear sequence, geometric sequence or a logarithmic sequence. For example, the species in a multi-analyte control comprising three species can be present in a ratio of 1:2:3 or 1:10:100. The person of ordinary skill will be able to select pre-determined desired ratios suitable to for a particular use of the multi-analyte controls disclosed herein.

In some embodiments, at least two species are present in the multi-analyte control in the same titer. In some embodiments, for example those embodiments where the multi-analyte control comprises at least three species, at least two species are present in the multi-analyte control at the same titer and at least two species are present in different titers.

In some embodiments of the multi-analyte controls of the disclosure comprising pathogenic species, each different species of pathogen in the mixture is present at a concentration of between about 5 and 10 million infectious units per mL (IU/mL). In some embodiments, each different species in the mixture is present at a concentration of between about 50 and 5 million IU/mL, between about 500 and 5 million IU/mL, between about 500 and 1 million IU/mL, between about 500 and 100,000 IU/mL, between about 500 and 10,000 IU/mL, or between about 1,000 and 10,000 IU/mL. In some embodiments, each species is the multi-analyte control is present at 0 IU/mL, 100 IU/mL, 1,000 IU/mL, 5,000 IU/mL, 10,000 IU/mL, 100,000 IU/mL or 1,000,000 IU/mL.

In some embodiments, multi-analyte controls are added to the sample at a final concentration of between 100 IU/mL and 1,000,000 IU/mL, between 100 IU/mL and 100,000 IU/mL, between 1,000 IU/mL and 1,000,000 IU/mL, between 1,000 IU/mL and 100,000 IU/mL, between 100 IU/mL and 10,000 IU/mL or between 1,000 IU/mL and 10,000 IU/mL. In some embodiments, multi-analyte controls are added to the sample at a final concentration of 100 IU/mL, 150 IU/mL, 1,000 IU/mL, 5,000 IU/mL, 10,000 IU/mL, or 100,000 IU/mL. In some embodiments, multi-analyte controls can be added to multiple samples at a range of concentrations, for example 0 IU/mL, 100 IU/mL, 1,000 IU/mL, 5,000 IU/mL, 10,000 IU/mL, and 100,000 IU/mL. As a further example, multi-analyte controls can be added to a sample at concentrations of 1 IU/mL, 100 IU/mL, 150 IU/mL, 1,000 IU/mL, 5,000 IU/mL, 10,000 IU/mL and 100,000 IU/mL. In some embodiments, this range of concentrations is used to calculate a calibration curve for determining the titer of a target organism in a sample.

As used herein, "titer" or "physical titer" refers to the concentration of an organism in a solution. For infectious organisms, "titer" or "infectious titer" refers to concentration infectious units per volume of solution (e.g., infectious units, or IU, per mL or μL). Methods of measuring titer will be readily apparent to one of ordinary skill in the art. For example, physical titers can be measured by measuring the concentration of organisms in a solution. This can be done using direct methods such as counting, or indirect methods, for example by measuring the level of a protein specific to the organism in question. Physical titers can be indicated as numbers of cells, organisms, particles or units per volume of solution (e.g. unit/mL). Methods of measuring infectious titer will be known to the person of ordinary skill in the art. For example, infectious titer for viruses can determined using assays routine in the art such as plaque forming assays. In a plaque forming assay, dilutions of virus stock are prepared and plated onto susceptible cell monolayers, and the number of infected cells that form viral plaques are counted. Bacterial titers can be determined by plating and measuring the number colonies formed, by counting methods, spectrophotometry or by any other method known in the art.

In some embodiments of the multi-analyte controls of the disclosure, the species of organisms in the multi-analyte control comprise species of viruses, bacteria, fungi, eukaryotic parasites or a combination thereof.

In some embodiments, the species of organisms in the multi-analyte control consist essentially of species of viruses. In some embodiments, the species of organisms in the multi-analyte control consist essentially of at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of viruses. In some embodiments, the species of organisms in the multi-analyte control consist essentially of 3, 4, 5, 6, 7, 8, 9, 10, 11, 12, 13, 14, 15, 16, 17, 18, 19 or 20 species of viruses. In some embodiments, the species of organisms in the multi-analyte control consist essentially of 11 species of viruses. In some embodiments, the species of organisms in the multi-analyte control comprise at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of viruses. In some embodiments, the species of organisms in the multi-analyte control comprise 3, 4, 5, 6, 7, 8, 9, 10, 11, 12, 13, 14, 15, 16, 17, 18, 19 or 20 species of viruses. In some embodiments, the species of organisms in the multi-analyte control comprise 11 species of viruses.

In some embodiments, the species of organisms in the multi-analyte control consist essentially of species of bacteria. In some embodiments, the species of organisms in the multi-analyte control consist essentially of at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of bacteria. In some embodiments, the species of organisms in the multi-analyte control consist essentially of 3, 4, 5, 6, 7, 8, 9, 10, 11, 12, 13, 14, 15, 16, 17, 18, 19 or 20 species of bacteria. In some embodiments, the species of organisms in the multi-analyte control comprise at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of bacteria. In some embodiments, the species of organisms in the multi-analyte control comprise 3, 4, 5, 6, 7, 8, 9, 10, 11, 12, 13, 14, 15, 16, 17, 18, 19 or 20 species of bacteria.

In some embodiments, the species of organisms in the multi-analyte control consist essentially of species of fungi. In some embodiments, the species of organisms in the multi-analyte control consist essentially of at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of fungi. In some embodiments, the species of organisms in the multi-analyte control consist essentially of 3, 4, 5, 6, 7, 8, 9, 10, 11, 12, 13, 14, 15, 16, 17, 18, 19 or 20 species of fungi. In some embodiments, the species of organisms in the multi-analyte control comprise at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of fungi. In some embodiments, the species of organisms in the multi-analyte control comprise 3, 4, 5, 6, 7, 8, 9, 10, 11, 12, 13, 14, 15, 16, 17, 18, 19 or 20 species of fungi.

In some embodiments, the species of organisms in the multi-analyte control consist essentially of species of viruses and bacteria. In some embodiments, the species of organisms in the multi-analyte control consist essentially of at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of viruses and bacteria. In some embodiments, the species of organisms in the multi-analyte control consist essentially of 3, 4, 5, 6, 7, 8, 9, 10, 11, 12, 13, 14, 15, 16, 17, 18, 19 or 20 species of viruses and bacteria. In some embodiments, the species of organisms in the multi-analyte control comprise at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of viruses and bacteria. In some embodiments, the species of organisms in the multi-analyte control comprise 3, 4, 5, 6, 7, 8, 9, 10, 11, 12, 13, 14, 15, 16, 17, 18, 19 or 20 species of viruses and bacteria.

In some embodiments, the species of organisms in the multi-analyte control consist essentially of species of viruses and fungi. In some embodiments, the species of organisms in the multi-analyte control consist essentially of at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of viruses and fungi. In some embodiments, the species of organisms in the multi-analyte control consist essentially of 3, 4, 5, 6, 7, 8, 9, 10, 11, 12, 13, 14, 15, 16, 17, 18, 19 or 20 species of viruses and fungi. In some embodiments, the species of organisms in the multi-analyte control comprise at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of viruses and fungi. In some embodiments, the species of organisms in the multi-analyte control comprise 3, 4, 5, 6, 7, 8, 9, 10, 11, 12, 13, 14, 15, 16, 17, 18, 19 or 20 species of viruses and fungi.

In some embodiments, the species of organisms in the multi-analyte control consist essentially of bacteria and fungi. In some embodiments, the species of organisms in the multi-analyte control consist essentially of at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of bacteria and fungi. In some embodiments, the species of organisms in the multi-analyte control consist essentially of 3, 4, 5, 6, 7, 8, 9, 10, 11, 12, 13, 14, 15, 16, 17, 18, 19 or 20 species of bacteria and fungi. In some embodiments, the species of organisms in the multi-analyte control comprise at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of bacteria and fungi. In some embodiments, the species of organisms in the multi-analyte control comprise 3, 4, 5, 6, 7, 8, 9, 10, 11, 12, 13, 14, 15, 16, 17, 18, 19 or 20 species of bacteria and fungi.

In some embodiments, the species of organisms in the multi-analyte control consist essentially of species of viruses, bacteria and fungi. In some embodiments, the species of organisms in the multi-analyte control consist essentially of at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of viruses, bacteria and fungi. In some embodiments, the species of organisms in the multi-analyte control consist essentially of 3, 4, 5, 6, 7, 8, 9, 10, 11, 12, 13, 14, 15, 16, 17, 18, 19 or 20 species of viruses, bacteria and fungi. In some embodiments, the species of organisms in the multi-analyte control comprise at least 3, at least 4, at least 5, at least 6, at least 7, at least 8, at least 9, at least 10, at least 11, at least 12, at least 13, at least 14, at least 15, at least 20, at least 20 at least 25, at least 30, at least 35, at least 40, at least 45, at least 50, at least 75, at least 100, at least 150 or at least 200 species of viruses, bacteria and fungi. In some embodiments, the species of organisms in the multi-analyte control comprise 3, 4, 5, 6, 7, 8, 9, 10, 11, 12, 13, 14, 15, 16, 17, 18, 19 or 20 species of viruses, bacteria and fungi.

In some embodiments, the species of organisms in the multi-analyte control comprise one or more species of virus. All species of viruses are envisaged as eligible for inclusion in the multi-analyte controls of the disclosure. The viruses can be DNA viruses, RNA viruses or a combination thereof. In some embodiments, the virus is a DNA virus or an RNA virus. In some embodiments, a DNA virus comprises a double stranded DNA viral genome. In some embodiments, a DNA virus comprises a single stranded DNA viral genome. In some embodiments, an RNA virus comprises a double stranded RNA viral genome. In some embodiments, an RNA virus comprises a single stranded RNA viral genome. In some embodiments a single stranded RNA viral genome comprises a sense strand. In some embodiments a single stranded RNA viral genome comprises an anti-sense strand. In some embodiments, a virus is a type I, type II, type III, type IV, type V, type VI or type VII virus as determined by the Baltimore classification system.

In some embodiments, the multi-analyte control comprises a species of virus that is a human pathogen. Exemplary, but non limiting viruses that are human pathogens are shown in Table 2 below:

Table 2. Exemplary Human Viral Pathogens

| Virus | Genus and Family | Disease Examples |
|---|---|---|
| Adeno-associated virus | Dependovirus, Parvoviridae | None |
| Aichi virus | Kobuvirus, Picornaviridae | Gastroenteritis |
| Aleutian Disease virus | Parvovirus | Plasmacytosis |
| Australian bat lvssavirus | Lvssavirus, Rhabdoviridae | Fatal encephalitis |
| BK polvomavirus | Polvomavirus, Polvomaviridae | None |
| Banna virus | Seadornavirus, Reoviridae | Encephalitis |
| Barmah forest virus | Alphavirus, Togaviridae | Fever, joint pain |
| Bunvamwera virus | Orthobunyavirus, Bunvaviridae | Encephalitis |
| Bunvavirus La Crosse | Orthobunyavirus, Bunvaviridae | Encephalitis |
| Bunvavirus snowshoe hare | Orthobunyavirus, Bunvaviridae | Encephalitis |
| Cercopithecine herpesvirus | Lymphocryptovirus, Herpesviridae | Encephalitis |
| Chandipura virus | Vesiculovirus, Rhabdoviridae | Encephalitis |
| Chikungunva virus | Alphavirus, Togaviridae | Fever, joint pain |
| Cosavirus A | Cosavirus, Picornaviridae | |

(continued)

| Virus | Genus and Family | Disease Examples |
|---|---|---|
| Cowpox virus | Orthopoxvirus, Poxviridae | None |
| Coxsackievirus | Enterovirus, Picornaviridae | Meningitis, myocarditis, paralysis |
| Crimean-Congo hemorrhagic fever virus | Nairovirus, Bunvaviridae | Hemorrhagic fever |
| Dengue virus | Flavivirus, Flaviviridae | Hemorrhagic fever |
| Dhori virus | Thogotovirus, Orthomyxoviridae | Fever, encephalitis |
| Dugbe virus | Nairovirus, Bunvaviridae | Thrombocvtopaenia |
| Duvenhage virus | Lvssavirus, Rhabdoviridae | Fatal encephalitis |
| Eastern equine encephalitis virus | Alphavirus, Togaviridae | Encephalitis |
| Ebolavirus | Ebolavirus, Filoviridae | Hemorrhagic fever |
| Echovirus | Enterovirus, Picornaviridae | Common cold |
| Encephalomyocarditis virus | Cardiovirus, Picornaviridae | Encephalitis |
| Epstein-Barr virus | Lymphocryptovirus, Herpesviridae | Mononucleosis |
| European bat lvssavirus | Lvssavirus, Rhabdovirus | Fatal encephalitis |
| GB virus C/Hepatitis G virus | Pegivirus, Flaviviridae | None |
| Hantaan virus | Hantavirus, Bunvaviridae | Renal or respiratory syndrome |
| Hendra virus | Henipavirus, paramvxoviridae | Encephalitis |
| Hepatitis A virus | Hepatovirus, picornaviridae | Hepatitis |
| Hepatitis B virus | Orthohepadnavirus, Hepadnaviridae | Hepatitis |
| Hepatitis C virus | Hepacivirus, Flaviviridae | Hepatitis |
| Hepatitis E virus | Hepevirus, Unassigned | Hepatitis |
| Hepatitis delta virus | Deltavirus, Unassigned | Hepatitis |
| Herpes simplex virus 1 | Herpesviridae | Herpes |
| Herpes simplex virus 2 | Herpesviridae | Herpes |
| Horsepox virus | Orthopoxvirus, Poxviridae | None |
| Human adenovirus | Mastadenovirus, Adenoviridae | Respiratory, gastrointestinal |
| Human astrovirus | Mamastrovirus, Astroviridae | Gastroenteritis |
| Human coronavirus | Alphacoronavirus, Coronaviridae | Respiratory |
| Human cytomegalovirus | Cytomegalovirus, Herpesviridae | Mononucleosis, pneumonia |
| Human enterovirus 68, 70 | Enterovirus, Picornaviridae | Diarrhea, neurological disorder |
| Human herpesvirus 1 | Simplexvirus, Herpesviridae | Skin lesions |
| Human herpesvirus 2 | Simplexvirus, Herpesviridae | Skin lesions |
| Human herpesvirus 6 (A and B) | Roseolovirus, Herpesviridae | Skin lesions |
| Human herpesvirus 7 | Roseolovirus, Herpesviridae | Skin lesions |
| Human herpesvirus 8 | Rhadinovirus, Herpesviridae | Skin lymphoma |
| Human immunodeficiency virus | Lentivirus, Retroviridae | AIDS |
| Human papillomavirus 1 | Mupapillomavirus, Papillomaviridae | Skin warts |
| Human papillomavirus 2 | Alphapapillomavirus, Papillomaviridae | Skin warts |

(continued)

| Virus | Genus and Family | Disease Examples |
|---|---|---|
| Human papillomavirus 16,18 | Alphapapillomavirus, Papillomaviridae | Genital warts, |
| Human parainfluenza | Respirovirus, Paramyxoviridae | Respiratory |
| Human parvovirus B19 | Ervthrovirus, Parvoviridae | Skin lesion |
| Human respiratory syncytial virus | Pneumovirus, Paramvxoviridae | Respiratory |
| Human rhinovirus | Enterovirus, Picornaviridae | Respiratory |
| Human SARS coronavirus | Betacoronavirus, Coronaviridae | Respiratory |
| Human spumaretrovirus | Spumavirus, Retroviridae | None |
| Human T-lvmphotropic virus | Deltaretrovirus, Retroviridae | Leukemia |
| Human torovirus | Torovirus, Coronaviridae | Gastroenteritis |
| Influenza A virus | Influenzavirus A, Orthomyxoviridae | Flu |
| Influenza B virus | Influenzavirus B, Orthomyxoviridae | Flu |
| Influenza C virus | Influenzavirus C, Orthomyxoviridae | Flu |
| Isfahan virus | Vesiculovirus, Rhabdoviridae | encephalitis |
| JC polyomavirus (JC virus, human polvomavirus 2) | Polvomavirus, Polyomaviridae | Encephalitis |
| Japanese encephalitis virus | Flavivirus, Flaviviridae | Encephalitis |
| Junin arenavirus | Arenavirus, Arenaviridae | Hemorrhagic fever |
| KI Polvomavirus | Polvomavirus, Polvomaviridae | Encephalitis |
| Kunjin virus | Flavivirus, Flaviviridae | Encephalitis |
| Lagos bat virus | Lvssavirus, Rhabdoviridae | Fatal encephalitis |
| Lake Victoria marburgvirus | Marburgvirus, Filoviridae | Hemorrhagic fever |
| Langat virus | Flavivirus, Flaviviridae | Encephalitis |
| Lassa virus | Arenavirus, Arenaviridae | Hemorrhagic fever |
| Lordsdale virus | Norovirus, Caliciviridae | Gastroenteritis |
| Louping ill virus | Flavivirus, Flaviviridae | Encephalitis |
| Lymphocytic choriomeningitis virus | Arenavirus, Arenaviridae | Encephalitis |
| Machupo virus | Arenavirus, Arenaviridae | Encephalitis |
| Mavaro virus | Alphavirus, Togaviridae | Fever, joint pain |
| MERS coronavirus | Betacoronavirus, Coronaviridae | Respiratory |
| Measles virus | Morbilivirus, Paramvxoviridae | Fever, rash |
| Mengo encephalomyocarditis virus | Cardiovirus, Picornaviridae | Encephalitis |
| Merkel cell polvomavirus | Polvomavirus, Polyomaviridae | Merkel cell carcinoma |
| Mokola virus | Lvssavirus, Rhabdoviridae | Encephalitis |
| Molluscum contagiosum virus | Molluscipoxvirus, Poxviridae | Skin lesions |
| Monkeypox virus | Orthopoxvirus, Poxviridae | Skin lesions |
| Mumps virus | Rubulavirus, Paramvxoviridae | Mumps |
| Murray valley encephalitis virus | Flavivirus, Flaviviridae | Encephalitis |
| New York virus | Hantavirus, Bunvavirus | Hemorrhagic fever |
| Nipah virus | Henipavirus, Paramvxoviridae | Encephalitis |

(continued)

| Virus | Genus and Family | Disease Examples |
|---|---|---|
| Norwalk virus | Norovirus, Caliciviridae | Gastroenteritis |
| O'nyong-nyong virus | Alphavirus, Togaviridae | Fever, joint pain |
| Orf virus | Parapoxvirus, Poxviridae | Skin lesions |
| Oropouche virus | Orthobunyavirus, Bunvaviridae | Fever, joint pain |
| Pichinde virus | Arenavirus, Arenaviridae | Hemorrhagic fever |
| Poliovirus | Enterovirus, Picornaviridae | Poliomvelitis |
| Punta toro phlebovirus | Phlebovirus, Bunvaviridae | Hemorrhagic fever |
| Puumala virus | Hantavirus, Bunvavirus | Hemorrhagic fever |
| Rabies virus | Lvssavirus, Rhabdoviridae | Fatal encephalitis |
| Rift valley fever virus | Phlebovirus, Bunvaviridae | Hemorrhagic fever |
| Rosavirus A | Rosavirus, Picomaviridae | |
| Ross river virus | Alphavirus, Togaviridae | Fever, joint pain |
| Rotavirus A | Rotavirus, Reoviridae | Gastroenteritis |
| Rotavirus B | Rotavirus, Reoviridae | Gastroenteritis |
| Rotavirus C | Rotavirus, Reoviridae | Gastroenteritis |
| Rubella virus | Rubivirus, Togaviridae | Rubella |
| Sagivama virus | Alphavirus, Togaviridae | Fever, joint pain |
| Salivirus A | Salivirus, Picomaviridae | Gastroenteritis |
| Sandflv fever sicilian virus | Phlebovirus, Bunvaviridae | Hemorrhagic fever |
| Sapporo virus | Sapovirus, Caliciviridae | Gastroenteritis |
| Semliki forest virus | Alphavirus, Togaviridae | Fever, joint pain |
| Seoul virus | Hantavirus, Bunvavirus | Hemorrhagic fever |
| Simian foamv virus | Spumavirus, Retroviridae | None |
| Simian virus 5 | Rubulavirus, Paramyxoviridae | Undocumented |
| Sindbis virus | Alphavirus, Togaviridae | Fever, joint pain |
| Southampton virus | Norovirus, Caliciviridae | Gastroenteritis |
| St. louis encephalitis virus | Flavivirus, Flaviviridae | Encephalitis |
| Tick-borne powassan virus | Flavivirus, Flaviviridae | Encephalitis |
| Torque teno virus | Alphatorquevirus, Anelloviridae | None |
| Toscana virus | Phlebovirus, Bunvaviridae | Hemorrhagic fever |
| Uukuniemi virus | Phlebovirus, Bunvaviridae | Hemorrhagic fever |
| Vaccinia virus | Orthopoxvirus, Poxviridae | None |
| Varicella -zoster virus | Varicellovirus, Herpesviridae | Varicella |
| Variola virus | Orthopoxvirus, Poxviridae | Variola |
| Venezuelan equine encephalitis virus | Alphavirus, Togaviridae | Fever, joint pain |
| Vesicular stomatitis virus | Vesiculovirus, Rhabdoviridae | Encephalitis |
| Western equine encephalitis virus | Alphavirus, Togaviridae | Fever, joint pain |
| WU polvomavirus | Polyomavirus, Polvomaviridae | None |
| West Nile virus | Flavivirus, Flaviviridae | Hemorrhagic fever |

(continued)

| Virus | Genus and Family | Disease Examples |
|---|---|---|
| Yaba monkey tumor virus | Orthopoxvirus, Poxviridae | None |
| Yaba-like disease virus | Orthopoxvirus, Poxviridae | None |
| Yellow fever virus | Flavivirus, Flaviviridae | Hemorrhagic fever |
| Zika virus | Flavivirus, Flaviviridae | Fever, joint pain, rash |

In some embodiments, the species of organisms in the multi-analyte control comprise one or more species of bacteria. All species of bacteria are envisaged as eligible for inclusion in the multi-analyte controls of the disclosure. In some embodiments, the bacteria are gram positive bacteria or gram negative bacteria.

In some embodiments, the multi-analyte control comprises a species of bacteria that is a human pathogen. Exemplary, but non limiting bacteria that are human pathogens are shown in Table 3 below:

Table 3. Exemplary Human Bacterial Pathogens

| Disease | Bacterial Species |
|---|---|
| acne | Propionibacterium acne |
| Anthrax | Bacillus anthracis |
| Bacillary Angiomatosis/Hepatica peliosis | Bartonella henselae, Bartonella quintana |
| Bacterial Sepsis | Streptococcus agalactiae (group b strep.), Escherichia coli, Streptococcus pneumoniae |
| Bacterial vaginosis | Gardnerella vaginalis, Atopobium vaginae, Mycoplasma hominis, various anaerobic bacteria incl. Mobiluncus spp., Prevotella spp. |
| Balantidiasis | Balantidium coli |
| Boils | Staphylococcal spp. |
| Botulism | Clostridium botulinum |
| bronchitis | Mycoplasma pneumonia, Chlamydophila pneumoniae, S. pyogenes |
| Brucellosis | Brucella spp., Y. pestis |
| Bubonic plague | Yersinia pestis |
| Buruli ulcer | Mycobacterium ulcerans |
| Cat scratch disease | Bartonella henselae |
| Cellulitis | Streptococcus and Staphylococcus spp. |
| Chancroid | Haemophilus ducreyi |
| Chlamvdia | Chlamvdia trachomatis |
| Cholera | Vibrio cholera |
| Clostridium sordellii infection | Clostridium sordellii |
| Diarrheal illness | Camvlobacter jejuni |
| Ehrlichiosis | Ehrlichiachaffeensis, E. ewingii, E. muris eauclairensis |
| Endemic relapsing fever- | Borrelia sp. (15 different species; inc. B. hermsii, B. parkeri) |
| Flavimonas orvzihabitans infection | Flavimonas oryzihabitans |
| Folliculitis | Staphylococcus aureus; Staphylococcal spp. |
| Food poisoning | Escherichia coli, Staphylococcus aureus, Bacillus cereus, Clostridium perfringens, Clostridium botulinum |

(continued)

| Disease | Bacterial Species |
|---|---|
| Glanders | Burkholderia mallei |
| Gonorrhea | Neisseria gonorrhoeae |
| Granuloma Inguinale | Klebsiella granulomatis |
| Group B strep disease | Group B Streptococcus (GBS), or Streptococcus agalactiae |
| Haemophilus influenzae | Haemophilus influenzae |
| Hansen's disease | Mycobacterium leprae |
| health-care associated infections | Acinetobacter baumannii, Bacteroides fragilis, Burkholderia cepacia, Clostridium difficile, Clostridium sordellii, Carbapenem-resistant Enterobacteriaceae, Enterococcus faecalis, Klebsiella pneumonia, Methicillin-resistant Staphylococcus aureus, Morganella morganii, Mycobacterium abscessus Psuedomonas aeruginosa, Staphylococcus aureus, Stenotrophomonas maltophilia, Mycobacterium tuberculosis, Vancomyin-resistant Staphylococcus aureus, Vancomycin-resistant Enterococci, Streptococcus pneumoniae |
| Human granulocytic ehrlichiosis | Anaplasma phagocvtophilum |
| Human monocytic ehrlichiosis | Ehrlichia chaffeensis |
| Impetigo | Staphylococcal spp. |
| Intestinal infections | Campylobacter jejuni, Escherichia coli (EIEC), Escherichia coli (EHEC), Salmonella typhimurium, Salmonella typhi, Shigella dysenteriae type 1, Shigella sonnei/flexneri Yersinia enterocolitica, Clostridium difficile, Vibrio cholerae |
| Klebsiella infection | Klebsiella spp. |
| Legionnaires' disease | Legionella spp., Legionella pneumophila |
| Lemierre syndrome | Fusobacterium necrophorum |
| Listeria infection | Listeria monocytogenes |
| Lvme disease | Borrelia burgdorferi |
| Lvmphogranuloma Venereum | Chlamvdia trachomatis |
| Melioidosis | Burkholderia pseudomallei |
| Meningitis | Mycobacterium tuberculosis, Streptococcus agalactiae (Group b streptococcus), Escherichia coli, Listeria monocytogenes, Streptococcus agalactiae, Escherichia coli, Streptococcus pneumoniae, Haemophilus influenzae |
| Mycetoma | actinomycetes spp., Actinomadura pelletier, Actinomadura madurae |
| Mycobacterial infections | Mycobacterium Abscessus, M. avium, M. intracellulare, M. chelonae, M. fortuitum, M. Gordonae, M. Kansasii, M. malmoense, M. marinum, M. tuberculosis, M. xenopi |
| Necrotizing fasciitis | Group A. streptoccus and other spp.; S. aureus, S. pyogenes, Clostridium spp. |
| Nontuberculous mycobacterial lung disease | Mvcobacteria spp. |
| Osteomyelitis | Staphylococcus aureus, Streptococcus spp., Enterobacteriaceae |
| Pasteurella multocida infection | Pasteurella multocida |
| Pericarditis | Staphylococcus aureus, Streptococcus pneumoniae and other streptococci, Mycobacterium tuberculosis |
| Pinta | Treponema carateum |

(continued)

| Disease | Bacterial Species |
|---|---|
| Pvomvositis | Staphylococcus aureus |
| Q fever | Coxiella Burnetii |
| Rheumatic Fever | group A Streptococcusbacteria |
| Rocky mountain spotted fever | Rickettsia |
| Salmonella | Salmonella Tvphimurium |
| Sennetsu Fever | Noerickettsia sennetsu |
| Shigellosis | Shigella spp. |
| Staphylococcal food poisoning | Staphylococcus spp. |
| Stenotrophomonas maltophilia infection | Stenotrophomonas maltophilia |
| Streptococcal Group A invasive disease | Streptococcus Group A |
| Streptococcal Group B invasive disease | Streptococcus Group B |
| Syphilis | Treponema pallidum |
| Tetanus | Clostridium tetani, Clostridium tetanus |
| Tuberculosis | Mycobacterium tuberculosis |
| Tularemia | Francisella tularensis |
| Typhus | Rickettsia prowazekii, Orientia tsutsugamushi, Rickettsia tvphi |
| Ulcers | Helicobacter pylori |
| Urethritis, Cervicitis | Chlamydia trachomatis, Neisseria gonorrhoeae |
| Vibrio infection | Vibrio spp., V. parahaemolyticus, V. vulnificus, V. alginolyticus |
| Waterhouse-Friderichsen syndrome | Meningococcus spp. |
| Whipple disease | Trophervma whipplei |
| Whooping cough/ Pertussis | Bordetella pertussis |
| Yaws | Treponema pertenue |

Additional bacterial species, including representative commensal, symbiotic, pathogenic and drug resistant species, as well as species found in metagenomic samples, that can be included in multi-analyte controls are shown in Table 4 below:

Table 4. Exemplary Additional Bacterial Species

| Bacterial Genus and Species | Bacterial Genus and Species |
|---|---|
| Acinetobacter baumannii | Lactobacillus gasseri |
| Acinetobacter johnsonii | Lactobacillus jensenii |
| Acinetobacter spp. | Lactobacillus plantarum |
| Actinomyces odontolyticus | Listeria monocytogenes |
| Allobacillus halotolerans | Micrococcus luteus |
| Bacillus cereus | Mycoplasma hominis |
| Bacillus subtilis | Neisseria meningitidis |
| Bacteriodes fragilis | Porphyromonas gingivalis |
| Bacteroides vulgatus | Prevotella bivia |
| Bifidobacterium adolescentis | Prevotella melaninogenica |
| Chromobacter violaceum | Propionibacterium acnes |
| Clostridioides difficile | Proteus mirabilis |
| Clostridium beijerinckii | Providencia rettgeri |
| Clostridium perfringens | Pseudoalteromonas haloplanktis |
| Coagulase-negative staphylococci | Pseudomonas aeruginosa |
| Corynebacterium striatum | Pseudomonas fluorescens |

| Bacterial Genus and Species | Bacterial Genus and Species |
|---|---|
| Cutibacterium acnes | Pseudomonas spp. |
| Deinococcus radiodurans | Rhodobacter sphaeroides |
| Enterococcus faecalis | Salmonella enterica |
| Enterobacter cloacae | Serratia liquifaciens |
| Enterobacter spp. | Serratia marcescens |
| Escherichia coli | Serratia spp. |
| Fusobacterium nucleatum | Staphylococcus aureus |
| Gardnerella vaginalis | Staphylococcus aureus (MRSA, MSSA) |
| Haemophilus parainfluenzae | Staphylococcus epidermidis |
| Halobacillus halophilus | Streptococcus agalactiae |
| Haloferax volcanii | Streptococcus mitis |
| Helicobacter pylori | Streptococcus mutans |
| Imtechella halotolerans | Streptococcus pneumoniae |
| Klebsiella pneumoniae | Streptococcus pyogenes |
| Klebsiella spp | Streptococcus spp. |
| Lactobacillus fermentum | Veillonella parvula |
| | Yersinia enterocolitica |

In some embodiments, the multi-analyte control comprises one or more species of eukaryotic parasite. In some embodiments, the parasite is a human parasite. In some embodiments, the eukaryotic parasite is a multicellular parasite (e.g., a species of round worm). In some embodiments, the eukaryotic parasite is a single celled organism. In some embodiments, the eukaryotic parasite is a yeast or a fungus. In some embodiments, the eukaryotic parasite is a species that is not classified as a yeast or a fungus (for example, an amoeba).

In some embodiments, the multi-analyte control comprises one or more species of fungus that is a human pathogen. Exemplary, but non limiting fungi that are human pathogens are shown in Table 5 below:

Table 5. Exemplary Human Fungal Pathogens

| Fungal Species | Disease |
|---|---|
| Aspergillus spp., A. fumigatus, A. flavus, A. clavatus and others | Aspergillosis |
| Trichophyton, Epidermophvton, Microsporum, other spp. | Athlete's foot |
| Blastomyces spp. | Blastomycosis |
| Cryptococcus gattii | C. gattii infection |
| Cryptococcus neoformans | C. neoformans infection |
| Cryptococcus neoformans | C. neoformans infection |
| Candida albicans, C. auris, and other Candida spp. | Candidiasis |
| Candida and Aspergillus spp. | Endocartitis |
| Candida spp., other spp. | Fungal Eve infections |
| various spp. | Fungal nail infections |
| Histoplasma spp., H. capsulatum | Histoplasmosis |
| Cryptococcus neoformans, C. laurentii, C. albidus | Meningitis |
| Mucormycetes | Mucormycosis |
| Acremonium strictum, Aspergillus nidulans, Noetestudina rosatii, Phaeoacremonium krajdenii, Pseudallescheria boydii, Aspergillus terreus, Curvularia lunata, Cladophialophora bantiana, Exophiala jeanselmei, Leptosphaeria senegalensis, Leptosphaeria tompkinsii, Madurella grisea, Madurella mycetomatis, Pyrenochaeta romeroi, other spp. | Mycetoma |
| Pneumocystis jirovecii | Pneumocystis pneumonia |
| Pneumocystis jirovecii | Pneumonia |
| Stachvbotrvs chartarum | Respiratory problems |
| Trichophyton rubrum, Trichophyton tonsurans, Trichophyton interdigitale, and/or Trichophyton mentagrophytes, Microsporum canis, Epidermophyton floccosum | Ringworm |
| Sporothrix | Sporotrichosis |
| Talaromvces marneffei | Talaromvcosis |
| Coccidioides spp. | Valley Fever |

Additional fungal species that can be included in multi-analyte controls of the disclosure include, but are not limited to Cryptococcus neoformans and Saccharomyces cerevisiae.

In some embodiments, the multi-analyte control comprises a species of eukaryotic parasite. Exemplary, but non limiting eukaryotic parasites are shown in Table 6 below:

Table 6. Exemplary Human Eukaryotic Parasites

| Eukaryotic Parasite | Disease |
| --- | --- |
| Chistosoma mansoni, S. haematobium, S. japonicum, S. mekongi, S. intercalatum | Shistasomiasis |
| Filarioidea spp., Wuchereria bancrofti, Brugia malavi, Brugia timori | Filliariasis |
| Trypanosoma brucei | Trypanasomiasis |
| Leishmania makor | Leishmaniasis |
| Entamoeba histolytica | Amebiasis |
| Babesia bovis, B. divergens | Babesiosis |
| Trypanosoma cruzi | Chagas disease |
| Giradia lamblia | Giradiasis |
| Acanthamoeba culbertsoni | Granulomatous ameobic encephalitis |
| Necator americanus | Hookworm Infection |
| Bruga malavi | Lvmphatic filariasis |
| Plasmodium falciparum, P. vivax | Malaria |
| Toxoplasma gondii | Toxoplasmosis |
| Trichomonas vaginalis | Trichomoniasis |

In exemplary embodiments, Table 7 (adapted from the CDC's "Summary of Notifiable Diseases - United States, 2019" accessed on February 18, 2016 at wwwn.cdc.gov/nndss/conditions/notifiable/2019/) provides further exemplary disease causing pathogens that can be quantified using the methods and compositions described herein.

Table 7. Exemplary Disease-causing pathogens

| | |
|---|---|
| Anthrax pathogens | Listeriosis |
| Arboviral pathogens, neuroinvasive and nonneuroinvasive | Lujo virus |
| Babiosis | Lyme disease |
| Botulism pathogens | Malaria |
| Brucellosis pathogens | Marburg virus |
| California serogroup virus | Measles |
| California serogroup virus diseases | Meningococcal disease |
| Campyloberiosis | Mumps |
| Candida auris | New World Arenavirus |
| Chancroid | Novel influenza A virus infections |
| Chikungunya virus disease | Pertussis |
| Chlamydia trachomatis infection | Plague |
| Cholera | Poliomyelitis, paralytic |
| Cholera | Poliovirus infection, nonparalytic |
| Coccidioidomycosis | Powassan virus |
| Crimean-Congo hemorrhagic fever virus | Psittacosis |
| Cryptosporidiosis | Q fever |
| Cyclosporiasis | Rabies |
| Dengue fever | Rubella |
| Dengue hemorrhagic fever | Rubella, congenital syndrome |
| Dengue shock syndrome | Salmonellosis |
| Dengue Virus Infection | Severe acute respiratory syndrome-associated coronavirus (SARS-CoV) disease |

| | |
|---|---|
| Diphtheria | Shiga toxin-producing E. coli (STEC) |
| Eastern equine encephalitis virus | Shigellosis |
| Ebola virus | Smallpox |
| Ehrlichiosis/Anaplasmosis | Spotted fever rickettsiosis |
| Foodborne pathogens | St. Louis encephalitis virus |
| Giardiasis | Streptococcal toxic-shock syndrome |
| Gonorrhea | Streptococcus pneumoniae, invasive disease |
| Haemophilus influenzae, invasive disease | Syphilis |
| Hansen disease (leprosy) | Syphilis, congenital |
| Hantavirus pulmonary syndrome | Tetanus |
| Hemolytic uremic syndrome, post-diarrheal | Toxic-shock syndrome (other than streptococcal) |
| Hepatitis A, acute | Trichinellosis |
| Hepatitis B virus, perinatal infection | Tuberculosis |
| Hepatitis B, acute | Tularemia |
| Hepatitis B, chronic | Typhoid fever |
| Hepatitis C, acute | Vancomycin-intermediate (VISA) infection |
| Hepatitis C, chronic | Vancomycin-resistant (VRSA) infection |
| Hepatitis, viral | Varicella |
| Human Immunodeficiency Virus (HIV) | Vibriosis |
| Influenza-associated pediatric mortality | Viral hemorrhagic fevers |
| Lassa virus | West Nile virus |
| Legionellosis | Western equine encephalitis virus |
| Leptospirosis | Yellow fever |
| | Zika virus |

In some embodiments of the multi-analyte controls of the disclosure, the different species of organisms in the multi-analyte control are human pathogens. Exemplary species of human pathogens include any of the species listed in Tables 2-3 and 5-7. Using human known pathogens in the multi-analyte control allows the multi-analyte control to model the behavior of a target species in a sample whose titer is to be determined used the methods of the disclosure. The effectiveness of nucleic acid extraction, pathogen genome size, efficiency of library preparation, the number of sequence reads, and the composition of the sample all can affect the degree to which read counts from high throughput sequencing libraries reflect the initial titer of a target organism in a sample. A multi-analyte control comprising species similar to (e.g., same genera) or identical to target species in a sample allows the multi-analyte control to effectively control for parameters that affect the accuracy with which normalized reads reflect titer in the sample. In some embodiments, the multi-analyte control comprises one or more species of a target organism in a sample. The target organism can be known to be in the sample, or suspected to be in the sample (for example, the multi-analyte control comprises a panel of common human pathogens).

In some embodiments, the human pathogens comprise human pathogens that are commonly found in tissues used in blood or tissue transplants. Transplant acquired infections are a significant cause or morbidity and mortality in transplant recipients. Accordingly, the multi-analyte controls of the disclosure can be used as positive controls in screening transplant tissue and blood for the presence of one or more human pathogens, thereby mitigating the risk of infection from transplanted tissue or blood. Exemplary pathogens commonly found in human blood or tissue transplants include, but are not limited to, Cytomegalovirus (CMV), Epstein-Barr Virus (EBV), Adenovirus (ADV), BK Virus (BKV), JC Polyomavirus (JCV), Human Herpesvirus 6A (HHV6A), Human Herpesvirus 6B (HHV6B), Herpes simplex Virus type 1 (HSV1), Herpes simplex Virus type 2 (HSV2), Varicella-Zoster Virus (VZV) and Human Parvovirus B19 (B19), Human Immunodeficiency Virus (HIV), Hepatitis B, Hepatitis C, Human T-Lymphotropic Virus Types I and II (HTLV), West Nile virus, Zika virus, *Streptococcus species, Staphylococcus aureus, Enteroccocus faecalis, Bacillus cereus, Propionibacterium acnes, Serratia liquifaciens, Serratia marcescens, Yersinia enterocolitica, Enterobacter spp., Acinetobacter spp., Pseudomonas spp., E. coli, Klebsiella pneumonia, Proteus mirabilis, A. baumannii, Bacillus cereus, coagulate negative staphylococci, Streptococcus spp., Klebsiella spp., Serratia spp. Providencia rettgeri, Treponema pallidum, Trypanosoma cruzi* and *Babesia microti.*

In some embodiments, the multi-analyte control comprises or consists essentially of a mixture of 11 species of virus. An exemplary multi-analyte control comprises, or consists essentially of Cytomegalovirus (CMV), Epstein-Barr Virus (EBV), Adenovirus (ADV), BK Virus (BKV), JC Polyomavirus (JCV), Human Herpesvirus 6A (HHV6A), Human Herpesvirus 6B (HHV6B), Herpes simplex Virus type 1 (HSV1), Herpes simplex Virus type 2 (HSV2), Varicella-Zoster Virus (VZV) and Human Parvovirus B 19 (B19). In some embodiments, the multi-analyte control comprises or consists essentially of a mixture of 10 species of virus. An exemplary multi-analyte control comprises, or consists essentially of CMV (*e.g.* strain AD169), EBV, ADV (*e.g.* type 1), BKV (*e.g.* subtype 1b-2), JCV (*e.g.* type 1a), HHV6A (*e.g.* strain GS), HHV6B (*e.g.* strain Z-29), HSV1 (*e.g.* strain 95), HSV2 (*e.g.* strain 09) and VZV (*e.g.* strain 9/84). In some embodiments, the multi-analyte control comprises or consists essentially of CMV, EBV, ADV, BKV, JCV, HHV6A, HHV6B, HSV1, HSV2, and VZV. A further exemplary multi-analyte control comprises, or consists essentially of, Measles virus, West Nile virus, Zika virus, yellow fever virus, Sindbis virus, Variola virus, Norwalk virus, Rabies virus and human Rhinovirus. A yet further exemplary multi-analyte control comprises, or consists essentially of, Epstein-Barr virus, Human cytomegalovirus, Human respiratory syncytial virus, Variola virus, Influenza virus, Rubella virus, Mumps virus and Human SARS coronavirus.

Exemplary mixtures of organisms that can make up a multi-analyte control of the disclosure is shown in Table 8 below:

Table 8. Exemplary Multi-Analyte Controls

| Exemplary Multi-Analyte Control | Species |
|---|---|
| Virus Panel | Cytomegalovirus (CMV), Epstein-Barr Virus (EBV), Adenovirus (ADV), BK Virus (BKV), JC Polyomavirus (JCV), Human Herpesvirus 6A (HHV6A), Human Herpesvirus 6B (HHV6B), Herpes simplex Virus type 1 (HSV1), Herpes simplex Virus type 2 (HSV2), Varicella-Zoster Virus (VZV), Human Parvovirus B19 (B19) |
| Virus Panel | CMV, Hepatitis B, Hepatitis C, Vaccinia virus, Rubella virus, Rotavirus A, Rotavirus B, Mumps virus, Measles Virus, Influenza A, Influenza B, Influenza C, Human herpesvirus 6 |
| Virus Panel | Ebola virus, Variola, Yellow Fever Virus, Rabies virus, Machupo virus, Lassa virus, HIV, Dengue virus, Chickingunya virus |
| Virus Panel | CMV, EBV, ADV, BKV, JCV, HHV6A, HHV6B, HSV1, HSV2 and VZV |
| Virus + Bacteria Panel | CMV,EBV, ADV, BKV, JCV, HHV6A, HHV6B, HSV1, HSV2, VZV, B19, Staphylococcus aureus, Enteroccocus faecalis, Bacillus cereus, Propionibacterium acnes, Serratia liquifaciens, Serratia marcescens, Yersinia enterocolitica |
| Virus, Bacterial and Fungal Panel | CMV,EBV, ADV, BKV, JCV, HHV6A, HHV6B, HSV1, HSV2, VZV, B19, Staphylococcus aureus, Enteroccocus faecalis, Bacillus cereus, Propionibacterium acnes, Serratia liquifaciens, Serratia marcescens, Yersinia enterocolitica, Candida albicans, Aspergillis fumigatus, Cryptococcus neoformans |
| Multi-organism Panel | CMV,EBV, ADV, BKV, JCV, HHV6A, HHV6B, HSV1, HSV2, VZV, B19, Staphylococcus aureus, Enteroccocus faecalis, Bacillus cereus, Propionibacterium acnes, Serratia liquifaciens, Serratia marcescens, Yersinia enterocolitica, Candida albicans, Aspergillis fumigatus, Cryptococcus neoformans, Trypanosoma brucei, Leishmania makor, Giardia lamblia |

In some embodiments of the multi-analyte controls of the disclosure, the at least three different species of organisms in the multi-analyte control are not human pathogens. In some embodiments, the at least three different species of organisms in the multi-analyte control are not human pathogens, but are in the same genera as species that are human pathogens. Species in the same genera frequently have similar physical properties - for example, bacteria in the same genera may have similar cell walls, and viruses may have similar viral proteins. Species in the same genera can be expected to behave similarly when undergoing the sample processing methods described herein. By matching the species in multi-analyte controls to the same genera human pathogens, for example human pathogens commonly found in transplant tissues or blood, the multi-analyte controls may mimic the behavior of those human pathogenic species in the sample during sample processing. This could result in the superior ability of the methods of the disclosure to calculate the titer of one or more target organisms in a sample, for example a pathogen in a human clinical sample, using the multi-analyte controls and normalization controls of the disclosure.

In some embodiments of the multi-analyte controls of the disclosure, the species in the multi-analyte control comprise a mixture of species that are human pathogens and species that are not human pathogens.

In some embodiments, the multi-analyte control further comprises plasma. The plasma can be from any vertebrate animal. In some embodiments, the plasma is from a human, non-human primate, mouse, rat, rabbit, dog, cat, gerbil, sheep, pig, horse, cow or donkey. In some embodiments, the plasma is human plasma. In some embodiments, the plasma is synthetically produced.

Described herein are collections of nucleic acids extracted from any one of the multi-analyte controls of the disclosure. Methods of extracting nucleic acids can be extracted from the multi-analyte controls described herein will be readily apparent to one of ordinary skill in the art. Exemplary methods include, but are not limited to phenol/chloroform extraction followed by precipitation with ethanol or a similar suitable solvent, and commercially available kits such as the Qiagen and Zymo mini-prep kits. Nucleic acids extracted from multi-analyte controls can be used in any of the embodiments described herein. For example, nucleic acids extracted from multi-analyte controls can be added to a sample at the library preparation step, or processed and sequenced in parallel to the sample and used as a positive control or to generate a calibration curve.

Methods of Making Multi-Analyte Controls

Described herein are methods of making the multi-analyte controls described herein. In some embodiments, the method comprises: (a) selecting a panel of organisms to include in the multi-analyte control; (b) culturing each organism under appropriate culture conditions; (c) inactivating each organism and (d) mixing a predetermined amount of each inactivated organism with a suitable carrier, diluent or excipient to produce a multi-analyte control.

Appropriate methods of inactivating organisms in the multi-analyte control will be known to one of ordinary skill in the art. For example, many viruses can be inactivated with dry heat, steam, or a low pH, such as a pH of less than 4.0. Enveloped viruses can be inactivated by the addition of a solvent or detergent, which disrupts the viral envelope. Viruses can also be inactivated by exposure to chemical cross-linking agents such as formaldehyde. Methods of inactivating bacteria include ultraviolet light, radiation, heat, low PH, and treatment with chemicals such as ethylene oxide, formaldehyde and guanidium thiocyanate. Single celled eukaryotes, such as amoeba or yeast, can be inactivated with heat, or the application of chemical cross-linking agents such as formaldehyde.

In some embodiments, the organisms have been inactivated by chemical treatment, heat treatment, pH treatment or ultraviolet irradiation. In some embodiments, the chemical treatment comprises formaldehyde.

In some embodiments, known quantities of the inactivated organisms in the multi-analyte control are added to plasma. Plasma can be isolated from blood, *e.g.* by removal of blood cells, platelets and other cellular components by centrifugation and/or filtration. The plasma can be from any vertebrate animal. In some embodiments, the plasma is from a human, non-human primate, mouse, rat, rabbit, gerbil, dog, cat, cow, sheep, pig, horse or donkey. In some embodiments, the plasma is from a human. Adding the inactivated organisms to plasma allows the multi-analyte controls to closely mimic the behavior of experimental samples, such as human clinical samples comprising human plasma, during sample processing.

Methods of Using Multi-Analyte Controls

Described herein are methods of using the multi-analyte controls described herein. In some embodiments, multi-analyte controls of the disclosure can be processed in parallel with samples, for example clinical or experimental samples. Multi-analyte controls processed in in parallel with samples can be used to generate a calibration curve that is used ordinary skill in the art to determine the titer of one or more organisms in a sample comprising a mixture of organisms. For example, the sample can be a clinical sample comprising cells from a human host, and cells or particles from a non-host such as a viral, bacteria or fungal pathogen. In alternative embodiments, multi-analyte controls are added directly to a sample, such as a clinical or experimental sample, and are used as an internal standard to calculate the titer of one or more organisms in a sample comprising a mixture of organisms. In some embodiments, multi-analyte controls are used as positive controls for the methods of the disclosure.

In some embodiments, the multi-analyte controls are processed in parallel with, but separate from, a sample, to generate a high throughput sequencing library and a collection of reads therefrom. In some embodiments, normalization controls added to the multi-analyte control and the sample are used to normalize the reads from the multi-analyte control and the sample. In those embodiments where the multi-analyte control and the sample are processed in parallel (*i.e.,* the multi-analyte control is not "spiked into" the sample), the multi-analyte control comprises species that are human pathogens, species that are not human pathogens, or a combination thereof. In some embodiments, the normalized reads from the multi-analyte control are used to generate a calibration curve, and the calibration curve is used to calculate the titer of a target organism in the sample.

In some embodiments of the methods of the disclosure, the methods are used to quantify the level of expression of at least one target sequence for each species of organisms in the multi-analyte control. In some embodiments, the methods comprise (a) mixing a known amount of the normalization control with the multi-analyte control, (b) preparing a high throughput sequencing library, (c) sequencing said library to produce a collection of reads, (d) mapping reads from the collection of reads to the multi-analyte control or the normalization control, (e) determining the number of reads produced by each of the groups or subgroups of polynucleotides in the normalization control, (f) calculating a relationship between the starting concentration of each of the groups or subgroups of polynucleotides in the normalization control mixed with the multi-analyte control in (a) and the number of reads produced in (c), (g) modeling a relationship between reads and concentration of the at least one target sequence for each species in the multi-analyte control, and (h) calculating an initial concentration of the at least one target sequence for each species in the multi-analyte control from the number of reads produced by the target sequence using the model in (g). In some embodiments, the method further comprises extracting nucleic acids from the multi-analyte control. In some embodiments, the mixing step of (a) occurs prior to the library preparation step of (b), at the same time as the library preparation step of (b) or after as the library preparation step of (b). In some embodiments, the mixing step of (a) occurs prior to extracting nucleic acids from the sample.

In some embodiments, each of the different species of organisms in the multi-analyte control comprises at least one target sequence. In some embodiments, the at least one target sequence is a different target sequence for each species of organisms in the multi-analyte control. In some embodiments, each species in the multi-analyte control comprises a different target sequence or target sequences. In some embodiments, the at least one target sequence from each species of organisms in the multi-analyte control has less than 99% identity, less than 95% identity, less than 90% identity, less than 85% identity, less than 80% identity, less than 75% identity, less than 70% identity, less than 60% identity, less than 50% identity, less than 40% identity, less than 30% identity or less than 20% identity to the target sequence from any or more of the other species of organisms in the multi-analyte control.

In some embodiments the at least one target sequence from each species of organisms in the multi-analyte control is identical to, has less than 99% identity, less than 95% identity, less than 90% identity, less than 85% identity, less than 80% identity, less than 75% identity, less than 70% identity, less than 60% identity, less than 50% identity, less than 40% identity, less than 30% identity, less than 20% identity, less than 10% identity or less than 5% identity to a target sequence from the sample. In some embodiments, at least one target sequence from a species of organisms in the multi-analyte control is identical to a target sequence from the sample. In some embodiments, the multi-analyte control comprises at least one species that comprises a target sequence identical to a target sequence from the sample, and at least one species that comprises a target sequence that is not identical to a target sequence from the sample.

In some embodiments, the at least one target sequence for each species of organisms in the multi-analyte control comprises a genomic sequence. In some embodiments, the at least one target sequence from each species of organisms in the multi-analyte control comprises or consists of a reference genome of that species. In some embodiments, the at least one target sequence from each species of organisms in the multi-analyte control comprises or consists of a transcriptome of that species. In some embodiments, the at least one target sequence from each species of organisms in the multi-analyte control comprises a portion of a reference genome of that species. The at least one target sequence can be protein coding sequence, or non-coding sequence.

In some embodiments, mapping reads from the collection of reads to the multi-analyte control or the normalization control at step (d) further comprises mapping reads to each of the species of organisms in the multi-analyte control (d). Methods of mapping reads from a collection of high-throughput sequencing reads will be readily apparent to one of ordinary skill in the art. For example, where each organism in the multi-analyte control has a reference genome, reads from the collection of reads generated by high-throughput sequence can be mapped to the reference genome via BWA, Novoalign, Bowtie, SOAP2, BFAST), SSAHA2, MPscan, GASSST) or PerM algorithms. In some embodiments, mapping the reads to reference genome for each organism in the multi-analyte control comprises mapping the reads to the at least one target sequence for each species of organisms in the multi-analyte control.

In some embodiments, calculating the initial concentration of the at least one target sequence at (h) comprises calculating an initial concentration of at least one target sequence for each species in the multi-analyte control. The initial concentration of the at least on target sequence for each species in the multi-analyte control can be calculated from the relationship between read count and the initial concentration of each of the groups or subgroups in the normalization control that is determined using the normalization control compositions and methods of the disclosure.

Described herein are methods of quantifying the titer of a target organism in a sample. In some embodiments, the methods comprise (a) providing a sample comprising the target organism, wherein the target organism comprises at least one target sequence; (b) providing a multi-analyte control comprising known titers of at least three species of organisms, wherein the organisms have been inactivated; (c) mixing a known amount of the normalization control of the disclosure with the sample and with the multi-analyte control; (d) preparing high throughput sequencing libraries from the sample and the at least one multi-analyte control; (e) sequencing said libraries to produce a collection of sample reads and a collection of multi-analyte control reads; (f) normalizing the collection of sample reads and the collection of multi-analyte control reads from (e) using the normalization controls; (g) determining a relationship between normalized reads and the known titers of the species of organisms in the multi-analyte control; and (h) calculating a titer the target organism in the sample using the relationship determined in (g).

In some embodiments, the species of organisms each comprise at least one species-specific target sequence. In some embodiments, the at least one species-specific target sequence is different in each species in the multi-analyte control.

In some embodiments, normalizing the reads from the multi-analyte control comprises: (i) mapping reads from the collection of sample reads to the sample or the normalization control; (ii) mapping reads from the collection of multi-analyte control reads to the multi-analyte control or the normalization control; (iii) determining the number of reads produced by each of the groups or subgroups of polynucleotides in the normalization control for the collection of sample reads and the collection of multi-analyte control reads; (iv) calculating a relationship between the starting concentration of each of the groups or subgroups of polynucleotides in the normalization control and the number of reads produced at step (e) for both the sample and the multi-analyte control; and (v) determining the relationship between reads and concentration in the sample and in the multi-analyte control.

In some embodiments, determining the relationship between reads and titer in the multi-analyte control and the sample comprises: (i) calculating an initial concentration of the at least one species-specific target sequence from each of the species in the multi-analyte control; and (ii) calculating an initial concentration of the at least one target sequence in the sample. In some embodiments, the mapping at step (i) further comprises mapping the collection of sample reads to a reference genome. In some embodiments, the reference genome does not comprise the target sequence. In some embodiments, the reference genome is a human genome. For example, in a human clinical sample comprising human host cells and a pathogen, the pathogen comprising a target sequence, reads generated from the high-throughput sequencing library from the sample are mapped to the human genome as a reference genome. Reads that map to and align with the human genome are from nucleic acids isolated from the human cells, and do not comprise the target sequence. Reads that do not map to the human reference genome come from the target sequence, and are normalized using the methods of the disclosure. An advantage of this method is that the identity of the target organism that produces the target sequence does not necessarily need to be known ahead of time. Methods of the disclosure can be used to efficiently determine both the presence and titer of an unknown target organism in a sample, such as an unknown pathogen in a human clinical sample.

In some embodiments, the mapping at step further comprises mapping the collection of sample reads to a more than one reference genome. In some embodiments, a first reference genome does not comprise the target sequence, and a second reference genome comprises the target sequence. For example, in a human clinical sample comprising human host cells and a pathogen, the pathogen comprising a target sequence, reads generated from the high-throughput sequencing library from the sample are mapped to the human genome, which does not comprise the target sequence, and a second reference genome corresponding to the pathogen, which comprises the target sequence. In some embodiments, the mapping further comprises mapping to a species-specific reference genome for each of the species of organisms in the multi-analyte control.

In some embodiments, the relationship between normalized reads and the known titers of species of organisms in the multi-analyte control is a linear relationship. In some embodiments, the relationship is logarithmic. Methods of determining the relationship between normalize reads and known titers species of organisms in the multi-analyte control will be readily apparent to one of ordinary skill in the art.

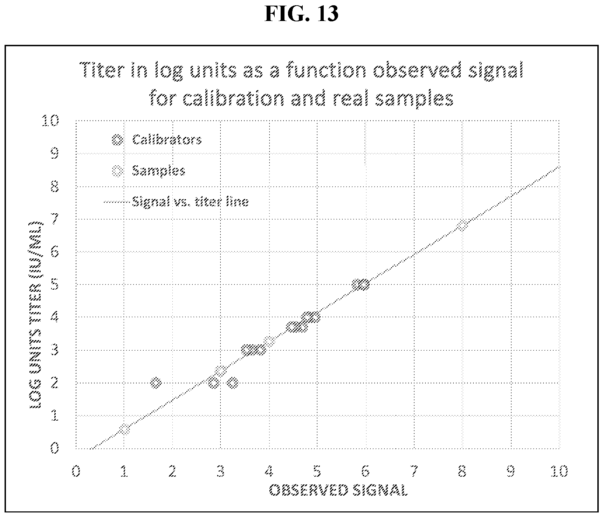

In some embodiments, multi-analyte controls are used to generate a calibration curve, which is used to determine the titer of an organism in a sample from normalized read count. An exemplary calibration curve is shown in FIG. 13, and shows as signal (normalized reads), versus log titer of the organisms in the multi-analyte controls used to generate the calibration curve. In some embodiments, the calibration curve is generated in parallel with a sample or samples (*e.g.* clinical or experimental samples). In some embodiments, the calibration curve is generated from multi-analyte controls that are analyzed at multiple different concentrations. For example, an exemplary calibration curve can be generated from multi-analyte controls diluted 1:10, 1:100, 1: 1000 etc. and then sequenced. In some embodiments, the calibration curve is generated from multi-analyte controls provided at 2 concentrations, at 3 concentrations, at 4 concentrations, at 5 concentrations, at 6 concentrations, at 7 concentrations, at 8 concentrations, at 9 concentrations or at 10 concentrations. In some embodiments, the calibration curve comprises a reference calibration curve. For example, normalized reads from a target sequence in a sample can be compared to a reference calibration curve to determine the titer of an organism in the sample corresponding to the target sequence. In some embodiments, for example those embodiments that employ a reference calibration curve, one or more multi-analyte controls are processed in parallel with the sample to validate the calibration curve and/or serve as positive controls. For example, two multi-analyte controls, at a high and a low concentration, are processed in parallel with the sample.

In some embodiments of the methods of the disclosure, the methods further comprise mixing a known amount of a multi-analyte control with the sample (a spike-in method). The multi-analyte controls are used as an internal standard to calculate the titer of one or more organisms in a sample comprising a mixture of organisms. For example, the sample can be a human clinical sample comprising host cells (human cells) and one or more of viral particles, or cells from bacterial or fungal pathogens (non-host). The multi-analyte control comprises organisms that are not human pathogens, and are therefore distinguishable from human pathogens in the sample using sequence differences.

In some embodiments, the multi-analyte control comprises organism that are in the same genera as human pathogens. Organisms that are related to human pathogens will behave similarly to human pathogens in the sample during processing, and thus provide a superior metric for calculating the titer of human pathogens in the sample. In some embodiments, an organism in the multi-analyte control comprises a target sequence with less than 99% identity, less than 95% identity, less than 90% identity, less than 85% identity, less than 80% identity, less than 75% identity, less than 70% identity, less than 60% identity, less than 50% identity, less than 40% identity, less than 30% identity or less than 20% identity to a sequence of a human pathogen.

*Formulations*

Normalization control and multi-analyte control compositions of the disclosure can be formulated in any acceptable carrier, diluent or excipient known in the art.

Normalization controls can be provided in solutions at any acceptable concentration, precipitate, or as a lyophilized composition. Nucleic acids, such as normalization controls, may be suspended in solutions including, but not limited to water, Tris buffer, Tris-EDTA buffer and Tris-EDTA with up to 0.5 M NaCl.

Exemplary buffers used in formulation of the disclosure include acetate, sulfate, hydrochloride, phosphate or free acid forms of Tris-(hydroxymethyl)aminomethane (TRIS), although alternative buffers of the same approximate ionic strength and pKa as TRIS may be used with equivalent results. In addition to the buffer salts, cofactor salts such as those of potassium (*e.g.*, potassium chloride or potassium acetate) and magnesium (*e.g.*, magnesium chloride or magnesium acetate) are included in the compositions.

Inactivated organisms in multi-analyte controls can be supplied in solution, freeze dried or lyophilized. If freeze dried or lyophilized, multi-analyte controls can be re-suspended in an appropriate volume of any acceptable carrier. Acceptable carriers include, but are not limited to water, phosphate buffered saline (PBS) or Ringer's solution.

*Methods of Depletion or Enrichment*

Normalization controls, multi-analyte controls, and methods of using same can be combined with the methods of enriching a sample for a target sequence of interest or depleting a sample of sequences targeted for depletion. In some embodiments, the methods of enrichment or depletion comprise nucleic acid-guided nuclease based methods, nucleotide modification based methods, or a combination thereof.

Accordingly, the disclosure provides methods of quantifying or identifying a target sequence in a sample comprising depleting the sample of sequences targeted for depletion, adding the normalization controls described herein, and sequencing the sample using NGS. Normalization controls can be added before, during or after enrichment of target sequences of interest or depletion of nucleic acids targeted for depletion.

In some embodiments, normalization controls are added to the sequencing sample prior to enrichment of target sequences of interest or depletion of sequences targeted depletion. In some embodiments, normalization controls can be designed to mimic those properties of target sequences of interest that are used to distinguish those sequences from sequences targeted for depletion. For example, if target sequences of interest are enriched using a nucleic acid-guided nuclease and a plurality of guide nucleic acids (gNAs) that target the sequences targeted for depletion, the normalization controls do not comprise sequences targeted by the plurality of gNAs. As a further example, if sequences targeted for depletion are depleted using nucleotide-modification based methods, normalization controls may comprise nucleotide modifications similar to the modifications of the targeted sequences of interest that are enriched.

Nucleotide Modification Based Methods

Described herein are methods of enriching a sample for target nucleic acids of interest relative to nucleic acids targeted for depletion, comprising using differences in nucleotide modification between the target nucleic acids of interest and the nucleic acids targeted for depletion. In some embodiments, the target nucleic acids comprise the target sequences in the sequencing sample, whose presence and/or titer are to be determined using the normalization controls, and optionally, multi-analyte controls of the instant disclosure. Normalization controls comprising the same nucleotide modifications as the target sequences of interest can be added to the sample prior to modification based depletion methods. Alternatively, normalization controls can be added to the sample following modification-based depletion.

Any type of nucleotide modification is envisaged as within the scope of the disclosure. Exemplary but non-limiting examples of nucleotide modifications of the disclosure are described below.

Nucleotide modifications used by the methods of the disclosure can occur on any nucleotide (adenine, cytosine, guanine, thymine or uracil, *e.g.*). These nucleotide modifications can occur on deoxyribonucleic acids (DNA) or ribonucleic acids (RNA). These nucleotide modifications can occur on double or single stranded DNA molecules, or on double or single stranded RNA molecules.

In some embodiments, the nucleotide modification comprises adenine modification or cytosine modification.

In some embodiments, the adenine modification comprises adenine methylation. In some embodiments, the adenine methylation comprises N6-methyladenine (6mA). In some embodiments, the adenine methylation comprises Dam methylation carried out by the *Deoxyadenosine methylase.* In some embodiments, the adenine methylation comprises EcoKI methylation. In some embodiments, the adenine modification comprises adenine modified at $N^6$ by glycine (momylation).

In some embodiments, the modification comprises cytosine modification. In some embodiments, the cytosine modification comprises 5-methylcytosine (5mC), 5-hydroxymethlcytosine (5hmC), 5-formylcytosine (5fC), 5-carboxylcytosine (5caC), 5-glucosylhydroxymethylcytosine (5ghmC) or 3-methylcytosine (3mC). In some embodiments, the cytosine methylation comprises 5-methylcytosine (5mC) or N4-methylcytosine (4mC). In some embodiments, the cytosine methylation comprises Dcm methylation, DNMT1 methylation, DNMT3A methylation or DNMT3B methylation. In some embodiments, the cytosine methylation comprises CpG methylation, CpA methylation, CpT methylation, CpC methylation or a combination thereof. In some embodiments, the cytosine methylation comprises CpG methylation. For example, CpG methylation can be used to selectively target an active region in a mammalian genome for depletion using the methods of the disclosure.

In some embodiments, the cytosine modification comprises 5-hydroxymethylcytosine (5hmC), 5-formylcytosine (5fC). 5-formylcytosine is an oxidized derivative of 5mC, 5-carboxylcytosine (5caC), 5-glucosylhydroxymethylcytosine or 3-methylcytosine.

In some embodiments of the methods of the disclosure, the methods employ at least a first modification-sensitive restriction enzyme and a second modification-sensitive restriction enzyme. In some embodiments, the first and second modification-sensitive restriction enzymes are the same. In some embodiments, the first and second modification-sensitive restriction enzymes are not the same. In some embodiment, the first or second modification-sensitive restriction enzyme is a single species of restriction enzyme (*e.g.*, AluI, or McrBC, but not both). In some embodiments, the first or second modification-sensitive restriction enzyme is a mixture of 2 or more species of modification- sensitive restriction enzymes (*e.g.*, a mixture of FspEI and AbaSI). In some embodiments of the methods of the disclosure, more than two different methods are combined, each using a different modification-sensitive restriction enzyme or cocktail of modification-sensitive restriction enzymes.

The term "modification-sensitive restriction enzyme", as used herein, refers to a restriction enzyme that is sensitive to the presence of modified nucleotides within or adjacent to the recognition site for the restriction enzyme. Alternatively, or in addition, the modification-sensitive restriction enzyme can be sensitive to modified nucleotides within the recognition site itself. The modification-sensitive restriction enzyme can be sensitive to modified nucleotides that are adjacent to the recognition site, for example, within 1-50 nucleotides, 5' or 3' of the recognition site. Nucleotide modifications of the disclosure can be within the recognition site itself, or comprise nucleotides adjacent to the recognition site (for example, within 1-50 nucleotides, 5' or 3' of the recognition site, or both).

Exemplary modifications capable of blocking or reducing the activity of modification-sensitive restriction enzymes include, but are not limited to, N6-methyladenine, 5-methylcytosine (5mC), 5-hydroxymethlcytosine (5hmC), 5-formylcytosine (5fC), 5-carboxylcytosine (5caC), 5-glucosylhydroxymethylcytosine, 3-methylcytosine (3mC), N4-methylcytosine (4mC) or combinations thereof. Exemplary modifications capable of blocking modification-sensitive restriction enzymes include modifications mediated by Dam, Dcm, EcoKI, DNMT1, DNMT3A, DNMT3B and TET enzymes.

In some embodiments, the modification comprises Dam methylation. Restriction enzymes that are blocked by Dam methylation include, but are not limited to, AlwI, BcgI, BclI, BsaBI, BspDI, BspEI, BspHI, ClaI, DpnII, HphI, Hpy188I, Hpy188III, MboI, MboII, NruI, Nt.AlwI, Taqα I and XbaI.

In some embodiments, the modification comprises Dcm methylation. Restriction enzymes that are blocked by Dcm methylation include, but are not limited to, Acc65I, AlwNI, ApaI, AvaI, AvaII, BanI, BsaI, BsaHI, BslI, BsmFI, BssKI, BstXI, EaeI, Esp3I, EcoO109I, MscI, NlaIV, PflMI, PspGI, PspOMI, Sau96I, ScrFI, SexAI, SfiI, SfoI and StuI.In some embodiments, the modification comprises CpG methylation. Restriction enzymes that are blocked by CpG methylation include, but are not limited to, AatII, AccII, AciI, AclI, AfeI, AgeI, Aor13HI, Aor51HI, AscI, AsiSI, AluI, AvaI, BceAI, BmgBI, BsaI, BsaHI, BsiEI, BsiWI, BsmBI, BspDI, BspT104I BsrFalphaI, BssHII, BstBI BstUI, Cfr10I, ClaI, CpoI, EagI, Esp3I, Eco52I, FauI, FseI, FspI, HaeII, HgaI, HhaI, HpaII, HpyCH4IV, Hpy99I, KasI, MluI, NaeI, NgoMIV, NotI, NruI, Nt.BsmAI, Nt.CviPII, NsbI, PmaCI, Psp1406I, PluTI, PmlI, PvuI, RsrII SacII, SalI, SmaI, SnaBI, SfoI, SgrAI, SmaI, SrfI, Sau3AI, TspMI and ZraI.

In some embodiments, a modification-sensitive restriction enzyme is active at a recognition site comprising at least one modified nucleotide and is not active at a recognition site that does not comprise at least one modified nucleotide. Exemplary modifications recognized by modification-sensitive restriction enzymes that cleave at recognition sites comprising one or more modified nucleotides include, but are not limited to, $N^6$-methyladenine, 5-methylcytosine (5mC), 5-hydroxymethlcytosine (5hmC), 5-formylcytosine (5fC), 5-carboxylcytosine (5caC), 5-glucosylhydroxymethylcytosine, 3-methylcytosine (3mC), N4-methylcytosine (4mC) or combinations thereof. Exemplary modifications recognized modification-sensitive restriction enzymes that specifically cleave recognition sites comprising one or more modified nucleotides include modifications mediated by Dam, Dcm, EcoKI, DNMT1, DNMT3A, DNMT3B and TET enzymes.

Exemplary but non-limiting modification-sensitive restriction enzymes that cleave at a recognition site comprising one or more modified nucleotides within or adjacent to the recognition site include, but are not limited to AbaSI, DpnI, FspEI, LpnPI, MspJI and McrBC.

In some embodiments, the modification comprises 5-glucosylhydroxymethylcytosine and the modification-sensitive restriction enzyme comprises AbaSI. AbaSI cleaves an AbaSI recognition site comprising a glucosylhydroxymethylcytosine, and does not cleave an AbaSI recognition site that does not comprise a glucosylhydroxymethylcytosine.

In some embodiments, the nucleotide modification comprises 5-hydroxymethylcytosine and the modification-sensitive restriction enzyme comprises AbaSI and T4 phage β-glucosyltransferase. T4 Phage β-glucosyltransferase specifically transfers the glucose moiety of uridine diphosphoglucose (UDP-Glc) to the 5-hydroxymethylcytosine (5-hmC) residues in double-stranded DNA, for example, within the AbaSI recognition site, making a glucosylhydroxymethylcytosine modified AbaSI recognition site. AbaSI cleaves an AbaSI recognition site comprising glucosylhydroxymethylcytosine and does not cleave an AbaSI recognition site that does not comprise a glucosylhydroxymethylcytosine.

In some embodiments, the nucleotide modification comprises methylcytosine and the modification-sensitive restriction enzyme comprises McrBC. McrBC cleaves McrBC sites comprising methylcytosines, and does not cleave McrBC sites that do not comprise methylcytosines. The McrBC site can be modified with methylcytosines on one or both DNA strands. In some embodiments, McrBC also cleaves McrBC sites comprising hydroxymethylcytosines on one or both DNA strands. In some embodiments, the McrBC half sites are separated by up to 3,000 nucleotides. In some embodiments, the McrBC half sites are separated by 55-103 nucleotides.

In some embodiments, the modification comprises adenine methylation and the methods comprise digestion with DpnI. DpnI cleaves a GATC recognition site when the adenines on both strands of the GATC recognition are methylated. In some embodiments, DpnI GATC recognition sites comprising both adenine methylation and cytosine modification occur in bacterial DNA, but not in mammalian DNA. These recognition sites comprising both methylated adenines and modified cytosines can be selectively cleaved by DpnI in a sample (*e.g.*, of mixed bacterial and mammalian DNA), and then treated with T4 polymerase to replace methylated adenines and modified cytosines at the cleaved ends with unmodified adenines and cytosines. T4 polymerase catalyzes the synthesis of DNA in the 5' to 3' direction, in the presence of a template, primer and nucleotides. T4 polymerase will incorporate unmodified nucleotides into the newly synthesized DNA. This produces a sample that now comprises unmodified cytosines in the nucleic acids of interest and modified cytosines in the nucleic acids targeted for depletion. These differences in modified cytosines can be used to enrich for nucleic acids of interest using the methods of the disclosure.

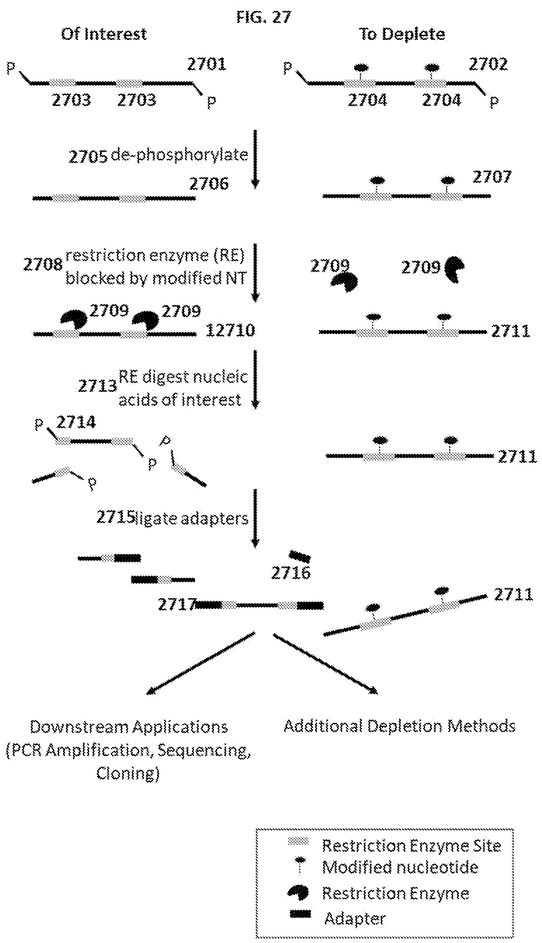

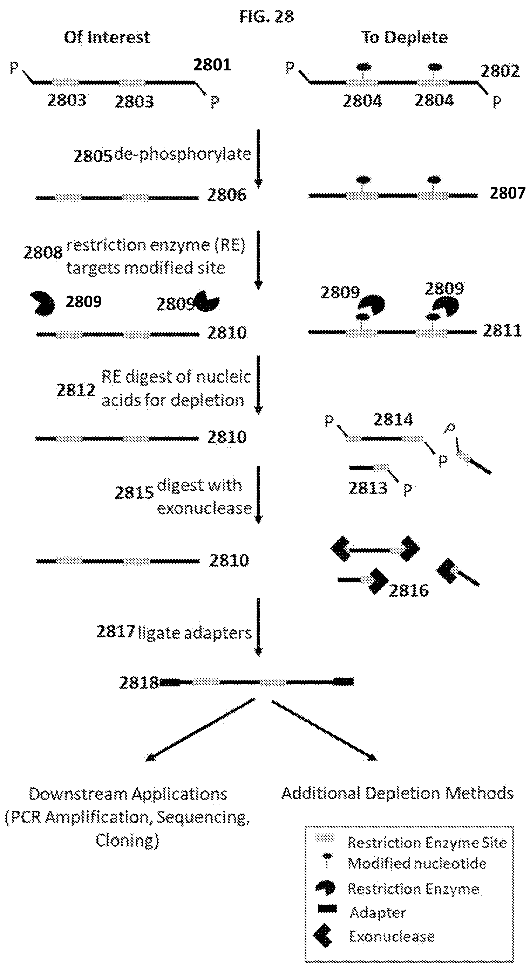

In some embodiments of the methods of the disclosure, the nucleic acids in the sample are terminally dephosphorylated, so that contacting the nucleic acids in the sample with a modification-sensitive restriction enzyme produces either nucleic acids of interest or nucleic acids targeted for depletion with exposed terminal phosphates than can be used in the methods of the disclosure to enrich the sample for nucleic acids of interest. For example, these exposed terminal phosphates can be used to target the nucleic acids for depletion for degradation by an exonuclease (FIG. 28 or the nucleic acids of interest for adapter ligation (FIG. 27).

As used herein, the term "terminally dephosphorylated" refers to nucleic acids that have had the terminal phosphate groups removed from the 5' and 3' ends of the nucleic acid molecule. In some embodiments, the nucleic acids in the sample are terminally dephosphorylated using a phosphatase, such as an alkaline phosphatase. Exemplary phosphatases of the disclosure include, but are not limited to shrimp alkaline phosphatase (SAP), recombinant shrimp alkaline phosphatase (rSAP), calf intestine alkaline phosphatase (CIP) and Antarctic phosphatase.

As used herein, the term "exonuclease" refers to a class of enzymes successively remove nucleotides from the 3' or 5' ends of a nucleic acid molecule. The nucleic acid molecule can be DNA or RNA. The DNA or RNA can be single stranded or double stranded. Exemplary exonucleases include, but are not limited to Lambda nuclease, Exonuclease I, Exonuclease III and BAL-31. Exonucleases can be used to selectively degrade nucleic acids targeted for depletion using the methods of the disclosure (FIG. 28, *e.g.*).

Described herein are adapters that are ligated to the 5' and 3' ends of the nucleic acids in the sample or the nucleic acids of interest, and optionally, the normalization controls. In some embodiments, the adapters are ligated to normalization control sequences. In other embodiments, normalization controls comprising adapter sequences are synthesized *de novo.*

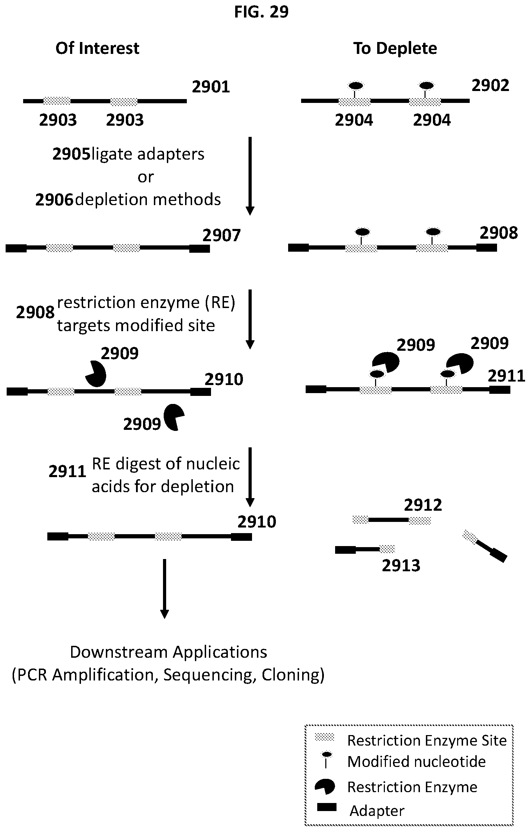

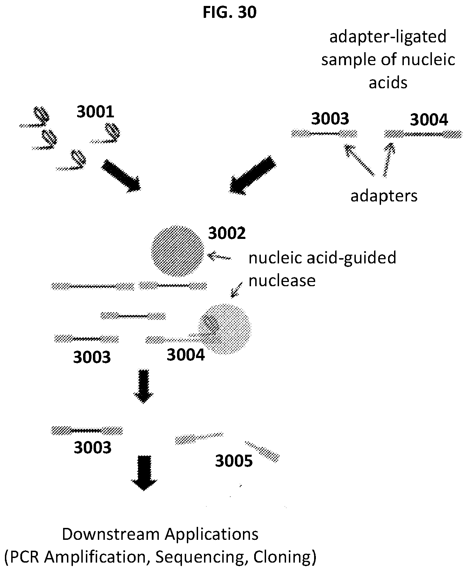

In some embodiments of the methods of the disclosure, adapters are ligated to all the nucleic acids in the sample, and then differences in nucleotide modification are used to selectively cleave the nucleic acids targeted for depletion, producing nucleic acids of interest that are adapter ligated on both ends and nucleic acids targeted for depletion that are adapter ligated on one end (FIG. 29, FIG. 30). In some embodiments, differences in nucleotide modification are used to selectively deplete the nucleic acids targeted for depletion, and then adapters are ligated to the target nucleic acids of interest (FIG. 28). In some embodiments, differences in nucleotide modification are used to produce nucleic acids of interest with exposed terminal phosphates, which are used to ligate adapters to the target nucleic acids of interest (FIG. 27).

In some embodiments of the methods of the disclosure, adapters are ligated to the 5' and 3' ends of the nucleic acids in the sample. In some embodiments, the adapters further comprise intervening sequence between the 5' terminal end and/or the 3' terminal end.

In some embodiments the adapter is a nucleic acid that is ligatable to both strands of a double-stranded DNA molecule.

In some embodiments, adapters are ligated prior to depletion/enrichment. In other embodiments, adapters are ligated at a later step.

Non-limiting examples of adapter include linear, linear Y-shaped, or hairpin adapters. In some embodiments, the adapters comprise a polyG sequence.

In various embodiments the adapter may be a hairpin adapter *i.e.,* one molecule that base pairs with itself to form a structure that has a double-stranded stem and a loop, where the 3' and 5' ends of the molecule ligate to the 5' and 3' ends of the double-stranded DNA molecule of the fragment, respectively.

Alternately, the adapter may be a Y-adapter ligated to one end or to both ends of a fragment, also called a universal adapter. Alternately, the adapter may itself be composed of two distinct oligonucleotide molecules that are base paired with one another. Additionally, a ligatable end of the adapter may be designed to be compatible with overhangs made by cleavage by a restriction enzyme, or it may have blunt ends or a 5' T overhang. In some embodiments, the restriction enzyme is a modification-sensitive restriction enzyme.

The adapter may include double-stranded as well as single-stranded molecules. Thus, the adapter can be DNA or RNA, or a mixture of the two. Adapters containing RNA may be cleavable by RNase treatment or by alkaline hydrolysis.

Adapters can be 10 to 100 bp in length although adapters outside of this range are usable without deviating from the present disclosure.

An adapter may be configured for a next generation sequencing platform, for example for use on an Illumina sequencing platform such as HiSeq or MiSeq, or for use on an IonTorrent platform, or for use with Nanopore technology. In some embodiments, the adapters comprise sequencing adapters (*e.g.*, Illumina sequencing adapters). In some embodiments, the adapters comprise unique molecular identifier (UMI) sequences, sometimes referred to as barcodes. In some embodiments, the UMI sequences comprise a sequence that is unique to each original nucleic acid molecule (*e.g.*, a random sequence). In some embodiments, the adapters comprise multiple distinct sequences, such as a UMI unique to each nucleic acid molecule, a barcode shared among nucleic acid molecules from a particular source, and a sequencing adapter.

The nucleic acids targeted for depletion can be depleted by differential adapter attachment. In some embodiments, adapters are attached to nucleic acids of a sample, and subsequently one or more adapters are removed from nucleic acids targeted for depletion based on their modification status. For example, nucleic acids targeted for depletion with adapters attached to both ends can be cleaved by a modification-sensitive restriction enzyme, thereby producing nucleic acids targeted for depletion with adapters attached to only one end. Subsequent steps (*e.g.*, amplification) can be used to target only nucleic acids with adapters attached to both ends, thereby depleting the nucleic acids targeted for depletion. In another example, the nucleic acids of the sample are treated (*e.g.,* by dephosphorylation) such that only cleaved nucleic acids are able to have adapters attached; subsequently, nucleic acids of interest can be cleaved by a modification-sensitive restriction enzyme (*e.g.*, thereby exposing a phosphate group) and adapters can be attached. Subsequent steps (*e.g.*, amplification) can be used to target only nucleic acids with adapters attached, thereby depleting the nucleic acids targeted for depletion.

The nucleic acids targeted for depletion can be depleted by digestion, such as digestion with an exonuclease.

The nucleic acids targeted for depletion can be depleted by size selection. For example, a modification-sensitive restriction enzyme can be used to cleave either the nucleic acids of interest or the nucleic acids targeted for depletion, and subsequently the nucleic acids of interest can be separated from the nucleic acids targeted for depletion based on size differences due to the cleavage.

In some cases, the nucleic acids targeted for depletion are depleted without the use of size selection.

Protocol 1: Exemplary methods of the application described herein are depicted in **FIG. 27.** A sample of nucleic acids comprising target nucleic acids of interest (**2701**) and nucleic acids targeted for depletion (**2702**), and optionally normalization controls, is terminally dephosphorylated (**2705**) to produce unphosphorylated nucleic acids of interest (**2706**) and nucleic acids targeted for depletion (**2707**). In some embodiments, the nucleic acids are fragmented prior to dephosphorylation. In some embodiments, the nucleic acids in the sample are terminally dephosphorylated with a phosphatase, for example recombinant shrimp alkaline phosphatase (rSAP). In some embodiments, both the nucleic acids of interest and the nucleic acids targeted for depletion comprise one or more recognition sites for a modification-sensitive restriction enzyme (**2703**, **2704,** respectively). In the nucleic acids of interest, the recognition sites for the modification-sensitive restriction enzyme do not comprise modified nucleotides (**2703**), or alternatively, contain modified nucleotides less frequently than the corresponding recognition sites of the nucleic acids targeted for depletion. In the nucleic acids targeted for depletion, the recognition sites for the modification-sensitive restriction enzyme comprise modified nucleotides within or adjacent to the restriction site (**2704**), or alternatively, comprise modified nucleotides more frequently than the corresponding recognition sites of the nucleic acids of interest. Activity of the modification-sensitive restriction enzyme (**2709**) is blocked by the presence of modified nucleotides within or adjacent to its cognate recognition site (**2708**), thereby targeting the activity of the modification-sensitive restriction enzyme to the nucleic acids of interest (compare **2710** and **2711**). In some embodiments, the modification-sensitive restriction enzyme (**2709**) comprises AatII, AccII, Aor13HI, Aor51HI, BspT104I, BssHII, Cfr10I, ClaI, CpoI, Eco52I, HaeII, HapII, HhaI, MluI, NaeI, NotI, NruI, NsbI, PmaCI, Psp1406I, PvuI, SacII, SalI, SmaI, SnaBI, AluI or Sau3AI. In some embodiments, the modification-sensitive restriction enzyme (**2709**) comprises AluI or Sau3AI. Digesting the sample with the modification-sensitive restriction enzyme (**2713**) produces nucleic acids of interest with terminal phosphates at the 5' and 3' ends of the terminal phosphates (**2714**). These terminal phosphates are used to ligate adapters (**2715**, ligation step; **116,** adapters) to the ends of the nucleic acids of interest, producing nucleic acids of interest that are adapter ligated on both ends (**2717**). In contrast, the nucleic acids targeted for depletion are not adapter ligated (**2711**). These adapters can be used for downstream applications, for example adapter-mediated PCR amplification, sequencing (*e.g.* high throughput sequencing), and quantification of the nucleic acids of interest in the sample and/or cloning. This depletes the nucleic acids targeted for depletion by selectively ligating adapters to the nucleic acids of interest. This depletion can be accomplished without the use of size selection. Alternatively, the adapter ligated nucleic acids of interest are subjected to one or more of the additional enrichment methods described herein. For example, the adapter ligated nucleic acids are subjected to additional modification-dependent enrichment methods of the disclosure (for example, the methods depicted in **FIG. 29**). Alternatively, or in addition, the adapter ligated nucleic acids are subjected to nucleic acid-guided nuclease based enrichment methods of the disclosure (for example, the methods depicted in **FIG. 30**).

Protocol 2: Exemplary methods of the application described herein are depicted in **FIG. 28.** A sample of nucleic acids comprising target nucleic acids of interest (**2801**) and nucleic acids targeted for depletion (**2802**), and optionally normalization controls, is terminally dephosphorylated (**2805**) to produce unphosphorylated nucleic acids of interest (**2806**) and nucleic acids targeted for depletion (**2807**). In some embodiments, the nucleic acids are fragmented prior to dephosphorylation. In some embodiments, the nucleic acids in the sample are terminally dephosphorylated with a phosphatase, for example recombinant shrimp alkaline phosphatase (rSAP). In some embodiments, both the nucleic acids of interest and the nucleic acids targeted for depletion comprise one or more recognition sites for a modification-sensitive restriction enzyme (**2803** and **2804,** respectively). In the nucleic acids of interest, the recognition sites for the modification-sensitive restriction enzyme do not comprise modified nucleotides (**2803**), or alternatively, contain modified nucleotides less frequently than the corresponding recognition sites of the nucleic acids targeted for depletion. In the nucleic acids targeted for depletion, the recognition sites for the modification-sensitive restriction enzyme comprise modified nucleotides within or adjacent to the restriction site (**2804**), or alternatively, comprise modified nucleotides more frequently than the corresponding recognition sites of the nucleic acids of interest. The modification-sensitive restriction enzyme (**2809**) cuts its cognate recognition site when there are one or more modified nucleotides within or adjacent to the recognition site (**2808**), and does not cut its cognate recognition site when the recognition site does not comprise one or more modified nucleotides (**2808**), thereby targeting the activity of the modification-sensitive restriction enzyme to the nucleic acids targeted for depletion (compare **2810** and **2811**). In some embodiments, the modification-sensitive restriction enzyme comprises AbaSI, FspEI, LpnPI, MspJI or McrBC. In some embodiments, the modification-sensitive restriction enzyme is FspEI. In some embodiments, the modification-sensitive restriction enzyme is MspJI. Digestion of the sample with the modification-sensitive restriction enzyme (**2812**) produces nucleic acids targeted for depletion with terminal phosphates one end (**2813**) or both the 5' and 3' ends of the nucleic acid (**2814**). In contrast, the nucleic acids of interest, which were not cut by the modification-sensitive restriction enzyme, do not have exposed terminal phosphates at the 5' and or 3' ends of the nucleic acids (compare **2810** with **2813-2814**). The sample is then digested with an exonuclease (**2815**, digestion step; **2816** exonuclease) which uses the terminal phosphates in the nucleic acids targeted for depletion to remove successive nucleotides from the ends of the nucleic acids molecules, thus depleting the nucleic acids targeted for depletion from the sample. This depletion can be accomplished without the use of size selection. Following exonuclease digestion, adapters are ligated to the nucleic acids of interest (**2817**), which, lacking terminal phosphates, have not been digested by the exonuclease. This produces nucleic acids of interest that are adapter ligated on both ends (**2818**). These adapters can be used for downstream applications, for example adapter-mediated PCR amplification, sequencing (*e.g.* high throughput sequencing), and quantification of the nucleic acids of interest in the sample and/or cloning. Alternatively, the adapter ligated nucleic acids of interest are subjected to one or more of the additional enrichment methods described herein. For example, the adapter ligated nucleic acids are subjected to additional modification-dependent enrichment methods of the disclosure (for example, the methods depicted in **FIG. 29**). Alternatively, or in addition, the adapter ligated nucleic acids are subjected to nucleic acid-guided nuclease based enrichment methods of the disclosure (for example, the methods depicted in **FIG. 30**).

Protocol 3: Exemplary methods of the application described herein are depicted in **FIG. 29.** A sample of nucleic acids comprising nucleic acids of interest (**2901**) and nucleic acids targeted for depletion (**2902**), and optionally normalization controls, is adapter-ligated (**2905**), or is subjected to enrichment methods of the disclosure (**2906**) (*e.g.,* the methods depicted in **FIG. 27** or **FIG. 28**) that produce adapter-ligated nucleic acids of interest (**2907**) and adapter-ligated nucleic acids targeted for depletion (**2908**). In some embodiments, both the nucleic acids of interest and the nucleic acids targeted for depletion comprise one or more recognition sites for a modification-sensitive restriction enzyme (**2903** and **2904**, respectively). In the nucleic acids of interest, the recognition sites for the modification-sensitive restriction enzyme do not comprise modified nucleotides (**2903**), or alternatively, contain modified nucleotides less frequently than the corresponding recognition sites of the nucleic acids targeted for depletion. In the nucleic acids targeted for depletion, the recognition sites for the modification-sensitive restriction enzyme comprise modified nucleotides within or adjacent to the restriction site (**2904**), or alternatively, comprise modified nucleotides more frequently than the corresponding recognition sites of the nucleic acids of interest. The modification-sensitive restriction enzyme (**2909**) cuts its cognate recognition site when there are one or more modified nucleotides within or adjacent to the recognition site (**2908**), and does not cut its cognate recognition site when the recognition site does not comprise one or more modified nucleotides (**2908**), thereby targeting the activity of the modification-sensitive restriction enzyme to the nucleic acids targeted for depletion (compare **2910** and **2911**). In some embodiments, the modification-sensitive restriction enzyme comprises AbaSI, FspEI, LpnPI, MspJI or McrBC. In some embodiments, the modification-sensitive restriction enzyme is FspEI. In some embodiments, the modification-sensitive restriction enzyme is MspJI. The sample is digested with the modification-sensitive restriction enzyme (**2911**), producing nucleic acids targeted for depletion that are not adapter ligated (**2912**), or are adapter ligated on only one end (**2913**). This depletes the nucleic acids targeted for depletion by selectively removing adapters from the nucleic acids targeted for depletion. This depletion can be accomplished without the use of size selection. In contrast, the nucleic acids of interest, which were not cut by the modification-sensitive restriction enzyme, are adapter ligated on both ends (contrast **2910** with **2912-2913**). These adapters can be used for downstream applications, for example adapter-mediated PCR amplification, sequencing (*e.g.* high throughput sequencing), and quantification of the nucleic acids of interest in the sample and/or cloning.

Protocol 4: Exemplary methods of the application described herein are depicted in **FIG. 30.** A plurality of gNAs (**3001**) are used to target a nucleic acid-guided nuclease (**3002**) to nucleic acids targeted for depletion (**3003**) in a sample of adapter-ligated nucleic acids. The adapter ligated nucleic acids are generated by any of the methods of enrichment described herein that use modification-sensitive restriction enzymes to deplete nucleic acids targeted for depletion from a sample, either before or after an initial adapter ligation. In this method, the gNAs are specifically targeted to the nuclei acids targeted for depletion (**3003**), and not the nucleic acids of interest (**3004**), which are therefore not cut by the nucleic acid-guided nuclease (**3002**). Cleavage by the nucleic acid-guided nuclease results in nucleic acids targeted for depletion that are adapter ligated on one end (**3005**), and nucleic acids of interest that are adapter ligated on both ends (**3003**). These adapters can be used for downstream applications, for example adapter-mediated PCR amplification, sequencing (*e.g.* high throughput sequencing), quantification of the nucleic acids of interest in the sample and cloning.

Any of the methods described herein can be used as a stand-alone method to deplete nucleic acids targeted for depletion from a sample, thereby enriching for nucleic acids of interest. Alternatively, the methods described herein can be combined to achieve a greater degree of enrichment than any individual method in alone.

While particular combinations of methods, and orders of combinations of methods, are described herein, these are in no way intended to limit the ways in which the methods of the disclosure can be combined. Any method of enriching a sample for nucleic acids of interest of the disclosure that produces adapter ligated nucleic acids of interest as a product of the method can be combined with any additional methods of the disclosure that use adapter ligated nucleic acids as its starting substrate.

Nucleic Acid-Guided Nuclease based Enrichment Methods

Described herein are nucleic acid-guided nuclease based methods for enriching for target sequences that can be combined with the normalization controls and multi-analyte controls, and optionally other methods of enrichment or depletion described herein. Nucleic acid-guided nuclease based enrichment methods are methods that employ nucleic acid-guided nucleases to enrich a sample for sequences of interest. Nucleic acid-guided nuclease based enrichment methods are described in WO/2016/100955, WO/2017/031360, WO/2017/100343, WO/2017/147345 and WO/2018/227025 the contents of each of which are herein incorporated by reference in their entirety.

Normalization and optionally, multi-analyte controls, can be added to the sample prior to subjecting the sample to nucleic acid-guided nuclease based methods, or after. In some embodiments, the normalization controls comprise sequences that are not the same as the sequences of the targeting sequences of the gNAs described herein.

The term "nucleic acid-guided nuclease-gNA complex" refers to a complex comprising a nucleic acid-guided nuclease protein and a guide nucleic acid (gNA, for example a gRNA or a gDNA). For example, the "Cas9-gRNA complex" refers to a complex comprising a Cas9 protein and a guide RNA (gRNA). The nucleic acid-guided nuclease may be any type of nucleic acid-guided nuclease, including but not limited to a wild type nucleic acid-guided nuclease, a catalytically dead nucleic acid-guided nuclease, or a nucleic acid-guided nuclease-nickase. When the nucleic-acid guided nuclease is a CRISPR/Cas nucleic-acid guided nuclease, the complex can be referred to as a "CRISPR/Cas system protein-gNA complex."

Methods of the present disclosure can utilize nucleic acid-guided nucleases. As used herein, a "nucleic acid-guided nuclease" is any nuclease that cleaves DNA, RNA or DNA/RNA hybrids, and which uses one or more guide nucleic acids (gNAs) to confer specificity. Nucleic acid-guided nucleases include CRISPR/Cas system proteins as well as non-CRISPR/Cas system proteins. The nucleic acid-guided nucleases described herein can be DNA guided DNA nucleases; DNA guided RNA nucleases; RNA guided DNA nucleases; or RNA guided RNA nucleases. The nucleases can be endonucleases. The nucleases can be exonucleases. In one embodiment, the nucleic acid-guided nuclease is a nucleic acid-guided-DNA endonuclease. In one embodiment, the nucleic acid-guided nuclease is a nucleic acid-guided-RNA endonuclease.

In some embodiments, the modification-based enrichment methods and the nucleic acid-guided nuclease based enrichment methods of the disclosure deplete different nucleic acids in the sample, thereby achieving a greater degree of enrichment for the nucleic acids of interest than either approach alone.

Described herein are pluralities (interchangeably referred to as libraries, or collections) of guide nucleic acids (gNAs). In some embodiments, the normalization controls do not comprise a sequence that is identical to or highly similar to a sequence of any of the gNAs in the plurality of gNAs.

The term "guide nucleic acid" refers to a guide nucleic acid (gNA) that is capable of forming a complex with a nucleic acid guided nuclease, and optionally, additional nucleic acid(s). The gNA may exist as an isolated nucleic acid, or as part of a nucleic acid-guided nuclease-gNA complex, for example a Cas9-gRNA complex.

As used herein, a plurality of gNAs denotes a mixture of gNAs containing at least $10^2$ unique gNAs. In some embodiments a plurality of gNAs contains at least $10^2$ unique gNAs, at least $10^3$ unique gNAs, at least $10^4$ unique gNAs, at least $10^5$ unique gNAs, at least $10^6$ unique gNAs, at least $10^7$ unique gNAs, at least $10^8$ unique gNAs, at least $10^9$ unique gNAs or at least $10^{10}$ unique gNAs. In some embodiments a collection of gNAs contains a total of at least $10^2$ unique gNAs, at least $10^3$ unique gNAs, at least $10^4$ unique gNAs or at least $10^5$ unique gNAs.

In some embodiments, a collection of gNAs comprises a first NA segment comprising a targeting sequence; and a second NA segment comprising a nucleic acid-guided nuclease system (*e.g.*, CRISPR/Cas system) protein-binding sequence. In some embodiments, the first and second segments are in 5'- to 3'-order'. In some embodiments, the first and second segments are in 3'- to 5'-order'.

In some embodiments, the size of the first segment varies from 12-100 bp, 12-75 bp, 12-50 bp, 12-30 bp, 12-25 bp, 12-22 bp, 12-20 bp, 12-18 bp, 12-16 bp, 14-250 bp, 14-100 bp, 14-75 bp, 14-50 bp, 14-30 bp, 14-25 bp, 14-22 bp, 14-20 bp, 14-18 bp, 14-17 bp, 14-16 bp, 15-250 bp, 15-100 bp, 15-75 bp, 15-50 bp, 15-30 bp, 15-25 bp, 15-22 bp, 15-20 bp, 15-18 bp, 15-17 bp, 15-16 bp, 16-250 bp, 16-100 bp, 16-75 bp, 16-50 bp, 16-30 bp, 16-25 bp, 16-22 bp, 16-20 bp, 16-18 bp, 16-17 bp, 17-250 bp, 17-100 bp, 17-75 bp, or 17-50 bp, 17-30 bp, 17-25 bp, 17-22 bp, 17-20 bp, 17-18 bp, 18-250 bp, 18-100 bp, 18-75 bp, 18-50 bp, 18-30 bp, 18-25 bp, 18-22 bp, 18-20 bp, 19-250 bp, 19-100 bp, 19-75 bp, or 19-50 bp, 19-30 bp, 19-25 bp, or 19-22 bp across the plurality of gNAs. In some particular embodiments, the size of the first segment is 15 bp, 16 bp, 17 bp, 18 bp, 19 bp, or 20 bp.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the first segments in the plurality are 15-50 bp.

In some embodiments, the plurality of gNAs comprises targeting sequences which can base-pair with a target sequence in the nucleic acids targeted for depletion, wherein the target sequence in the nucleic acids targeted for depletion is spaced at least every 1 bp, at least every 2 bp, at least every 3 bp, at least every 4 bp, at least every 5 bp, at least every 6 bp, at least every 7 bp, at least every 8 bp, at least every 9 bp, at least every 10 bp, at least every 11 bp, at least every 12 bp, at least every 13 bp, at least every 14 bp, at least every 15 bp, at least every 16 bp, at least every 17 bp, at least every 18 bp, at least every 19 bp, 20 bp, at least every 25 bp, at least every 30 bp, at least every 40 bp, at least every 50 bp, at least every 100 bp, at least every 200 bp, at least every 300 bp, at least every 400 bp, at least every 500 bp, at least every 600 bp, at least every 700 bp, at least every 800 bp, at least every 900 bp, at least every 1000 bp, at least every 2500 bp, at least every 5000 bp, at least every 10,000 bp, at least every 15,000 bp, at least every 20,000 bp, at least every 25,000 bp, at least every 50,000 bp, at least every 100,000 bp, at least every 250,000 bp, at least every 500,000 bp, at least every 750,000 bp, or even at least every 1,000,000 bp across a genome or transcriptome targeted for depletion in the sample.

In some embodiments, the plurality of gNAs comprises a first NA segment comprising a targeting sequence; and a second NA segment comprising a nucleic acid-guided nuclease system (*e.g.*, CRISPR/Cas system) protein-binding sequence, wherein the gNAs in the plurality can have a variety of second NA segments with various specificities for protein members of the nucleic acid-guided nuclease system (*e.g.*, CRISPR/Cas system). For example a collection of gNAs as provided herein, can comprise members whose second segment comprises a nucleic acid-guided nuclease system (*e.g.*, CRISPR/Cas system) protein-binding sequence specific for a first nucleic acid-guided nuclease system (*e.g.*, CRISPR/-Cas system) protein; and also comprises members whose second segment comprises a nucleic acid-guided nuclease system (*e.g.*, CRISPR/Cas system) protein-binding sequence specific for a second nucleic acid-guided nuclease system (*e.g.*, CRISPR/Cas system) protein, wherein the first and second nucleic acid-guided nuclease system (*e.g.*, CRISPR/Cas system) proteins are not the same. In some embodiments, a plurality of gNAs as provided herein comprises members that exhibit specificity for a Cas9 protein and another protein selected from the group consisting of Cpf1, Cas3, Cas8a-c, Cas10, CasX, CasY, Cas13, Cas14, Cse1, Csy1, Csn2, Cas4, Csm2, and Cm5. The order of the first NA segment comprising a targeting sequence and the second NA segment comprising a nucleic acid-guided nuclease system protein-binding sequence will depend on the nucleic acid-guided nuclease system protein. The appropriate 5' to 3' arrangement of the first and second NA segments and choice of nucleic acid-guided nuclease system proteins will be apparent to one of ordinary skill in the art.

In some embodiments the gNAs comprise DNA and RNA. In some embodiments, the gNAs consist of DNA (gDNAs). In some embodiments, the gNAs consist of RNA (gRNAs).

In some embodiments, the gNA comprises a gRNA and the gRNA comprises two sub-segments, which encode for a crRNA and a tracrRNA. In some embodiment, the crRNA does not comprise the targeting sequences plus the extra sequence which can hybridize with tracrRNA. In some embodiments, the crRNA comprises an extra sequence which can hybridize with tracrRNA. In some embodiments, the two sub-segments are independently transcribed. In some embodiments, the two sub-segments are transcribed as a single unit. In some embodiments, the DNA encoding the crRNA comprises the targeting sequence 5' of the sequence GTTTTAGAGCTATGCTGTTTTG (SEQ ID NO: 1). In some embodiments, the DNA encoding the tracrRNA comprises the sequence GGAACCATTCAAAACAGCATAGCAAGTTA AAATAAGGCTAGTCCGTTATCA ACTTGAAAAAGTGGCACCGAGTCGGTGCTTTTTTT (SEQ ID NO: 2).

As used herein, a targeting sequence is one that directs the gNA to a target sequence in a nucleic acid targeted for depletion in a sample. For example, a targeting sequence targets any of the non-host sequences described herein.

Described herein are gNAs and pluralities of gNAs that comprise a segment that comprises a targeting sequence.

In some embodiments, the targeting sequence comprises or consists of DNA. In some embodiments, the targeting sequence comprises or consists of RNA.

In some embodiments, the targeting sequence comprises RNA, and shares at least 70% sequence identity, at least 75% sequence identity, at least 80% sequence identity, at least 85% sequence identity, at least 90% sequence identity, at least 95% sequence identity, or shares 100% sequence identity to a sequence 5' to a PAM sequence on a sequence of interest, except that the RNA comprises uracils instead of thymines. In some embodiments, the targeting sequence comprises RNA, and shares at least 70% sequence identity, at least 75% sequence identity, at least 80% sequence identity, at least 85% sequence identity, at least 90% sequence identity, at least 95% sequence identity, or shares 100% sequence identity to a sequence 3' to a PAM sequence on a sequence of interest, except that the RNA comprises uracils instead of thymines. In some embodiments, the PAM sequence is AGG, CGG, TGG, GGG or NAG. In some embodiments, the PAM sequence is TTN, TCN or TGN.

In some embodiments, the targeting sequence comprises RNA and is complementary to the strand opposite to a sequence of nucleotides 5' to a PAM sequence. In some embodiments, the targeting sequence is at least 70% complementary, at least 75% complementary, at least 80% complementary, at least 85% complementary, at least 90% complementary, at least 95% complementary, or is 100% complementary to the strand opposite to a sequence of nucleotides 5' to a PAM sequence. In some embodiments, the targeting sequence comprises RNA and is complementary to the strand opposite to a sequence of nucleotides 3' to a PAM sequence. In some embodiments, the targeting sequence is at least 70% complementary, at least 75% complementary, at least 80% complementary, at least 85% complementary, at least 90% complementary, at least 95% complementary, or is 100% complementary to the strand opposite to a sequence of nucleotides 3' to a PAM sequence. In some embodiments, the PAM sequence is AGG, CGG, TGG, GGG or NAG. In some embodiments, the PAM sequence is TTN, TCN or TGN.

Different CRISPR/Cas system proteins recognize different PAM sequences. PAM sequences can be located 5' or 3' of a targeting sequence. For example, Cas9 can recognize an NGG PAM located on the immediate 3' end of a targeting sequence. Cpf1 can recognize a TTN PAM located on the immediate 5' end of a targeting sequence. All PAM sequences recognized by all CRISPR/Cas system proteins are envisaged as being within the scope of the disclosure. It will be readily apparent to one of ordinary skill in the art which PAM sequences are compatible with a particular CRISPR/Cas system protein.

Described herein are gNAs and pluralities of gNAs comprising a segment that comprises a nucleic acid-guided nuclease protein-binding sequence. The nucleic acid-guided nuclease can be a nucleic acid-guided nuclease system protein (*e.g.*, CRISPR/Cas system). A nucleic acid-guided nuclease system can be an RNA-guided nuclease system. A nucleic acid-guided nuclease system can be a DNA-guided nuclease system.

A nucleic acid-guided nuclease protein-binding sequence is a nucleic acid sequence that binds any protein member of a nucleic acid-guided nuclease system. For example, a CRISPR/Cas protein-binding sequence is a nucleic acid sequence that binds any protein member of a CRISPR/Cas system.

In some embodiments, CRISPR/Cas system proteins can be from any bacterial or archaeal species. In some embodiments, the CRISPR/Cas system protein is isolated, recombinantly produced, or synthetic. In some embodiments, examples of CRISPR/Cas system proteins can be naturally occurring or engineered versions. In some embodiments, nucleic acid-guided nuclease system proteins (*e.g.*, CRISPR/Cas system proteins) can be from any bacterial or archaeal species. In some embodiments, naturally occurring CRISPR/Cas system proteins can belong to CAS Class I Type I, III, or IV, or CAS Class II Type II or V, and can include Cas9, Cas3, Cas8a-c, Cas10, CasX, CasY, Cas13, Cas14, Cse1, Csy1, Csn2, Cas4, Csm2, Cmr5, Csf1, C2c2, and Cpf1. In an exemplary embodiment, the CRISPR/Cas system protein comprises Cas9. In an exemplary embodiment, the CRISPR/Cas system protein comprises Cpf1.

In some embodiments, the nucleic acid-guided nuclease system proteins (*e.g.*, CRISPR/Cas system proteins) are from, or are derived from nucleic acid-guided nuclease system proteins (*e.g.,* CRISPR/Cas system proteins) from *Streptococcus pyogenes, Staphylococcus aureus, Neisseria meningitidis, Streptococcus thermophiles, Treponema denticola, Francisella tularensis, Pasteurella multocida, Campylobacter jejuni, Campylobacter lari, Mycoplasma gallisepticum, Nitratifractor salsuginis, Parvibaculum lavamentivorans, Roseburia intestinalis, Neisseria cinerea, Gluconacetobacter diazotrophicus, Azospirillum, Sphaerochaeta globus, Flavobacterium columnare, Fluviicola taffensis, Bacteroides coprophilus, Mycoplasma mobile, Lactobacillus farciminis, Streptococcus pasteurianus, Lactobacillus johnsonii, Staphylococcus pseudintermedius, Filifactor alocis, Legionella pneumophila, Suterella wadsworthensis Corynebacter diphtheria, Acidaminococcus, Lachnospiraceae bacterium* or *Prevotella.*

In some embodiments, the nucleic acid-guided nuclease system protein-binding sequence comprises a gNA (*e.g.,* gRNA) stem-loop sequence. Different CRISPR/Cas system proteins are compatible with different nucleic acid-guided nuclease system protein-binding sequences. It will be readily apparent to one of ordinary skill in the art which CRISPR/Cas system proteins are compatible with which nucleic acid-guided nuclease system protein-binding sequences.

In some embodiments, a double-stranded DNA sequence encoding the gNA (*e.g.,* gRNA) stem-loop sequence comprises the following DNA sequence on one strand (5'>3', GTTTTAGAGCTAGAAATAGCAAGTTAAAATAAGGCTA GTCCGTTATCAACTT GAAAAAGTGGCACCGAGTCGGTGCTTTTTTT) (SEQ ID NO: 3), and its reverse-complementary DNA on the other strand (5'>3', AAAAAAAGCACCGACTCGGTGCCACTTTTTCAAGTTGATAACGGACTAGCC TTATTTTAACTTGCTATTTCTAGCTCTAAAAC) (SEQ ID NO: 4).

In some embodiments, a single-stranded DNA sequence encoding the gNA (*e.g.,* gRNA) stem-loop sequence comprises the following DNA sequence: (5'>3', AAAAAAAGCACCGACTCGGTGCCACTTTTTCAAGTTGATAACGGA CTAGCC TTATTTTAACTTGCTATTTCTAGCTCTAAAAC) (SEQ ID NO: 4), wherein the single-stranded DNA serves as a transcription template.

In some embodiments, the gNA (*e.g.,* gRNA) stem-loop sequence comprises the following RNA sequence: (5'>3', GUUUUAGAGCUAGAAAUAGCAAGUUAAAAUAAGGCUAGUCCGUUAUCAA CUUGAAAAAGUGGCACCGAGUCGGUGCUUUUUUU) (SEQ ID NO: 5).

In some embodiments, a double-stranded DNA sequence encoding the gNA (*e.g.,* gRNA) stem-loop sequence comprises the following DNA sequence on one strand (5'>3', GTTTTAGAGCTATGCTGGAAACAGCATAGCAAGTTA AAATAAGGCTAGTCC GTTATCAACTTGAAAAAGTGGCACCGAGTCGGTGCTTTTTTTC) (SEQ ID NO: 6), and its reverse-complementary DNA on the other strand (5'>3', GAAAAAAAGCACCGACTCGGTGCCACTTTTTCAAGTTGA TAACGGACTAGC CTTATTTTAACTTGCTATGCTGTTTCCAGCATAGCTCTAAAAC) (SEQ ID NO: 7).

In some embodiments, a single-stranded DNA sequence encoding the gNA (*e.g.,* gRNA) stem-loop sequence comprises the following DNA sequence: (5' >3', GAAAAAAAGCACCGACTCGGTGCCACTTTTTCAAGTTGATAACG GACTAGC CTTATTTTAACTTGCTATGCTGTTTCCAGCATAGCTCTAAAAC) (SEQ ID NO: 7), wherein the single-stranded DNA serves as a transcription template.

In some embodiments, the gNA (*e.g.,* gRNA) stem-loop sequence comprises the following RNA sequence: (5'>3', GUUUUAGAGCUAUGCUGGAAACAGCAUAGCAAGUUAAAAUAAGGCUAGU CCGUUAUCAACUUGAAAAAGUGGCACCGAGUCGGUGCUUUUUUUC) (SEQ ID NO: 8).

In some embodiments, the CRISPR/Cas system protein is a Cpf1 protein. In some embodiments, the Cpf1 protein is isolated or derived from Franciscella species or Acidaminococcus species. In some embodiments, the gNA (*e.g.,* gRNA) CRISPR/Cas system protein-binding sequence comprises the following RNA sequence: (5'>3', AAUUUCUACUGUUGUAGAU) (SEQ ID NO: 9).

In some embodiments, the CRISPR/Cas system protein is a Cpf1 protein. In some embodiments, the Cpf1 protein is isolated or derived from Franciscella species or Acidaminococcus species. In some embodiments, a DNA sequence encoding the gNA (*e.g.,* gRNA) CRISPR/Cas system protein-binding sequence comprises the following DNA sequence: (5'>3', AATTTCTACTGTTGTAGAT) (SEQ ID NO: 10). In some embodiments, the DNA is single stranded. In some embodiments, the DNA is double stranded.

In some embodiments, provided herein is a gNA (*e.g.,* gRNA) comprising a first NA segment comprising a targeting sequence and a second NA segment comprising a nucleic acid-guided nuclease (*e.g.*, CRISPR/Cas) system protein-binding sequence. In some embodiments, the size of the first segment is 15 bp, 16 bp, 17 bp, 18 bp, 19 bp or 20 bp. In some embodiments, the second segment comprises a single segment, which comprises the gRNA stem-loop sequence. In some embodiments, the gRNA stem-loop sequence comprises the following RNA sequence: (5' >3', GUUUUAGAGCUAGAAAUAGCAAGUUAAAAUAAGGCUAGUCCGUUAUCAA CUUGAAAAAGUGGCACCGAGUCGGUGCUUUUUUU) (SEQ ID NO: 5). In some embodiments, the gRNA stem-loop sequence comprises the following RNA sequence: (5'>3', GUUUUAGAGCUAUGCUGGAAACAGCAUAGCAAGUUAAAAUAAGGCUAGU CCGUUAUCAACUUGAAAAAGUGGCACCGAGUCGGUGCUUUUUUUC) (SEQ ID NO: 8). In some embodiments, the second segment comprises two sub-segments: a first RNA sub-segment (crRNA) that forms a hybrid with a second RNA sub-segment (tracrRNA), which together act to direct nucleic acid-guided nuclease (*e.g.*, CRISPR/Cas) system protein binding. In some embodiments, the sequence of the second sub-segment comprises GUUUUAGAGCUAUGCUGUUUUG. In some embodiments, the first RNA segment and the second RNA segment together form a crRNA sequence. In some embodiments, the other RNA that will form a hybrid with the second RNA segment is a tracrRNA. In some embodiments the tracrRNA comprises the sequence of 5' >3', GGAACCAUUCAAAACAGCAUAGCAA-GUUAAAAUAAGGCUAGUCCGUUAU CAACUUGAAAAAGUGGCACCGAGUCGGUGCUUUUUU (SEQ ID NO: 11).

In some embodiments, provided herein is a gNA (*e.g.,* gRNA) comprising a first NA segment comprising a targeting sequence and a second NA segment comprising a nucleic acid-guided nuclease (*e.g.*, CRISPR/Cas) system protein-binding sequence. In some embodiments, for example those embodiments wherein the CRISPR/Cas system protein is a Cpf1 system protein, the second segment is 5' of the first segment. In some embodiments, the size of the first segment is 20 bp. In some embodiments, the size of the first segment is greater than 20 bp. In some embodiments, the size of the first segment is greater than 30 bp. In some embodiments, the second segment comprises a single segment, which comprises the gRNA stem-loop sequence. In some embodiments, the gRNA stem-loop sequence comprises the following RNA sequence: (5'>3', AAUUUCUACUGUUGUAGAU) (SEQ ID NO: 9).

A CRISPR/Cas system protein may be at least 60% identical (*e.g.*, at least 70%, at least 80%, or 90% identical, at least 95% identical or at least 98% identical or at least 99% identical) to a wild type CRISPR/Cas system protein. The CRISPR/Cas system protein may have all the functions of a wild type CRISPR/Cas system protein, or only one or some of the functions, including binding activity and nuclease activity.

The term "CRISPR/Cas system protein-associated guide NA" refers to a guide NA (gNA). The CRISPR/Cas system protein -associated guide NA may exist as isolated NA, or as part of a CRISPR/Cas system protein-gNA complex.

In some embodiments, the CRISPR/Cas system protein is an RNA-guided DNA nuclease. In some embodiments, the DNA cleaved by the CRISPR/Cas system protein is double stranded. Exemplary RNA-guided DNA nucleases that cut double stranded DNA include, but are not limited to Cas9, Cpf1, CasX and CasY. Further exemplary RNA-guided DNA nucleases include Cas10, Csm2, Csm3, Csm4, and Csm5. In some embodiments, Cas10, Csm2, Csm3, Csm4, and Csm5 form a ribonucleoprotein complex with a gRNA.

In some embodiments, the CRISPR/Cas System protein nucleic acid-guided nuclease is or comprises Cas9. The Cas9 of the present disclosure can be isolated, recombinantly produced, or synthetic. In some embodiments, the Cas9 protein is thermostable. Examples of Cas9 proteins that can be used in the embodiments herein can be found in F.A. Ran, L. Cong, W.X. Yan, D. A. Scott, J.S. Gootenberg, A.J. Kriz, B. Zetsche, O. Shalem, X. Wu, K.S. Makarova, E.V. Koonin, P.A. Sharp, and F. Zhang; "In vivo genome editing using Staphylococcus aureus Cas9," Nature 520, 186-191 (09 April 2015) doi:10.1038/nature14299). In some embodiments, the Cas9 is a Type II CRISPR system derived from *Streptococcus pyogenes, Staphylococcus aureus, Neisseria meningitidis, Streptococcus thermophiles, Treponema denticola, Francisella tularensis, Pasteurella multocida, Campylobacter jejuni, Campylobacter lari, Mycoplasma gallisepticum, Nitratifractor salsuginis, Parvibaculum lavamentivorans, Roseburia intestinalis, Neisseria cinerea, Gluconacetobacter diazotrophicus, Azospirillum, Sphaerochaeta globus, Flavobacterium columnare, Fluviicola taffensis, Bacteroides coprophilus, Mycoplasma mobile, Lactobacillus farciminis, Streptococcus pasteurianus, Lactobacillus johnsonii, Staphylococcus pseudintermedius, Filifactor alocis, Legionella pneumophila, Suterella wadsworthensis,* or *Corynebacter diphtheria.*

In some embodiments, the Cas9 is a Type II CRISPR system derived from S. *pyogenes* and the PAM sequence is NGG located on the immediate 3' end of the target specific guide sequence. The PAM sequences of Type II CRISPR systems from exemplary bacterial species can also include: *Streptococcus pyogenes* (NGG), *Staph aureus* (NNGRRT), *Neisseria meningitidis* (NNNNGATT), *Streptococcus thermophilus* (NNAGAA) and *Treponema denticola* (NAAAAC), which are all usable without deviating from the present disclosure.

In one exemplary embodiment, Cas9 sequence can be obtained, for example, from the pX330 plasmid (available from Addgene), re-amplified by PCR then cloned into pET30 (from EMD biosciences) to express in bacteria and purify the recombinant 6His tagged protein.

A "Cas9-gNA complex" refers to a complex comprising a Cas9 protein and a guide NA. A Cas9 protein may be at least 60% identical (*e.g.*, at least 70%, at least 80%, or 90% identical, at least 95% identical or at least 98% identical or at least 99% identical) to a wild type Cas9 protein, *e.g.,* to the *Streptococcus pyogenes* Cas9 protein. The Cas9 protein may have all the functions of a wild type Cas9 protein, or only one or some of the functions, including binding activity, nuclease activity, and nuclease activity.

The term "Cas9-associated guide NA" refers to a guide NA as described above. The Cas9-associated guide NA may exist isolated, or as part of a Cas9-gNA complex.

In some embodiments, the CRISPR/Cas system protein nucleic acid-guided nuclease is or comprises a Cpf1 system protein. Cpf1 system proteins of the present disclosure can be isolated, recombinantly produced, or synthetic. In some embodiments, the Cpf1 protein is thermostable.

Cpf1 system proteins are Class II, Type V CRISPR system proteins. In some embodiments, the Cpf1 protein is isolated or derived from *Francisella tularensis.* In some embodiments, the Cpf1 protein is isolated or derived from *Acidaminococcus, Lachnospiraceae bacterium* or *Prevotella.*

Cpf1 system proteins bind to a single guide RNA comprising a nucleic acid-guided nuclease system protein-binding sequence (*e.g.*, stem-loop) and a targeting sequence. The Cpf1 targeting sequence comprises a sequence located immediately 3' of a Cpf1 PAM sequence in a target nucleic acid. Unlike Cas9, the Cpf1 nucleic acid-guided nuclease system protein-binding sequence is located 5' of the targeting sequence in the Cpf1 gRNA. Cpf1 can also produce staggered rather than blunt ended cuts in a target nucleic acid. Following targeting of the Cpf1 protein-gRNA protein complex to a target nucleic acid, Francisella derived Cpf1, for example, cleaves the target nucleic acid in a staggered fashion, creating an approximately 5 nucleotide 5' overhang 18-23 bases away from the PAM at the 3' end of the targeting sequence. In contrast, cutting by a wild type Cas9 produces a blunt end 3 nucleotides upstream of the Cas9 PAM.

An exemplary Cpf1 gRNA stem-loop sequence comprises the following RNA sequence: (5'>3', AAUUUCUACUGUUGUAGAU) (SEQ ID NO: 9).

A "Cpf1 protein-gNA complex" refers to a complex comprising a Cpf1 protein and a guide NA (*e.g.* a gRNA). Where the gNA is a gRNA, the gRNA may be composed of a single molecule, *i.e.,* one RNA ("crRNA") which hybridizes to a target and provides sequence specificity.

A Cpf1 protein may be at least 60% identical (*e.g.,* at least 70%, at least 80%, or 90% identical, at least 95% identical or at least 98% identical or at least 99% identical) to a wild type Cpf1 protein. The Cpf1 protein may have all the functions of a wild type Cpf1 protein, or only one or some of the functions, including binding activity and nuclease activity.

Cpf1 system proteins recognize a variety of PAM sequences. Exemplary PAM sequences recognized by Cpf1 system proteins include, but are not limited to TTN, TCN and TGN. Additional Cpf1 PAM sequences include, but are not limited to TTTN. One feature of Cpf1 PAM sequences is that they have a higher A/T content than the NGG or NAG PAM sequences used by Cas9 proteins.

### *Computer Systems and Software*

The methods described herein may be used in the context of a computer system or as part of software or computer-executable instructions that are stored in a computer-readable storage medium.

In some embodiments, a system (*e.g.*, a computer system) may be used to implement certain features of some of the embodiments of the invention.

In certain embodiments, the system may include one or more memory and/or storage devices. The memory and storage devices may be one or more computer-readable storage media that may store computer-executable instructions that implement at least portions of the various embodiments of the invention.

Described herein is a system for designing a plurality of normalization control polynucleotide sequences. In some embodiments, the system comprises a computer-readable storage medium which stores computer-executable instructions comprising: (i) instructions for importing at least one reference sequence; (ii) instructions for generating a plurality of reference sequence fragments from at least one reference sequence; (iii) instructions for generating a distribution of at least one parameter as a function of number of reference sequence fragments; (iv) instructions for dividing the distribution into bins; (v) instructions for selecting a plurality of reference sequence fragment from at least a subset of the bins; and (vi) instructions for shuffling the plurality of reference sequence fragments to generate shuffled sequences; thereby generating a plurality of normalization polynucleotide sequences. In some embodiments the system comprises a computer-readable storage medium which stores computer-executable instructions comprising: (i) instructions for importing at least one reference sequence; (ii) instructions for generating a plurality of reference sequence fragments from at least one reference sequence; (iii) instructions for generating a distribution of at least one parameter as a function of number of reference sequence fragments; (iv) instructions for dividing the distribution into bins; (v) instructions for selecting at least two reference sequence fragments from each of at least a subset of the bins, wherein the at least two reference sequence fragments are either non-contiguous in the reference sequence, or from different reference sequences; and (vi) instructions for concatenating the at least two reference sequence fragments from each bin; thereby generating a plurality of normalization polynucleotide sequences. In some embodiments, the system further comprises a processor which is configured to perform steps comprising: (a) receiving a set of input files comprising the at least at least one reference sequence; and (b) executing the computer-executable instructions stored in the computer-readable storage medium. In some embodiments, the parameter comprises least one of (1) percent GC content, (2) entropy, (3) complexity, (4) EIIP, (5) length, or a combination thereof.

The computing system may include one or more central processing units ("processors"), memory, input/output devices, *e.g.* keyboard and pointing devices, touch devices, display devices, storage devices, *e.g.* disk drives, and network adapters, *e.g.* network interfaces, that are connected to an interconnect. The interconnect is an abstraction that represents any one or more separate physical buses, point-to-point connections, or both, connected by appropriate bridges, adapters, or controllers. The interconnect, therefore, may include, for example a system bus, a peripheral component interconnect (PCI) bus or PCI-Express bus, a HyperTransport or industry standard architecture (ISA) bus, a small computer system interface (SCSI) bus, a universal serial bus (USB), IIC (12C) bus, or an Institute of Electrical and Electronics Engineers (IEEE) standard 1394 bus, also referred to as Firewire.

In addition, data structures and message structures may be stored or transmitted via a data transmission medium, *e.g.* a signal on a communications link. Various communications links may be used, *e.g.* the Internet, a local area network, a wide area network, or a point-to-point dial-up connection. Thus, computer readable media can include computer-readable storage media, *e.g.* non-transitory media, and computer-readable transmission media.

The instructions stored in memory can be implemented as software and/or firmware to program one or more processors to carry out the actions described above. In some embodiments of the invention, such software or firmware may be initially provided to the processing system by downloading it from a remote system through the computing system, *e.g.* via the network adapter.

The various embodiments introduced herein can be implemented by, for example, programmable circuitry, *e.g.* one or more microprocessors, programmed with software and/or firmware, entirely in special-purpose hardwired, *i.e.* non-programmable, circuitry, or in a combination of such forms. Special purpose hardwired circuitry may be in the form of, for example, one or more ASICs, PLDs, FPGAs, etc.

Some portions of the detailed description may be presented in terms of algorithms, which may be symbolic representations of operations on data bits within a computer memory. These algorithmic descriptions and representations are those methods used by those skilled in the data processing arts to most effectively convey the substance of their work to others skilled in the art. An algorithm is here, and generally, conceived to be a self-consistent sequence of operations leading to a desired result. The operations are those requiring physical manipulations of physical quantities. Usually, though not necessarily, these quantities take the form of electrical or magnetic signals capable of being stored, transferred, combined, compared, and otherwise manipulated. It has proven convenient at times, principally for reasons of common usage, to refer to these signals as bits, values, elements, symbols, characters, terms, numbers, or the like.

The algorithms and displays presented herein are not inherently related to any particular computer or other apparatus. Various general purpose systems may be used with programs in accordance with the teachings herein, or it may prove convenient to construct more specialized apparatus to perform the methods of some embodiments.

Moreover, while embodiments have been described in the context of fully functioning computers and computer systems, those skilled in the art will appreciate that the various embodiments are capable of being distributed as a program product in a variety of forms, and that the disclosure applies equally regardless of the particular type of machine or computer-readable media used to actually effect the distribution.

Further examples of machine-readable storage media, machine-readable media, or computer-readable (storage) media include but are not limited to recordable type media such as volatile and non-volatile memory devices, floppy and other removable disks, hard disk drives, optical disks (*e.g.*, Compact Disk Read-Only Memory (CD ROMS), Digital Versatile Disks, (DVDs), etc.), among others, and transmission type media such as digital and analog communication links.

### *Kits and Articles of Manufacture*

Described herein are kits and articles of manufacture comprising any one or more of the normalization control compositions described herein and/or reagents used for making or using any of the normalization control compositions described herein. In some embodiments, an exemplary kit comprises a DNA normalization control composition described herein. In some embodiments, an exemplary kit comprises an RNA normalization control composition described herein. In some embodiments, an exemplary kit comprises a combination of DNA and RNA normalization control compositions described herein. In some embodiments, the kit further comprises instructions for using the normalization control compositions, for example, instructions for dilution, concentrations, and adding the normalization controls to the sample.

In some embodiments of the kits described herein, the kit comprises any one or more of the multi-analyte controls of the disclosure, and reagents for their use. In some embodiments, the kit further comprises instructions for using the multi-analyte controls, for example, instructions for dilution, concentrations, and adding the multi-analyte controls to the sample, or processing the multi-analyte controls in parallel with a sample. Exemplary multi-analyte controls provided with a kit of the disclosure include the multi-analyte control comprising at least three species of organisms that have been inactivated. In some embodiments, the kit further comprises reagents for diluting the multi-analyte controls. In some embodiments, the kit further comprises instructions for use of the multi-analyte controls.

In some embodiments, the kit comprises normalization controls, multi-analyte controls, as well as reagents and instructions for their use. In some embodiments, the kit further comprises positive controls. Exemplary positive controls include inactivated organisms at known concentration (*e.g.*, 1 IU/mL, 10 IU/mL, 100 IU/mL, 1,000 IU/mL, 10,000 IU/mL, 100,000 IU/mL or 1,000,000 IU/mL) that can used to positively control for or calibrate the normalization controls and multi-analyte controls described herein. In some embodiments, the positive controls comprise viruses, bacteria, fungi, single-celled eukaryotes or any combination thereof.

Described herein are kits and articles of manufacture comprising any one or more the reagents used to deplete a sample of sequences targeted for depletion, or enrich a sample for sequences of interest prior to NGS sequencing, as described herein. In some embodiments, the kits comprise any one or more of the compositions described herein, not limited to adapters, gNAs (*e.g.,* gRNAs or gDNAs), gNA collections (*e.g.,* gRNA or gDNA pluralities), modification-sensitive restriction enzymes, controls and the like.

In exemplary embodiments, the kit comprises gRNAs wherein the gRNAs are targeted to any host genomic or cDNA sequence described herein. In further exemplary embodiments, the kit comprises of gRNAs wherein the gRNAs are targeted to human genomic or other sources of DNA sequences.

The present disclosure also describes all essential reagents and instructions for carrying out the methods of enriching a sample for nucleic acids of interest using differences in nucleotide modification, as described herein.

The present disclosure also describes kits comprising normalization controls, multi-analyte controls, reagents used to deplete a sample of sequences targeted for depletion, or enrich a sample for sequences of interest prior to NGS sequencing, as well as reagents and instructions for their use.

Also described herein is computer software monitoring the information before and after enriching a sample using the methods provided herein. In one exemplary embodiment, the software can compute and report the abundance of sequences of nucleic acids targeted for depletion in the sample before and after applying the methods described herein, to assess the level of off-target depletion, and wherein the software can check the efficacy of targeted-depletion/enrichment/capture/partitioning/labeling/regulation/editing by comparing the abundance of the sequence of interest before and after processing the sample using the methods of enrichment provided herein.

In some embodiments of the kits of the disclosure, the kits comprise both normalization controls, multi-analyte controls, reagents for sample enrichment and/or depletion, instructions for their use, and reagents for their use such as acceptable diluents.

In some embodiments of the kits of the disclosure, the multi-analyte controls comprise or consists of a mixture of 11 species of virus. In some embodiments, the viruses consist of Cytomegalovirus (CMV), Epstein-Barr Virus (EBV), Adenovirus (ADV), BK Virus (BKV), JC Polyomavirus (JCV), Human Herpesvirus 6A (HHV6A), Human Herpesvirus 6B (HHV6B), Herpes simplex Virus type 1 (HSV1), Herpes simplex Virus type 2 (HSV2), Varicella-Zoster Virus (VZV) and Human Parvovirus B19 (B19).

In some embodiments of the kits of the disclosure, the multi-analyte controls comprise or consists of a mixture of 10 species of virus. In some embodiments, the viruses consist of CMV, EBV, ADV, BKV, JCV, HHV6A, HHV6B, HSV1, HSV2 and VZV.

Every maximum numerical limitation given throughout this disclosure includes every lower numerical limitation, as if such lower numerical limitations were expressly written herein. Every minimum numerical limitation given throughout this disclosure will include every higher numerical limitation, as if such higher numerical limitations were expressly written herein. Every numerical range given throughout this disclosure will include every narrower numerical range that falls within such broader numerical range, as if such narrower numerical ranges were all expressly written herein.

The values disclosed herein are not to be understood as being strictly limited to the exact numerical values recited. Instead, unless otherwise specified, each such value is intended to mean both the recited value and a functionally equivalent range surrounding that value. For example, a value disclosed as "20 $\mu$m" is intended to mean "about 20 $\mu$m."

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document cited herein, the meaning or definition assigned to that term in this document shall govern.

## EXAMPLES

In order that the invention disclosed herein may be more efficiently understood, examples are provided below. It should be understood that these examples are for illustrative purposes only and are not to be construed as limiting the invention in any manner.

### *Example 1: RNA normalization controls*

RNA normalization controls can be made by transcribing randomly generated DNA sequences. The randomly generated sequences were synthesized as a gBlock, with terminal T7 promoter sequences for transcription, and XhoI restriction sites to linearize the random DNA template. Three versions of the design shown below were created, termed RNA_NC_1, RNA_NC_2 and RNA_NC_3. Each version was created with the following design, but with different randomly generated sequences:

5'-TGAAGAACTGCGGCAGG (SEQ ID NO: 12) (Mito array primer) + 175 bp random sequence (designed using Random DNA Generator) + T7 RNA Polymerase promoter + XhoI site + 250bp random sequence (designed using Random DNA Generator) + T7 RNA Polymerase promoter + XhoI site + 450 bp random sequence (designed using Random DNA Generator) + T7 RNA Polymerase promoter + (Ribo array primer) GGTCTGCACTTCCAGCT-3' (SEQ ID NO: 13).

The sequence of the Ribo array primer comprises a sequence 5'-AGCTGGAAGTGCAGACC-3' (SEQ ID NO: 20).

The sequence of the T7 promoter comprises a sequence of 5' - TAATACGACTCACTATAGGG -3' (SEQ ID NO: 15).

The total length of the design was 981bp. The three versions of the design with different random sequences were ordered as gBlocks (gene blocks), and were called RNA_NC_1, RNA_NC_2 and RNA_NC_3. The RNA_NC_1, RNA_NC_2, RNA_NC_3 gBlocks were digested with XhoI to produce three linear DNA fragments, and the resulting mixture of three fragments transcribed with T7 RNA polymerase to produce three RNA NCs. gBlock RNA_NC_1 resulted in 3 RNA fragments: RNA_NC_1.1, RNA_NC_1.2, RNA_NC_1.3. gBlock RNA_NC_2 resulted in 3 RNA fragments: RNA_NC_2.1, RNA_NC_2.2, RNA_NC_2.3. gBlock RNA_NC_3 resulted in 3 RNA fragments: RNA_NC_3.1, RNA_NC_3.2, RNA_NC_3.3. T7 transcription of the DNA fragments corresponding to RNA NC_1.1, RNA_NC_2.1 and RNA_NC_3.1 NC RNAs resulted in NC RNAs that comprised a sequence complementary to Mito array primer, a sequence complementary to 175 bp random DNA sequence and a terminal GGG sequence introduced by the T7 polymerase during transcription. T7 transcription of the DNA fragments corresponding to NC RNAs RNA_NC_1.2, RNA_NC_2.2 and RNA_NC_3.2 resulted in NC RNAs that comprised a sequence complementary to the 250 bp random sequence and a terminal GGG sequence introduced during transcription. T7 transcription of the DNA fragments corresponding to NC RNAs RNA_NC_1.3, RNA_NC_2.3 and RNA_NC_3.3 resulted in NC RNAs that comprised a sequence complementary to the 450 bp random sequence and a terminal GGG sequence introduced during transcription.

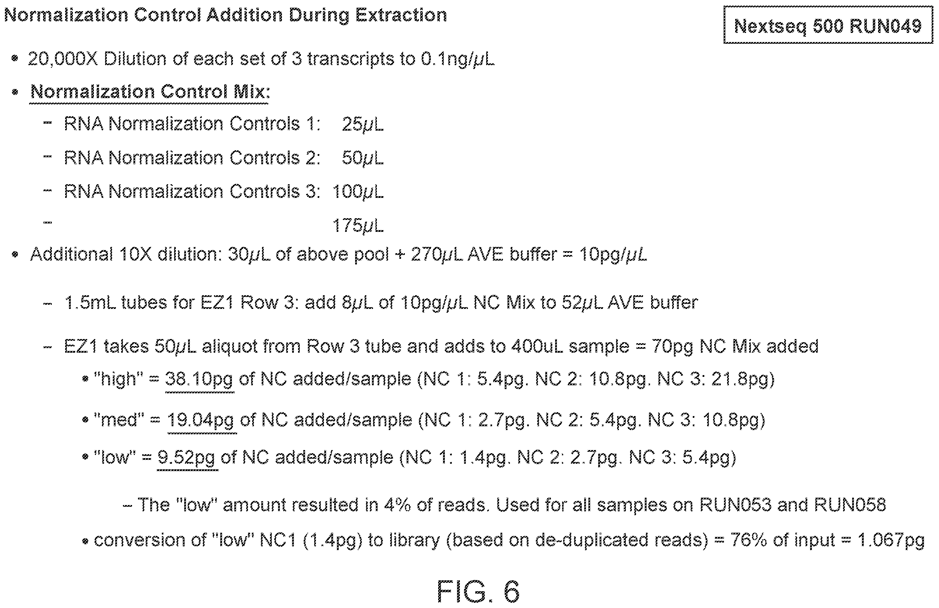

A protocol for the addition of NC controls during sample extraction is shown in FIG. 6. The output of each of the three NC transcription reactions (sets of RNA Normalization Controls 1, 2, and 3, also called NC 1, NC 2 and NC 3 or RNA_NC_1, RNA_NC_2 and RNA_NC_3, each containing the three transcripts described above) was diluted 20,000X to 0.1 ng/$\mu$L. The three sets of NC transcripts were then mixed at a ratio of 1:2:4 by mixing 25 $\mu$L of RNA_NC_1, 50 $\mu$L of RNA_NC_2 and 50 $\mu$L of RNA_NC_3, for a final volume of 175 $\mu$L. This NC mix contained 9 different transcripts total, 3 comprising the 3 different 175 bp random sequences, 3 comprising the 3 different the 250 bp random sequences and 3 comprising the 3 different 450 bp random sequences. The NC mix was then diluted an additional 10x, by added 30 $\mu$L of the mix to 270 $\mu$L of AVE buffer to produce a 10 pg/$\mu$L NC mix.

Nucleic acids were extracted from Negative Plasma samples (Exact Diagnostics) using the EZ1 virus Mini Kit (Qiagen). 8 $\mu$L of the 10 pg/$\mu$L NC mix and 52 $\mu$L of AVE buffer were added to the EZ1 kit Row 3 1.5 mL tubes. A 50 $\mu$L aliquot was taken from the row 3 tubes, and added to 400 $\mu$L of sample, resulting in 70 pg of NC mix being added to the sample during extraction.

The results of adding three different amounts of NC mix to a Negative Plasma sample during extraction were assayed. For the "high" amount of NCs, 38.10 pg of NC mix were added per sample (NC 1: 5.4 pg, NC2: 10.8 pg, NC 3: 21.8 pg). For the "medium" amount of NCs, 19.04 pg of NC mix were added per sample (NC 1: 2.7 pg, NC2: 5.4 pg, NC3: 10.8 pg). For the "low" amount of NCs, 9. 52 pg of NC mix were added per sample (NC 1: 1.4 pg, NC2: 2.7 pg, NC3: 5.4 pg). The "low" amount resulted in 4% of reads following NextSeq 500 next generation sequencing. The mass of "low" NC 1 converted to library, based on de-duplicated reads, was 1.076 pg, or 76% of input.

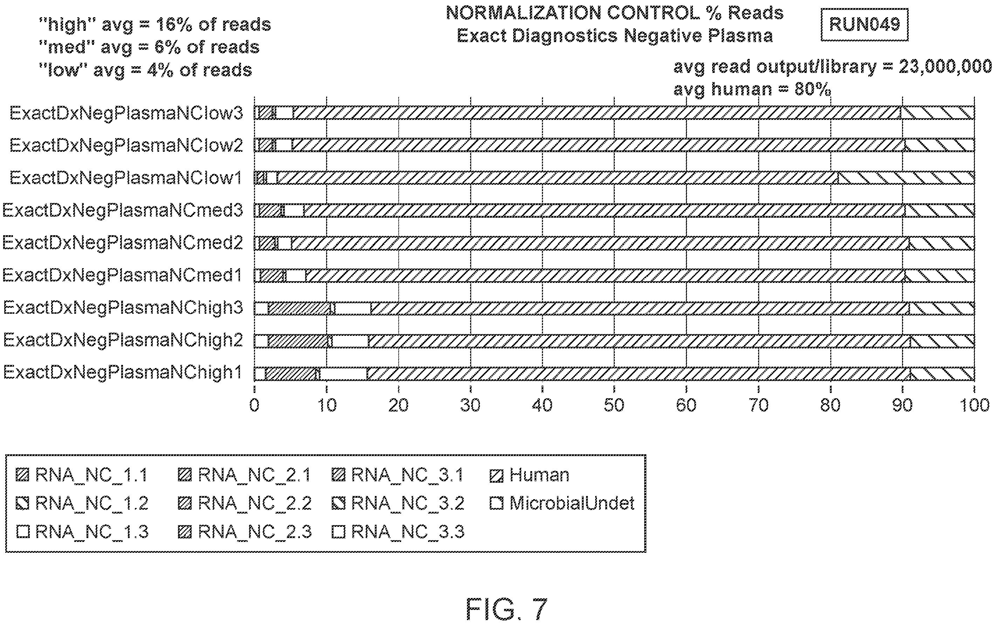

FIG. 7 summarizes the representation of the RNA Normalization Controls in RNA-Seq libraries prepared from Exact Diagnostics Negative Plasma with NCs added during nucleic acid extraction. The average read output per library was 23,000,000 reads, with an average human representation of 80%. The "high" level addition of the RNA Normalization Control (38.10 pg) resulted in reads representing 16% of the total reads, on average, in these libraries. The "medium" level addition of the RNA Normalization Control (19.04 pg) resulted in reads representing 6% of the total reads, on average, in these libraries. The "low" level addition of the RNA Normalization Control (9.52 pg) resulted in reads representing 4% of the total reads, on average, in these libraries. The conversion rate for the RNA_NC 1 fragments at "low" input was 76% (1.067 pg).

*Example 2: DNA normalization controls*

DNA NCs can be made using randomly generated sequence. Below, is an example of a design of DNA NCs. Three versions of this design were made with different randomly generated sequences:

5'-TGAAGAACTGCGGCAGG (SEQ ID NO: 12) (Mito array primer) + random sequence using Random DNA Generator (200bp) + XhoI site + random sequence using Random DNA Generator (300bp) + XhoI site + random sequence using Random DNA Generator (500bp) + T7 RNA Polymerase promoter + (Ribo array primer) GGTCTGCACTTCCAGCT-3' (SEQ ID NO: 13).

From 5' to 3' the sequence of the Ribo array primer is 5'-AGCTGGAAGTGCAGACC-3' (SEQ ID NO: 20). The total length of the design was 1048 bp. The three versions of the design were ordered as gBlocks (gene blocks), termed gBlock DNA_NC_1, gBlock DNA_NC_2 and gBlock DNA_NC_3. XhoI digestion of gBlock DNA NC_1 resulted in 3 fragments: DNA NC_1.1, DNA NC_1.2 and DNA_NC_1.3. Digestion of gBlock DNA_NC_2 resulted in 3 fragments: DNA_NC_2.1, DNA_NC_2.2 and DNA_NC_2.3. Digestion of gBlock DNA_NC_3 resulted in 3 fragments: DNA_NC_3.1, DNA_NC_3.2 and DNA_NC_3.3. Fragments DNA NC_1.1, DNA_NC_2.1 and DNA_NC_3.1 each comprised a DNA sequence of the Mito array primer, a 200 bp random sequence (different for each), and a XhoI half site. Fragments DNA_NC_1.2, DNA_NC_2.2 and DNA_NC_3.2 comprised a 300 bp random sequence and flanked by two XhoI half sites. Fragments DNA_NC_1.3, DNA_NC_2.3 and DNA_NC_3.3 comprised a XhoI half site, a 500 bp random sequence, a T7 promoter sequence and the Ribo array primer sequence.

Normalization controls were added to the sample prior to extraction of genomic DNA. Normalization controls were then indexed in parallel with the rest of the sequencing library.

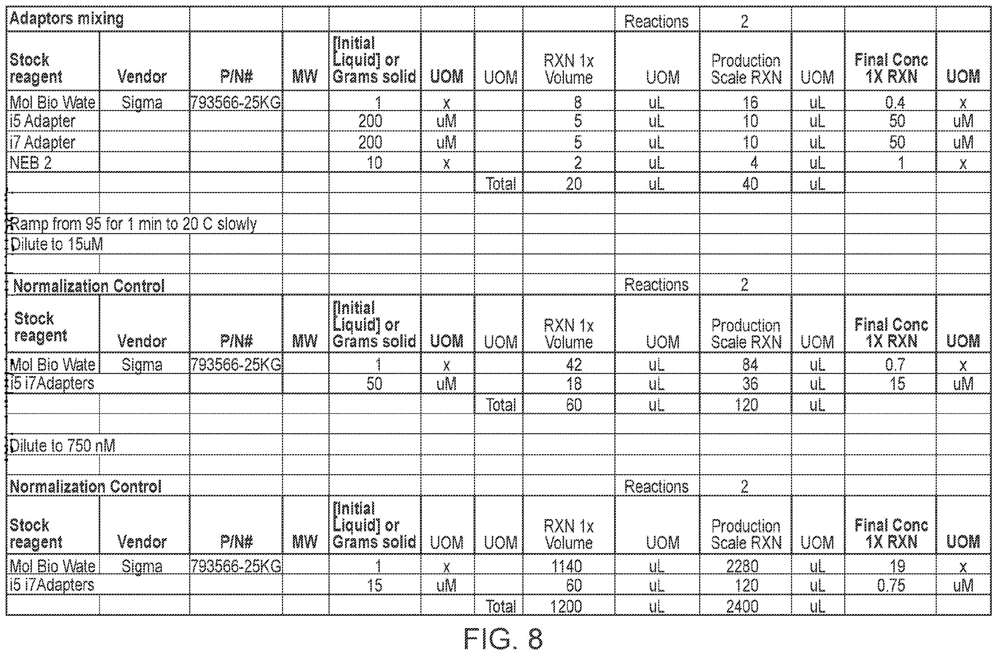

DNA libraries with normalization controls were made using a dual indexing strategy with i7 and i5 adapters. Primers with the i7 and i5 sequences as described below were resuspended at a concentration of 200 $\mu$M. 5 $\mu$L of i5 and i7 primers were mixed in a total volume of 20 $\mu$L in NEB buffer 2 (FIG. 8). The i5 and i7 primer mix was then placed in a thermocycler, ramped to 95 °C for 1 minute, then slowly cooled to 20 °C. The i5/i7 mix was then diluted to a concentration of 15 $\mu$M in a total volume of 1200 $\mu$L (for 1 reaction). i7/i5 adapters were further diluted 1:20 to give a total concentration of 750 nM.

*Dual indexing primers*

i701-UM16 primer: 5' phos/GAT CGG AAG AGC ACA CGT CTG AAC TCC AGT CAC TAC AGG TCN NNN NNA TCT CGT (SEQ ID NO: 21)
i702-UM16 primer: 5'phos/GAT CGG AAG AGC ACA CGT CTG AAC TCC AGT CAC AGT TAC ATN NNN NNA TCT CGT (SEQ ID NO: 22)
i501-UM16 primer: AAT GAT ACG GCG ACC GAG ATC TAC ACN NNN NNC CAG TAC AAC ACT CTT TCC CTA (SEQ ID NO: 23)
i502-UM16 primer: 5' AAT GAT ACG GCG ACC ACC GAG ATG TAC CAN NNN NNG TGC TTA TAC ACT CTT TGG CTA N (SEQ ID NO: 24)

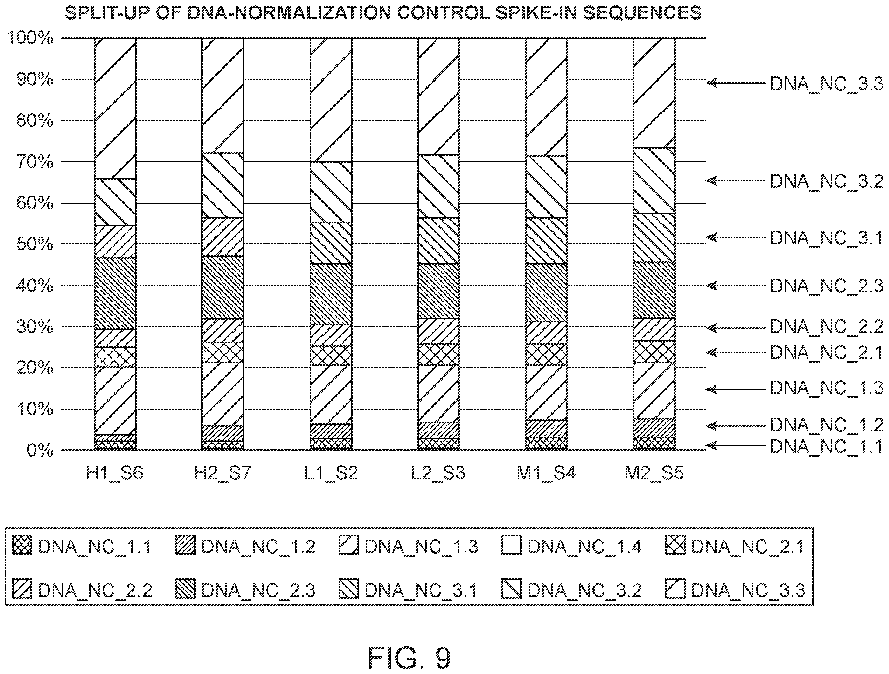

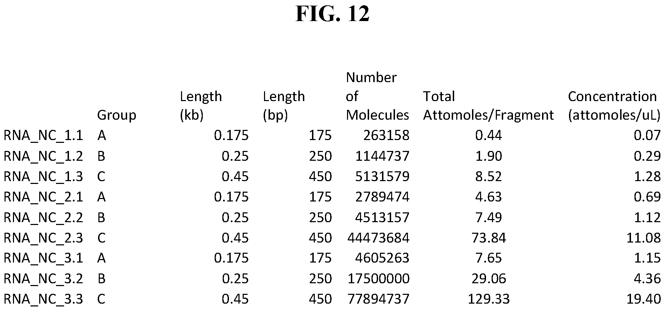

FIG. 9 shows the percent NC reads in an exemplary DNA library sequenced with NCs included. NC controls were added to the sample at the concentrations shown in FIG. 12.

*Example 3: Quantifying levels of non-host in a sample*

In this example, determining the ratio of non-host to NC is used to subtract out the effect of differing host background quantities on non-host quantification in a sample of mixed host and non-host nucleic acids.

The NC is composed of a set of nucleic acids, either separately DNA or RNA or as mixtures of both DNA and RNA. The NCs of various sizes are mixed to provide a linear distribution of different lengths and base compositions to simulate different variability within an NGS library. These nucleic acids contained in a NC can be synthetically manufactured, or from cultured mixtures of native nucleic acids. The fragments can represent very specific sizes, nucleic acid compositions, or cover a very broad range of sizes of fragments at various concentrations. Sequence motifs can be included to understand if the library process is biased for sequence repeats or repetitive sequences

The NC are one or more organisms. For example, the NC comprises polynucleotides extracted from one or more organisms or species of organisms, such as viruses, bacteria, fungi or eukaryotes, that simulate one or more properties of the non-host sequences in a mixed host/non-host sample.

The NC is used to determine the relative amounts of different nucleic acids in a sample. For example, normalization controls are used to determine the abundance value of a pathogen (a non-host) in a sample from an infected host

(*e.g.* a human host infected with a virus or bacteria). The abundance value for the pathogen is determined by dividing NGS reads that map to the pathogen by the number of NGS reads that map to the NCs to compensate for differing background host content. This value is mapped to a standard curve, which was generated and normalized the same way, and the pathogen titer is inferred from the standard curve. Using such methods, there is not necessarily a need to determine an absolute pathogen load.

NCs are used to determine the absolute amounts of different nucleic acids in a sample. For example, the NC is used to determine the absolute amount of a pathogen (a non-host) in a sample from an infected host (*e.g.,* a sample from a human host infected with a virus or bacteria). An abundance value for the pathogen is determined by dividing the number of NGS reads that map to the pathogen by the number of NGS reads that map to the NCs to compensate for differing background host contents. Based on this ratio and the input quantity of NC (*e.g.,* copies of bacteriophage or polynucleotides), the relative pathogen genome copy is determined. This value is then corrected for pathogen genome size to derive an "absolute" pathogen abundance in the starting material.

In some cases, the NC comprises T4 phage nucleic acid sequences, and the pathogen comprises cytomegalovirus. Because the T4 phage and CMV genomes are both around 200 kb, the two are about the same size, and it is assumed that plaque forming units/mL (pfu/mL, T4) and copies/mL (cp/mL, CMV) are equivalent. If, after next generation sequencing, there are 2 CMV reads for every 1 read of T4 phage NC, and the NC was added at a concentration of 100 pfu/ml, then the CMV must have been at a concentration of 200 cp/ml. If the CMV were 1/2 the genome size of T4, then the CMV and T4 would be present at approximately the same concentration (100 pfu/ml or cp/ml) in the initial sample.

### Example 4: Use of a Multi-Analyte Control to Determine Viral Titer

Normalization controls of the disclosure can be used to determine the titer of a panel of viruses in a multi-analyte control, which is used to calculate the titer of a virus in an experimental sample.

In this example, the multi-analyte control is a mixture of 11 viruses that are commonly found human transplant samples: Cytomegalovirus (CMV), Epstein-Barr Virus (EBV), Adenovirus (ADV), BK Virus (BKV), JC Polyomavirus (JCV), Human Herpesvirus 6A (HHV6A), Human Herpesvirus 6B (HHV6B), Herpes simplex Virus type 1 (HSV1), Herpes simplex Virus type 2 (HSV2), Varicella-Zoster Virus (VZV) and Human Parvovirus B19 (B19). The mixture of 11 viruses is used to build a linearity panel of different titers, e.g., 0, 100, 1,000 and 10,000 infectious units (IU)/mL, and spiked into plasma. The multi-analyte control can be spiked into plasma at different levels to generate a calibration panel of multi-analyte controls. Normalization controls are added to the sample and the multi-analyte controls in the calibration panel, the nucleic acids are extracted, and a high throughput sequencing library or libraries are then generated from the experimental sample and the multi-analyte controls. The reads are mapped to the different viruses in the multi-analyte control panel or the normalization controls, and the normalization controls are used to normalize the read count from each of the viruses in the multi-analyte control. Normalized read count are then plotted against titer for the multi-analyte control to generate a standard curve (FIG. 13), and the equation for the relationship between normalized reads and log (IU/mL) is calculated. In this example (FIG. 13), this relationship is linear. Viral signal from the experimental samples is similarly normalized with the normalization controls, and the relationship calculated from the multi-analyte control is used to calculate viral titer in the experimental sample.

The multi-analyte control is also used as a positive external control that goes through the full sample preparation process, separate from the experimental samples. High and low concentration multi-analyte controls can also be included with experimental samples to ensure that a prior calibration panel, *e.g.* a reference panel, is still applicable to current conditions.

### Example 5: Generating Normalization Control Sequences Using Sequence Shuffling

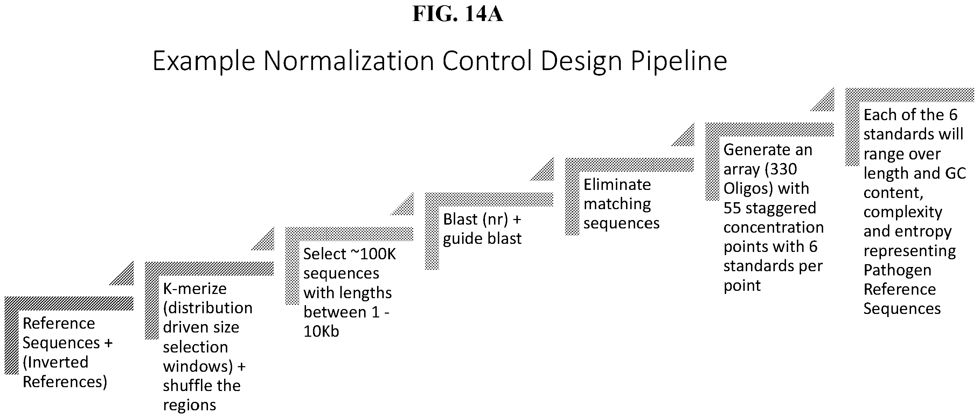

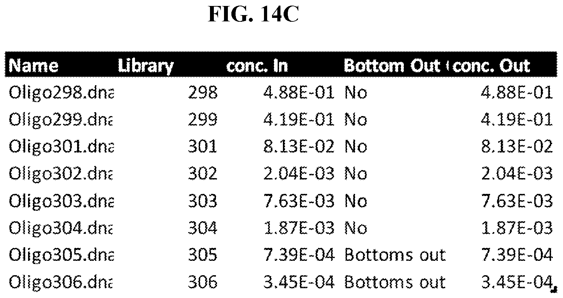

One approach to designing normalization controls is through a base-shuffling approach (Cantor Shuffling). A diagram of this design process is shown in FIG. 14A. In a shuffling approach, all the same bases of target sequence(s) are preserved in the same ratio, and the parameters of target sequence(s) are preserved, but the sequence of the normalization controls is shuffled and does not map to the target sequence(s).

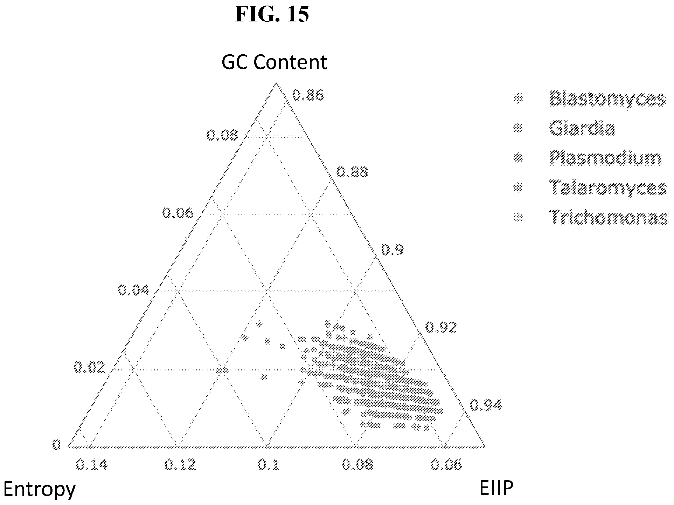

Approximately 1200-2000 Reference Genomes representing potential pathogens were used to generate a distribution of GC content, entropy, complexity, and electron-ion interaction potential (EIIP) with respect to Genome length. A ternary plot showing the interaction between GC content, entropy and EIIP for the reference genomes is shown in FIG. 15.

Sequences were obtained from length bins which were in the 10th, 30th, 50th, 70th, 80th and 90th percentile of the x axis (length) with respect to the different distributions (GC content, Entropy, Complexity, EIIP). Each sequences was shuffled within itself by 20-25 mer windows and placed in a repository of sequences for potential an array. Each sequence was also shuffled within itself by a 100 mer window and placed in a repository of sequences to use as a "map control."

The potential array and "map control" were partitioned against the reference genomes, and blasted against NCBI non-redundant sequence database (NR) and the guide sequences. Sequences from the potential array that mapped were eliminated. Normalization controls were designed in 55 staggered concentrations.

The normalization controls were informatically spike in to Probit (1000 cp/ml), Clinical samples, Identify and quantify Oligo, Identify and quantify pathogens. The "map control" sequences were mapped against references to prove that shuffling works.

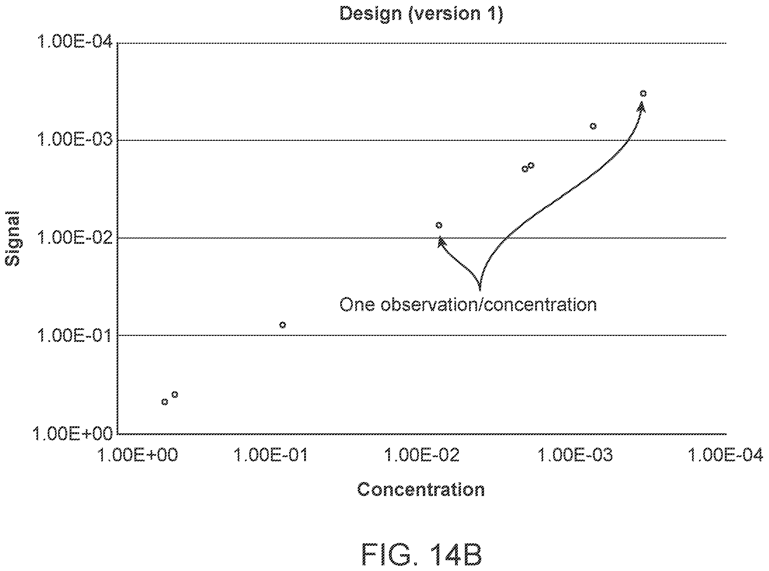

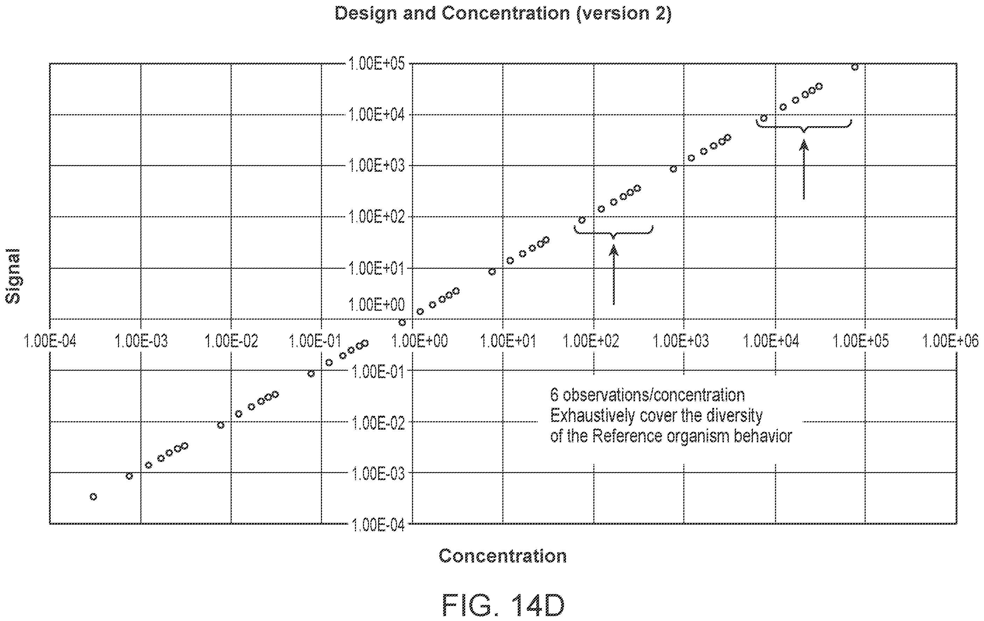

An example of the normalization controls designed with 1-3 oligo per log concentration is shown in FIGS. 14B-14C. An example normalization controls designed with each order of magnitude in concentration represented by 6 concentrations with at least one different oligo per log concentration, and 7-8 orders of magnitude covered, is shown in FIG. 14D.

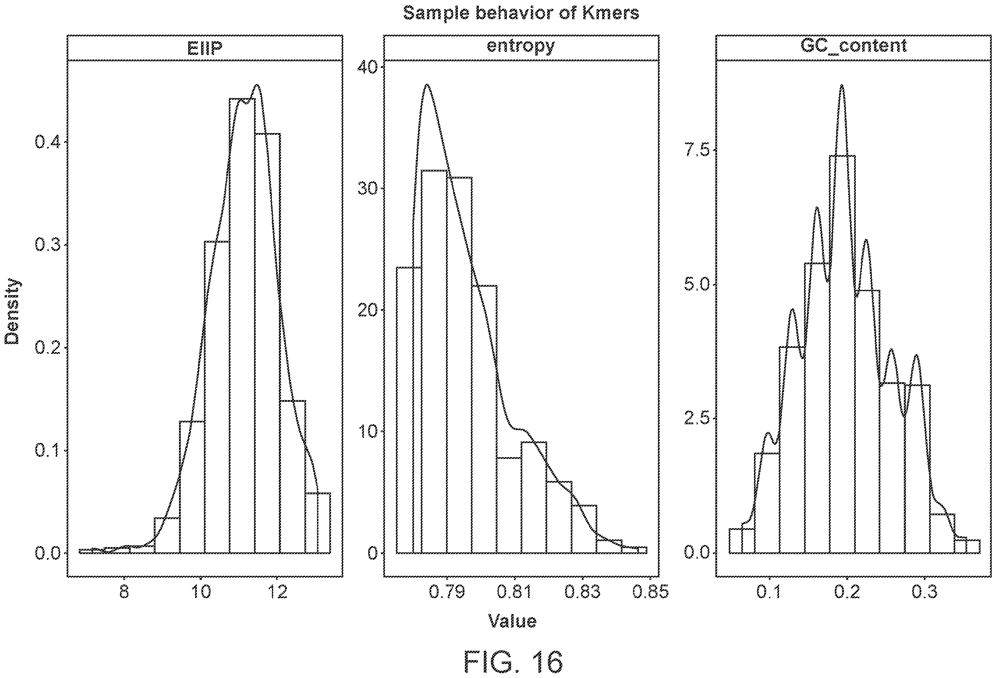

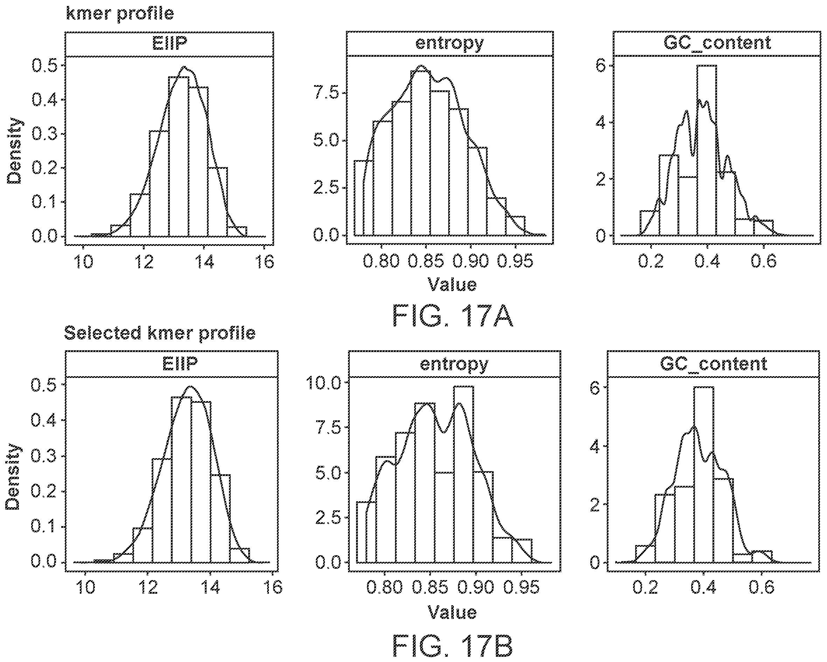

FIGS. 16-17 show the distribution of EIIP, entropy and GC content in Kmers generated using the panel of Reference Genomes. 3.2 billion Kmers were generated, and the initial profile was built using 2 billion Kmers (FIG. 16), 10 million Kmers (FIG. 17A) and 1.3 million Kmers (FIG. 17B). Kmers were shuffled to mimic the distribution of EIIP, entropy and GC content seen in Reference Genomes.

Multiprocessing in python can be used to perform processing on the Kmer dataset (for example, Process and Pool). Pandas and Process are used to do similar operations on large files. Datamash in python was used on 1.2 billion lines of Kmers (divided into 7 files) to obtain the percentile values of the features. AWK was used to perform selections and write the selected Kmers to file, which was used to build oligos.

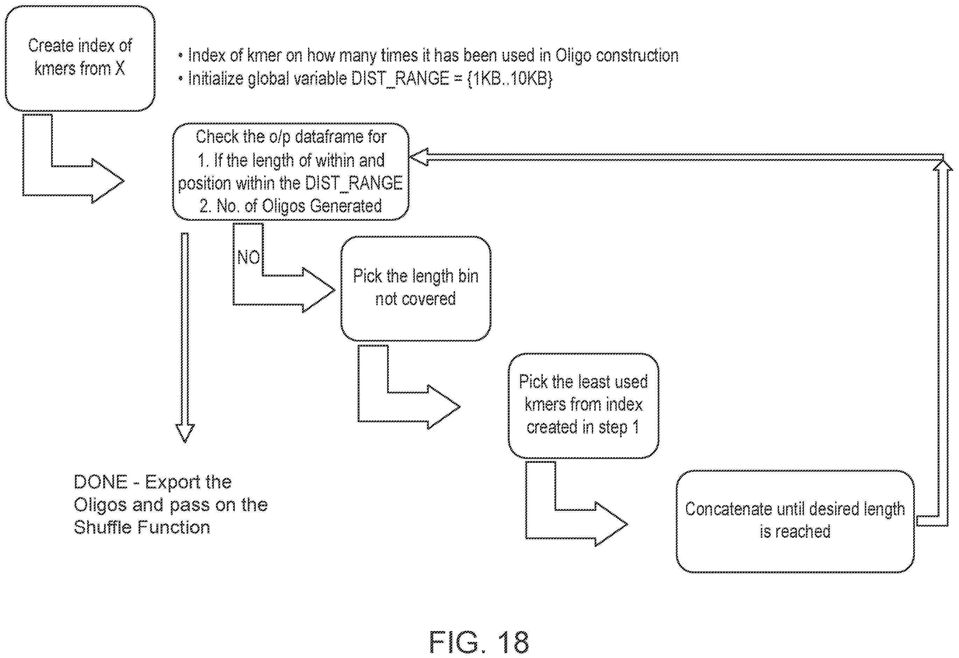

An exemplary pipeline for processing Kmers and generating output oligos is shown in FIG. 18. Kmers are indexed to show how many times they have been used in normalization control oligo construction and the global variable DIST_RANGE = {1KB...10KB} is initiated. The output data frame (of normalization control oligos) is checked to see if length and position are within the DIST_RANGE, and the number of oligos generated. If NO, then the length bin not covered is selected, then the least used Kmers from the index created in step 1 are selected. These are concatenated until the designed normalization control length is reached, and the process is repeated until the desired number of oligos are generated. The sequences are then passed on to the Shuffle function.

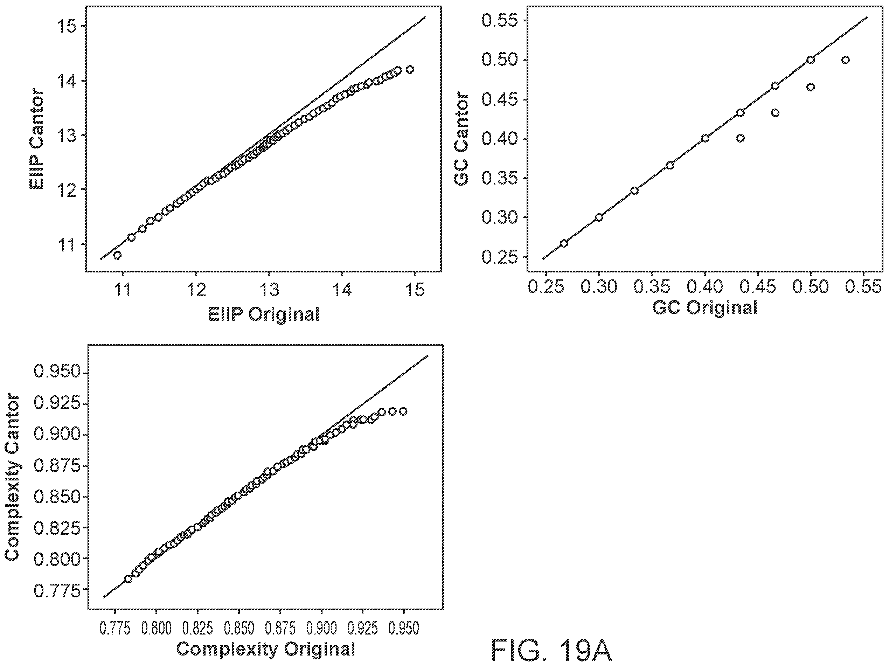

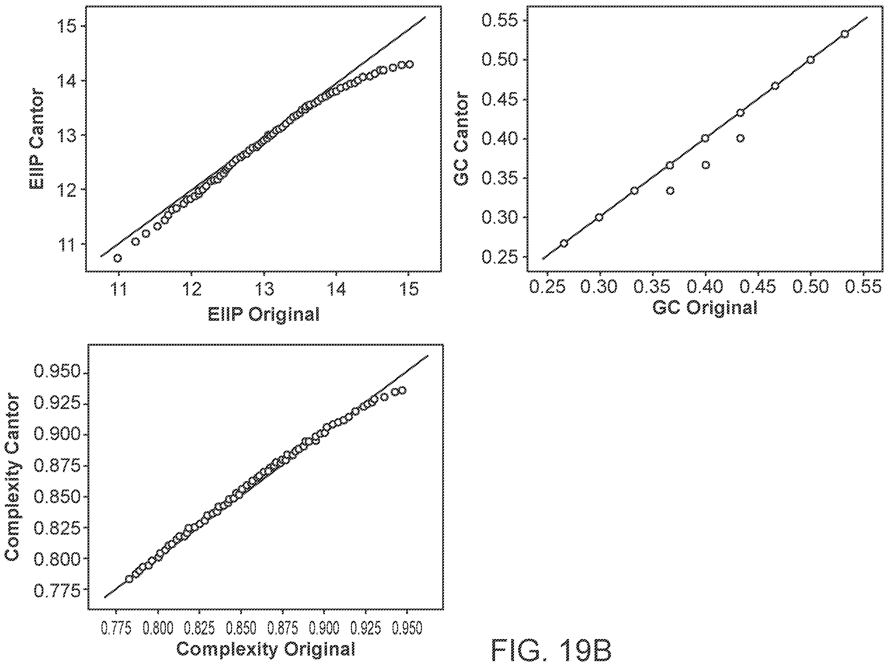

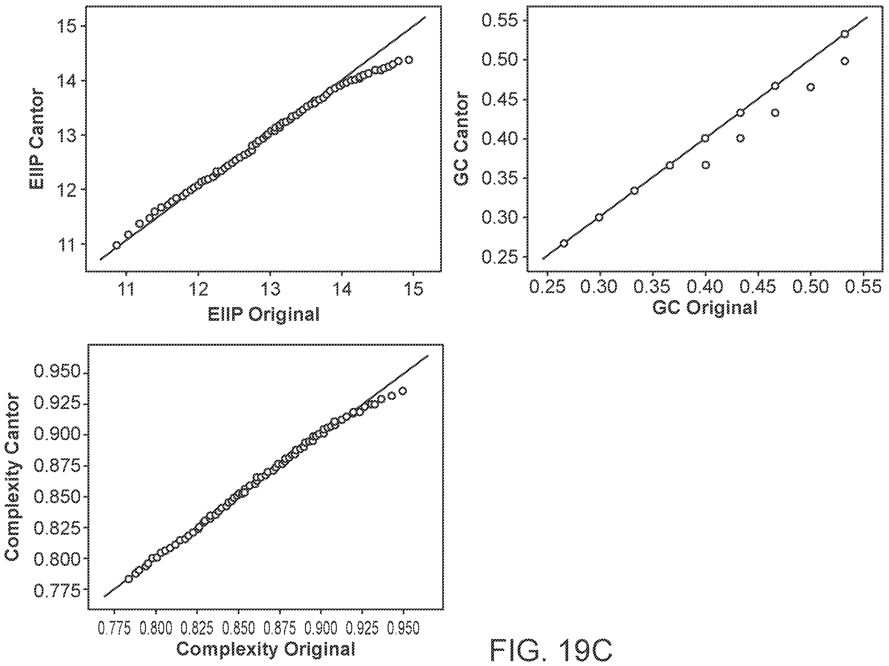

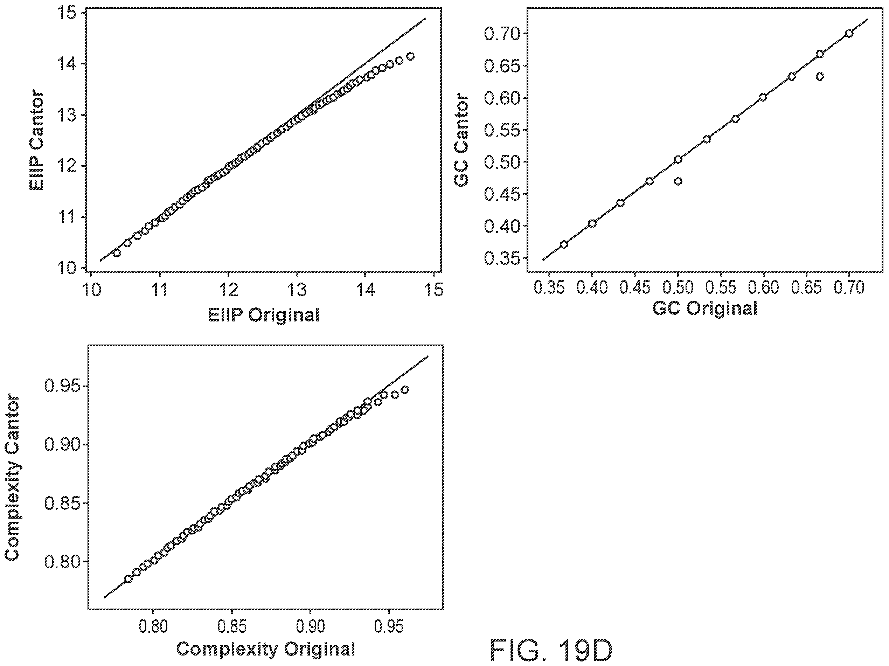

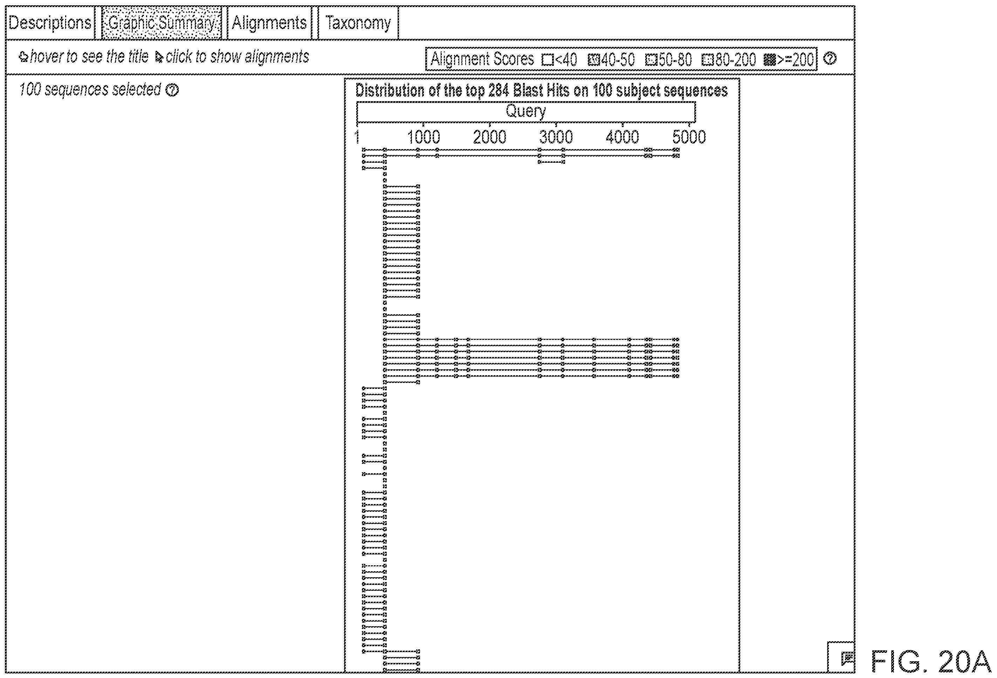

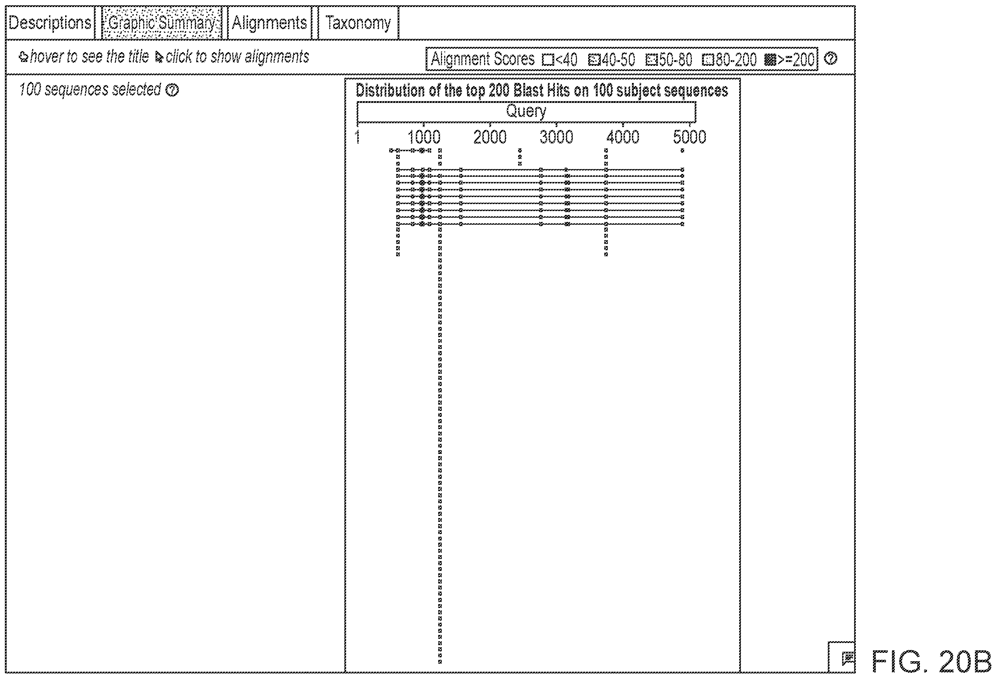

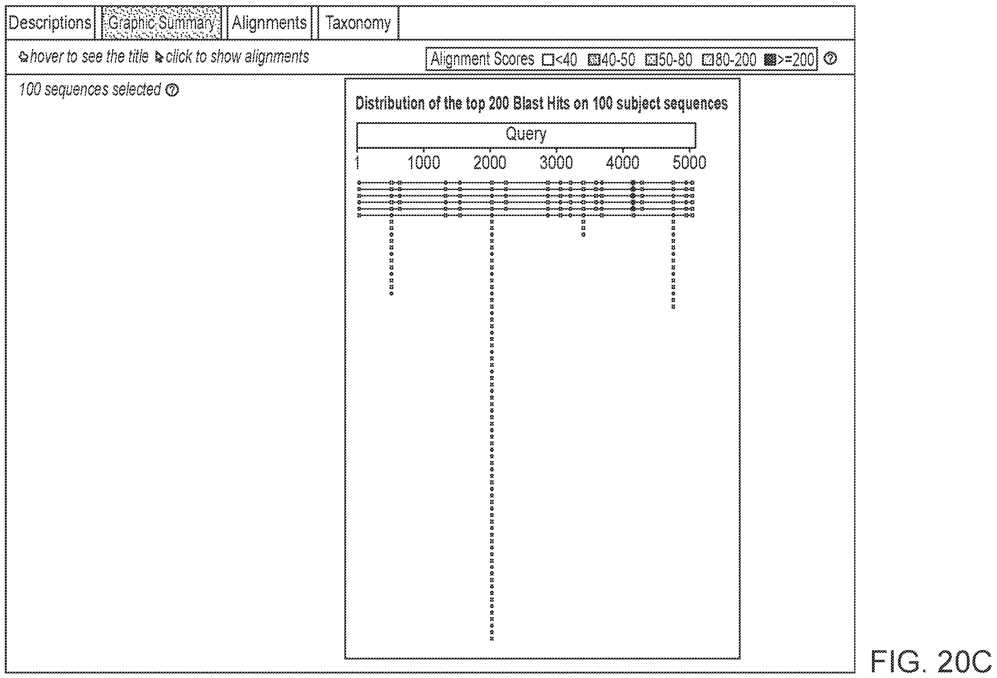

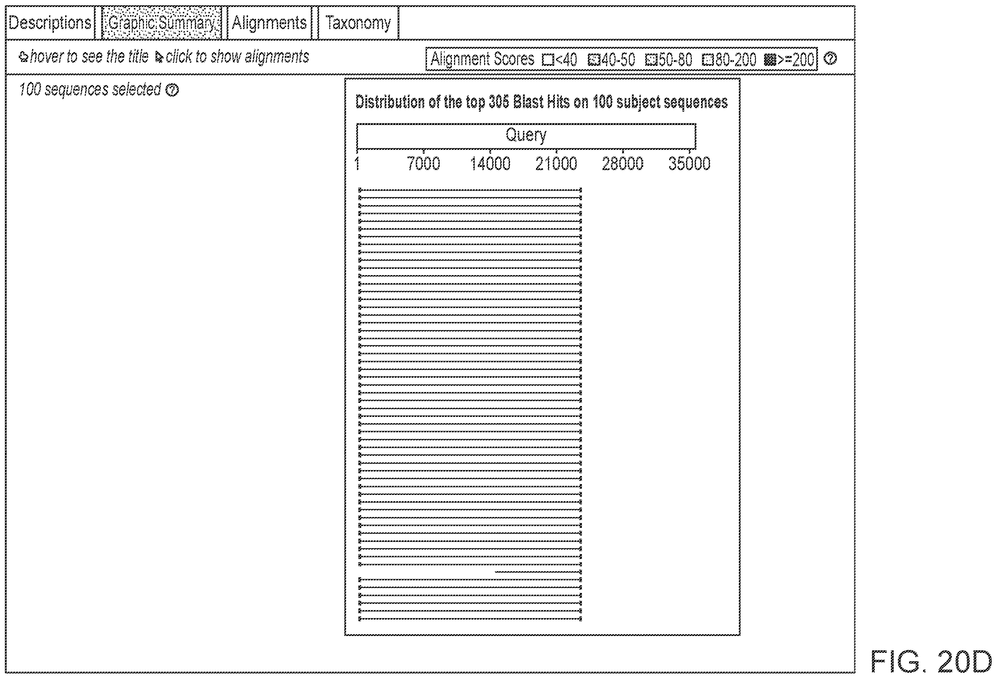

FIGS. 19A-19D show that normalization controls generated by Cantor shuffling mimic EIIP, complexity and GC content for BK polyomavirus isolate CH-1, JC polyomavirus strain NIID 12-31, human polyomavirus 1 strain BK 2 and human adenovirus C. FIGS. 20A-20D show that normalization controls do not Blast to BK polyomavirus isolate CH-1, JC polyomavirus strain NIID 12-31, human polyomavirus 1 strain BK 2 and human adenovirus C using the National Center for Biotechnology Information (NCBI) BLAST Nucleotide Sequence server (blast.ncbi.nlm.nih.gov/Blast.cgi?PAGE_TYPE=-BlastSearch).

*Example 6: Generating Normalization Control Sequences Using a Sliding Window (Exhaustive Kmer Approach)*

An alternative approach to the Cantor shuffling described in Example 5 is to use natural sequences from target sequences. In this approach, a sliding window is taken across the natural sequences to generate staggered, overlapping Kmers. Non-adjacent Kmers were then concatenated to produce normalization control sequences. If a Kmer size of around 31 or 32 bp is used, then sequencing reads of about 150 bp will encompass multiple Kmers, and normalization control sequences can be identified through the juxtaposition of Kmers which are not adjacent in target sequence(s).

In this approach, a single genome can be represented by selecting multiple sequences spread throughout the genome that are representative how that genome would fragment. Further, individual normalization controls can incorporate Kmers from multiple organisms, and match the parameters of multiple organisms. For example, a normalization control oligo can contain a long Kmer, and within the long Kmer may be shorter Kmers containing sequences from other organisms.

Approximately 1200-2000 Reference Genomes were used to generate staggered Kmers, and Kmer distributions were calculated as described above for Example 5.

The Kmers were first selected based on percent GC content: *i.e.,* a set number of percentages of GC content were picked to include in the normalization controls. Kmers at the selected percent GC contents were next concatenated to generate 1 kilobase (KB) Oligos on GC bins. The GC content of concatenated sequences was checked, as was entropy. Sequences were then passed homopolymer filters, and blasted (Blastn and guide blast) to produce 785,000 oligos, each 1Kb in length.

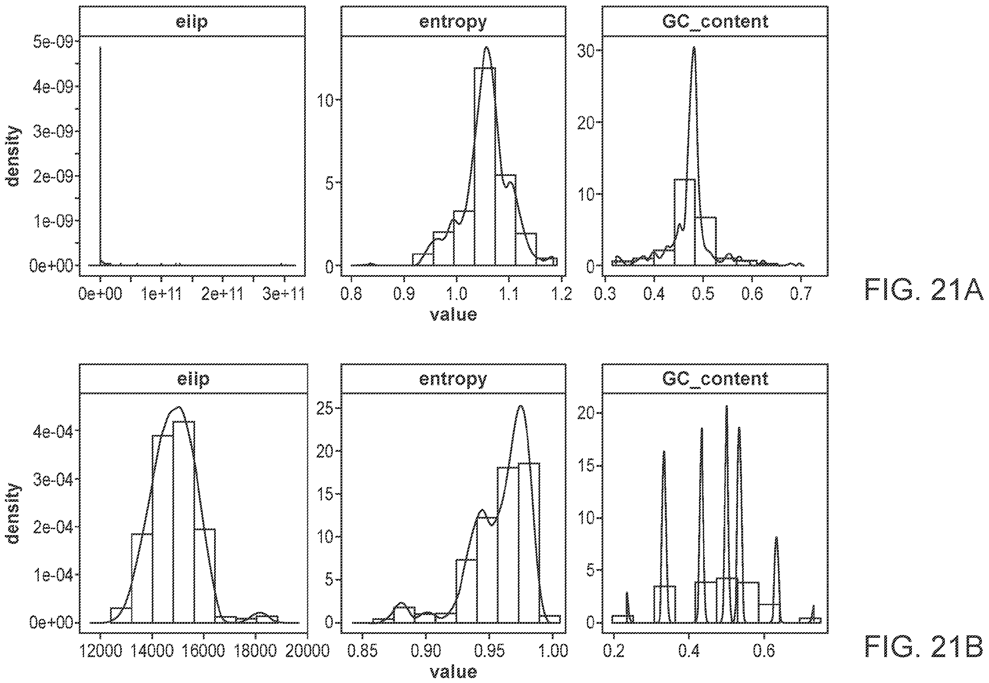

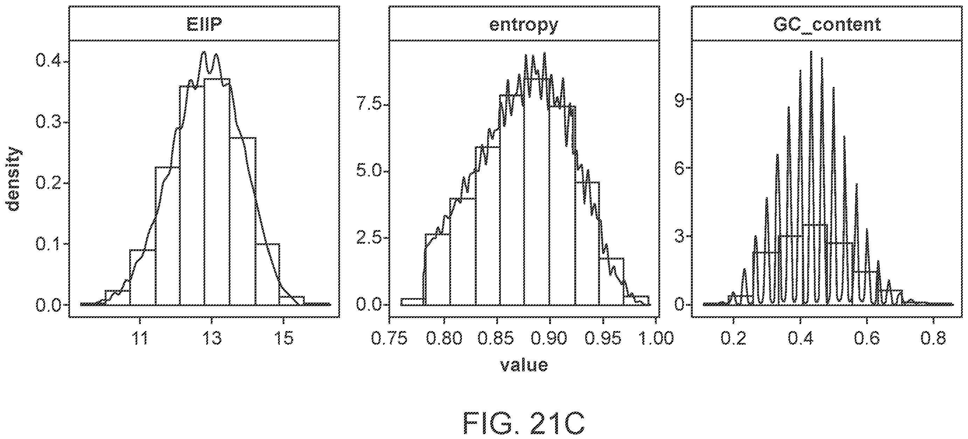

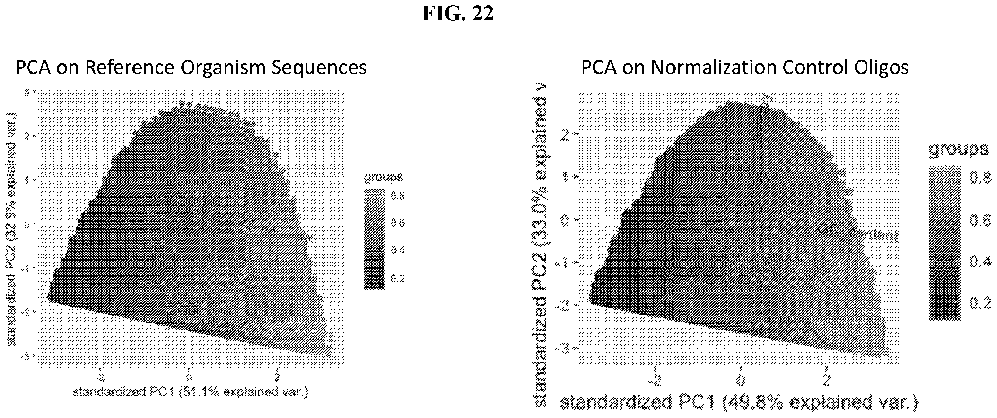

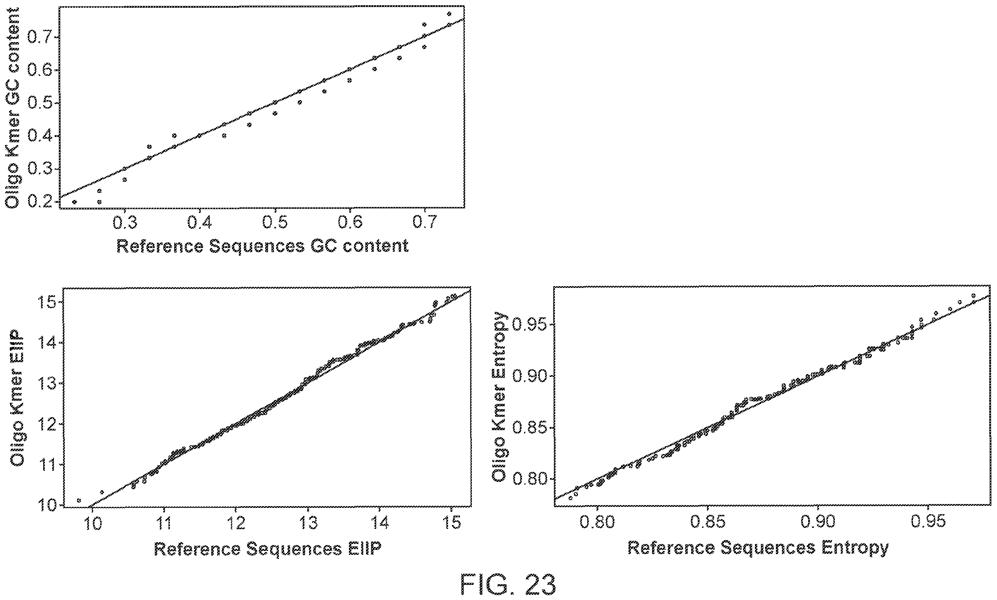

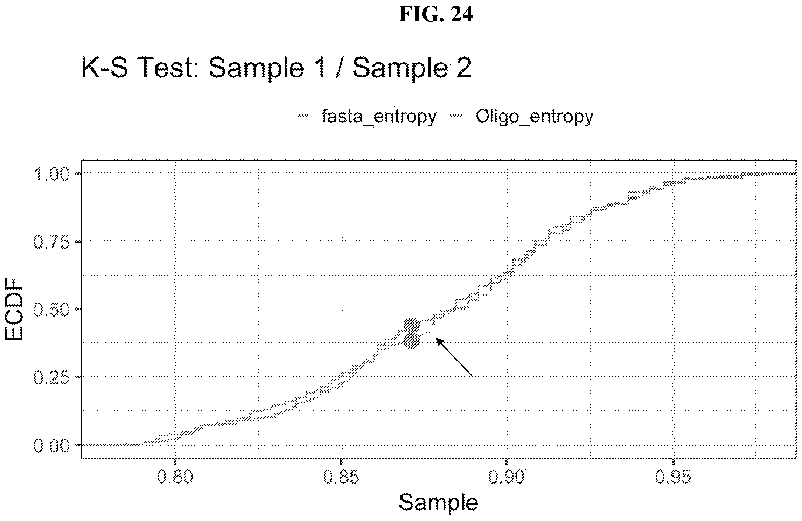

FIGS. 21A-21C show distribution of EIIP, entropy and GC content of Kmers generated at selected GC content percentages. When Principle Component Analysis (PCA) was performed on both Reference Genome sequences and normalization control sequences generated using these methods, both showed a similar correlation between entropy, EIIP and GC content (FIG. 22). Further, entropy, EIIP and GC content of normalization controls generated using this method correlated with entropy, EIIP and GC content of Reference Genome sequences (FIG. 23). In addition, a Kolmogorov Smirnov (KS) test was run comparing probability distributions of entropies of the Reference Genome sequences to the normalization controls (FIG. 24).

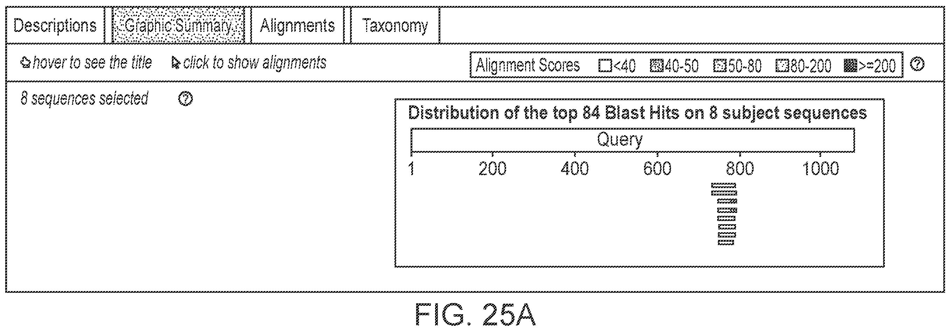

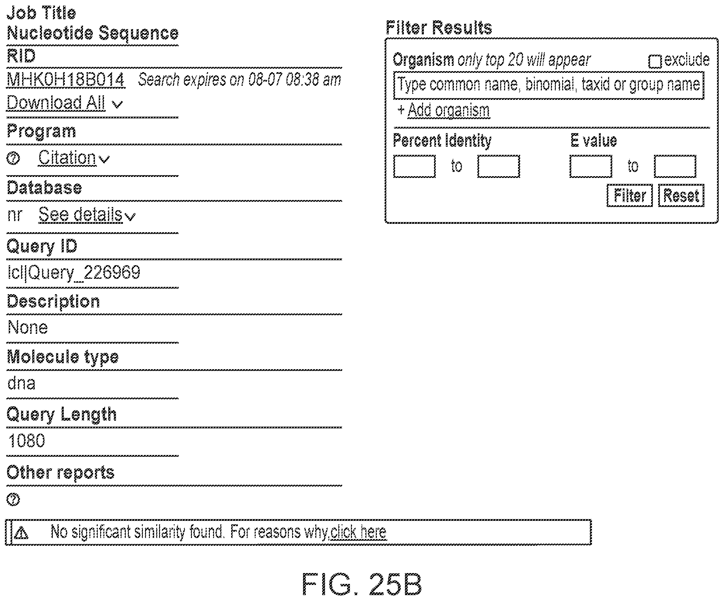

That 785,000 normalization control oligo sequences did not align to the Reference Genome sequences was verified by BLAST using the NCBI BLAST Nucleotide Sequences server (FIG. 25A-25B). The head of the normalization control oligo always BLASTed to Pseudomonas at about 750 bp for 50-80 bases (FIG. 25A). The tail of the file had no BLAST hits (FIG. 25). All 785,000 normalization control oligo sequences can be mass BLASTed, and only those sequences with no BLAST hits can be selected for use as normalization controls.

**Claims**

1. A method of making a normalization control comprising RNA molecules, comprising:

   a. synthesizing at least three groups of DNA molecules, wherein each DNA molecule in the at least three groups of DNA molecules comprises, from 5' to 3', a first component sequence, a sequence complementary to a promoter sequence, a first restriction enzyme site, a second component sequence, a second sequence complementary to a promoter sequence, a second restriction site, a third component sequence and a third sequence complementary to a promoter sequence;
   b. digesting each of the at least three groups of DNA molecules with a restriction enzyme that cuts at the first and second restriction enzyme sites to produce at least three groups of digested DNA molecules;
   c. *in vitro* transcribing each of the at least three groups of digested DNA molecules to produce at least three populations of RNA molecules;
   d. quantifying the amount of RNA in each of the at least three populations of RNA molecules; and
   e. mixing a pre-determined amount of each of the at least three populations of RNA molecules, thereby producing a normalization control comprising RNA molecules.

2. The method of claim 1, wherein the first, second and third component sequences are each between about 15 bp and about 1200 bp, between about 150 bp and about 500 bp, or between about 100 bp and about 600 bp.

3. The method of any one of the preceding claims, wherein the first, second and third component sequences of each of the DNA molecules are random sequences.

4. The method of any one of the preceding claims, wherein the first, second and third component sequences of each of the DNA molecules have at least one property in common with a target sequence in a sample.

5. The method of any one of the preceding claims, wherein *in vitro* transcribing comprises incorporating modified nucleotides into the at least three populations of RNA molecules.

6. The method of any one of the preceding claims, wherein the sequences of the first and second restriction sites are the same.

7. The method of any one of the preceding claims, wherein the restriction sites are restriction sites that are recognized and cleaved by a type II restriction endonuclease.

8. The method of any one of the preceding claims, wherein neither the first nor the second restriction site occur in any of the first, second and third component sequences.

9. The method of any one of the preceding claims, wherein the first promoter sequence, the second promoter sequence and the third promoter sequence comprise the same promoter sequence.

10. The method of any one of the preceding claims, wherein the promoter sequence comprises a promoter sequence selected from the group consisting of a T7 promoter sequence, an SP6 promoter sequence or a T3 promoter sequence.

11. The method of any one of the preceding claims, wherein the method further comprises diluting the normalization controls to a concentration of between about 0.001 and about 500 pg/μL.

12. The method of any one of the preceding claims, wherein the pre-determined amounts of each of the populations of RNA molecules are not equal.

13. The method of any one of the preceding claims, wherein the pre-determined amounts of the at least three populations of RNA molecules are a ratio of 1:2:4 in the normalization control.

14. The method of any one of the preceding claims, wherein the sequence of the first and second restriction sites is selected from the group consisting of a sequence of an EcoRI site, a BamHI site, a HindIII site, a NotI site, a PvuII site, a SmaI site, an EcoRV site, a KpnI site, a PstI site, a SaiI site, a ScaI site, an SpeI site, an SphI site, a StuI site, and an XbaI site.

15. The method of any one of claims 1-13, wherein the restriction enzyme used to digest the at least three groups of DNA molecules comprises EcoRI, BamHI, HindIII, NotI, PvuII, SmaI, EcoRV, KpnI, PstI, SalI, ScaI, SpeI, SphI, StuI or XbaI.

**Patentansprüche**

1. Verfahren zur Herstellung einer Normalisierungskontrolle, die RNA-Moleküle umfasst, umfassend:

   a. Synthetisieren von mindestens drei Gruppen von DNA-Molekülen, wobei jedes DNA-Molekül in den mindestens drei Gruppen von DNA-Molekülen von 5' nach 3' eine erste Komponentensequenz, eine zu einer Promotorsequenz komplementäre Sequenz, eine erste Restriktionsenzymstelle, eine zweite Komponentensequenz, eine zweite zu einer Promotorsequenz komplementäre Sequenz, eine zweite Restriktionsstelle, eine dritte Komponentensequenz und eine dritte zu einer Promotorsequenz komplementäre Sequenz umfasst;
   b. Verdauen jeder der mindestens drei Gruppen von DNA-Molekülen mit einem Restriktionsenzym, das an den ersten und zweiten Restriktionsenzymstellen schneidet, um mindestens drei Gruppen von verdauten DNA-Molekülen zu erzeugen;
   c. In-vitro-Transkribieren jeder der mindestens drei Gruppen von verdauten DNA-Molekülen, um mindestens drei Populationen von RNA-Molekülen zu erzeugen;
   d. Quantifizieren der RNA-Menge in jeder der mindestens drei Populationen von RNA-Molekülen; und
   e. Mischen einer vorbestimmten Menge jeder der mindestens drei Populationen von RNA-Molekülen, wodurch eine RNA-Moleküle-umfassende Normalisierungskontrolle hergestellt wird.

2. Verfahren nach Anspruch 1, wobei die erste, zweite und dritte Komponentensequenz jeweils zwischen etwa 15 bp und etwa 1200 bp, zwischen etwa 150 bp und etwa 500 bp oder zwischen etwa 100 bp und etwa 600 bp liegen.

3. Verfahren nach einem der vorhergehenden Ansprüche, wobei die erste, zweite und dritte Komponentensequenz jedes der DNA-Moleküle Zufallssequenzen sind.

4. Verfahren nach einem der vorhergehenden Ansprüche, wobei die erste, zweite und dritte Komponentensequenz jedes der DNA-Moleküle mindestens eine Eigenschaft mit einer Zielsequenz in einer Probe gemeinsam haben.

5. Verfahren nach einem der vorhergehenden Ansprüche, wobei die *In-vitro*-Transkription den Einbau von modifizierten Nukleotiden in die mindestens drei Populationen von RNA-Molekülen umfasst.

6. Verfahren nach einem der vorhergehenden Ansprüche, wobei die Sequenzen der ersten und zweiten Restriktionsstellen gleich sind.

7. Verfahren nach einem der vorhergehenden Ansprüche, wobei die Restriktionsstellen Restriktionsstellen sind, die von einer Typ-II-Restriktionsendonuklease erkannt und gespalten werden.

8. Verfahren nach einem der vorhergehenden Ansprüche, wobei weder die erste noch die zweite Restriktionsstelle in einer der ersten, zweiten und dritten Komponentensequenzen vorkommt.

9. Verfahren nach einem der vorhergehenden Ansprüche, wobei die erste Promotorsequenz, die zweite Promotorsequenz und die dritte Promotorsequenz die gleiche Promotorsequenz umfassen.

10. Verfahren nach einem der vorhergehenden Ansprüche, wobei die Promotorsequenz eine Promotorsequenz umfasst, die ausgewählt ist aus der Gruppe bestehend aus einer T7-Promotorsequenz, einer SP6-Promotorsequenz oder einer T3-Promotorsequenz.

**11.** Verfahren nach einem der vorhergehenden Ansprüche, wobei das Verfahren ferner das Verdünnen der Normalisierungskontrollen auf eine Konzentration zwischen etwa 0,001 und etwa 500 pg/$\mu$L umfasst.

**12.** Verfahren nach einem der vorhergegangenen Ansprüche, wobei die vorbestimmten Mengen jeder der Populationen von RNA-Molekülen nicht gleich sind.

**13.** Verfahren nach einem der vorhergehenden Ansprüche, wobei die vorbestimmten Mengen der mindestens drei Populationen von RNA-Molekülen in der Normalisierungskontrolle ein Verhältnis von 1:2:4 aufweisen.

**14.** Verfahren nach einem der vorhergehenden Ansprüche, wobei die Sequenz der ersten und zweiten Restriktionsstellen ausgewählt ist aus der Gruppe bestehend aus einer Sequenz einer EcoRI-Stelle, einer BamHI-Stelle, einer HindIII-Stelle, einer NotI-Stelle, einer PvuII-Stelle, einer SmaI-Stelle, einer EcoRV-Stelle, einer KpnI-Stelle, einer PstI-Stelle, einer SalI-Stelle, einer ScaI-Stelle, einer SpeI-Stelle, einer SphI-Stelle, einer StuI-Stelle und einer XbaI-Stelle.

**15.** Verfahren nach einem der Ansprüche 1-13, wobei das Restriktionsenzym, das zum Verdauen der mindestens drei Gruppen von DNA-Molekülen verwendet wird, EcoRI, BamHI, HindIII, NotI, PvuII, SmaI, EcoRV, KpnI, PstI, SalI, ScaI, SpeI, SphI, StuI oder XbaI umfasst.

**Revendications**

**1.** Procédé de réalisation de témoins de normalisation comprenant des molécules d'ARN, comprenant :

> a. la synthèse d'au moins trois groupes de molécules d'ADN, dans lequel chaque molécule d'ADN dans les au moins trois groupes de molécules d'ADN comprend, de 5' à 3', une première séquence de composants, une séquence complémentaire d'une séquence de promoteurs, un premier site d'enzyme de restriction, une deuxième séquence de composants, une deuxième séquence complémentaire d'une séquence de promoteurs, un deuxième site d'enzyme de restriction, une troisième séquence de composants et une troisième séquence complémentaire d'une séquence de promoteurs ;
> b. la digestion de chacun des au moins trois groupes de molécules d'ADN avec une enzyme de restriction qui coupe au niveau des premier et deuxième sites d'enzyme de restriction pour produire au moins trois groupes de molécules d'ADN digérées ;
> c. la transcription in vitro de chacun des au moins trois groupes de molécules d'ADN digérées pour produire au moins trois populations de molécules d'ARN ;
> d. la quantification de la quantité d'ARN dans chacune des au moins trois populations de molécules d'ARN ; et
> e. le mélange d'une quantité prédéterminée de chacune des au moins trois populations de molécules d'ARN, en produisant ainsi un témoin de normalisation comprenant des molécules d'ARN.

**2.** Procédé selon la revendication 1, dans lequel les première, deuxième et troisième séquences de composants sont chacune entre environ 15 bp et environ 1200 bp, entre environ 150 bp et environ 500 bp, ou entre environ 100 bp et environ 600 bp.

**3.** Procédé selon l'une quelconque des revendications précédentes, dans lequel les première, deuxième et troisième séquences de composants de chacune des molécules d'ADN sont des séquences aléatoires.

**4.** Procédé selon l'une quelconque des revendications précédentes, dans lequel les première, deuxième et troisième séquences de composants de chacune des molécules d'ADN présentent au moins une propriété en commun avec une séquence cible dans un échantillon.

**5.** Procédé selon l'une quelconque des revendications précédentes, dans lequel la transcription in vitro comprend l'incorporation de nucléotides modifiés dans les au moins trois populations de molécules d'ARN.

**6.** Procédé selon l'une quelconque des revendications précédentes, dans lequel les séquences des premier et deuxième sites d'enzyme de restriction sont les mêmes.

**7.** Procédé selon l'une quelconque des revendications précédentes, dans lequel les sites d'enzyme de restriction sont des sites d'enzyme de restrictions qui sont reconnus et clivés par une endonucléase de restriction de type II.

8. Procédé selon l'une quelconque des revendications précédentes, dans lequel ni le premier site d'enzyme de restriction ni le deuxième site d'enzyme de restriction n'est présent dans l'une quelconque des première, deuxième et troisième séquences de composants.

9. Procédé selon l'une quelconque des revendications précédentes, dans lequel la première séquence de promoteurs, la deuxième séquence de promoteurs et la troisième séquence de promoteurs comprennent la même séquence de promoteurs.

10. Procédé selon l'une quelconque des revendications précédentes, dans lequel la séquence de promoteurs comprend une séquence de promoteurs choisie dans le groupe comprenant une séquence de promoteurs T7, une séquence de promoteurs SP6 ou une séquence de promoteurs T3.

11. Procédé selon l'une quelconque des revendications précédentes, dans lequel le procédé comprend en outre la dilution des témoins de normalisation à une concentration entre environ 0,001 et environ 500 pg/$\mu$l.

12. Procédé selon l'une quelconque des revendications précédentes, dans lequel les quantités prédéterminées de chacune des populations de molécules d'ARN ne sont pas égales

13. Procédé selon l'une quelconque des revendications précédentes, dans lequel les quantités prédéterminées des au moins trois populations de molécules d'ARN sont présentes dans un rapport de 1:2:4 dans le témoin de normalisation.

14. Procédé selon l'une quelconque des revendications précédentes, dans lequel la séquence des premier et deuxième sites d'enzyme de restriction est choisie dans le groupe constitué d'une séquence d'un site EcoRI, d'un site BamHI, d'un site HinIII, d'un site NotI, d'un site PvuII, d'un site SmaI, d'un site EcoRV, d'un site KpnI, d'un site PstI, d'un site SalI, d'un site ScaI, d'un site SpeI, d'un site SphI, d'un site StuI et d'un site XbaI.

15. Procédé selon l'une quelconque des revendications 1 à 13, dans lequel l'enzyme de restriction utilisée pour digérer les au moins trois groupes de molécules d'ADN comprend EcoRI, BamHI, HinIII, NotI, PvuII, SmaI, EcoRV, KpnI, PstI, SalI, ScaI, SpeI, SphI, StuI ou XbaI.

## FIG. 1

Normalization Control ✓

| Control | Reads | Expected Ratio | Actual | QC Pass/Fail |
|---|---|---|---|---|
| GalileoNormC.100 | 124 | - | - | - |
| GalileoNormC.1000 | 1,108 | 10x | 8.9x | Pass |
| GalileoNormC.10000 | 9,978 | 10x | 9.0x | Pass |
| GalileoNormC.100000 | 101,390 | 10x | 10.2x | Pass |

FIG. 2

NC-normalized CMV abundance vs titer

$R^2 = .9888$

FIG. 3

EP 4 296 373 B1

Percent abundances (y-axis arbitrary) of a virus to demonstrate the different transformations

Legend: Viral load; f(r) read evidence; Raw read evidence; f(r) + Δ(r) read evidence2

## FIG. 4

Raw reads

Viral load = mx + b

f(r) reads

FIG. 5

Normalization Control Addition During Extraction

- 20,000X Dilution of each set of 3 transcripts to 0.1ng/$\mu$L

- **Normalization Control Mix:**

  - RNA Normalization Controls 1:   25$\mu$L

  - RNA Normalization Controls 2:   50$\mu$L

  - RNA Normalization Controls 3:  100$\mu$L

  - ─                                    175$\mu$L

- Additional 10X dilution: 30$\mu$L of above pool + 270$\mu$L AVE buffer = 10pg/$\mu$L

  - 1.5mL tubes for EZ1 Row 3: add 8$\mu$L of 10pg/$\mu$L NC Mix to 52$\mu$L AVE buffer

  - EZ1 takes 50$\mu$L aliquot from Row 3 tube and adds to 400uL sample = 70pg NC Mix added

    - "high" = 38.10pg of NC added/sample (NC 1: 5.4pg. NC 2: 10.8pg. NC 3: 21.8pg)

    - "med" = 19.04pg of NC added/sample (NC 1: 2.7pg. NC 2: 5.4pg. NC 3: 10.8pg)

    - "low" = 9.52pg of NC added/sample (NC 1: 1.4pg. NC 2: 2.7pg. NC 3: 5.4pg)

      - The "low" amount resulted in 4% of reads. Used for all samples on RUN053 and RUN058

    - conversion of "low" NC1 (1.4pg) to library (based on de-duplicated reads) = 76% of input = 1.067pg

FIG. 6

"high" avg = 16% of reads
"med" avg = 6% of reads
"low" avg = 4% of reads

NORMALIZATION CONTROL % Reads
Exact Diagnostics Negative Plasma

RUN049

avg read output/library = 23,000,000
avg human = 80%

FIG. 7

| Adaptors mixing | | | | | | | | Reactions | 2 | | | |
|---|---|---|---|---|---|---|---|---|---|---|---|---|
| Stock reagent | Vendor | P/N# | MW | [Initial Liquid] or Grams solid | UOM | UOM | RXN 1x Volume | UOM | Production Scale RXN | UOM | Final Conc 1X RXN | UOM |
| Mol Bio Wate | Sigma | 793566-25KG | | 1 | x | | 8 | uL | 16 | uL | 0.4 | x |
| i5 Adapter | | | | 200 | uM | | 5 | uL | 10 | uL | 50 | uM |
| i7 Adapter | | | | 200 | uM | | 5 | uL | 10 | uL | 50 | uM |
| NEB 2 | | | | 10 | x | | 2 | uL | 4 | uL | 1 | x |
| | | | | | | Total | 20 | uL | 40 | uL | | |
| | | | | | | | | | | | | |
| Ramp from 95 for 1 min to 20 C slowly | | | | | | | | | | | | |
| Dilute to 15uM | | | | | | | | | | | | |
| | | | | | | | | | | | | |
| Normalization Control | | | | | | | | Reactions | 2 | | | |
| Stock reagent | Vendor | P/N# | MW | [Initial Liquid] or Grams solid | UOM | UOM | RXN 1x Volume | UOM | Production Scale RXN | UOM | Final Conc 1X RXN | UOM |
| Mol Bio Wate | Sigma | 793566-25KG | | 1 | x | | 42 | uL | 84 | uL | 0.7 | x |
| i5 i7Adapters | | | | 50 | uM | | 18 | uL | 36 | uL | 15 | uM |
| | | | | | | Total | 60 | uL | 120 | uL | | |
| | | | | | | | | | | | | |
| Dilute to 750 nM | | | | | | | | | | | | |
| | | | | | | | | | | | | |
| Normalization Control | | | | | | | | Reactions | 2 | | | |
| Stock reagent | Vendor | P/N# | MW | [Initial Liquid] or Grams solid | UOM | UOM | RXN 1x Volume | UOM | Production Scale RXN | UOM | Final Conc 1X RXN | UOM |
| Mol Bio Wate | Sigma | 793566-25KG | | 1 | x | | 1140 | uL | 2280 | uL | 19 | x |
| i5 i7Adapters | | | | 15 | uM | | 60 | uL | 120 | uL | 0.75 | uM |
| | | | | | | Total | 1200 | uL | 2400 | uL | | |

FIG. 8

EP 4 296 373 B1

SPLIT-UP OF DNA-NORMALIZATION CONTROL SPIKE-IN SEQUENCES

FIG. 9

EP 4 296 373 B1

| Fasta Header | GC content | Total Count | Length |
|---|---|---|---|
| >Oligo177_RNA_Normalization_Controls_1_with_T7_RNA_polymerase_promoter_for_IVT | 45.41284404 | 218 | 436 |
| >Oligo178_RNA_Normalization_Controls_2_with_T7_RNA_polymerase_promoter_for_IVT | 46.78899083 | 218 | 436 |
| >Oligo179_RNA_Normalization_Controls_3_with_T7_RNA_polymerase_promoter_for_IVT | 43.57798165 | 218 | 436 |
| >Oligo180_RNA_Normalization_Controls_4_with_T7_RNA_polymerase_promoter_for_IVT | 46.73913043 | 276 | 552 |
| >Oligo181_RNA_Normalization_Controls_5_with_T7_RNA_polymerase_promoter_for_IVT | 43.84057971 | 276 | 552 |
| >Oligo182_RNA_Normalization_Control_6_with_T7_RNA_polymerase_promoter_for_IVT | 40.57971014 | 276 | 552 |
| >Oligo183_RNA_Normalization_Control_7_with_T7_RNA_polymerase_promoter_for_IVT | 44.35318275 | 487 | 974 |
| >Oligo184_RNA_Normalization_Control_8_with_T7_RNA_polymerase_promoter_for_IVT | 43.32648871 | 487 | 974 |
| >Oligo185_RNA_Normalization_Control_9_with_T7_RNA_polymerase_promoter_for_IVT | 43.53182752 | 487 | 974 |
| >Oligo186_RNA_Normalization_Control_1 | 49.77777778 | 225 | 450 |
| >Oligo187_RNA_Normalization_Control_2 | 52 | 225 | 450 |
| >Oligo188_RNA_Normalization_Control_3 | 49.77777778 | 225 | 450 |
| >Oligo189_RNA_Normalization_Control_4 | 42.15686275 | 306 | 612 |
| >Oligo190_RNA_Normalization_Control_5 | 43.46405229 | 306 | 612 |
| >Oligo191_RNA_Normalization_Control_6 | 41.50326797 | 306 | 612 |
| >Oligo192_RNA_Normalization_Control_7 | 45.64796905 | 517 | 1034 |
| >Oligo193_RNA_Normalization_Control_8 | 46.61508704 | 517 | 1034 |
| >Oligo194_RNA_Normalization_Control_9 | 44.87427466 | 517 | 1034 |
| >Oligo241_manorm1seg1 | 47.05882353 | 255 | 510 |
| >Oligo242_manorm2seg1 | 48.62745098 | 255 | 510 |
| >Oligo243_manorm3seg1 | 53.7254902 | 255 | 510 |
| >Oligo244_manorm1seg2 | 51.7571885 | 313 | 626 |
| >Oligo245_manorm2seg2 | 51.7571885 | 313 | 626 |
| >Oligo246_manorm3seg2 | 44.7284345 | 313 | 626 |
| >Oligo247_manorm2seg3 | 50.57251908 | 524 | 1048 |
| >Oligo248_manorm3seg3 | 49.42748092 | 524 | 1048 |
| >Oligo249_manorm4seg3 | 48.85496183 | 524 | 1048 |
| >Oligo250_dnanorm1seg1 | 46.66666667 | 225 | 450 |
| >Oligo251_dnanorm2seg1 | 55.55555556 | 225 | 450 |
| >Oligo252_dnanorm3seg1 | 54.22222222 | 225 | 450 |
| >Oligo253_dnanorm1seg2 | 49.34640523 | 306 | 612 |
| >Oligo254_dnanorm2seg2 | 50.65359477 | 306 | 612 |
| >Oligo255_dnanorm3seg2 | 49.01960784 | 306 | 612 |
| >Oligo256_dnanorm1seg3 | 49.9032882 | 517 | 1034 |
| >Oligo257_dnanorm2seg3 | 48.54932302 | 517 | 1034 |
| >Oligo258_dnanorm3seg3 | 48.54932302 | 517 | 1034 |

FIG. 10

| Oligo number | Final Conc ng/ul | Ratio to total amt of DNA |
|---|---|---|
| Oligo186 | 4.20E-03 | 0.420 |
| Oligo187 | 4.21E-04 | 0.042 |
| Oligo188 | 2.11E-04 | 0.021 |
| Oligo189 | 2.86E-03 | 0.286 |
| Oligo190 | 4.29E-04 | 0.043 |
| Oligo191 | 1.43E-04 | 0.014 |
| Oligo192 | 9.68E-04 | 0.097 |
| Oligo193 | 4.84E-04 | 0.048 |
| Oligo194 | 9.68E-05 | 0.010 |
| Oligo250 | 4.22E-05 | 0.004 |
| Oligo251 | 2.11E-05 | 0.002 |
| Oligo253 | 4.31E-05 | 0.004 |
| Oligo254 | 1.43E-05 | 0.001 |
| Oligo256 | 4.84E-05 | 0.005 |
| Oligo257 | 9.71E-06 | 0.001 |
| Lib Size | | |

FIG. 11

# FIG. 12

| | Group | Length (kb) | Length (bp) | Number of Molecules | Total Attomoles/Fragment | Concentration (attomoles/uL) |
|---|---|---|---|---|---|---|
| RNA_NC_1.1 | A | 0.175 | 175 | 263158 | 0.44 | 0.07 |
| RNA_NC_1.2 | B | 0.25 | 250 | 1144737 | 1.90 | 0.29 |
| RNA_NC_1.3 | C | 0.45 | 450 | 5131579 | 8.52 | 1.28 |
| RNA_NC_2.1 | A | 0.175 | 175 | 2789474 | 4.63 | 0.69 |
| RNA_NC_2.2 | B | 0.25 | 250 | 4513157 | 7.49 | 1.12 |
| RNA_NC_2.3 | C | 0.45 | 450 | 44473684 | 73.84 | 11.08 |
| RNA_NC_3.1 | A | 0.175 | 175 | 4605263 | 7.65 | 1.15 |
| RNA_NC_3.2 | B | 0.25 | 250 | 17500000 | 29.06 | 4.36 |
| RNA_NC_3.3 | C | 0.45 | 450 | 77894737 | 129.33 | 19.40 |

**FIG. 13**

Titer in log units as a function observed signal for calibration and real samples

⊕ Calibrators
⊙ Samples
—— Signal vs. titer line

LOG UNITS TITER (IU/ML)

OBSERVED SIGNAL

## FIG. 14A

## Example Normalization Control Design Pipeline

Reference Sequences + (Inverted References)

K-merize (distribution driven size selection windows) + shuffle the regions

Select ~100K sequences with lengths between 1 - 10Kb

Blast (nr) + guide blast

Eliminate matching sequences

Generate an array (330 Oligos) with 55 staggered concentration points with 6 standards per point

Each of the 6 standards will range over length and GC content, complexity and entropy representing Pathogen Reference Sequences

FIG. 14B

## FIG. 14C

| Name | Library | conc. In | Bottom Out | conc. Out |
|---|---|---|---|---|
| Oligo298.dna | 298 | 4.88E-01 | No | 4.88E-01 |
| Oligo299.dna | 299 | 4.19E-01 | No | 4.19E-01 |
| Oligo301.dna | 301 | 8.13E-02 | No | 8.13E-02 |
| Oligo302.dna | 302 | 2.04E-03 | No | 2.04E-03 |
| Oligo303.dna | 303 | 7.63E-03 | No | 7.63E-03 |
| Oligo304.dna | 304 | 1.87E-03 | No | 1.87E-03 |
| Oligo305.dna | 305 | 7.39E-04 | Bottoms out | 7.39E-04 |
| Oligo306.dna | 306 | 3.45E-04 | Bottoms out | 3.45E-04 |

Design and Concentration (version 2)

6 observations/concentration
Exhaustively cover the diversity
of the Reference organism behavior

Signal

Concentration

FIG. 14D

## FIG. 15

Sample behavior of Kmers

FIG. 16

kmer profile

FIG. 17A

Selected kmer profile

FIG. 17B

- Index of kmer on how many times it has been used in Oligo construction
- Initialize global variable DIST_RANGE = {1KB..10KB}

Create index of kmers from X

Check the o/p dataframe for
1. If the length of within and position within the DIST_RANGE
2. No. of Oligos Generated

NO

Pick the length bin not covered

Pick the least used kmers from index created in step 1

Concatenate until desired length is reached

DONE - Export the Oligos and pass on the Shuffle Function

FIG. 18

FIG. 19A

FIG. 19B

FIG. 19C

FIG. 19D

| Descriptions | Graphic Summary | Alignments | Taxonomy |

🖑 *hover to see the title*  🖱 *click to show alignments*   | Alignment Scores ☐<40 ▨40-50 ☐50-80 ☐80-200 ▧>=200 | ⑦

100 sequences selected ⑦

**Distribution of the top 284 Blast Hits on 100 subject sequences**

Query

| 1 | 1000 | 2000 | 3000 | 4000 | 5000 |

FIG. 20A

| Descriptions | Graphic Summary | Alignments | Taxonomy |

☞hover to see the title ☞click to show alignments     | Alignment Scores  ☐<40  ☒40-50  ☒50-80  ☐80-200  ■>=200 | ⊘

100 sequences selected ⊘

**Distribution of the top 200 Blast Hits on 100 subject sequences**

## FIG. 20B

EP 4 296 373 B1

| Descriptions | Graphic Summary | Alignments | Taxonomy |

*hover to see the title* *click to show alignments*     Alignment Scores □<40 ▨40-50 ▨50-80 ▢80-200 ■>=200 ⊙

*100 sequences selected* ⊙

**Distribution of the top 200 Blast Hits on 100 subject sequences**

Query

1   1000   2000   3000   4000   5000

FIG. 20C

| Descriptions | Graphic Summary | Alignments | Taxonomy |

↳ *hover to see the title* ↳ *click to show alignments*

Alignment Scores ☐<40 ▨40-50 ▨50-80 ☐80-200 ■>=200 ⊙

100 sequences selected ⊙

**Distribution of the top 305 Blast Hits on 100 subject sequences**

Query

1    7000    14000    21000    28000    35000

FIG. 20D

FIG. 21A

FIG. 21B

FIG. 21C

**FIG. 22**

PCA on Reference Organism Sequences

PCA on Normalization Control Oligos

EP 4 296 373 B1

FIG. 23

**FIG. 24**

K-S Test: Sample 1 / Sample 2

fasta_entropy    Oligo_entropy

Descriptions | Graphic Summary | Alignments | Taxonomy

🖑 *hover to see the title*　▷ *click to show alignments*　　Alignment Scores　☐<40　▨40-50　▦50-80　▨80-200　▨▨>=200　②

*8 sequences selected*　②

**Distribution of the top 84 Blast Hits on 8 subject sequences**

Query

1　　200　　400　　600　　800　　1000

FIG. 25A

**Job Title**

**Nucleotide Sequence**

**RID**

MHKOH18B014  *Search expires on 08-07 08:38 am*

Download All ∨

**Program**

⊘  Citation ∨

**Database**

nr  See details ∨

**Query ID**

lcl|Query_226969

**Description**

None

**Molecule type**

dna

**Query Length**

1080

**Other reports**

⊘

| ⚠  No significant similarity found. For reasons why.click here |
| --- |

**FIG. 25B**

EP 4 296 373 B1

## FIG. 26

### Comparison of Cantor Shuffling and Exhaustive Kmer Approaches

| Parameters | Cantor Shuffling | Exhaustive search |
|---|---|---|
| p value and D statistic(ks test - entropy) | 1.0, 0.06 - 0.08 | 0.7212,0.0566 |
| p value and D statistic (ks test - eiip) | 1.0,0.01 - 0.044 | 0.58, 0.0633 |
| p value (mann whitney test -  GC content) | 0.0039 -  0.49 | 0.2672 |
| Done on all References Genomes? | NO | YES |
| Time taken to build | | 3-4 days |
| Any further shuffling required? | YES - to mask regions | NO |
| features calculated on all References Genomes? | NO | YES |

**FIG. 27**

Of Interest                    To Deplete

Downstream Applications     Additional Depletion Methods
(PCR Amplification, Sequencing,
Cloning)

Restriction Enzyme Site
Modified nucleotide
Restriction Enzyme
Adapter

## FIG. 28

## FIG. 29

**Of Interest**                                    **To Deplete**

2901                                                2902

2903    2903                                        2904    2904

2905 ligate adapters
or
2906 depletion methods

2907                                                2908

2908 restriction enzyme (RE)
targets modified site

2909                                                2909    2909

2910                                                2911

2909

2911 RE digest of nucleic
acids for depletion

2912

2910

2913

Downstream Applications
(PCR Amplification, Sequencing, Cloning)

> Restriction Enzyme Site
> Modified nucleotide
> Restriction Enzyme
> Adapter

FIG. 30

adapter-ligated sample of nucleic acids

3004

3003

adapters

3001

3002

nucleic acid-guided nuclease

3004

3003

3005

3003

Downstream Applications (PCR Amplification, Sequencing, Cloning)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016100955 A
- WO 2017031360 A
- WO 2017100343 A
- WO 2017147345 A
- WO 2018227025 A

**Non-patent literature cited in the description**

- **I-ESHKOWITZ et al.** *PLOS ONE*, 2016, vol. 11 (4), 0153782
- **TATUSOVA** ; **MADDEN**. *FEMS Microbiol Lett.*, 1999, vol. 174, 247-250
- *Bioinformatics*, 2011, vol. 27, 1061-1067
- **VOLKERDING et al.** *Clin Chem*, 2009, vol. 55, 641-658
- **METZKER M**. *Nature Rev*, 2010, vol. 11, 31-46
- **MARGULIES, M. et al.** *Nature*, 2005, vol. 437, 376-380
- **SONI G V** ; **MELLER A.** *Clin Chem*, 2007, vol. 53, 1996-2001
- **BENTLEY et al.** *Nature*, 2009, vol. 6, 53-59
- **F.A. RAN** ; **L. CONG** ; **W.X. YAN** ; **D. A. SCOTT** ; **J.S. GOOTENBERG** ; **A.J. KRIZ** ; **B. ZETSCHE** ; **O. SHALEM** ; **X. WU** ; **K.S. MAKAROVA**. In vivo genome editing using Staphylococcus aureus Cas9. *Nature*, 09 April 2015, vol. 520, 186-191